# EUROPEAN PATENT APPLICATION

(11) **EP 2 397 482 A1**
(43) Date of publication of application: **21.12.2011**
(21) Application number: 10382174.0
(22) Date of filing: 15.06.2010
(51) Int. Cl.: C07D 487/04, C07D 519/00, A61K 31/522, A61P 29/00

(54) **Heteroaryl imidazolone derivatives as jak inhibitors**

(71) Applicant: Almirall, S.A., 08022 Barcelona (ES)
(72) Inventor: Eastwood, Paul Robert, 08980, Sant Feliu de Llobregat (ES); Gonzalez Rodriguez, Jacob, 08980, Sant Feliu de Llobregat (ES); Gomez Castillo, Elena, 08980, Sant Feliu de Llobregat (Barcelona) (ES); Bach Tana, Jordi, 08980, Sant Feliu de Llobregat (ES)
(74) Representative: Elzaburu Marquez, Alberto

(57) **Abstract**

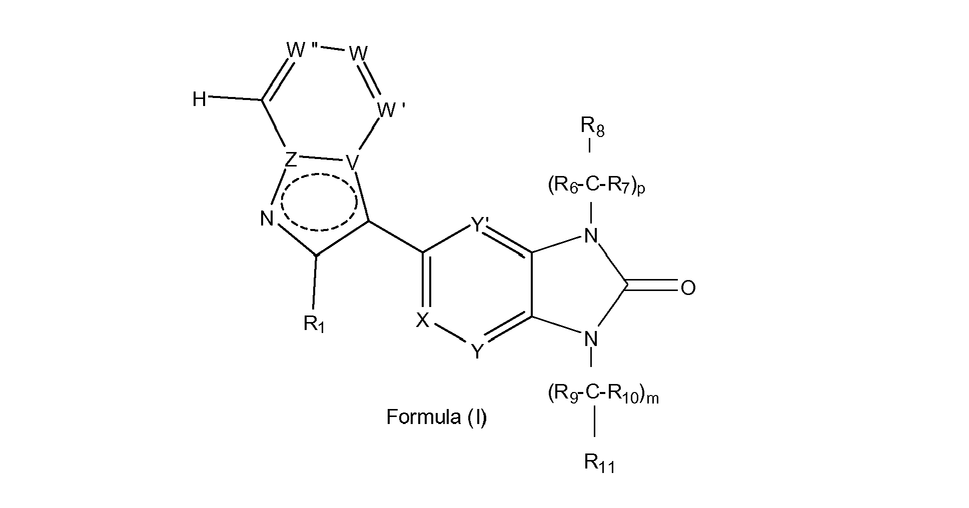

New heteroaryl imidazolone derivatives having the chemical structure of formula (I) are disclosed; as well as process for their preparation, pharmaceutical compositions comprising them and their use in therapy as inhibitors of Janus Kinases (JAK).

## Description

Cytokines have critical functions in regulating many aspects of immunity and inflammation, ranging from the development and differentiation of immune cells to the suppression of immune responses. Type I and type II cytokine receptors lack intrinsic enzymatic activity capable of mediating signal transduction, and thus require association with tyrosine kinases for this purpose. The JAK family of kinases comprises four different members, namely JAK1, JAK2, JAK3 and TYK2, which bind to type I and type II cytokine receptors for controlling signal transduction (Murray PJ, (2007). The JAK-STAT signalling pathway: input and output integration. J Immunol, 178: 2623). Each of the JAK kinases is selective for the receptors of certain cytokines. In this regard, JAK-deficient cell lines and mice have validated the essential role of each JAK protein in receptor signalling: JAK1 in class II cytokine receptors (IFN and IL-10 family), those sharing the gp130 chain (IL-6 family) and the common gamma chain (IL-2, IL-4, IL-7, IL-9, IL-15 and IL-21) (Rodig et at. (1998). Disruption of the JAK1 gene demonstrates obligatory and nonredundant roles of the Jaks in cytokine-induced biological response. Cell, 93:373; Guschin et at. (1995). A major role for the protein tyrosine kinase JAK1 in the JAK/STAT signal transduction pathway in response to interleukin-6. EMBO J. 14: 1421; Briscoe et at. (1996). Kinase-negative mutants of JAK1 can sustain intereferon-gamma-inducible gene expression but not an antiviral state. EMBO J. 15:799); JAK2 in hematopoietic factors (Epo, Tpo, GM-CSF, IL-3, IL-5) and type II IFNs (Parganas et al., (1998). JAK2 is essential for signalling through a variety of cytokine receptors. Cell, 93:385); JAK3 in receptors sharing the common gamma chain (IL-2 family) (Park et al., (1995). Developmental defects of lymphoid cells in JAK3 kinase-deficient mice. Immunity, 3:771; Thomis et al., (1995). Defects in B lymphocyte maturation and T lymphocyte activation in mice lacking JAK3. Science, 270:794; Russell et al, (1995). Mutation of JAK3 in a partient with SCID: Essential role of JAK3 in lymphoid development. Science, 270:797); and Tyk2 in the receptors of IL-12, IL-23, IL-13 and type I IFNs (Karaghiosoff et al., (2000). Partial impairment of cytokine responses in Tyk2-deficient mice. Immunity, 13:549; Shimoda et al., (2000). Tyk2 plays a restricted role in IFNg signaling, although it is required for IL-12-mediated T cell function. Immunity, 13:561; Minegishi et al., (2006). Human Tyrosine kinase 2 deficiency reveals its requisite roles in multiple cytokine signals involved in innate and acquired immunity. Immunity, 25:745).

Receptor stimulation leads sequentially to JAK activation by phosphorylation, receptor phosphorylation, STAT protein recruitment and STAT activation and dimerization. The STAT dimer then functions as a transcription factor, translocating to the nucleus and activating the transcription of multiple response genes. There are seven STAT proteins identified: STAT1, STAT2, STAT3, STAT4, STAT5a, STAT5b and STAT6. Each particular cytokine receptor associates preferentially with a particular STAT protein. Some associations are independent of cell type (ex: IFNg- STAT1) while others may be cell type dependent (Murray PJ, (2007). The JAK-STAT signaling pathway: input and output integration. J Immunol, 178: 2623).

The phenotype of deficient mice has provided insights on the function of each JAK and the cytokine receptors signaling through them. JAK3 associates exclusively with the common gamma chain of the receptors for IL-2, IL-4, IL-7, IL-9, IL-15 and IL-21 cytokines. By virtue of this exclusive association, JAK3 knock out mice and common gamma chain deficient mice have an identical phenotype (Thomis et al., (1995). Defects in B lymphocyte maturation and T lymphocyte activation in mice lacking JAK3. Science, 270:794; DiSanto et at., (1995). Lymphoid development in mice with a targeted deletion of the interleukin 2 receptor gamma chain. PNAS, 92:377). Moreover, this phenotype is shared to a great extent with SCID patients that hold mutations/defects in the common gamma chain or JAK3 genes (O'Shea et al, (2004). JAK3 and the pathogenesis of severe combined immunodeficiency. Mol Immunol, 41: 727). JAK3-deficient mice are viable but display abnormal lymphopoiesis which leads to a reduced thymus size (10-100 fold smaller than wild type). JAK3-deficient peripheral T cells are unresponsive and have an activated/memory cell phenotype (Baird et al, (1998). T cell development and activation in JAK3-deficient mice. J. Leuk. Biol. 63: 669). The thymic defect in these mice strongly resembles that seen in IL-7 and IL-7 receptor knockout mice, suggesting that the absence of IL-7 signaling accounts for this defect in JAK3 -/-mice (von Freeden-Jeffry et al, (1995). Lymphopenia in Interleukin (IL)-7 Gene-deleted Mice Identifies IL-7 as a non-redundant Cytokine. J Exp Med, 181:1519; Peschon et al, (1994). Early lymphocyte expansion is severely impaired in interleukin 7 receptor-deficient mice. J Exp Med, 180: 1955). These mice, like SCID humans, have no NK cells, probably due to the absence of IL-15 signaling, a survival factor for these cells. JAK3 knockout mice, unlike SCID patients, show deficient B cell lymphopoiesis while in human patients, B cells are present in circulation but are not responsive leading to hypoglobulinemia (O'Shea et al, (2004). JAK3 and the pathogenesis of severe combined immunodeficiency. Mol Immunol, 41: 727). This is explained by species-specific differences in IL-7 function in B and T cell development in mice and humans. On the other hand, Grossman et al. (1999. Dysregulated myelopoiesis in mice lacking JAK3. Blood, 94:932:939) have shown that the loss of JAK3 in the T-cell compartment drives the expansion of the myeloid lineages leading to dysregulated myelopoiesis.
JAK2-deficient mice are embrionically lethal, due to the absence of definitive erythropoiesis. Myeloid progenitors fail to respond to Epo, Tpo, IL-3 or GM-CSF, while G-CSF and IL-6 signaling are not affected. JAK2 is not required for the generation, amplification or functional differentiation of lymphoid progenitors (Parganas et al., (1998). JAK2 is essential for signaling through a variety of cytokine receptors. Cell, 93:385).
JAK1-deficient mice die perinatally due to a nursing defect. JAK1 binds exclusively to the gp130 chain shared by the IL-6 cytokine family (i.e. LIF, CNTF, OSM, CT-1) and along with JAK3, is an essential component of the receptors sharing the common gamma chain, by binding to the non-shared receptor subunit. In this regard, JAK1-deficient mice show similar hematopoiesis defects as JAK3-deficient mice. In addition, they show defective responses to neurotrophic factors and to all interferons (class II cytokine receptors) (Rodig et al, (1998). Disruption of the JAK1 gene demonstrates obligatory and non-redundant roles of the Jaks in cytokine-induced biological response. Cell, 93:373).
Finally, Tyk2-deficient mice show an impaired response to IL-12 and IL-23 and only partially impaired to IFN-alpha (Karaghiosoff et al., (2000). Partial impairment of cytokine responses in Tyk2-deficient mice. Immunity, 13:549; Shimoda et al., (2000). Tyk2 plays a restricted role in IFNg signaling, although it is required for IL-12-mediated T cell function. Immunity, 13:561). However, human Tyk2 deficiency demonstrates that Tyk2 is involved in the signaling from IFN-α, IL-6, IL-10, IL-12 and IL-23 (Minegishi et al., (2006). Human Tyrosine kinase 2 deficiency reveals its requisite roles in multiple cytokine signals involved in innate and acquired immunity. Immunity, 25:745).

The role of JAK kinases in transducing the signal from a myriad of cytokines makes them potential targets for the treatment of diseases in which cytokines have a pathogenic role, such as inflammatory diseases, including but not limited to allergies and asthma, chronic obstructive pulmonary disease (COPD), psoriasis, autoimmune diseases such as rheumatoid arthritis, amyotrophic lateral sclerosis and multiple sclerosis, uveitis, transplant rejection, as well as in solid and hematologic malignancies such as myeloproliferative disorders, leukemia and lymphomas.

Inhibition of JAK kinases, especially JAK1 and JAK3, could give rise to potent immunosuppression which could be used therapeutically to prevent transplant rejection. In this regard, the JAK inhibitor CP-690550 has shown efficacy in several animal models of transplantation (heretopic heart transplantation in mice, cardiac allografts implanted in the ear of mice, renal allotransplantation in cynomolgous monkeys, aorta and tracheal transplantation in rats) by prolonging the mean survival time of grafts (West K (2009). CP-690550, a JAK3 inhibitor as an immunosuppressant for the treatment of rheumatoid arthritis, transplant rejection, psoriasis and other immune-mediated disorders. Curr. Op. Invest. Drugs 10: 491).

In rheumatoid joints, an imbalance between pro and anti-inflammatory cytokine activities favours the induction of autoimmunity, followed by chronic inflammation and tissue destruction. In this regard, the pathogenic role of IL-6 in rheumatoid arthritis (RA) has been validated clinically by the use of the anti-IL-6R antibody tocilizumab. IL-6 activates the transcription factor STAT3, through the use of JAK1 binding to the gp130 receptor chain (Heinrich et al., (2003). Principles of interleukin (IL)-6-type cytokine signaling and its regulation. Biochem J. 374: 1). Constitutive STAT3 mediates the abnormal growth and survival properties of RA synoviocytes (Ivashkiv and Hu (2003). The JAK/STAT pathway in rheumatoid arthritis: pathogenic or protective? Arth & Rheum. 48:2092). Other cytokines that have been implicated in the pathogenesis of arthritis include IL-12 and IL-23, implicated in Th1 and Th17 cell proliferation, respectively; IL-15, and GM-CSF (McInnes and Schett, (2007). Cytokines in the pathogenesis of rheumatoid arthritis. Nature Rew Immunol. 7:429.). The receptors for these cytokines also utilize JAK proteins for signal transduction, making JAK inhibitors potential pleiotropic drugs in this pathology. Consequently, administration of several JAK inhibitors in animal models of murine collagen-induced arthritis and rat adjuvant-induced arthritis has shown to reduce inflammation, and tissue destruction (Milici et al., (2008). Cartilage preservation by inhibition of Janus kinase 3 in two rodent models of rheumatoid arthritis. Arth. Res. 10:R14).

Inflammatory bowel disease (IBD) encloses two major forms of intestinal inflammation: ulcerative colitis and Crohn's disease. Growing evidence has shown that multiple cytokines, including interleukins and interferons, are involved in the pathogenesis of IBD (Strober et al, (2002). The immunology of mucosal models of inflammation. Annu Rev Immunol. 20: 495). Activation of the IL-6/STAT3 cascade in lamina propia T cells has been shown to induce prolonged survival of pathogenic T cells (Atreya et al, (2000). Blockade of interleukin 6 trans signaling suppresses T-cell resistance against apoptosis in chronic intestinal inflammation: Evidence in Crohn's disease and experimental colitis in vivo. Nature Med. 6:583). Specifically, STAT3 has been shown to be constitutively active in intestinal T cells of Crohn's disease patients and a JAK inhibitor has been shown to block the constitutive activation of STAT3 in these cells (Lovato et al, (2003). Constitutive STAT3 activation in intestinal T cells from patients with Crohn's disease. J Biol Chem. 278:16777). These observations indicate that the JAK-STAT pathway plays a pathogenic role in IBD and that a JAK inhibitor could be therapeutic in this setting.

Multiple sclerosis is an autoimmune demyelinating disease characterized by the formation of plaques in the white matter. The role of cytokines in the generation of multiple sclerosis has long been known. Potential therapies include blockade of IFN-g, IL-6, IL-12 and IL-23 (Steinman L. (2008). Nuanced roles of cytokines in three major human brain disorders. J Clin Invest. 118:3557), cytokines that signal through the JAK-STAT pathways. Use of tyrphostin, a JAK inhibitor, has been shown to inhibit IL-12-induced phosphorylation of STAT3, and to reduce the incidence and severity of active and passive experimental autoimmune encephalitis (EAE) (Bright et al., (1999) Tyrphostin B42 inhibits IL-12-induced tyrosine phosphorylation and activation of Janus kinase-2 and prevents experimental allergic encephalomyelitis. J Immunol. 162:6255). Another multikinase inhibitor, CEP701, has been shown to reduce secretion of TNF-alpha, IL-6 and IL-23 as well as the levels of phospho-STAT1, STAT3, and STAT5 in peripheral DCs of mice with EAE, significantly improving the clinical course of EAE in mice (Skarica et al, (2009). Signal transduction inhibition of APCs diminishes Th17 and Th1 responses in experimental autoimmune encephalomyelitis. J. Immunol. 182:4192.).

Psoriasis is a skin inflammatory disease which involves a process of immune cell infiltration and activation that culminates in epithelial remodeling. The current theory behind the cause of psoriasis states the existence of a cytokine network that governs the interaction between immune and epithelial cells (Nickoloff BJ. (2007). Cracking the cytokine code in psoriasis, Nat Med, 13:242). In this regard, IL-23 produced by dendritic cells is found elevated in psoriatic skin, along with IL-12. IL-23 induces the formation of Th17 cells which in turn produce IL-17 and IL-22, the last one being responsible for epidermis thickening. IL-23 and IL-22 induce the phosphorylation of STAT-3, which is found abundantly in psoriatic skin. JAK inhibitors may thus be therapeutic in this setting. In accordance, a JAK1/3 inhibitor, R348, has been found to attenuate psoriasiform skin inflammation in a spontaneous T cell-dependent mouse model of psoriasis (Chang et al., (2009). JAK3 inhibition significantly attenuates psoriasiform skin inflammation on CD18 mutant PL/J mice. J Immunol. 183:2183).

Th2 cytokine-driven diseases such as allergy and asthma could also be a target of JAK inhibitors. IL-4 promotes Th2 differentiation, regulates B-cell function and immunoglobulin class switching, regulates eotaxin production, induces expression of IgE receptor and MHC II on B cells, and stimulates mast cells. Other Th2 cytokines like IL-5 and IL-13 can also contribute to eosinophil recruitment in bronchoalveolar lavage by stimulating eotaxin production. Pharmacological inhibition of JAK has been shown to reduce the expression of IgE receptor and MHCII induced by IL-4 stimulation on B cells (Kudlacz et al., (2008). The JAK3 inhibitor CP-690550 is a potent anti-inflammatory agent in a murine model of pulmonary eosinophilia. European J. Pharm. 582: 154). Furthermore, JAK3-deficient mice display poor eosinophil recruitment and mucus secretion to the airway lumen upon OVA challenge, as compared to wild type mice (Malaviya et al, (2000). Treatment of allergic asthma by targeting Janus kinase 3-dependent leukotriene synthesis in mast cells with 4-(3', 5'- dibromo-4'-hydroxyphenyl)amino-6,7-dimethoxyquinazoline (WHI-P97). JPET 295:912.). In this regard, systemic administration of the CP-690550 JAK inhibitor in mice has been shown to reduce the eosinophil count as well as the levels of eotaxin and IL13 in BAL in a murine model of pulmonary eosinophilia (Kudlacz et al., (2008). The JAK3 inhibitor CP-690550 is a potent anti-inflammatory agent in a murine model of pulmonary eosinophilia. European J. Pharm. 582:154).

There is increasing evidence that cytokines play a pathogenetic role in ocular inflammatory disease such as uveitis or dry eye syndrome. Some cytokines implicated in experimental autoimmune uveitis, such as IL-2, IL-6, IL-12 and IFNg, would be amenable to JAK inhibition (Vallochi et al, (2007). The role of cytokines in the regulation of ocular autoimmune inflammation. Cytok Growth Factors Rev. 18:135). In this regard, drugs or biologicals that interfere with IL-2 signaling such as cyclosporine or anti-IL-2 receptor antibody (daclizumab) have shown efficacy in the treatment of keratoconjuctivitis sicca and refractory uveitis, respectively (Lim et al, (2006). Biologic therapies for inflammatory eye disease. Clin Exp Opht 34:365). Similarly, allergic conjunctivitis, a common allergic eye disease characterized by conjuctival congestion, mast cell activation and eosinophil infiltration, could benefit from JAK inhibition. STAT6-deficient mice, showing decreased TH2-mediated immune responses which are normally triggered by IL-4, do not develop the classical early and late phase responses, suggesting that IL-4 pathway abrogation through JAK inhibition may be therapeutic in this setting (Ozaki et al, (2005). The control of allergic conjunctivitis by suppression of cytokine signaling (SOCS)3 and SOCS5 in a murine model. J Immunol, 175:5489).

There is growing evidence of the critical role of STAT3 activity in processes involved in tumorigenesis like cell cycle dysregulation, promotion of uncontrolled growth, induction of survival factors and inhibition of apoptosis (Siddiquee et al., (2008). STAT3 as a target for inducing apoptosis in solid and haematological tumors. Cell Res. 18: 254). Antagonism of STAT3 by means of dominant-negative mutants or antisense oligonucleotides has shown to promote apoptosis of cancer cells, inhibition of angiogenesis and up-regulation of host immunocompetence. Inhibition of constitutively active STAT3 in human tumors by means of JAK inhibitors may provide a therapeutic option to the treatment of this disease. In this regard, the use of the JAK inhibitor tyrphostin has been shown to induce apoptosis of malignant cells and inhibit cell proliferation in vitro and in vivo (Meydan et al., (1996). Inhibition of acute lymphoblastic leukemia by a JAK-2 inhibitor. Nature, 379:645).

Hematological malignancies with dysregulated JAK-STAT pathways may benefit from JAK inhibition. Recent studies have implicated dysregulation of JAK2 kinase activity by chromosomal translocations and mutations within the pseudokinase domain (such as the JAK2V617F mutation) in a spectrum of myeloproliferative diseases (Ihle and Gililand, 2007), including polycythemia vera, myelofibrosis and essential thrombocythemia. In this regard, several JAK inhibitors that tackle JAK2 potently, such as TG-1 01209 (Pardanani et al., (2007). TG101209, a small molecular JAK2-selective inhibitor potently inhibits myeloproliferative disorder-associated JAK2V617F and MPLW515L/K mutations Leukemia. 21:1658-68), TG101348 (Wernig et al, (2008). Efficacy of TG101348, a selective JAK2 inhibitor, in treatment of a murine model of JAK2V617F-induced polycythemia vera. Cancer Cell, 13: 311), CEP701, (Hexner et al, (2008). Lestaurtinib (CEP701) is a JAK2 inhibitor that suppresses JAK2/STAT5 signaling and the proliferation of primary erythroid cells from patients with myeloproliferative disorders. Blood, 111: 5663), CP-690550 (Manshouri et al, (2008). The JAK kinase inhibitor CP-690550 suppresses the growth of human polycythemia vera cells carrying the JAK2V617F mutation. Cancer Sci, 99:1265), and CYT387 (Pardanani et al., (2009). CYT387, a selective JAK1/JAK2 inhibitor: invitro assessment of kinase selectivity and preclinical studies using cell lines and primary cells from polycythemia vera patients. Leukemia, 23:1441) have been proposed for treating myeloproliferative diseases on the basis of their antiproliferative activity on cells carrying the JAK2V617F mutation. Similarly, T-cell leukemia due to human T-cell leukemia virus (HTLV-1) transformation is associated with JAK3 and STAT5 constitutive activation (Migone et al, (1995). Constitutively activated JAK-STAT pathway in T cells transformed with HTLV-I. Science, 269: 79) and JAK inhibitors may be therapeutic in this setting (Tomita et al, (2006). Inhibition of constitutively active JAK-STAT pathway suppresses cell growth of human T-cell leukemia virus type I-infected T cell lines and primary adult T-cell leukemia cells. Retrovirology, 3:22). JAK1-activating mutations have also been identified in adult acute lymphoblastic leukemia of T cell origin (Flex et al, (2008). Somatically acquired JAK1 mutations in adult acute lymphoblastic leukemia. J. Exp. Med. 205:751-8) pointing to this kinase as a target for the development of novel antileukemic drugs.

Conditions in which targeting of the JAK pathway or modulation of the JAK kinases, particularly JAK1, JAK2 and JAK3 kinases, are contemplated to be therapeutically useful for the treatment or prevention of diseases include: neoplastic diseases (e.g. leukemia, lymphomas, solid tumors); transplant rejection, bone marrow transplant applications (e.g., graft- versus-host disease); autoimmune diseases (e.g. diabetes, multiple sclerosis, rheumatoid arthritis, inflammatory bowel disease); respiratory inflammation diseases (e.g. asthma, chronic obstructive pulmonary disease), inflammation-linked ocular diseases or allergic eye diseases (e.g. dry eye, glaucoma, uveitis, diabetic retinopathy, allergic conjunctivitis or age-related macular degeneration) and skin inflammatory diseases (e.g., atopic dermatitis or psoriasis).

In view of the numerous conditions that are contemplated to benefit by treatment involving modulation of the JAK pathway or of the JAK kinases it is immediately apparent that new compounds that modulate JAK pathways and use of these compounds should provide substantial therapeutic benefits to a wide variety of patients.

Provided herein are novel heteroaryl imidazolone derivatives for use in the treatment of conditions in which targeting of the JAK pathway or inhibition of JAK kinases can be therapeutically useful.

The compounds described in the present invention are simultaneously potent JAK1, JAK2 and JAK3 inhibitors, i.e. pan-JAK inhibitors. This property makes them useful for the treatment or prevention of pathological conditions or diseases such as myeloproliferative disorders (such as polycythemia vera, essential thrombocythemia or myelofibrosis), leukemia, lymphomas and solid tumors; bone marrow and organ transplant rejection; or immune-mediated diseases such as autoimmune and inflammation diseases, including rheumatoid arthritis, multiple sclerosis, inflammatory bowel disease (such as ulcerative colitis or Crohn's disease), inflammation-linked ocular diseases or allergic eye diseases (such as dry eye, uveitis, or allergic conjunctivitis), allergic rhinitis, asthma, chronic obstructive pulmonary disease (COPD), and skin inflammatory diseases (such as atopic dermatitis or psoriasis).

It has now been found that certain heteroaryl imidazolone derivatives are novel and potent JAK inhibitors and can therefore be used in the treatment or prevention of these diseases.

Thus the present invention is directed to new heteroaryl imidazolone derivatives of formula (I), or a pharmaceutically acceptable salt, or solvate, or N-oxide, or stereoisomer or deuterated derivative thereof: 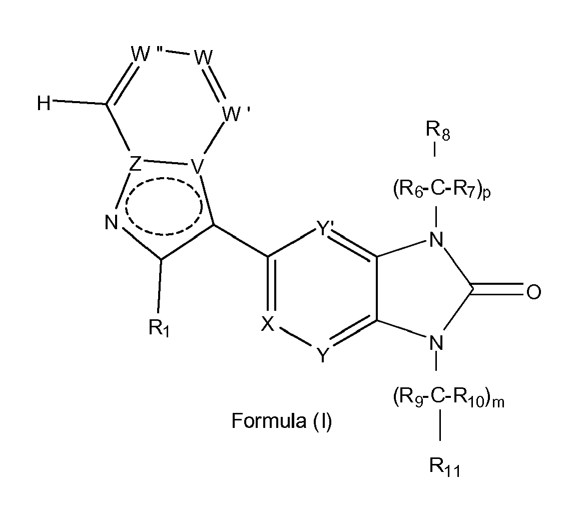 wherein
m is 0 or an integer from 1 to 3;
p is 0 or an integer from 1 to 3;
Z and V independently represent a nitrogen atom or a carbon atom, wherein at least one of Z and V represents a nitrogen atom, and the other represents a carbon atom; W represents a nitrogen atom or a -CR₃ group;
W' represents a nitrogen atom or a -CR₂ group;
W" represents a nitrogen atom or a -CR₄ group;
X and Y independently represent a nitrogen atom or a -CR₅ group, wherein at least one of X and Y represents a -CR₅ group;
Y' represents a nitrogen atom or a -CR₅, group;
R₁, R₂, R₃, R₄ and R₅ independently represent a hydrogen atom, a halogen atom, a hydroxyl group, a cyano group, a linear or branched C₁-C₆ alkyl group, a C₂-C₄ alkenyl group, a C₂-C₄ alkynyl group, a C₁-C₄ haloalkyl group, a C₁-C₄ hydroxyalkyl group, a C₃-C₁₀ cycloalkyl group, a C₃-C₁₀ cycloalkenyl group, a monocyclic or polycyclic C₅-C₁₄ aryl group, a 5- to 14- membered heteroaryl group containing at least one heteroatom selected from O, S and N, a 5- to 14- membered heterocyclyl group containing at least one heteroatom selected from O, S and N, a bicyclyl group containing a monocyclic C₅-C₉ aryl or heteroaryl group bonded directly to a 5- to 9- membered cycloalkyl or heterocyclyl group, said heteroaryl or heterocyclyl group containing at least one heteroatom selected from O, S and N, an aza-bicycloalkyl group having up to 12 carbon atoms or an aza-bicycloalkenyl group having up to 12 carbon atoms, a -(CH₂)qSR₁₅ group, a -(CH₂)qSOR₁₅ group, a -(CH₂)qS(O)₂R₁₅ group, a -(CH₂)qS(O)₂NR₁₅R₁₆ group, a -(CH₂)_{q}NR₁₅S(O)₂R₁₆ group, a -(CH₂)qN R₁₅S(O)₂N R₁₆ group, a -(CH₂)_{q}OR₁₅ group, a -(CH₂)_{q}C(O)OR₁₅ group, a -(CH₂)_{q}O-C(O)R₁₅ group, a - (CH₂)_{q},C(O)-(CH₂)_{q}-R₁₅ group, a -(CH₂)_{q}NR₁₅R₁₆ group, a -(CH₂)_{q}CH(R₁₅)NR₁₆R₁₇ group, a -(CH₂)_{q},C(O)-(CH₂)_{q}-NR₁₅R₁₆ group, a -(CH₂)_{q},NR₁₅C(O)-(CH₂)q-R₁₆ group or a -(CH₂)_{q},NR₁₅C(O)-(CH₂)_{q}-NR₁₆R₁₇ group, wherein each q and q' are independently 0, 1 or 2,
   wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from substituents Ra, and the alkyl groups are unsubstituted or substituted by one or more substituents selected from Rb;
R₅, represents a hydrogen atom, a halogen atom, a hydroxyl group, a cyano group, a linear or branched C₁-C₆ alkyl group, a C₂-C₄ alkenyl group, a C₂-C₄ alkynyl group, a C₁-C₄ haloalkyl group, a C₁-C₄ hydroxyalkyl group or a C₃-C₁₀ cycloalkyl group;
R₆, R₇, R₉ and R₁₀ each independently represent a hydrogen atom, a hydroxyl group, a C₁-C₄ hydroxyalkyl group, a -(C₁-C₄ alkyl)-O-(C₁-C₄ alkyl) group or a linear or branched C₁-C₆ alkyl group, wherein said alkyl group is optionally substituted by one or more substituents selected from a cyano group, a C₁-C₄ haloalkyl group, a C₁-C₄ hydroxyalkyl group, a C₁-C₄ alkoxycarbonyl group, a C₃-C₇ cycloalkyl group, a phenyl group or a 6 membered, saturated N-containing heterocyclyl ring;
R₈ and R₁₁ each independently represent a hydrogen atom, a halogen atom, a cyano group, a linear or branched C₁-C₆ alkyl group, a C₂-C₄ alkenyl group, a C₂-C₄ alkynyl group, a C₁-C₄ haloalkyl group, a C₁-C₄ hydroxyalkyl group, a C₃-C₁₀ cycloalkyl group, a C₃-C₁₀ cycloalkenyl group, a monocyclic or polycyclic C₅-C₁₄ aryl group, a 5- to 14-membered heteroaryl group containing at least one heteroatom selected from O, S and N, a 5- to 14- membered heterocyclyl group containing at least one heteroatom selected from O, S and N, a bicyclyl group containing a monocyclic C₅-C₉ aryl or heteroaryl group fused to a 5- to 9- membered cycloalkyl or heterocyclyl group, said heteroaryl or heterocyclyl group containing at least one heteroatom selected from O, S and N, a bicyclyl group containing a C₃-C₁₀ cycloalkyl group fused to a 5- to 9-membered heterocyclyl group containing at least one heteroatom selected from O, S and N, an aza-bicycloalkyl group having up to 12 carbon atoms or a aza-bicycloalkenyl group having up to 12 carbon atoms, a -(CH₂)ₙSR₁₅ group, a -(CH₂)ₙSOR₁₅ group, a -(CH₂)ₙS(O)₂R₁₅ group, a -(CH₂)ₙS(O)₂NR₁₅R₁₆ group, a -(CH₂)ₙNR₁₅S(O)₂R₁₆ group, a -(CH₂)ₙNR₁₅S(O)₂NR₁₆ group, a -(CH₂)ₙOR₁₅ group, a -(CH₂)ₙC(O)OR₁₅ group, a -(CH₂)ₙO-C(O)R₁₅ group, a -(CH₂)ₙ,C(O)-(CH₂)ₙ-R₁₅ group, a -(CH₂)ₙ,C(OH)(J)-(CH₂)ₙK group, a -(CH₂)ₙNR₁₅R₁₆ group, a -(CH₂)ₙCH(R₁₅)NR₁₆R₁₇ group, a -(CH₂)ₙ,C(O)-(CH₂)ₙ-NR₁₅R₁₅ group, a -(CH₂)ₙ,NR₁₅C(O)-(CH₂)n-R₁₆ group or a -(CH₂)ₙ,NR₁₅C(O)-(CH₂)ₙ-NR₁₆R₁₇ group, wherein each n and n' are independently 0 or 1; and J represents a hydrogen atom or a methyl group, K represents a hydroxyl group, a methyl group or a -NR'R" group, and R' and R" each independently represent a hydrogen atom, a linear or branched C₁-C₆ alkyl group, a C₁-C₄ haloalkyl group or a C₁-C₄ hydroxyalkyl group,
   wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from Ra, -(C₁-C₄ alkyl)-CN groups, or -(C₁-C₄ alkyl)-C(O)NR'R" groups wherein R' and R" are the same or different and are selected from hydrogen atoms and linear or branched C₁-C₄ alkyl groups; and the alkyl groups are unsubstituted or substituted by one or more substituents selected from Rb;
Ra is a halogen atom, a cyano group, a hydroxyl group, an oxo group, a linear or branched C₁-C₆ alkyl group, a C₁-C₄ haloalkyl group, C₁-C₄ alkoxy group, a C₁-C₄ hydroxyalkyl group, a C₃-C₇ cycloalkyl or a C₃-C₇ cycloalkenyl group unsubstituted or substituted by one or more substituents selected from substituents Re, a monocyclic or polycyclic C₅-C₁₄ aryl group unsubstituted or substituted by one or more substituents selected from substituents Re, a 5- to 14- membered heteroaryl group containing at least one heteroatom selected from O, S and N unsubstituted or substituted by one or more substituents selected from substituents Re, a 5- to 14- membered heterocyclyl group containing at least one heteroatom selected from O, S and N unsubstituted or substituted by one or more substituents selected from substituents Re, a -(CH₂)_{q}SR₁₂ group, a -(CH₂)_{q}SOR₁₂ group, a -(CH₂)_{q}S(O)₂R₁₂ group, a -(CH₂)_{q}S(O)₂NR₁₂R₁₃ group, a -(CH₂)_{q}NR₁₂S(O)₂R₁₃ group, a -(CH₂)_{q}NR₁₂S(O)₂NR₁₃ group, a -(CH₂)_{q}OR₁₂ group, a -(CH₂)_{q}C(O)OR₁₂ group, a -(CH₂)_{q}O-C(O)R₁₂ group, a -(CH₂)_{q},C(O)-(CH₂)_{q}-R₁₂ group, a -(CH₂)_{q}NR₁₂R₁₃ group, a -(CH₂)qCH(R₁₂)NR₁₃R₁₄ group, a -(CH₂)_{q},C(O)-(CH₂)_{q}-NR₁₂R₁₃ group, a -(CH₂)_{q},NR₁₂C(O)-(CH₂)_{q}-R₁₃ group or a -NR₁₂C(O)-(CH₂)_{q}-NR₁₃R₁₄ group, wherein each q and q' are independently 0, 1 or 2;
Rb is a cyano group, a C₁-C₄ haloalkyl group, C₁-C₄ alkoxy group, a C₁-C₄ hydroxyalkyl group, a C₃-C₇ cycloalkyl or a C₃-C₇ cycloalkenyl group unsubstituted or substituted by one or more substituents selected from substituents Re, a monocyclic or polycyclic C₅-C₁₄ aryl group unsubstituted or substituted by one or more substituents selected from substituents Re, a 5- to 14- membered heteroaryl group containing at least one heteroatom selected from O, S and N unsubstituted or substituted by one or more substituents selected from substituents Re, a 5- to 14- membered heterocyclyl group containing at least one heteroatom selected from O, S and N unsubstituted or substituted by one or more substituents selected from substituents Re, a -(CH₂)_{q}SR₁₂ group, a -(CH₂)_{q}SOR₁₂ group, a -(CH₂)_{q}S(O)₂R₁₂ group, a -(CH₂)_{q}S(O)₂NR₁₂R₁₃ group, a -(CH₂)_{q}NR₁₂S(O)₂R₁₃ group, a -(CH₂)_{q}NR₁₂S(O)₂NR₁₃ group, a -(CH₂)_{q}OR₁₂ group, a -(CH₂)_{q}C(O)OR₁₂ group, a -(CH₂)_{q}O-C(O)R₁₂ group, a -(CH₂)_{q},C(O)-(CH₂)_{q}-R₁₂ group, a -(CH₂)_{q}NR₁₂R₁₃ group, a -(CH₂)_{q}CH(R₁₂)NR₁₃R₁₄ group, a -(CH₂)_{q},C(O)-(CH₂)_{q}-NR₁₂R₁₃ group, a -(CH₂)_{q},NR₁₂C(O)-(CH₂)_{q}-R₁₃ group or a -NR₁₂C(O)-(CH₂)_{q}-NR₁₃R₁₄ group, wherein each q and q' are independently 0, 1 or 2;
R₁₂, R₁₃ and R₁₄ each independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, a bicyclyl group containing a monocyclic C₅-C₆ aryl or heteroaryl group bonded directly to a 5- to 6- membered cycloalkyl or heterocyclyl group which heteroaryl or heterocyclyl group contains 1, 2 or 3 nitrogen atoms, the haloalkyl, hydroxyalkyl, alkoxycarbonyl, cycloalkyl, phenyl, heteroaryl, heterocyclyl and bicyclyl group being unsubstituted or substituted by one or more substituents selected from substituents Rc, and the alkyl groups being unsubstituted or substituted by one or more substituents selected from substituents Rd;
Rc is a halogen atom, a hydroxyl group, a cyano group, a linear or branched C₁-C₆ alkyl group, a C₁-C₄ haloalkyl group, a C₁-C₄ alkoxy group, a C₁-C₄ hydroxyalkyl group, a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃-C₇ heterocycloalkyl ketone group containing 1, 2 or 3 nitrogen atoms, wherein said phenyl group is unsubstituted or substituted by one or more halogen atoms, and wherein said heteroaryl, heterocyclyl and heterocycloalkyl ketone groups are unsubstituted or substituted by one or more linear or branched C₁-C₃ alkyl groups;
Rd is a cyano group, a C₁-C₄ haloalkyl group, a C₁-C₄ alkoxy group, a C₁-C₄ hydroxyalkyl group, a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃-C₇ heterocycloalkyl ketone group containing 1, 2 or 3 nitrogen atoms, wherein said phenyl group is unsubstituted or substituted by one or more halogen atoms, and wherein said heteroaryl, heterocyclyl and heterocycloalkyl ketone groups are unsubstituted or substituted by one or more linear or branched C₁-C₃ alkyl groups;
Re is a halogen atom, a hydroxyl group, a cyano group, a linear or branched C₁-C₆ alkyl group or a C₁-C₄ haloalkyl group;
R₁₅, R₁₆, and R₁₇ each independently represents a hydrogen atom, a cyano group, a linear or branched C₁-C₆ alkyl group, a C₁-C₄ haloalkyl group, a C₁-C₄ hydroxyalkyl group, a C₁-C₄ alkoxycarbonyl group, a C₃-C₇ cycloalkyl group, a monocyclic or polycyclic C₅-C₁₄ aryl group, a 5- to 14- membered heteroaryl group containing at least one heteroatom selected from O, S and N, or a 5- to 14- membered heterocyclyl group containing at least one heteroatom selected from O, S and N, wherein the haloalkyl, hydroxyalkyl, alkoxycarbonyl, cycloalkyl, aryl, heteroaryl and heterocyclyl groups are unsubstituted or substituted by one or more substituents selected from the substituents Ra, and the alkyl groups are unsubstituted or substituted by one or more substitutents selected from Rb;
provided that the compound of formula (1) does not carry a -(CH₂)ₙSR₁₅ group, a - (CH₂)ₙSOR₁₅ group, a -(CH₂)ₙS(O)₂R₁₅ group, a -(CH₂)ₙ S(O)₂NR₁₅R₁₆ group, a - (CH₂)ₙNR₁₅S(O)₂R₁₆ group, a -(CH₂)ₙNR₁₅S(O)₂NR₁₆ group, a -(CH₂)ₙOR₁₅ group, a - (CH₂)ₙC(O)OR₁₅ group, a -(CH₂)ₙO-C(O)R₁₅ group, a -(CH₂)ₙ,C(O)-(CH₂)ₙ-R₁₅ group, a - (CH₂)ₙNR₁₅R₁₆ group, a -(CH₂)ₙCH(R₁₅)NR₁₆R₁₇ group, a -(CH₂)ₙ,C(O)-(CH₂)ₙ-NR₁₅R₁₆ group, a -(CH₂)ₙ,NR₁₅C(O)-(CH₂)ₙ-R₁₆ group or a -(CH₂)ₙ,NR₁₅C(O)-(CH₂)ₙ-NR₁₆R₁₇ group bonded directly to an imidazolone nitrogen atom.

The invention further provides synthetic processes and intermediates described herein, which are useful for preparing said compounds.

The invention also provides a pharmaceutical composition comprising the compounds of the invention and a pharmaceutically-acceptable diluent or carrier.

The invention also provides a compound of the invention for use in the treatment of the human or animal body by therapy.

The invention is also directed to the compounds of the invention as described herein, for use in the treatment of a pathological condition or disease susceptible to amelioration by inhibition of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

The invention also provides a method of treatment of a pathological condition or disease susceptible to amelioration by inhibition of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis; comprising administering a therapeutically effective amount of the compounds of the invention or a pharmaceutical composition of the invention to a subject in need of such treatment.

The invention also provides use of a compound of the invention in the manufacture of a medicament for the treatment of the human or animal body by therapy, in particular wherein the medicament is for the treatment of a pathological condition or disease susceptible to amelioration by inhibition of Janus Kinases (JAK), more in particular wherein the pathological condition or disease is selected from myeloproliferative disorders, leukemia, lymphoid malignancies and solid tumors; bone marrow and organ transplant rejection; immune-mediated diseases and inflammatory diseases, and still more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis

The invention also provides a combination product comprising of (i) the compounds of the invention as described herein; and (ii) one or more additional active substances which are known to be useful in the treatment of myeloproliferative disorders (such as polycythemia vera, essential thrombocythemia or myelofibrosis), leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease (such as ulcerative colitis or Crohn's disease), dry eye, uveitis, allergic conjunctivitis, allergic rhinitis, asthma, chronic obstructive pulmonary disease (COPD), atopic dermatitis and psoriasis.

As used herein the term C₁-C₆ alkyl embraces linear or branched radicals having 1 to 6 carbon atoms, preferably 1 to 4 carbon atoms. Examples include methyl, ethyl, n-propyl, i-propyl, n-butyl, sec-butyl, t-butyl, n-pentyl, 1-methylbutyl, 2-methylbutyl, isopentyl, 1-ethylpropyl, 1,1-dimethylpropyl, 1,2-dimethylpropyl, n-hexyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl, 3-methylpentyl and iso-hexyl radicals.

As used herein, the term C₂-C₄ alkenyl embraces optionally substituted, linear or branched, mono or polyunsaturated radicals having 2 to 4 carbon atoms. Examples include vinyl, allyl, 1-propenyl, isopropenyl, 1-butenyl, 2-butenyl and 3-butenyl radicals.

As used herein, the term C₂-C₄ alkynyl embraces optionally substituted, linear or branched, mono or polyunsaturated radicals having 2 to 4 carbon atoms. Examples include 1-propynyl, 2-propynyl, 1-butynyl, 2-butynyl and 3-butynyl radicals.

When it is mentioned that alkyl, alkenyl or alkynyl radicals may be optionally substituted it is meant to include linear or branched alkyl, alkenyl or alkynyl radicals as defined above, which may be unsubstituted or substituted in any position by one or more substituents, for example by 1, 2 or 3 substituents. When two or more substituents are present, each substituent may be the same or different.

A said optionally substituted alkyl group is typically unsubstituted or substituted by 1, 2 or 3 substituents which may be the same or different. Typically, substituents on an alkyl group are themselves unsubstituted. Preferred substituents on the alkyl groups are halogen atoms and hydroxy groups, and are more preferably halogen atoms.

A said optionally substituted alkenyl group is typically unsubstituted or substituted by 1, 2 or 3 substituents which may be the same or different. Typically, substituents on an alkenyl group are themselves unsubstituted. Preferred substituents on the alkenyl groups are halogen atoms and hydroxy groups, and are more preferably halogen atoms.

A said optionally substituted alkynyl group is typically unsubstituted or substituted by 1, 2 or 3 substituents which may be the same or different. Typically, substituents on an alkynyl group are themselves unsubstituted. Preferred substituents on the alkynyl groups are halogen atoms and hydroxy groups, and are more preferably halogen atoms.

As used herein, the term C₁-C₄ haloalkyl group is an alkyl group, for example a C₁₋₄ or C₁₋₂ alkyl group, which is bonded to one or more, preferably 1, 2 or 3 halogen atoms. Preferably, said haloakyl group is chosen from -CCl₃ and -CF₃.

As used herein, the term C₁-C₄ hydroxyalkyl embraces linear or branched alkyl radicals having 1 to 4 carbon atoms, any one of which may be substituted by one or more, preferably 1 or 2, more preferably 1 hydroxyl radicals. Examples of such radicals include hydroxymethyl, hydroxyethyl, hydroxypropyl, and hydroxybutyl.

As used herein, the term C₁-C₄ alkoxy (or alkyloxy) embraces linear or branched oxy-containing radicals each having alkyl portions of 1 to 4 carbon atoms.

When it is mentioned that alkoxy radicals may be optionally substituted it is meant to include linear or branched alkoxy radicals as defined above, which may be unsubstituted or substituted in any position by one or more substituents, for example by 1, 2 or 3 substituents. When two or more substituents are present, each substituent may be the same or different. A said optionally substituted alkoxy group is typically unsubstituted or substituted by 1, 2 or 3 substituents which may be the same or different. Typically, the substituents on an alkoxy group are themselves unsubstituted. Preferred alkoxy radicals include methoxy, ethoxy, n-propoxy, i-propoxy, n-butoxy, sec-butoxy, t-butoxy, trifluoromethoxy, difluoromethoxy, hydroxymethoxy, 2-hydroxyethoxy and 2-hydroxypropoxy.

As used herein, the term C₁-C₄ alkoxycarbonyl group embraces radicals of formula -C(O)O(C₁-C₄ alkyl), wherein said C₁-C₄ alkyl is a linear or branched hydrocarbon radical having 1 to 4 carbon atoms. Examples include methoxycarbonyl, ethoxycarbonyl, n-propyloxycarbonyl, i-propyloxycarbonyl, n-butyloxycarbonyl, sec-butyloxycarbonyl and tert-butyloxycarbonyl radicals.

As used herein, the term C₃-C₁₀ cycloalkyl embraces saturated monocyclic or polycyclic carbocyclic radicals having from 3 to 10 carbon atoms, preferably from 3 to 7 carbon atoms.

When it is mentioned that cycloalkyl radicals may be optionally substituted it is meant to include linear or branched cycloalkyl radicals as defined above, which may be unsubstituted or substituted in any position by one or more substituents, for example by 1, 2 or 3 substituents. When two or more substituents are present, each substituent may be the same or different. A said optionally substituted C₃-C₁₀ cycloalkyl radical is typically unsubstituted or substituted by 1, 2 or 3 substituents which may be the same or different. When a C₃-C₁₀ cycloalkyl radical carries 2 or more substituents, the substituents may be the same or different. Typically the substituents on a C₃-C₁₀ cycloalkyl group are themselves unsubstituted. Polycyclic cycloalkyl radicals contain two or more fused cycloalkyl groups, preferably two cycloalkyl groups. Typically, polycyclic cycloalkyl radicals are selected from decahydronaphthyl (decalyl), bicyclo[2.2.2]octyl, adamantly, camphyl or bornyl groups.
Examples of monocyclic cycloalkyl groups include cyclopropyl, cyclobutyl, cyclopentyl, cyclohexyl, cycloheptyl, cyclooctyl, cyclononyl and cyclodecyl.

As used herein, the term C₃-C₁₀ cycloalkenyl embraces partially unsaturated carbocyclic radicals having from 3 to 10 carbon atoms, preferably from 3 to 7 carbon atoms.

When it is mentioned that cycloalkenyl radicals may be optionally substituted it is meant to include linear or branched cycloalkenyl radicals as defined above, which may be unsubstituted or substituted in any position by one or more substituents, for example by 1, 2 or 3 substituents. When two or more substituents are present, each substituent may be the same or different. A said optionally substituted C₃-C₁₀ cycloalkenyl radical is typically unsubstituted or substituted by 1, 2 or 3 substituents which may be the same or different. When a C₃-C₁₀ cycloalkenyl radical carries 2 or more substituents, the substituents may be the same or different. Typically, the substituents on a cycloalkenyl group are themselves unsubstituted.
Examples include cyclobutenyl, cyclopentenyl, cyclohexenyl, cycloheptenyl, cyclooctenyl, cyclononenyl and cyclodecenyl.

As used herein, the term C₅-C₁₄ aryl radical embraces typically a C₅-C₁₄, preferably C₆-C₁₄, more preferably C₆-C₁₀ monocyclic or polycyclic aryl radical such as phenyl, naphthyl, anthranyl and phenanthryl. Phenyl is preferred. A said optionally substituted C₅-C₁₄ aryl radical is typically unsubstituted or substituted by 1, 2 or 3 substituents which may be the same or different. When a C₅-C₁₄ aryl radical carries 2 or more substituents, the substituents may be the same or different. Unless otherwise specified, the substituents on a C₅-C₁₄ aryl group are typically themselves unsubstituted.

As used herein, the term 5- to 14- membered heteroaryl radical embraces typically a 5-to 14- membered ring system, preferably a 5- to 10- membered ring system, more preferably a 5- to 6- membered ring system, comprising at least one heteroaromatic ring and containing at least one heteroatom selected from O, S and N. A 5- to 14-membered heteroaryl radical may be a single ring or two or more fused rings wherein at least one ring contains a heteroatom.

A said optionally substituted 5- to 14- membered heteroaryl radical is typically unsubstituted or substituted by 1, 2 or 3 substituents which may be the same or different. When a 5- to 14- membered heteroaryl radical carries 2 or more substituents, the substituents may be the same or different. Unless otherwise specified, the substituents on a 5- to 14- membered heteroaryl radical are typically themselves unsubstituted.

Examples of 5- to 14- membered heteroaryl radicals include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidinyl and the various pyrrolopyridyl radicals.

As used herein, the term 5- to 14-membered heterocyclyl radical embraces typically a non-aromatic, saturated or unsaturated C₅-C₁₄ carbocyclic ring system, preferably C₅-C₁₀ carbocyclic ring system, more preferably C₅-C₆ carbocyclic ring system, in which one or more, for example 1, 2, 3 or 4 of the carbon atoms preferably 1 or 2 of the carbon atoms are replaced by a heteroatom selected from N, O and S. A heterocyclyl radical may be a single ring or two or more fused rings wherein at least one ring contains a heteroatom. A 5- to 14-membered heterocyclyl radical may be a spiro compound consisting of two or more rings, preferably two rings, which rings are connected through a single atom wherein at least one ring contaims a heteroatom.

A said optionally substituted 5- to 14-membered heterocyclyl radical is typically unsubstituted or substituted by 1, 2 or 3 substituents. Typically, the substituents on a 5 to 14-membered heterocyclyl radical are themselves unsubstituted.

Where a 5- to 14-membered heterocyclyl radical carries 2 or more substituents, the substituents may be the same or different.

Examples of 5- to 14-membered heterocyclyl radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, 4,5-dihydro-oxazolyl, 2-benzofuran-1(3H)-one, 1,3-dioxol-2-one, 3-aza-tetrahydrofuranyl and 1,4-dioxospiro[4.5]decanyl.

As used herein, the term 6-membered saturated N-containing heterocyclic group is a C₆ saturated carbocyclic ring system in which one of the carbon atoms is replaced by N and optionally in which 1, 2, or 3, preferably 1 or 2, further carbon atoms are replaced by heteroatoms selected from N and O.

A said 6-membered saturated N-containing heterocyclic group is typically unsubstituted or substituted by 1, 2 or 3 substituents which may be the same or different. Typically, the substituents on a 6-membered saturated N-containing heterocyclic group are themselves unsubstituted, unless otherwise specified.

Examples of 6-membered saturated N-containing heterocyclic group include piperidyl and piperazinyl.

As used herein, the term C₃-C₇ heterocycloalkyl ketone group embraces typically a non-aromatic, saturated or unsaturated C₃-C₇ carbocyclic ring system, in which one of the carbon atoms is replaced by a C=O group and 1, 2 or 3, preferably 1 or 2, more preferably 1, further carbon atoms are replaced by a heteroatom selected from N, O and S, and preferably are replaced by N. Examples include pyridone groups.

As used herein, the term aza-bicycloalkyl group having up to 12 carbon atoms denotes a fused ring system consisting of a cycloalkyl group and a N-containing heterocyclyl group. A said N-containing heterocyclyl grop is typically a 6-membered saturated N-containing heterocyclyl group, as defined herein.

As used herein, the term aza-bicycloalkenyl group having up to 12 carbon atoms embraces an aza-bicycloalkyl group, as defined herein, containing at least one unsaturated carbon-carbon bond.

As used herein, a bicyclyl group containing a monocyclic C₅-C₉ aryl or heteroaryl group bonded directly to a 5- to 9- membered cycloalkyl or heterocyclyl group typically refers to groups where a monocyclic C₅-C₉ aryl or heteroaryl group is bonded to a 5- to 9-membered cycloalkyl or heterocyclyl group by a single bond.

As used herein, a bicyclyl group containing a monocyclic C₅-C₉ aryl or heteroaryl group fused to a 5- to 9- membered cycloalkyl or heterocyclyl group typically refers to a moiety containing a bond which is shared between a monocyclic C₅-C₉ aryl or heteroaryl group and a 5- to 9- membered cycloalkyl or heterocyclyl group. Examples include chromanyl groups or 1,2,3,4-tetrahydronaphthalenyl groups.

As used herein, a bicyclyl group containing a C₃-C₁₀ cycloalkyl group fused to a 5- to 9-membered heterocyclyl group typically refers to a moiety containing a bond which is shared between a C₃-C₁₀ cycloalkyl group and a 5- to 9- membered heterocyclyl group Examples include 5,6,7,8-tetrahydroquinolinyl groups and 3,4-dihydro-2H-pyrano[2,3-b]pyridinyl groups.

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

Typically when a cyclic radical is bridged by an alkylene or alkylenedioxy radical, the bridging alkylene radical is bonded to the ring at non-adjacent atoms.

As used herein, the term halogen atom embraces chlorine, fluorine, bromine and iodine atoms. A halogen atom is typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclyl amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) -is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

The dotted line in the ring containing Z, V and N in the compounds of formula (I) denotes that there are two double bounds in the ring, whose position may vary depending on which Z or V represents a nitrogen atom or a carbon atom. Thus, when Z represents a nitrogen atom, the ring is represented by the formula 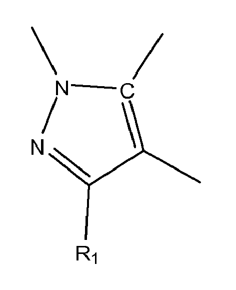 and when V represents a nitrogen atom, the ring is represented by the formula

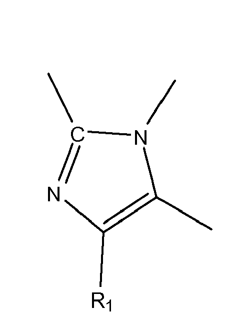

Typically, in the compound of formula (I) R₁ represents a hydrogen atom, a halogen atom, a hydroxyl group, a cyano group, a linear or branched C₁-C₆ alkyl group, a C₁-C₄ haloalkyl group, a C₁-C₄ hydroxyalkyl group, a C₃-C₁₀ cycloalkyl group or a -(CH₂)ₙNR'R" group; wherein n is 0 or 1, and R' and R" are the same or different and each represents a hydrogen atom, a linear or branched C₁-C₆ alkyl group, a C₁-C₄ haloalkyl group or C₁-C₄ hydroxyalkyl group;
R₂ and R₄ are the same or different and each represent a hydrogen atom, a halogen atom, a hydroxyl group, a cyano group, a linear or branched C₁-C₆ alkyl group, a C₁-C₄ haloalkyl group, a C₁-C₄ hydroxyalkyl group, or a C₃-C₁₀ cycloalkyl group;
R₃ represents a hydrogen atom, a halogen atom, a hydroxyl group, a cyano group, a linear or branched C₁-C₆ alkyl group, a C₁-C₄ haloalkyl group, a C₁-C₄ hydroxyalkyl group, a C₃-C₁₀ cycloalkyl group, a -(CH₂)_{q},C(O)-(CH₂)_{q}-R group or a -(CH₂)_{q,}C(O)-(CH₂)_{q}-NR'R" group, wherein each q and q' are independently 0, 1 or 2, R represents a hydrogen atom, or a linear or branched C₁-C₆ alkyl group, a C₁-C₄ haloalkyl group, a C₁-C₄ hydroxyalkyl group, or a cyano group and R' and R" are the same or different and each represents a hydrogen atom, a linear or branched C₁-C₆ alkyl group, a C₁-C₄ haloalkyl group, or C₁-C₄ hydroxyalkyl group;
R₅ represents a hydrogen atom, a halogen atom, a hydroxyl group, a cyano group, a linear or branched C₁-C₆ alkyl group, a C₁-C₄ haloalkyl group, a C₁-C₄ hydroxyalkyl group, a C₃-C₁₀ cycloalkyl group, a 5- to 10- membered heterocyclyl group, a C₆-C₁₀ aryl group, or a 5- to 10- membered heteroaryl group, wherein said heterocyclyl, aryl and heteroaryl groups are unsubstituted or substituted by one or more substituents selected from a halogen atom, a linear or branched C₁-C₆ alkyl group, a cyano group, a hydroxyl group or a C₁-C₄ alkoxy group;
For the avoidance of doubt, when more than one R₅ groups are present, they may be the same or different.
R₅, represents a hydrogen atom, a halogen atom, a hydroxyl group, a cyano group, a linear or branched C₁-C₆ alkyl group, a C₁-C₄ haloalkyl group, a C₁-C₄ hydroxyalkyl group or a C₃-C₇ cycloalkyl group;
R₆, R₇, R₉ and R₁₀ are the same or different and each represent a hydrogen atom, a C₁-C₄ haloalkyl group, a C₁-C₄ hydroxyalkyl group, a -(C₁-C₄ alkyl)-O-(C₁-C₄ alkyl) group or a linear or branched C₁-C₆ alkyl group;
R₈ and R₁₁ each independently represent a hydrogen atom, a linear or branched C₁-C₆ alkyl group, a C₁-C₄ haloalkyl group, a C₁-C₄ hydroxyalkyl group, a C₃-C₁₀ cycloalkyl group, a C₃-C₁₀ cycloalkenyl group, a C₆-C₁₀ aryl group, a 5- to 10- membered heteroaryl group containing 1, 2 or 3 heteroatoms selected from N, O and S, a 5- to 10-membered heterocyclyl group containing 1, 2 or 3 heteroatoms selected from N, O and S, -L-Het-R"', -L-NR'R", -L-A, -A-SO₂-R', -A-SO-R"', -A-A', -A-L'-C(O)NR'R", -A-(C₁-C₄ alkyl)--CN, -A-L'-NR'R", -A-L'-OR', -A-NR'R", -A-C(O)-Het'-L-CN, -A-C(O)-NR'R", -A-C(O)-(O)_{z}-A", -A-C(O)-(O)_{z}-R', -A-C(O)-(O)_{z}-L-A"', -A-C(O)-(O)_{z}R"', -A-C(O)-(O)_{z}-L-CN, -A-C(O)-L'-Het-R' group, or a -(CH₂)ₙ,C(OH)(J)-(CH₂)ₙK group, wherein z is 0 or 1, n and n' are independently 0 or 1, J represents a hydrogen atom or a methyl group, K represents a hydroxyl group, a methyl group or a -NR'R" group; and wherein R' and R" are the same or different and each represents a hydrogen atom or linear or a branched C₁-C₆ alkyl group, a C₁-C₄ haloalkyl group or a C₁-C₄ hydroxyalkyl group, and R'" represents a linear or branched C₁-C₆ alkyl group, a C₁-C₄ haloalkyl group or a C₁-C₄ hydroxyalkyl group; the heterocyclyl and heteroaryl groups being optionally fused to a phenyl group or to a pyridyl group; the cycloalkyl group being optionally fused to a 1,3-dioxolane group; and wherein the cycloalkyl, cycloalkenyl, heterocyclyl, aryl and heteroaryl groups being unsubstituted or substituted by one or more substituents selected from a halogen atom, a hydroxyl group, a cyano group, a linear or branched C₁-C₆ alkyl group, or a C₁-C₄ alkoxy group, and wherein
L is a linear or branched C₁-C₆ alkylene group, L' is a linear C₁-C₂ alkylene group;
Het represents O or NR^{IV}, and Het' represents NR^{IV}, wherein R^{IV} is a hydrogen atom, a linear or branched C₁-C₄ alkyl group, a C₁-C₄ haloalkyl group, or C₁-C₄ hydroxyalkyl group;
A, represents a C₃-C₁₀ cycloalkyl group, C₃-C₁₀ cycloalkenyl group, a 5- to 10-membered heterocyclyl group, a C₆-C₁₀ aryl group, or a 5- to 10- membered heteroaryl group, wherein the cycloalkyl, cycloalkenyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group;
A' represents a C₃-C₇ cycloalkyl group, C₃-C₇ cycloalkenyl group, a 5- to 10- membered heterocyclyl group, a C₆-C₁₀ aryl group, or a 5- to 10- membered heteroaryl group, wherein the cycloalkyl, cycloalkenyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group;
A" represents a C₃-C₇ cycloalkyl group, a phenyl group, a 5- to 6-membered monocyclic heteroaryl group containing 1, 2 or 3 heteroatoms selected from N, O and S, a 5- to 6- membered heterocyclyl group containing 1, 2 or 3 nitrogen atoms, wherein the cycloalkyl, phenyl, heteroaryl and heterocyclyl groups are unsubstituted or substituted by one or more substituents selected from a halogen atom, a hydroxyl group, a cyano group, a linear or branched C₁-C₆ alkyl group, or a C₁-C₄ alkoxy group;
A"' represents a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, wherein said phenyl group is unsubstituted or substituted by one or more halogen atoms, and wherein said heteroaryl and heterocyclyl groups are unsubstituted or substituted by one or more linear or branched C₁-C₃ alkyl groups.

In one embodiment, in the compound of formula (I), Z represents a nitrogen atom and V represents a carbon atom.

In other embodiment, in the compound of formula (I), Z represents a carbon atom and V represents a nitrogen atom.

Typically, in the compound of formula (I) R₁ represents a hydrogen atom, a halogen atom, a cyano group, a linear or branched C₁-C₆ alkyl group, a C₃-C₇ cycloalkyl group or a -(CH₂)ₙ-NR'R" group, wherein n is 0 or 1, and R' and R" are the same or different and each represents a hydrogen atom, a linear or branched C₁-C₆ alkyl group, a C₁-C₄ haloalkyl group or C₁-C₄ hydroxyalkyl group; preferably R₁ represents a hydrogen atom, a linear or branched C₁-C₃ alkyl group or a -NR'R" group, wherein R' and R" are the same or different and each represents a hydrogen atom or a linear or branched C₁-C₃ alkyl group; more preferably R₁ represents a hydrogen atom or a -NH₂ group; most preferably R₁ represents a hydrogen atom.

Typically, in the compound of formula (I) R₂ represents a hydrogen atom, a halogen atom, a cyano group, a linear or branched C₁-C₆ alkyl group or a C₃-C₇ cycloalkyl group; preferably R₂ represents a hydrogen atom or a halogen atom; more preferably R₂ represents a hydrogen atom.

Typically, in the compound of formula (I) R₃ represents a hydrogen atom, a halogen atom, a cyano group, a linear or branched C₁-C₆ alkyl group, a C₃-C₇ cycloalkyl group or a -(CH₂)_{q},C(O)-(CH₂)_{q}-NR'R" group, wherein q and q' are independently 0 or 1, and R' and R" are the same or different and each represents a hydrogen atom, a linear or branched C₁-C₆ alkyl group or a C₁-C₄ haloalkyl group; preferably R₃ represents a hydrogen atom, a halogen atom, a cyano group or a -C(O)-NH₂ group; more preferably R₃ represents a hydrogen atom, a halogen atom or a cyano group; most preferably R₃ represents a halogen atom or a cyano group.

Typically, in the compound of formula (I) R₄ represents a hydrogen atom, a halogen atom, a hydroxyl group, a cyano group, a linear or branched C₁-C₆ alkyl group or a C₃-C₇ cycloalkyl group; preferably R₄ represents a hydrogen atom, a halogen atom or a hydroxyl group; more preferably R₄ represents a hydrogen atom or a halogen atom; most preferably R₄ represents a hydrogen atom.

Typically, in the compound of formula (I) R₅ and R₅' each independently represent a hydrogen atom, a halogen atom, a hydroxyl group, a cyano group or a linear or branched C₁-C₆ alkyl group; preferably R₅ and R₅' independently represent a hydrogen atom, a halogen atom or a linear or branched C₁-C₃ alkyl group; more preferably R₅ and R₅' independently represent a hydrogen atom or a halogen atom; most preferably R₅ and R₅' independently represent a hydrogen atom.

Typically, in the compound of formula (I), not more than one of R₁, R₂, R₃, R₄ and R₅ represents a substituent selected from cycloalkyl, cycloalkenyl, aryl, heteroaryl, heterocyclyl, bicyclyl, aza-bicycloalkyl, aza-bicycloalkenyl, -(CH₂)_{q}SOR₁₅ group, a - (CH₂)qS(O)₂R₁₅ group, a -(CH₂)_{q}S(O)₂NR₁₅R₁₆ group, a -(CH₂)_{q}NR₁₅S(O)₂R₁₆ group, a - (CH₂)_{q}NR₁₅S(O)₂NR₁₆ group, a -(CH₂)_{q}OR₁₅ group, a -(CH₂)_{q}C(O)OR₁₅ group, a -(CH₂)_{q}O-C(O)R₁₅ group, a -(CH₂)_{q},C(O)-(CH₂)_{q}-R₁₅ group, a -(CH₂)_{q},C(O)-(CH₂)_{q}-NR₁₅R₁₆ group, a -(CH₂)_{q},NR₁₅C(O)-(CH₂)_{q}-R₁₆ group and a -(CH₂)_{q},NR₁₅C(O)-(CH₂)_{q}-NR₁₆R₁₇ group, wherein each q and q' are independently 0, 1 or 2.

Typically, when R₁, R₂, R₃, R₄ or R₅ represents a cycloalkyl, cycloalkenyl, aryl, heteroaryl, heterocyclyl, bicyclyl, aza-bicycloalkyl or aza-bicycloalkenyl group substituted by one or more substituents selected from substituents Ra, not more than 1, preferably none, of the Ra substituents are a substituent selected from a cycloalkyl group, a cycloalkenyl, an aryl group, a heteroaryl group, a heterocyclyl group, a - (CH₂)_{q}SOR₁₂ group, a -(CH₂)_{q}S(O)₂R₁₂ group, a -(CH₂)_{q}S(O)₂NR₁₂R₁₃ group, a -(CH₂)_{q}NR₁₂S(O)₂R₁₃ group, a -(CH₂)_{q}NR₁₂S(O)₂NR₁₃ group, a -(CH₂)_{q}C(O)OR₁₂ group, a -(CH₂)_{q}O-C(O)R₁₂ group, a -(CH₂)_{q},C(O)-(CH₂)_{q}-R₁₂, a -(CH₂)_{q},C(O)-(CH₂)_{q}-NR₁₂R₁₃ group, a -(CH₂)_{q},NR₁₂C(O)-(CH₂)_{q}-R₁₃ group and a -NR₁₂C(O)-(CH₂)_{q}-NR₁₃R₁₄ group, wherein each q and q' are independently 0, 1 or 2.

Typically, when R₁, R₂, R₃, R₄ or R₅ is substituted by a -(CH₂)_{q}SOR₁₂ group, a - (CH₂)_{q}S(O)₂R₁₂ group, a -(CH₂)_{q}S(O)₂NR₁₂R₁₃ group, a -(CH₂)_{q}NR₁₂S(O)₂R₁₃ group, a - (CH₂)_{q}NR₁₂S(O)₂NR₁₃ group, a -(CH₂)_{q}C(O)OR₁₂ group, a -(CH₂)_{q}O-C(O)R₁₂ group, a - (CH₂)_{q},C(O)-(CH₂)_{q}-R₁₂, a -(CH₂)_{q}^{,}C(O)-(CH₂)_{q}-NR₁₂R₁₃ group, a -(CH₂)_{q}NR₁₂C(O)-(CH₂)_{q}-R₁₃ group and a -NR₁₂C(O)-(CH₂)_{q}-NR₁₃R₁₄ group, wherein each q and q' are independently 0, 1 or 2, and R₁₂, R₁₃ and R₁₄ representys a cycloalkyl, phenyl, heteroaryl, heterocyclyl or bicyclyl group substituted by one or more substituents selected from substituents Rc, not more than one, preferably none, of the Rc substituents are a substituent selected from a cycloalkyl group, a phenyl group, a heteroaryl group, a heterocyclyl group and a heterocycloalkyl ketone group.

Typically, in the compound of formula (I) R₆ and R₇ each independently represent a hydrogen atom, a hydroxyl group, a C₁-C₄ hydroxyalkyl group, a -(C₁-C₄ alkyl)-O-(C₁-C₄ alkyl) group or a linear or branched C₁-C₄ alkyl group; preferably R₆ and R₇ independently represent a hydrogen atom, a hydroxyl group, a C₁-C₃ hydroxyalkyl group, a -(C₁-C₃ alkyl)-O-(C₁-C₃ alkyl) group or a linear or branched C₁-C₃ alkyl group; more preferably R₆ and R₇ independently represent a hydrogen atom, a C₁-C₂ hydroxyalkyl group, a -(C₁-C₂ alkyl)-O-(C₁-C₂ alkyl) group or a methyl group; most preferably R₆ and R₇ independently represent a hydrogen atom or a methyl group.

Typically, in the compound of formula (I) R₈ or R₁₁ independently represent a hydrogen atom, a linear or branched C₁-C₆ alkyl group, a C₁-C₄ haloalkyl group, a C₁-C₄ hydroxyalkyl group, a C₃-C₁₀ cycloalkyl group, a C₆-C₁₀ aryl group, a 5- to 10-membered heteroaryl group containing 1, 2 or 3 heteroatoms selected from N, O and S, a 5- to 10- membered heterocyclyl group containing 1, 2 or 3 heteroatoms selected from N, O and S, -L-Het-R"', -L-A, -A-SO₂-R', -A-A', -A-L-CN, -A-L'-NR'R", -A-L'-OR', -A-NR'R", -A-C(O)-NR'R", -A-C(O)-(O)_{z}-R', -A-C(O)-L'-Het-R' group, or a -(CH₂)ₙ,C(OH)(J)-(CH₂)ₙK group, wherein z is 0 or 1, n and n' are independently 0 or 1, J represents a hydrogen atom or a methyl group, K represents a hydroxyl group, a methyl group or a -NR'R" group; and wherein R' and R" are the same or different and each represents a hydrogen atom or linear or a branched C₁-C₃ alkyl group, a C₁-C₃ haloalkyl group or a C₁-C₃ hydroxyalkyl group, and R"' represents a linear or branched C₁-C₃ alkyl group, a C₁-C₃ haloalkyl group or a C₁-C₃ hydroxyalkyl group; the heterocyclyl and heteroaryl groups being optionally fused to a phenyl group or to a pyridyl group; the cycloalkyl group being optionally fused to a 1,3-dioxolane group; and wherein the cycloalkyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group.

Typically, L is a linear or branched C₁-C₆ alkylene group. Preferably, L is a linear or branched C₁-C₅ alkylene group; more preferably, L is a linear or branched C₁-C₃ alkylene group.

Typically, Het represents O or NR^{IV} and Het' represents NR^{IV}, wherein R^{IV} is a hydrogen atom or a linear or branched C₁-C₄ alkyl group, preferably a hydrogen atom or a straight or branched C₁-C₂ alkyl group. Preferably, Het represents O.

Typically, A represents a C₃-C₆ cycloalkyl, 5- to 6- membered heterocyclyl, phenyl, 5-to 6- membered heteroaryl group, the cycloalkyl, heterocyclyl, phenyl and heteroaryl groups being unsubstituted or substituted by 1, 2 or 3 halogen atoms, or hydroxyl, cyano, linear or branched C₁-C₂ alkyl, or C₁-C₂ alkoxy groups.

Typically, A is a 5- to 6-membered heterocyclyl group, phenyl or C₃-C₆ cycloalkyl group, said heterocyclyl, phenyl and cycloalkyl groups being unsubstituted or substituted by 1, 2 or 3, preferably 1 or 2, halogen atoms or hydroxyl or C₁-C₂ alkyl groups. Preferably, A is a piperidinyl, phenyl or cyclohexyl group, which piperidinyl, phenyl and cyclohexyl groups are unsubstituted or substituted by one halogen atom, or hydroxyl group or C₁-C₂ alkyl group.

Typically, A' is phenyl group or a 5- or 6-membered heteroaryl group, which phenyl and heteroaryl groups are unsubstituted or substituted by 1, 2 or 3 halogen atoms, or cyano, hydroxy or C₁-C₂ alkyl groups. Preferably, A' is a phenyl or pyridinyl group, which is unsubstituted or substituted by 1 or 2 halogen atoms or cyano groups.

Typically, A" is a 5- to 6-membered heterocyclyl group containing 1, 2 or 3 nitrogen atoms, C₃-C₆ cycloalkyl or 5- or 6-membered monocyclic heteroaryl group containing 1, 2 or 3 nitrogen atoms, which heterocyclyl, cycloalkyl and heteroaryl groups are unsubstituted or substituted by 1, 2 or 3, halogen atoms, or cyano, hydroxy or C₁-C₂ alkyl groups. Preferably, A" is a pyrrolidinyl, cyclopropyl or pyridinyl group, which pyrrolidinyl, cyclopropyl and pyridinyl groups are unsubstituted or substituted by 1 or 2 halogen atoms or cyano groups.

Typically, A'" is a 5- to 6-membered monocyclic heteroaryl group containing 1, 2 or 3 nitrogen atoms, which heteroaryl group is unsubstituted or substituted by 1, 2 or 3, preferably 1 or 2 halogen atoms or hydroxy or C₁-C₂ alkyl groups. Preferably, A"' is an imidazolyl group.

Preferably in the compounds of formula (I), R₈ or R₁₁ independently represent a hydrogen atom, a linear or branched C₁-C₆ alkyl group, a C₁-C₄ haloalkyl group, a C₁-C₄ hydroxyalkyl group, a C₃-C₁₀ cycloalkyl group, a C₆-C₁₀ aryl group, a 5- to 10-membered heteroaryl group containing 1, 2 or 3 heteroatoms selected from N, O and S, a 5- to 10- membered heterocyclyl group containing 1, 2 or 3 heteroatoms selected from N, O and S, -L-Het-R"', -L-A, -A-SO₂-R', -A-A', -A-L-CN, -A-L'-NR'R", -A-L'-OR', -A-NR'R", -A-C(O)-NR'R", -A-C(O)-(O)_{z}-R', -A-C(O)-L'-Het-R' group, or a -(CH₂)ₙ,C(OH)(J)-(CH₂)ₙK group, wherein z is 0 or 1, n and n' are independently 0 or 1, J represents a hydrogen atom or a methyl group, K represents a hydroxyl group, a methyl group or a -NR'R" group; and wherein R' and R" are the same or different and each represents a hydrogen atom or linear or a branched C₁-C₃ alkyl group, a C₁-C₃ haloalkyl group or a C₁-C₃ hydroxyalkyl group, and R"' represents a linear or branched C₁-C₃ alkyl group, a C₁-C₃ haloalkyl group or a C₁-C₃ hydroxyalkyl group; the heterocyclyl and heteroaryl groups being optionally fused to a phenyl group or to a pyridyl group; the cycloalkyl group being optionally fused to a 1,3-dioxolane group; and wherein the cycloalkyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group, and wherein
L is a linear or branched C₁-C₃ alkylene group, L' is a linear C₁-C₂ alkylene group,
Het represents O or NR^{IV}, wherein R^{IV} is a hydrogen atom, a linear or branched C₁-C₄ alkyl group, a C₁-C₄ haloalkyl group, or C₁-C₄ hydroxyalkyl group,
A represents a C₃-C₁₀ cycloalkyl group, a 5- to 10- membered heterocyclyl group, a C₆-C₁₀ aryl group, or a 5- to 10- membered heteroaryl group, the cycloalkyl, heterocyclyl, aryl and heteroaryl groups being unsubstituted or substituted by one or more substituents selected from a halogen atom, a hydroxyl group, a cyano group, a linear or branched C₁-C₆ alkyl group, or a
   C₁-C₄ alkoxy group;
A' represents a C₃-C₇ cycloalkyl group, a 5- to 10- membered heterocyclyl group, a C₆-C₁₀ aryl group, or a 5- to 10- membered heteroaryl group, the cycloalkyl, heterocyclyl, aryl and heteroaryl groups being unsubstituted or substituted by one or more substituents selected from a halogen atom, a hydroxyl group, a cyano group, a linear or branched C₁-C₆ alkyl group, or a C₁-C₄ alkoxy group.

Preferably, in the compound of formula (I) R₈ and R₁₁ each independently represent a hydrogen atom, a linear or branched C₁-C₆ alkyl group, a C₁-C₄ hydroxyalkyl group, a C₃-C₇ cycloalkyl group, a C₃-C₇ cycloalkenyl group, a phenyl group, a 5- to 6-membered monocyclic heteroaryl group containing 1, 2 or 3 heteroatoms selected from N, O and S, a 5- to 7- membered heterocyclyl group containing 1, 2 or 3 heteroatoms selected from N, O and S, a bicyclyl group containing a phenyl group or a pyridyl group fused to a 5- to 7- membered heterocyclyl group containing at least one heteroatom selected from O, S and N, a bicyclyl group containing a phenyl group or a pyridyl group fused to a C₅-C₇ cycloalkyl group, a bicyclyl group containing a C₅-C₇ cycloalkyl group fused to a 1,3 dioxolane group, a -S(O)R' group, a -S(O)₂R' group, a -(CH₂)ₙOR"' group, a -(CH₂)ₙC(O)OR' group, a -(CH₂)_{n'}C(O)-(CH₂)ₙ-R' group, a -(CH₂)ₙ,C(OH)(J)-(CH₂)ₙK group, a -(CH₂)ₙR"'NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₆ alkyl group, a C₁-C₄ haloalkyl group or a C₁-C₄ hydroxyalkyl group; and R'" represents a linear or branched C₁-C₆ alkyl group, a C₁-C₄ haloalkyl group or a C₁-C₄ hydroxyalkyl group; and wherein the cycloalkyl, cycloalkenyl, phenyl, heteroaryl, heterocyclyl and bicyclyl groups are unsubstituted or substituted by one or more substituents selected from a halogen atom, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 4- to 5- membered heterocyclyl group containing 1, 2 or 3 heteroatoms selected from N, O and S, a -S(O)R' group, a -S(O)₂R' group, a -(CH₂)ₙOR' group, a -(CH₂)ₙC(O)OR' group,
a -(CH₂)_{n'}C(O)-(CH₂)ₙ-R' group, a -(CH₂)ₙNR'R" group, a -(CH₂)ₙCH(R"')NR'R" group, a -(CH₂)_{n'}C(O)(CH₂)ₙNR'R" group or a -(CH₂)ₙCN group; wherein each n and n' are independently 0 or 1, and R' and R" each independently represent a hydrogen atom, a linear or branched C₁-C₆ alkyl group, a C₁-C₄ haloalkyl group or a C₁-C₄ hydroxyalkyl group, and R'" represents a linear or branched C₁-C₆ alkyl group, C₁-C₄ haloalkyl group or a C₁-C₄ hydroxyalkyl group.

More preferably, in the compound of formula (I) R₈ and R₁₁ each independently represent a hydrogen atom, a linear or branched C₁-C₆ alkyl group, a C₁-C₄ hydroxyalkyl group, a C₃-C₇ cycloalkyl group, a C₃-C₇ cycloalkenyl group, a phenyl group, a pyridyl group, a 5- to 7- membered heterocyclyl group containing 1, 2 or 3 heteroatoms selected from N, O and S, a bicyclyl group containing a phenyl group or a pyridyl group bounded directly to a 5- to 7- membered heterocyclyl group containing at least one heteroatom selected from O, S and N, a bicyclyl group containing a phenyl group or a pyridyl group fused to a C₅-C₇ cycloalkyl group, a bicyclyl group containing a C₅-C₇ cycloalkyl group fused to a 1,3 dioxolane group, a -(CH₂)ₙOR"' group, a -(CH₂)ₙC(O)OR' group, a -(CH₂)_{n'}C(O)-(CH₂)ₙ-R' group, a -(CH₂)ₙ,C(OH)(J)-(CH₂)ₙK group, a -(CH₂)ₙNR'R" group, a -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₆ alkyl group, a C₁-C₄ haloalkyl group or a C₁-C₄ hydroxyalkyl group, and R'" represents a linear or branched C₁-C₆ alkyl group, a C₁-C₄ haloalkyl group or a C₁-C₄ hydroxyalkyl group; and wherein the cycloalkyl, cycloalkenyl, phenyl, pyridyl, heterocyclyl and bicyclyl groups are unsubstituted or substituted by one or more substituents selected from a halogen atom, a linear or branched C₁-C₃ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 4- to 5- membered heterocyclyl group containing 1, 2 or 3 heteroatoms selected from N, O and S, a -S(O)R' group, a -S(O)₂R' group, a -(CH₂)ₙOR' group, a -C(O)OR' group, a -(CH₂)_{n'}C(O)-(CH₂)ₙ-R' group, a - (CH₂)ₙNR'R" group, a -(CH₂)_{n'}C(O)(CH₂)ₙNR'R" group or a -(CH₂)ₙCN group; wherein each n and n' are independently 0 or 1, and R' and R" each independently represent a hydrogen atom, a linear or branched C₁-C₆ alkyl group, a C₁-C₄ haloalkyl group or a C₁-C₄ hydroxyalkyl group.

Even more preferably, in the compound of formula (I) R₈ represents a linear or branched C₁-C₃ alkyl group, a C₁-C₄ hydroxyalkyl group, a C₃-C₇ cycloalkyl group, a C₃-C₇ cycloalkenyl group, a phenyl group, a pyridyl group, a pyrimidinyl group, a morpholinyl group, a piperidyl group, a tetrahydropyranyl group, a chromanyl group, a 3,4-dihydro-2H-pyrano[2,3-b]pyridyl group, a 1,2,3,4-tetrahydronaphthalenyl group, a 5,6,7,8-tetrahydroquinolinyl group, a 1,4-dioxaspiro[4.5]decanyl group, a -(CH₂)ₙOR"' group, a -(CH₂)ₙC(O)OR' group, a -(CH₂)_{n'}C(O)-(CH₂)ₙ-R' group,
a -(CH₂)_{n'}C(OH)(J)-(CH₂)ₙK group, a -(CH₂)ₙNR'R" group, -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₃ alkyl group or a C₁-C₄ haloalkyl group, and R'" represents a linear or branched C₁-C₃ alkyl group or a C₁-C₄ haloalkyl group; and wherein the cycloalkyl, cycloalkenyl, phenyl, pyridyl, pyrimidinyl, morpholinyl, piperidyl, tetrahydropyranyl, chromanyl and 3,4-dihydro-2H-pyrano[2,3-b]pyridyl 1,2,3,4-tetrahydronaphthalenyl, 5,6,7,8-tetrahydroquinolinyl and 1,4-dioxaspiro[4.5]decanyl groups are unsubstituted or substituted by one or more substituents selected from a halogen atom, a linear or branched C₁-C₃ alkyl group, a - S(O)₂R' group, a -(CH₂)ₙOR' group, a -(CH₂)ₙC(O)OR' group, a -(CH₂)_{n'}C(O)-(CH₂)ₙ-R' group, a -(CH₂)_{n'}C(O)(CH₂)ₙNR'R" group, a -(CH₂)ₙNR'R" group or a -(CH₂)ₙCN group; wherein each n and n' are independently 0 or 1, and R' and R" each independently represent a hydrogen atom, a linear or branched C₁-C₃ alkyl group or a C₁-C₄ haloalkyl group.

More preferably, when R₈ is an alkyl or hydroxyalkyl group, it is an unsubstituted alkyl or hydroxyalkyl group; when R₈ is a cycloalkyl, cycloalkenyl, phenyl or pyridyl group, it is unsubstituted or substituted by one or more substituents selected from a halogen atom, a linear or branched C₁-C₃ alkyl group, a -S(O)₂R' group, a -(CH₂)ₙOR' group, a -(CH₂)ₙC(O)OR' group, a -(CH₂)_{n'}C(O)-(CH₂)ₙ-R' group, a -(CH₂)_{n'}C(O)(CH₂)ₙNR'R" group, a -(CH₂)ₙNR'R" group or a -(CH₂)ₙCN group; wherein each n and n' are independently 0 or 1, and R' and R" each independently represent a hydrogen atom, a linear or branched C₁-C₃ alkyl group or a C₁-C₄ haloalkyl group.

When R₈ is a heterocyclyl group it is preferably a 5- or 6-membered heterocyclyl group containing one or two heteroatoms selected from N and O, more preferably containing one or two nitrogen atoms. Piperidinyl, morpholinyl and tetrahydropyranyl group are preferred. Substituents on a piperadinyl group may be present on any ring atom but are preferably present on the nitrogen atom.

Even more preferably, in the compound of formula (I) R₁₁ represents a hydrogen atom, a linear or branched C₁-C₃ alkyl group, a C₁-C₄ hydroxyalkyl group, a phenyl group, a morpholinyl group, a tetrahydropyranyl group, a -(CH₂)ₙOR"' group, a -(CH₂)_{n'}C(OH)(J)-(CH₂)ₙK group, a -(CH₂)ₙNR'R" group or a -(CH₂)ₙCH(R"')NR'R" group; wherein each n and n' are independently 0 or 1; and J represents a hydrogen atom or a methyl group; and K represents a hydroxyl group, a methyl group or a - NR'R" group; and R' and R" each independently represent a hydrogen atom, a linear or branched C₁-C₃ alkyl group or a C₁-C₄ haloalkyl group, and R'" represents a linear or branched C₁-C₃ alkyl group or a C₁-C₄ haloalkyl group; and wherein the phenyl, morpholinyl and tetrahydropyranyl groups are unsubstituted or substituted by one or more substituents selected from a halogen atom, a linear or branched C₁-C₃ alkyl group or a -(CH₂)ₙOR' group; wherein n is 0 or 1, and R' represents a hydrogen atom, a linear or branched C₁-C₃ alkyl group or a C₁-C₄ haloalkyl group.

More preferably R₁₁ is a hydrogen atom, a C₁-C₃ alkyl group or a C₁-C₄ hydroxyalkyl group.

Typically, when R₈ or R₁₁ represents a cycloalkyl, cycloalkenyl, aryl, heteroaryl, heterocyclyl, bicyclyl, aza-bicycloalkyl or aza-bicycloalkenyl group substituted by one or more substituents selected from Ra, not more than one, preferably none, of the Ra substituents represents a substituent selected from a cycloalkyl group, a cycloalkenyl, an aryl group, a heteroaryl group, a heterocyclyl group, a -(CH₂)_{q}SOR₁₂ group, a - (CH₂)_{q}S(O)₂R₁₂ group, a -(CH₂)_{q}S(O)₂NR₁₂R₁₃ group, a -(CH₂)_{q}NR₁₂S(O)₂R₁₃ group, a - (CH₂)_{q}NR₁₂S(O)₂NR₁₃ group, a -(CH₂)_{q}C(O)OR₁₂ group, a -(CH₂)_{q}O-C(O)R₁₂ group, a - (CH₂)_{q},C(O)-(CH₂)_{q}-R₁₂, a -(CH₂)_{q},C(O)-(CH₂)_{q}-NR₁₂R₁₃ group, a -(CH₂)_{q}NR₁₂C(O)-(CH₂)_{q}-R₁₃ group and a -NR₁₂C(O)-(CH₂)_{q}-NR₁₃R₁₄ group, wherein each q and q' are independently 0, 1 or 2.

Typically, when R₈ or R₁₁ is substituted by a -(CH₂)_{q}SOR₁₂ group, a -(CH₂)_{q}S(O)₂R₁₂ group, a -(CH₂)_{q}S(O)₂NR₁₂R₁₃ group, a -(CH₂)_{q}NR₁₂S(O)₂R₁₃ group, a -(CH₂)_{q}NR₁₂S(O)₂NR₁₃ group, a -(CH₂)_{q}C(O)OR₁₂ group, a -(CH₂)_{q}O-C(O)R₁₂ group, a -(CH₂)_{q},C(O)-(CH₂)_{q}-R_{12.} a -(CH₂)_{q},C(O)-(CH₂)_{q}-NR₁₂R₁₃ group, a -(CH₂)_{q}NR₁₂C(O)-(CH₂)_{q}-R₁₃ group and a -NR₁₂C(O)-(CH₂)_{q}-NR₁₃R₁₄ group, wherein each q and q' are independently 0, 1 or 2, and R₁₂, R₁₃ and R₁₄ representys a cycloalkyl, phenyl, heteroaryl, heterocyclyl or bicyclyl group substituted by one or more substituents selected from substituents Rc, not more than one, preferably none, of the Rc substituents is a substituent selected from a cycloalkyl group, a phenyl group, a heteroaryl group, a heterocyclyl group and a heterocycloalkyl ketone group.

For the avoidance of doubt, when more than one Ra groups are present, they may be the same or different.

Typically, in the compound of formula (I) R₉ and R₁₀ each independently represent a hydrogen atom, a hydroxyl group, a C₁-C₄ hydroxyalkyl group, a -(C₁-C₄ alkyl)-O-(C₁-C₄ alkyl) group or a linear or branched C₁-C₄ alkyl group; preferably R₉ and R₁₀ independently represent a hydrogen atom, a hydroxyl group, a C₁-C₃ hydroxyalkyl group, a -(C₁-C₃ alkyl)-O-(C₁-C₃ alkyl) group or a linear or branched C₁-C₃ alkyl group; more preferably R₉ and R₁₀ independently represent a hydrogen atom, a C₁-C₂ hydroxyalkyl group, a -(C₁-C₂ alkyl)-O-(C₁-C₂ alkyl) group or a methyl group; most preferably R₉ and R₁₀ independently represent a hydrogen atom or a methyl group.

Typically, in the compounds of formula (I), m is 0, 1 or 2, preferably 0 or 1.

Typically, in the compounds of formula (I), p is 0, 1 or 2, preferably 0 or 1.

In a particularly preferred embodiment, the compounds of formula (I) are those 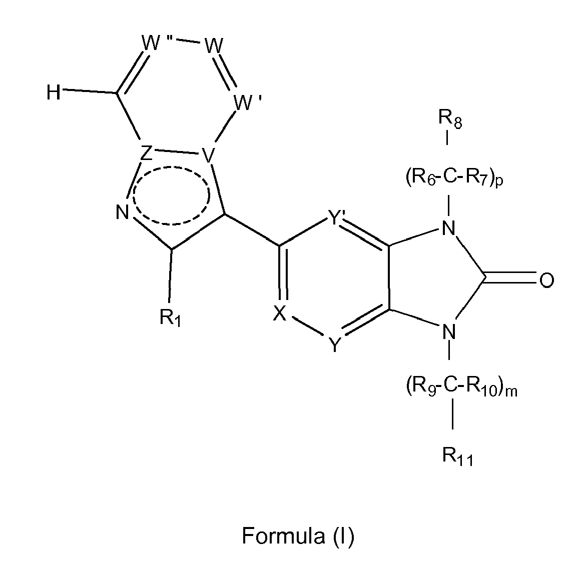 wherein
m is 0, 1 or 2;
p is 0, 1 or 2;
one of Z and V represents a nitrogen atom, and the other represents a carbon atom;
W represents a nitrogen atom or a -CR₃ group;
W' represents a nitrogen atom or a -CR₂ group;
W" represents a nitrogen atom or a -CR₄ group;
X and Y independently represent a nitrogen atom or a -CR₅ group, wherein at least one of X and Y represents a -CR₅ group;
Y' represents a nitrogen atom or a -CR₅, group;
R₁ represents a hydrogen atom or a -NH₂ group;
R₂ represents a hydrogen atom or a halogen atom;
R₃ represents a hydrogen atom, a halogen atom, a cyano group or a -C(O)-NH₂ group;
R₄ represents a hydrogen atom or a halogen atom;
R₅ represents a hydrogen atom or a halogen atom;
R₅, represents a hydrogen atom;
R₆ and R₇ independently represent a hydrogen atom, a methyl group, a C₁-C₂ hydroxyalkyl group or a -(C₁-C₂ alkyl)-O-(C₁-C₂ alkyl) group;
R₉ and R₁₀ independently represent a hydrogen atom, a methyl group, a C₁-C₂ hydroxyalkyl group or a -(C₁-C₂ alkyl)-O-(C₁-C₂ alkyl) group;
R₈ and R₁₁ independently represent a hydrogen atom, a linear or branched C₁-C₃ alkyl group, a C₁-C₄ hydroxyalkyl group, a C₃-C₇ cycloalkyl group, a C₃-C₇ cycloalkenyl group, a phenyl group, a pyridyl group, a pyrimidinyl group, a morpholinyl group, a piperidyl group, a tetrahydropyranyl group, a chromanyl group, a 3,4-dihydro-2H-pyrano[2,3-b]pyridyl group, a 1,2,3,4-tetrahydronaphthalenyl group, a 5,6,7,8-tetrahydroquinolinyl group, a 1,4-dioxaspiro[4.5]decanyl group, a -(CH₂)ₙOR"' group, a -(CH₂)ₙC(O)OR' group, a -(CH₂)_{n'}C(O)-(CH₂)ₙ-R' group, a -(CH₂)_{n'}C(OH)(J)-(CH₂)ₙK group, a -(CH₂)ₙNR'R" group, a -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₄ alkyl group or a C₁-C₄ haloalkyl group, and R'" represents a linear or branched C₁-C₃ alkyl group or a C₁-C₄ haloalkyl group; and wherein the cycloalkyl, cycloalkenyl, phenyl, pyridyl, pyrimidinyl, morpholinyl, piperidyl, tetrahydropyranyl, chromanyl, 3,4-dihydro-2H-pyrano[2,3-b]pyridyl 1,2,3,4-tetrahydronaphthalenyl, 5,6,7,8-tetrahydroquinolinyl and 1,4-dioxaspiro[4.5]decanyl groups are unsubstituted or substituted by one or two substituents selected from a halogen atom, a linear or branched C₁-C₃ alkyl group, a linear or branched C₁-C₃ haloalkyl group, an oxo group, a -S(O)₂R' group, a -(CH₂)ₙOR' group, a -(CH₂)ₙC(O)OR' group, a -(CH₂)_{n'}C(O)-(CH₂)ₙ-R' group, a -(CH₂)_{n'}C(O)(CH₂)ₙNR'R" group, a -(CH₂)ₙNR'R" group or a -(CH₂)ₙCN group; wherein each n and n' are independently 0 or 1, and R' and R" each independently represent a hydrogen atom, a linear or branched C₁-C₄ alkyl group or a C₁-C₄ haloalkyl group.

In a further particularly preferred embodiment, the compound of the invention is of formula (I-a) 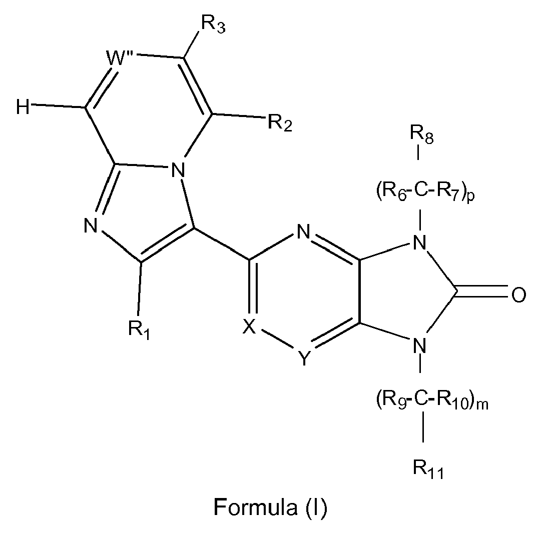 wherein
m is 0, 1 or 2;
p is 0, 1 or 2;
W" represents a nitrogen atom or a -CR₄ group; preferably W" represents a -CR₄ group;
X and Y independently represent a nitrogen atom or a -CR₅ group, wherein at least one of X and Y represents a -CR₅ group; preferably X represents a nitrogen atom or a -CR₅ group and Y represents a -CR₅ group;
R₁ represents a hydrogen atom or a -NH₂ group;
R₂ represents a hydrogen atom or a halogen atom;
R₃ represents a hydrogen atom, a halogen atom, a cyano group or a -C(O)-NH₂ group;
R₄ represents a hydrogen atom;
R₅ represents a hydrogen atom or a halogen atom; preferably R₅ represents a hydrogen atom;
R₆ and R₇ independently represent a hydrogen atom, a methyl group, a C₁-C₂ hydroxyalkyl group or a -(C₁-C₂ alkyl)-O-(C₁-C₂ alkyl) group; preferably R₆ and R₇ independently represent a hydrogen atom or a methyl group;
R₉ and R₁₀ independently represent a hydrogen atom, a methyl group, a C₁-C₂ hydroxyalkyl group or a -(C₁-C₂ alkyl)-O-(C₁-C₂ alkyl) group; preferably R₉ and R₁₀ independently represent a hydrogen atom or a methyl group;
R₈ represents a linear or branched C₁-C₃ alkyl group, a C₁-C₄ hydroxyalkyl group, a C₃-C₇ cycloalkyl group, a C₃-C₇ cycloalkenyl group, a phenyl group, a pyridyl group, a pyrimidinyl group, a morpholinyl group, a piperidyl group, a tetrahydropyranyl group, a chromanyl group, a 3,4-dihydro-2H-pyrano[2,3-b]pyridyl group, a 1,2,3,4-tetrahydronaphthalenyl group, a 5,6,7,8-tetrahydroquinolinyl group, a 1,4-dioxaspiro[4.5]decanyl group, a -(CH₂)ₙOR"' group, a -(CH₂)ₙC(O)OR' group, a -(CH₂)ₙ,C(O)-(CH₂)ₙ-R' group, a -(CH₂)ₙ,C(OH)(J)-(CH₂)ₙK group, a -(CH₂)ₙNR'R" group, a -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₃ alkyl group or a C₁-C₄ haloalkyl group, and R'" represents a linear or branched C₁-C₃ alkyl group or a C₁-C₄ haloalkyl group; and wherein the cycloalkyl, cycloalkenyl, phenyl, pyridyl, pyrimidinyl, morpholinyl, piperidyl, tetrahydropyranyl, chromanyl and 3,4-dihydro-2H-pyrano[2,3-b]pyridyl 1,2,3,4-tetrahydronaphthalenyl, 5,6,7,8-tetrahydroquinolinyl and 1,4-dioxaspiro[4.5]decanyl groups are unsubstituted or substituted by one or more substituents selected from a halogen atom, a linear or branched C₁-C₃ alkyl group, a -S(O)₂R' group, a -(CH₂)ₙOR' group, a -(CH₂)ₙC(O)OR' group, a -(CH₂)_{n'}C(O)-(CH₂)ₙ-R' group, a -(CH₂)_{n'}C(O)(CH₂)ₙNR'R" group, a -(CH₂)ₙNR'R" group or a -(CH₂)ₙCN group; wherein each n and n' are independently 0 or 1, and R' and R" each independently represent a hydrogen atom, a linear or branched C₁-C₃ alkyl group or a C₁-C₄ haloalkyl group.
R₁₁ represents a hydrogen atom, a linear or branched C₁-C₃ alkyl group, a C₁-C₄ hydroxyalkyl group, a phenyl group, a morpholinyl group, a tetrahydropyranyl group, a -(CH₂)ₙOR"' group, a -(CH₂)_{n'}C(OH)(J)-(CH₂)ₙK group, a -(CH₂)ₙNR'R" group or a -(CH₂)ₙCH(R"')NR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₃ alkyl group or a C₁-C₄ haloalkyl group, and R'" represents a linear or branched C₁-C₃ alkyl group or a C₁-C₄ haloalkyl group; and wherein the phenyl, morpholinyl and tetrahydropyranyl groups are unsubstituted or substituted by one or more substituents selected from a halogen atom, a linear or branched C₁-C₃ alkyl group or a -(CH₂)ₙOR' group; wherein n is 0 or 1, and R' represents a hydrogen atom, a linear or branched C₁-C₃ alkyl group or a C₁-C₄ haloalkyl group.

In an alternative particularly preferred embodiment, the compound of the invention is of formula (I-b): 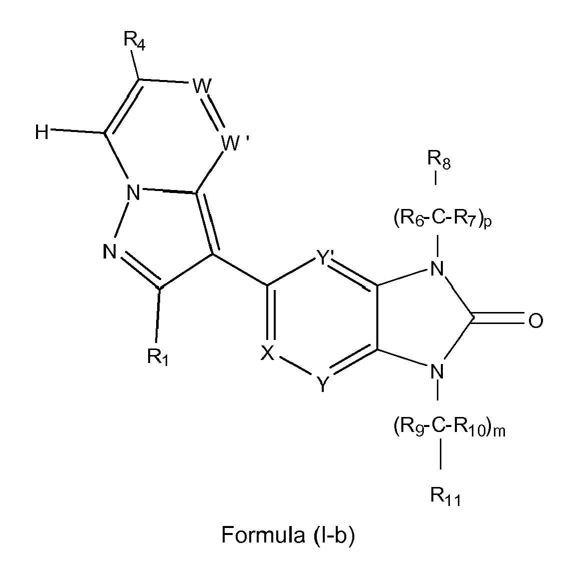 wherein
m is 0;
p is 0 or 1;
W represents a nitrogen atom or a -CR₃ group;
W' represents a nitrogen atom or a -CR₂ group; preferably W' represents a -CR₂ group;
X represents a nitrogen atom or a -CR₅ group;
Y represents a -CR₅ group;
Y' represents a nitrogen atom or a -CR₅, group;
R₁ represents a hydrogen atom;
R₂ represents a hydrogen atom or a halogen atom;
R₃ represents a hydrogen atom;
R₄ represents a hydrogen atom or a halogen atom;
R₅ represents a hydrogen atom or a halogen atom;
R₅, represents a hydrogen atom;
R₆ and R₇ independently represent a hydrogen atom or a methyl group;
R₈ represents a cyclohexyl group, a phenyl group, a pyridyl group, a chromanyl group, a tetrahydropyranyl group, wherein the cyclohexyl, phenyl, pyridyl, chromanyl and tetrahydropyranyl groups are unsubstituted or substituted by one or more substituents selected from a halogen atom, a linear or branched C₁-C₃ alkyl group or a -(CH₂)ₙCN group, wherein n is 0 or 1;
R₁₁ represents a hydrogen atom or a C₁-C₄ hydroxyalkyl group.

Particular individual compounds of the invention include:
3-[8-Oxo-9-(tetrahydro-2*H-*pyran-4-yl)-8,9-dihydro-7*H-*purin-2-yl]imidazo[1,2-*a*]pyridine-6-carbonitrile;
3-(9-Cyclohexyl-8-oxo-8,9-dihydro-7*H*-purin-2-yl)imidazo[1,2-*a*]pyridine-6-carbonitrile;
3-[3-(2-Methylcyclohexyl)-2-oxo-2,3-dihydro-1*H*-imidazo[4,5-*b*]pyridin-5-yl]imidazo[1,2-*a*]pyridine-6-carbonitrile;
3-{9-[(4*R*)-8-Fluoro-3,4-dihydro-2*H*-chromen-4-yl]-8-oxo-8,9-dihydro-7*H*-purin-2-yl}imidazo[1,2-*a*]pyridine-6-carbonitrile;
3-[7-Methyl-8-oxo-9-(tetrahydro-2*H*-pyran-4-yl)-8,9-dihydro-7*H*-purin-2-yl]imidazo[1,2-*a*]pyridine-6-carbonitrile;
3-(9-Benzyl-7-methyl-8-oxo-8,9-dihydro-7*H*-purin-2-yl)imidazo[1,2-*a*]pyridine-6-carbonitrile;
3-[7-(2-Morpholin-4-ylethyl)-8-oxo-9-(tetrahydro-2H-pyran-4-yl)-8,9-dihydro-7H-purin-2-yl]imidazo[1,2-a]pyridine-6-carbonitrile;
3-[7-[2-(Dimethylamino)ethyl]-8-oxo-9-(tetrahydro-2*H*-pyran-4-yl)-8,9-dihydro-7*H*-purin-2-yl]imidazo[1,2-*a*]pyridine-6-carbonitrile;
3-[7-(2-Hydroxyethyl)-8-oxo-9-(tetrahydro-2*H*-pyran-4-yl)-8,9-dihydro-7*H*-purin-2-yl]imidazo[1,2-*a*]pyridine-6-carbonitrile;
3-[7-(2-Hydroxy-2-methylpropyl)-8-oxo-9-(tetrahydro-2*H*-pyran-4-yl )-8, 9-dihydro-7*H-*purin-2-yl]imidazo[1,2-*a*]pyridine-6-carbonitrile;
3-[7-[(2*R*)-2,3-Dihydroxypropyl]-8-oxo-9-(tetrahydro-2*H*-pyran-4-yl)-8,9-dihydro-7*H-*purin-2-yl]imidazo[1,2-*a*]pyridine-6-carbonitrile;
3-[7-[(2*S*)-3-(Dimethylamino)-2-hydroxypropyl]-8-oxo-9-(tetrahydro-2*H*-pyran-4-yl)-8,9-dihydro-7*H*-purin-2-yl]imidazo[1,2-*a*]pyridine-6-carbonitrile;
3-[7-(2-Methoxyethyl)-8-oxo-9-(tetrahydro-2H-pyran-4-yl)-8,9-dihydro-7*H*-purin-2-yl]imidazo[1,2-*a*]pyridine-6-carbonitrile;
3-[7-(2-Aminoethyl)-8-oxo-9-(tetrahydro-2*H*-pyran-4-yl)-8,9-dihydro-7*H*-purin-2-yl]imidazo[1,2-*a*]pyridine-6-carbonitrile;
3-(8-Oxo-9-(tetrahydro-2*H*-pyran-4-yl)-7-f2-[(2,2,2-trifluoroethyl)amino]ethyl}-8,9-dihydro-7*H*-purin-2-yl)imidazo[1,2-*a*]pyridine-6-carbonitrile;
2-(2-(6-Cyanoimidazo[1,2-a]pyridin-3-yl)-8-oxo-9-(tetrahydro-2H-pyran-4-yl)-8,9-dihydro-7H-purin-7-yl)acetic acid;
3-[7-(2,4-Dimethoxybenzyl)-8-oxo-9-(tetrahydro-2*H*-pyran-4-yl)-8,9-dihydro-7*H*-purin-2-yl]imidazo[1,2-*a*]pyridine-6-carbonitrile;
2-(6-Fluoroimidazo[1,2-*a*]pyridin-3-yl)-9-(tetrahydro-2*H*-pyran-4-yl)-7,9-dihydro-8*H-*purin-8-one;
3-[8-Oxo-9-(tetrahydro-2*H*-pyran-4-yl)-8,9-dihydro-7*H*-purin-2-yl]imidazo[1,2-*a*]pyridine-6-carbonitrile;
9-Cyclohexyl-2-(6-fluoroimidazo[1,2-a]pyridin-3-yl)-7H-purin-8(9H)-one;
9-[(4*R*)-8-Fluoro-3,4-dihydro-2*H*-chromen-4-yl]-2-(6-fluoroimidazo[1,2-a]pyridin-3-yl)-7,9-dihydro-8*H*-purin-8-one;
2-(6-Fluoroimidazo[1,2-*a*]pyridin-3-yl)-9-[(1*S*)-1-phenylethyl]-7,9-dihydro-8*H*-purin-8-one;
2-(6-Fluoroimidazo[1,2-*a*]pyridin-3-yl)-9-[(1*R*)-1-phenylethyl]-7,9-dihydro-8*H*-purin-8-one;
2-(6-Fluoroimidazo[1,2-a]pyridin-3-yl)-9-(pyridin-3-ylmethyl)-7,9-dihydro-8H-purin-8-one;
2-(6-Fluoroimidazo[1,2-a]pyridin-3-yl)-9-(5,6,7,8-tetrahydroquinolin-5-yl)-7H-purin-8(9H)-one;
2-(6-Fluoroimidazo[1,2-a]pyridin-3-yl)-9-(pyridin-2-ylmethyl)-7H-purin-8(9H)-one;
2-(6-Fluoroimidazo[1,2-a]pyridin-3-yl)-9-((5-fluoropyridin-2-yl)methyl)-7H-purin-8(9H)-one;
(R)-2-(6-Fluoroimidazo[1,2-a]pyridin-3-yl)-9-(1-(5-fluoropyridin-2-yl)ethyl)-7H-purin-8(9H)-one;
(R)-2-(6-Fluoroimidazo[1,2-a]pyridin-3-yl)-9-(1-(5-fluoropyridin-2-yl)ethyl)-7-(2-hydroxyethyl)-7H-purin-8(9H)-one;
2-(6-Fluoroimidazo[1,2-a]pyridin-3-yl)-9-(1-(5-fluoropyridin-2-yl)-2-methoxyethyl)-7H-purin-8(9H)-one;
(R)-2-(6-Fluoroimidazo[1,2-a]pyridin-3-yl)-9-(1-(pyridin-2-yl)ethyl)-7H-purin-8(9H)-one;
(R)-2-(6-Fluoroimidazo[1,2-a]pyridin-3-yl)-9-(1-(5-fluoropyrimidin-2-yl)ethyl)-7H-purin-8(9H)-one;
(S)-2-(6-Fluoroimidazo[1,2-a]pyridin-3-yl)-9-(1-(5-fluoropyridin-2-yl)ethyl)-7H-purin-8(9H)-one;
(S)-2-(6-Fluoroimidazo[1,2-a]pyridin-3-yl)-9-(1-(5-fluoropyridin-2-yl)-2-hydroxyethyl)-7H-purin-8(9H)-one;
1-(2-(6-Fluoroimidazo[1,2-a]pyridin-3-yl)-8-oxo-7H-purin-9(8H)-yl)cyclohexanecarbonitrile;
(1 s,4s)-Ethyl 4-(2-(6-fluoroimidazo[1,2-a]pyridin-3-yl)-8-oxo-7H-purin-9(8H)-yl)cyclohexanecarboxylate;
(1 r,4r)-4-(2-(6-Fluoroimidazo[1,2-a]pyridin-3-yl)-8-oxo-7H-purin-9(8H)-yl)cyclohexanecarboxylic acid;
(1 r,4r)-4-(2-(6-Fluoroimidazo[1,2-a]pyridin-3-yl)-8-oxo-7H-purin-9(8H)-yl)-N-methylcyclohexanecarboxamide;
9-(2,2-Dimethyltetrahydro-2*H*-pyran-4-yl)-2-(6-fluoroimidazo[1,2-a]pyridin-3-yl)-7,9-dihydro-8H-purin-8-one;
9-(2,2-Dimethyl-3,4-dihydro-2H-pyrano[2,3-b]pyridin-4-yl)-2-(6-fluoroimidazo[1,2-a]pyridin-3-yl)-7H-purin-8(9H)-one;
2-(6-Fluoroimidazo[1,2-a]pyridin-3-yl)-9-(1,4-dioxaspiro[4.5]decan-8-yl)-7H-purin-8(9H)-one;
2-(6-Fluoroimidazo[1,2-a]pyridin-3-yl)-9-(4-oxocyclohexyl)-7H-purin-8(9H)-one;
2-((r,4r)-4-(2-(6-Fluoroimidazo[1,2-a]pyridin-3-yl)-8-oxo-7H-purin-9(8H)-yl)cyclohexyl)acetonitrile;
2-((r,4r)-4-(2-(6-Fluoroimidazo[1,2-a]pyridin-3-yl)-7-(2-hydroxyethyl)-8-oxo-7H-purin-9(8H)-yl)cyclohexyl)acetonitrile;
2-(6-Fluoroimidazo[1,2-a]pyridin-3-yl)-9-((1r,4r)-4-(hydroxymethyl)cyclohexyl)-7H-purin-8(9H)-one;
2-(6-Fluoroimidazo[1,2-a]pyridin-3-yl)-9-((1r,4r)-4-hydroxycyclohexyl)-7H-purin-8(9H)-one;
2-(6-Fluoroimidazo[1,2-a]pyridin-3-yl)-9-((1R,4R)-4-hydroxy-1,2,3,4-tetrahydronaphthalen-1-yl)-7H-purin-8(9H)-one;
2-(6-Fluoroimidazo[1,2-a]pyridin-3-yl)-9-((1r,4r)-4-hydroxy-4-methylcyclohexyl)-7H-purin-8(9H)-one;
9-((1r,4r)-4-(Aminomethyl)cyclohexyl)-2-(6-fluoroimidazo[1,2-a]pyridin-3-yl)-7H-purin-8(9H)-one;
9-((1r,4r)-4-Aminocyclohexyl)-2-(6-fluoroimidazo[1,2-a]pyridin-3-yl)-7H-purin-8(9H)-one;
9-Cyclobutyl-2-(6-fluoroimidazo[1,2-a]pyridin-3-yl)-7H-purin-8(9H)-one;
(R)-2-(6-Fluoroimidazo[1,2-a]pyridin-3-yl)-9-(3-methylbutan-2-yl)-7H-purin-8(9H)-one;
2-(6-Fluoroimidazo[1,2-a]pyridin-3-yl)-9-(1-methoxypropan-2-yl)-7H-purin-8(9H)-one;
(R)-tert-Butyl 3-(2-(6-fluoroimidazo[1,2-a]pyridin-3-yl)-8-oxo-7H-purin-9(8H)-yl)butanoate;
(R)-3-(2-(6-Fluoroimidazo[1,2-a]pyridin-3-yl)-8-oxo-7H-purin-9(8H)-yl)butanoic acid;
(R)-3-(2-(6-Fluoroimidazo[1,2-a]pyridin-3-yl)-8-oxo-7H-purin-9(8H)-yl)butanamide;
9-(1-(2,2-Difluoroethyl)piperidin-4-yl)-2-(6-fluoroimidazo[1,2-a]pyridin-3-yl)-7H-purin-8(9H)-one;
9-(4,4-Difluorocyclohexyl)-2-(6-fluoroimidazo[1,2-a]pyridin-3-yl)-7,9-dihydro-8H-purin-8-one;
2-(6-Fluoroimidazo[1,2-a]pyridin-3-yl)-9-[(3R)-piperidin-3-yl]-7,9-dihydro-8H-purin-8-one;
2-(6-Fluoroimidazo[1,2-a]pyridin-3-yl)-9-[(3R)-1-(methylsulfonyl)piperidin-3-yl]-7,9-dihydro-8H-purin-8-one;
3-[2-Oxo-3-(tetrahydro-2*H*-pyran-4-yl)-2,3-dihydro-1*H*-imidazo[4,5-b]pyridin-5-yl]imidazo[1,2-*a*]pyridine-6-carbonitrile;
3-[1-Methyl-2-oxo-3-(tetrahydro-2*H*-pyran-4-yl)-2,3-dihydro-1*H*-imidazo[4,5-b]pyridin-5-yl]imidazo[1,2-*a*]pyridine-6-carbonitrile;
5-(6-Fluoroimidazo[1,2-*a*]pyridin-3-yl)-3-(tetrahydro-2*H*-pyran-4-yl)-1,3-dihydro-2*H-*imidazo[4,5-*b*]pyridin-2-one;
5-(6-Fluoroimidazo[1,2-*a*]pyridin-3-yl)-1-methyl-3-(tetrahydro-2*H*-pyran-4-yl)-1,3-dihydro-2*H*-imidazo[4,5-*b*]pyridin-2-one;
5-(6-Fluoroimidazo[1,2-*a*]pyridin-3-yl)-1-(2-hydroxyethyl)-3-(tetrahydro-2*H*-pyran-4-yl)-1,3-dihydro-2*H*-imidazo[4,5-*b*]pyridin-2-one;
*tert*-Butyl 4-[5-(6-fluoroimidazo[1,2-a]pyridin-3-yl)-2-oxo-1,2-dihydro-3*H*-imidazo[4,5-*b*]pyridin-3-yl]piperidine-1-carboxylate;
5-(6-Fluoroimidazo[1,2-a]pyridin-3-yl)-3-piperidin-4-yl-1,3-dihydro-2H-imidazo[4,5-b]pyridin-2-one;
5-(6-Fluoroimidazo[1,2-*a*]pyridin-3-yl)-3-[1-(methylsulfonyl)piperidin-4-yl]-1,3-dihydro-2*H*-imidazo[4,5-b]pyridin-2-one;
3-(1-Acetylpiperidin-4-yl)-5-(6-fluoroimidazo[1,2-*a*]pyridin-3-yl)-1,3-dihydro-2*H-*imidazo[4,5-*b*]pyridin-2-one;
6-Fluoro-5-(6-fluoroimidazo[1,2-a]pyridin-3-yl)-3-(tetrahydro-2H-pyran-4-yl)-1,3-dihydro-2H-imidazo[4,5-b]pyridin-2-one;
6-(6-Fluoroimidazo[1,2-a]pyridin-3-yl)-1-(tetrahydro-2*H*-pyran-4-yl)-1,3-dihydro-2*H-*imidazo[4,5-*b*]pyrazin-2-one;
2-(2-Amino-6-fluoroimidazo[1,2-a]pyridin-3-yl)-9-(tetrahydro-2H-pyran-4-yl)-7H-purin-8(9H)-one;
2-(Imidazo[1,2-a]pyrazin-3-yl)-9-(tetrahydro-2H-pyran-4-yl)-7H-purin-8(9H)-one;
2-Pyrazolo[1,5-*a*]pyrazin-3-yl-9-(tetrahydro-2*H*-pyran-4-yl)-7,9-dihydro-8*H*-purin-8-one;
9-[(1*R*)-1-Phenylethyl]-2-pyrazolo[1,5-*a*]pyrazin-3-yl-7,9-dihydro-8*H*-purin-8-one;
(*R*)-9-(1-(5-Fluoropyridin-2-yl)ethyl)-2-(pyrazolo[1,5-a]pyrazin-3-yl)-7H-purin-8(9H)-one;
2-(Pyrazolo[1,5-a]pyrazin-3-yl)-9-((tetrahydro-2H-pyran-4-yl)methyl)-7H-purin-8(9H)-one;
9-(4,4-Difluorocyclohexyl)-2-(pyrazolo[1,5-a]pyrazin-3-yl)-7H-purin-8(9H)-one;
9-(2,2-Dimethyltetrahydro-2H-pyran-4-yl)-2-(pyrazolo[1,5-a]pyrazin-3-yl)-7H-purin-8(9H)-one;
(R)-9-(8-Fluorochroman-4-yl)-2-(pyrazolo[1,5-a]pyrazin-3-yl)-7H-purin-8(9H)-one;
2-((1r,4r)-4-(8-Oxo-2-(pyrazolo[1,5-a]pyrazin-3-yl)-7H-purin-9(8H)-yl)cyclohexyl)acetonitrile;
2-((1r,4r)-4-(7-(2-Hydroxyethyl)-8-oxo-2-(pyrazolo[1,5-a]pyrazin-3-yl)-7H-purin-9(8H)-yl)cyclohexyl)acetonitrile;
5-(Pyrazolo[1,5-a]pyrazin-3-yl)-3-(tetrahydro-2H-pyran-4-yl)-1H-imidazo[4,5-b]pyridin-2(3H)-one;
6-Fluoro-5-pyrazolo[1,5-a]pyrazin-3-yl-3-(tetrahydro-2H-pyran-4-yl)-1,3-dihydro-2H-imidazo[4,5-b]pyridin-2-one;
2-(Pyrazolo[1,5-a]pyridin-3-yl)-9-(tetrahydro-2H-pyran-4-yl)-7H-purin-8(9H)-one;
2-(6-Fluoropyrazolo[1,5-a]pyridin-3-yl)-9-(tetrahydro-2H-pyran-4-yl)-7H-purin-8(9H)-one;
2-(4-Fluoropyrazolo[1,5-a]pyridin-3-yl)-9-(tetrahydro-2H-pyran-4-yl)-7H-purin-8(9H)-one;
2-(Pyrazolo[1,5-a]pyrimidin-3-yl)-9-(tetrahydro-2H-pyran-4-yl)-7H-purin-8(9H)-one;
6-(Pyrazolo[1,5-a]pyrimidin-3-yl)-1-(tetrahydro-2H-pyran-4-yl)-1H-imidazo[4,5-c]pyridin-2(3H)-one;
3-[8-Oxo-9-(tetrahydro-2*H*-pyran-4-yl)-8,9-dihydro-7*H*-purin-2-yl]imidazo[1,2-*a*]pyridine-6-carboxamide;
and pharmaceutically acceptable salts, or solvates, or N-oxides, or stereoisomers or deuterated derivatives thereof:
Of outstanding interest are:
   3-[3-(2-Methylcyclohexyl)-2-oxo-2,3-dihydro-1*H*-imidazo[4,5-*b*]pyridin-5-yl]imidazo[1,2-*a*]pyridine-6-carbonitrile;
   3-{9-[(4*R*)-8-Fluoro-3,4-dihydro-2*H*-chromen-4-yl]-8-oxo-8,9-dihydro-7*H*-purin-2-yl}imidazo[1,2-*a*]pyridine-6-carbonitriie;
   9-Cyclohexyl-2-(6-fluoroimidazo[1,2-a]pyridin-3-yl)-7H-purin-8(9H)-one;
   9-[(4*R*)-8-Fluoro-3,4-dihydro-2*H*-chromen-4-yl]-2-(6-fluoroimidazo[1,2-*a*]pyridin-3-yl)-7,9-dihydro-8*H*-purin-8-one;
   2-(6-Fluoroimidazo[1,2-*a*]pyridin-3-yl)-9-[(1*R*)-1-phenylethyl]-7,9-dihydro-8*H*-purin-8-one;
   2-(6-Fluoroimidazo[1,2-a]pyridin-3-yl)-9-(5,6,7,8-tetrahydroquinolin-5-yl)-7H-purin-8(9H)-one;
   2-(6-Fluoroimidazo[1,2-a]pyridin-3-yl)-9-((5-fluoropyridin-2-yl)methyl)-7H-purin-8(9H)-one;
   (R)-2-(6-Fluoroimidazo[1,2-a]pyridin-3-yl)-9-(1-(5-fluoropyridin-2-yl)ethyl)-7H-purin-8(9H)-one;
   (R)-2-(6-Fluoroimidazo[1,2-a]pyridin-3-yl)-9-(1-(5-fluoropyridin-2-yl)ethyl)-7-(2-hydroxyethyl)-7H-purin-8(9H)-one;
   (R)-2-(6-Fluoroimidazo[1,2-a]pyridin-3-yl)-9-(1-(pyridin-2-yl)ethyl)-7H-purin-8(9H)-one;
   (R)-2-(6-Fluoroimidazo[1,2-a]pyridin-3-yl)-9-(1-(5-fluoropyrimidin-2-yl)ethyl)-7H-purin-8(9H)-one;
   (S)-2-(6-Fluoroimidazo[1,2-a]pyridin-3-yl)-9-(1-(5-fluoropyridin-2-yl)-2-hydroxyethyl)-7H-purin-8(9H)-one;
   9-(2,2-Dimethyltetrahydro-2H-pyran-4-yl)-2-(6-fluoroimidazo[1,2-a]pyridin-3-yl)-7,9-dihydro-8H-purin-8-one;
   2-((1r,4r)-4-(2-(6-Fluoroimidazo[1,2-a]pyridin-3-yl)-8-oxo-7H-purin-9(8H)-yl)cyclohexyl)acetonitrile;
   2-((1r,4r)-4-(2-(6-Fluoroimidazo[1,2-a]pyridin-3-yl)-7-(2-hydroxyethyl)-8-oxo-7H-purin-9(8H)-yl)cyclohexyl)acetonitrile;
   2-(6-Fluoroimidazo[1,2-a]pyridin-3-yl)-9-((1r,4r)-4-(hydroxymethyl)cyclohexyl)-7H-purin-8(9H)-one;
   2-(6-Fluoroimidazo[1,2-a]pyridin-3-yl)-9-((1r,4r)-4-hydroxycyclohexyl)-7H-purin-8(9H)-one;
   2-(6-Fluoroimidazo[1,2-a]pyridin-3-yl)-9-((1R,4R)-4-hydroxy-1,2,3,4-tetrahydronaphthalen-1-yl)-7H-purin-8(9H)-one;
   2-(6-Fluoroimidazo[1,2-a]pyridin-3-yl)-9-((1r,4r)-4-hydroxy-4-methylcyclohexyl)-7H-purin-8(9H)-one;
   9-(4,4-Difluorocyclohexyl)-2-(6-fluoroimidazo[1,2-a]pyridin-3-yl)-7,9-dihydro-8H-purin-8-one;
   6-Fluoro-5-(6-fluoroimidazo[1,2-a]pyridin-3-yl)-3-(tetrahydro-2H-pyran-4-yl)-1,3-dihydro-2H-imidazo[4,5-b]pyridin-2-one;
   2-(2-Amino-6-fluoroimidazo[1,2-a]pyridin-3-yl)-9-(tetrahydro-2H-pyran-4-yl)-7H-purin-8(9H)-one;
   2-Pyrazolo[1,5-*a*]pyrazin-3-yl-9-(tetrahydro-2*H*-pyran-4-yl)-7,9-dihydro-8*H*-purin-8-one; 9-[(1*R*)-1-Phenylethyl]-2-pyrazolo[1,5-a]pyrazin-3-yl-7,9-dihydro-8*H*-purin-8-one;
   (R)-9-(1-(5-Fluoropyridin-2-yl)ethyl)-2-(pyrazolo[1,5-a]pyrazin-3-yl)-7H-purin-8(9H)-one;
   9-(4,4-Difluorocyclohexyl)-2-(pyrazolo[1,5-a]pyrazin-3-yl)-7H-purin-8(9H)-one;
   9-(2,2-Dimethyltetrahydro-2H-pyran-4-yl)-2-(pyrazolo[1,5-a]pyrazin-3-yl)-7H-purin-8(9H)-one;
   (R)-9-(8-Fluorochroman-4-yl)-2-(pyrazolo[1,5-a]pyrazin-3-yl)-7H-purin-8(9H)-one;
   2-((1r,4r)-4-(8-Oxo-2-(pyrazolo[1,5-a]pyrazin-3-yl)-7H-purin-9(8H)-yl)cyclohexyl)acetonitrile;
   2-((1r,4r)-4-(7-(2-Hydroxyethyl)-8-oxo-2-(pyrazolo[1,5-a]pyrazin-3-yl)-7H-purin-9(8H)-yl)cyclohexyl)acetonitrile;
   5-(Pyrazolo[1,5-a]pyrazin-3-yl)-3-(tetrahydro-2H-pyran-4-yl)-1H-imidazo[4,5-b]pyridin-2(3H)-one;
   6-Fluoro-5-pyrazolo[1,5-a]pyrazin-3-yl-3-(tetrahydro-2H-pyran-4-yl)-1,3-dihydro-2H-imidazo[4,5-b]pyridin-2-one;
   2-(Pyrazolo[1,5-a]pyridin-3-yl)-9-(tetrahydro-2H-pyran-4-yl)-7H-purin-8(9H)-one;
and pharmaceutically acceptable salts, solvates, N-oxides or deuterated derivatives thereof.

The compounds of the present invention may be prepared by methods such as those illustrated in the following Schemes. Solvents, temperatures, pressures and other reaction conditions may readily be selected by one of ordinary skill in the art. Starting materials are commercially available or can be readily prepared by one of ordinary skill in the art using known methods.

According to one embodiment of the present invention, compounds of general formula (I) may be prepared by the following synthetic route as illustrated in Figure 1.

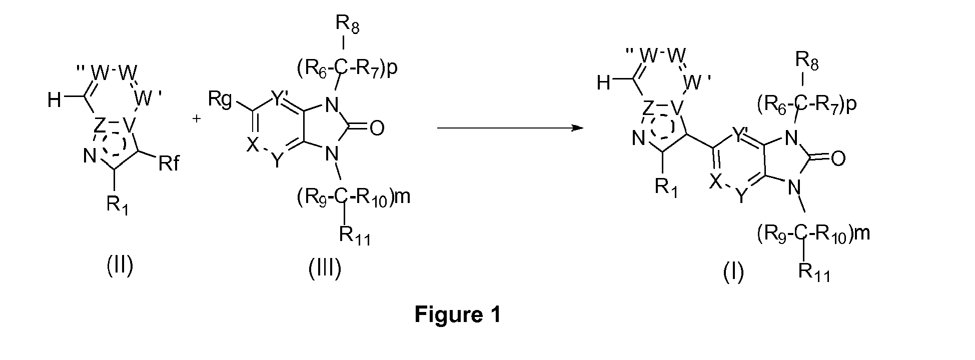

Compounds of formula (**II**) [in which V, W ,W ',W", Z and R₁ represent the groups as defined in the claims and R_{f} represents a hydrogen atom, a boronic acid or alkyl boronate or an alkyl tin residue such as tributyltin] may be reacted with compounds of formula (**III**) [in which X, Y, Y' and R₆-R₁₁ represent the groups as defined in the claims and Rg represents a chlorine or a bromine atom] under palladium-catalyzed coupling conditions with a suitable catalyst to furnish compounds of formula (**I**).
For example, compounds of formula (**II**) in which R_{f} represents a hydrogen atom may be reacted with compounds of formula (**III**) in the presence of a suitable catalyst such as tetrakis(triphenylphosphine)palladium(0) or the catalytically active species generated from palladium(II)acetate/triphenylphosphine in the presence of a base, for example potassium acetate or potassium carbonate, in a solvent such as 1,4-dioxane, ethanol or N,N'-dimethylacetamide or a mixture thereof at temperatures ranging from 100-160 °C with or without the use of microwave irradiation to give compounds of formula (I).
In another example, compounds of formula (**II**) in which R_{f} represents a boronic acid or boronate moiety may be reacted with compounds of formula (**III)** under Suzuki-Miyaura reaction conditions (Miyaura, N.; Suzuki, A. Chem. Rev. 1995, 95, 2457) to give compounds of formula (I). Such reactions may be catalyzed by a suitable palladium catalyst such as tetrakis(triphenylphospino)palladium (0), in a solvent such as 1,4-dioxane, in the presence of a base such as potassium carbonate or potassium acetate, at temperatures ranging from 80-120 °C with or without the use of microwave irradiation.
In yet another example, compounds of formula (**II**) in which R_{f} represents an alkyltin moiety, such as tributyltin, may be reacted with compounds of formula (**III**) in the presence of a suitable catalyst such as tetrakis(triphenylphosphine)palladium(0) in a solvent such as 1,4-dioxane at temperatures ranging from 80-130 °C with or without the use of microwave irradiation to give compounds of formula (**I**).
Compounds of general formula (**I**) in which the residue -(R₉-C-R₁₀)ₘ-R₁₁ or -(R₆-C-R₇)ₚ-R₈ contains a "protected" heteroatom, such as nitrogen or oxygen, may be "deprotected" by removal of the protecting group to give compounds of formula (I) in which the residue -(R₉-C-R₁₀)ₘ-R₁₁ or -(R₆-C-R₇)ₚ-R₈ contains the "deprotected" heteroatom. Typical examples of protecting groups for heteroatoms, such as nitrogen and oxygen, and their removal (deprotection) may be found in several textbooks, for example: Greene's Protective Groups in Organic Synthesis, ISBN: 0471697540. Furthermore said "deprotected" heteroatoms may be further functionalized by, for example, alkylation, amidation, sulfonamidation or arylation under standard reaction conditions.
In the particular cases of compounds of formula (**I**) in which m = 0 and R₁₁ represents an appropriate nitrogen protecting group such as the trimethylsilylethoxymethyl (SEM) moiety, then this group may be subsequently removed under appropriate conditions with, for example, tetrabutyl ammonium fluoride in a solvent such as tetrahydrofuran at temperatures ranging from ambient temperature to reflux to give compounds of formula (I) in which m = 0 and R₁₁ represents a hydrogen atom.

In a particular case, intermediate compounds of general sub-formula **(II-a)**, may be prepared by the following synthetic route as illustrated in Figure 2.

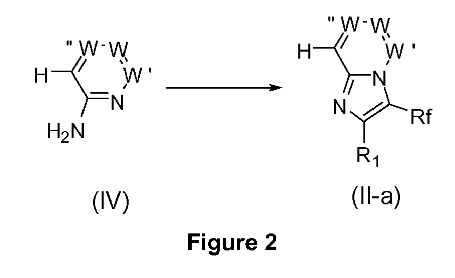

Treatment of heterocyclic amino compounds of formula **(IV)** with a suitable alkylating agent such as 2-chloro- or 2-bromoacetaldehyde in the presence of a base, for example sodium hydrogen carbonate, in a suitable solvent such as acetonitrile or propan-2-ol at temperatures ranging from ambient temperature to reflux gives rise to compounds of sub-formula **(II-a)**.

In yet another particular case, intermediate compounds of general sub-formulas **(IIb-c)** may be prepared by the following synthetic routes as illustrated in Figure 3.

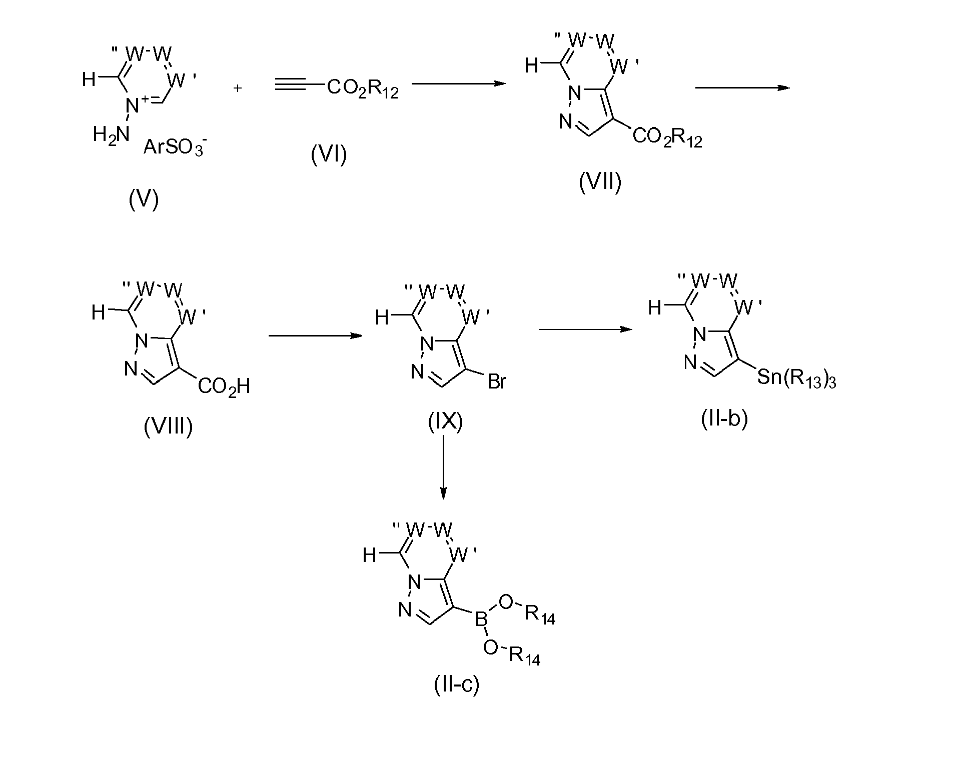

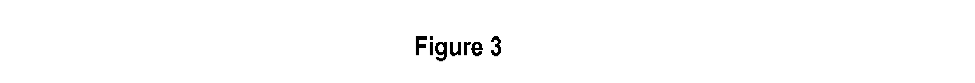

Reaction of salts of formula **(V)** with an acetylenic ester **(VI)** such as ethyl propiolate in the presence of a base, for example potassium carbonate, in a solvent such as *N,N'-*dimethylformamide at temperatures ranging from 0 °C to ambient temperature, furnishes compounds of formula **(VII)**.
Treatment of compounds of formula **(VII)** with a suitable base such as sodium hydroxide in a solvent such as ethanol at temperatures ranging from ambient temperature to reflux furnishes carboxylic acids of formula **(VIII)**.
Compounds of formula **(VIII)** may be transformed into bromo derivatives of formula **(IX)** by treatment with a brominating reagent such as N-bromosuccinimide in the presence of a base, such as sodium hydrogen carbonate, in a solvent such as N,N'-dimethylformamide at ambient temperature.
Treatment of bromo derivatives of formula **(IX)** with an appropriate hexa-alkyldistannane, such as 1,1,1,2,2,2-hexabutyldistannane, in the presence of a palladium catalyst, such as tetrakis(triphenylphosphine)palladium(0), in a solvent such as 1,4-dioxane at temperatures ranging from 80-130 °C with or without the use of microwave irradiation provides compounds of sub-formula **(II-b)**.
In another synthetic method, treatment of bromo derivatives of formula **(IX)** with an appropriate boron reagent such as bis(pinacolato)diboron with a palladium catalyst such as tetrakis(triphenylphospino)palladium (0), in a solvent such as 1,4-dioxane, in the presence of a base such as potassium acetate, at temperatures ranging from 80-120 °C with or without the use of microwave irradiation provides compounds of sub-formula **(II-c).**

Intermediate compounds of general formula **(III)** may be prepared by the following synthetic routes as illustrated in Figure 4.

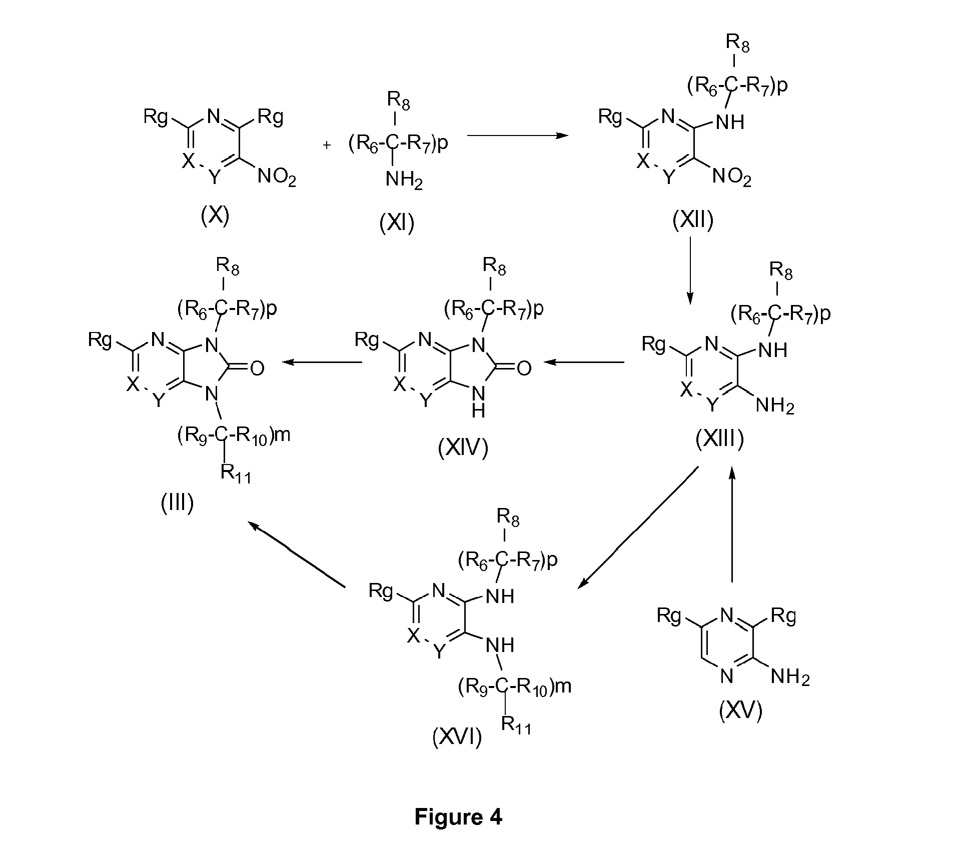

Compounds of formula (X) may be reacted with amines of formula **(XI),** in the presence of a base, such as N,N-diisopropylethylamine or triethylamine, in a solvent such as dichloromethane, chloroform or tetrahydrofuran at temperatures ranging from -78 °C to reflux to furnish compounds of formula **(XII).**
Compounds of formula **(XII)** may in turn be converted to compounds of formula **(XIII)** by treatment with tin (II) chloride in a solvent such as ethanol at temperatures ranging from 20-100 °C or by reduction with hydrogen gas at atmospheric pressure using a suitable catalyst such as platinum on carbon in the presence of an additive such as zinc bromide in a solvent such as ethyl acetate at ambient temperature.
Compounds of formula **(XIII)** may be converted into compounds of formula **(XIV)** by treatment with a suitable reagent such as 1,1'-carbonylbis-1H-imidazole in a solvent such as tetrahydrofuran or acetonitrile at temperatures ranging from ambient temperature to reflux.
Treatment of compounds of formula **(XIV)** with a suitable base such as sodium hydride or potassium carbonate in a solvent such as N,N'-dimethylformamide followed by addition of an electrophile, for example methyl iodide or (2-(chloromethoxy)ethyl)trimethylsilane at temperatures ranging from 0-100 °C furnishes compounds of formula **(III).**
In another synthetic pathway, particular compounds of formula **(XIII),** where X = CR₅ and Y = N, may be derived from bis-halopyrazines of formula **(XV)** by selective displacement of one of the halogen atoms with a nucleophile **(XI)** such as tetrahydro-2H-pyran-4-amine in the presence of a base, such as N,N-diisopropylethylamine, in a solvent such as n-butanol at temperatures ranging from 80-150 °C under microwave irradiation.
In yet another synthetic pathway, compounds of formula **(XIII)** may be transformed into compounds of formula **(XVI),** where R₉ = R₁₀ = H and m=1, by reaction under typical reductive amination conditions by reaction with an appropriate aldehyde R₁₁CHO, such as 2,4-dimethoxybenzaldehyde, in the presence of a reducing agent, such as sodium triacetoxyborohydride, and an acid such as acetic acid in a solvent such as dichloromethane or dichloroethane at ambient temperature.
Treatment of compounds of formula **(XVI)** with a reagent such as triphosgene in the presence of a base, such as triethylamine, in a solvent such as tetrahydrofuran at temperatures ranging from ambient temperature to reflux provides compounds of formula **(III).**

Compounds of formula **(XIV)** may also be prepared by the following synthetic route as illustrated in Figure 5.

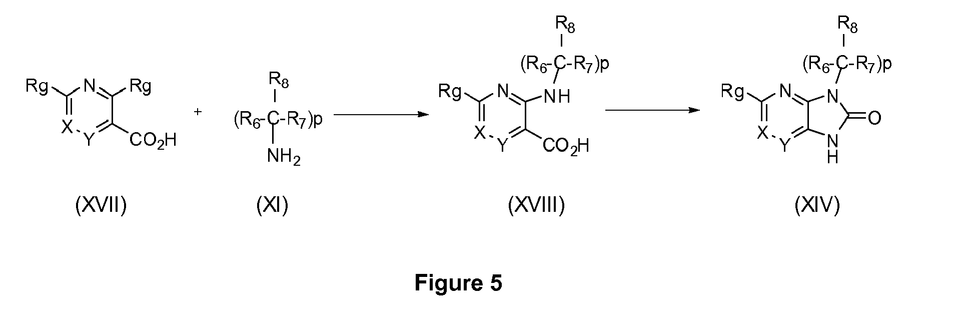

Compounds of formula **(XVIII)** may be accessed from compounds of formula **(XVII)** by selective displacement of one of the halogen atoms with a nucleophile **(XI)** such as tetrahydro-2H-pyran-4-amine in the presence of a base, such as N,N-diisopropylethylamine, in a solvent such as acetonitrile at temperatures ranging from 80-130 °C under microwave irradiation.
Compounds of formula **(XVIII)** may be converted into compounds of formula **(XIV)** by treatment with a reagent such as diphenylphosphoryl azide in the presence of a base such as triethylamine in a suitable solvent such as 1,4-dioxane at temperatures ranging from ambient temperature to reflux.

In another embodiment of the present invention, compounds of general formula **(I-c)** may be prepared by the following synthetic route as illustrated in Figure 6.

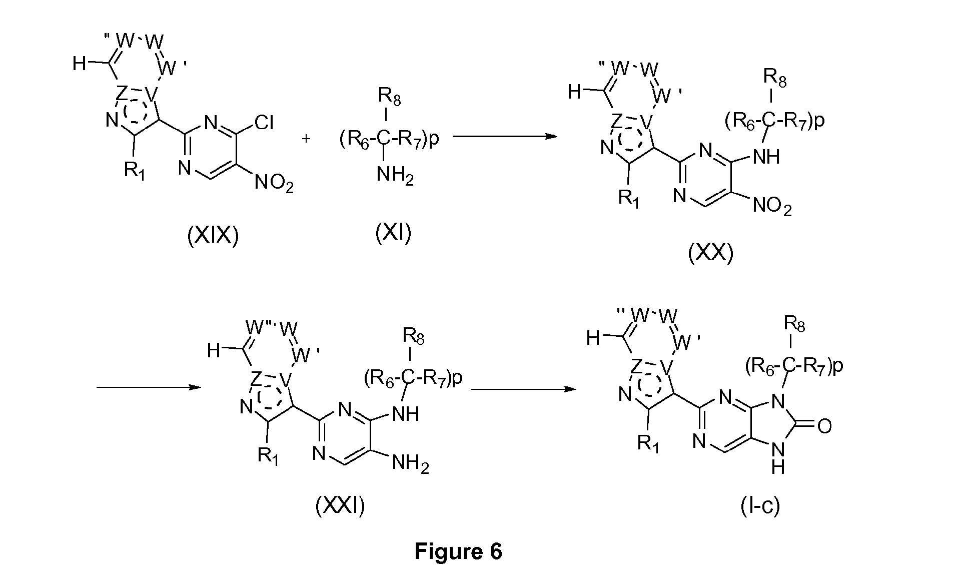

Compounds of formula **(XIX)** may be reacted with amines of formula **(XI),** in the presence of a base, such as N,N-diisopropylethylamine, in a solvent such as tetrahydrofuran at temperatures ranging from 0 °C to reflux to furnish compounds of formula **(XX)**.
Compounds of formula **(XX)** may in turn be converted to compounds of formula **(XXI)** by treatment with tin (II) chloride in a solvent such as ethanol at temperatures ranging from 20-100 °C or by reduction with hydrogen gas at atmospheric pressure using a suitable catalyst such as palladium or platinum on carbon in a solvent such as ethanol or methanol at ambient temperature.
Compounds of formula **(XXI)** may be converted into compounds of formula **(I-c)** by treatment with a suitable reagent such as 1,1'-carbonylbis-1*H*-imidazole in a solvent such as tetrahydrofuran or acetonitrile at temperatures ranging from ambient temperature to reflux.
Compounds of general formula **(I-c)** in which the residue -(R₆-C-R₇)ₚ-R₈ contains a "protected" heteroatom, such as nitrogen or oxygen, may be "deprotected" by removal of the protecting group to give compounds of formula **(I-c)** in which the residue -(R₆-C-R₇)p-R₈ contains the "deprotected" heteroatom. Furthermore said "deprotected" heteroatoms may be further functionalized by, for example, alkylation, amidation, sulfonamidation or arylation under standard conditions.

Intermediate compounds of general formula **(XIX)** may be prepared by the following synthetic route as illustrated in Figure 7.

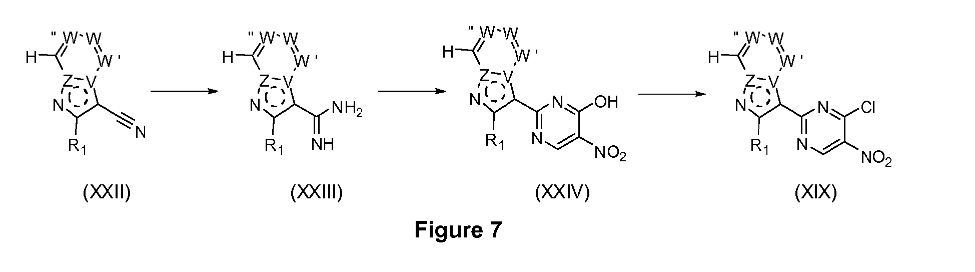

Treatment of nitriles of general formula **(XXII)** with catalytic sodium methoxide in methanol at ambient temperature followed by treatment of the corresponding imidate intermediates with ammonium chloride in methanol at refluxing temperatures furnishes amidine intermediates of formula **(XXIII).**
Reaction of amidines of formula **(XXIII)** with ethyl 3-(dimethylamino)-2-nitroacrylate, in the presence of triethylamine in a solvent such as ethanol at refluxing temperatures furnishes pyrimidones of formula **(XXIV).**
Treatment of compounds of formula **(XXIV)** with a suitable chlorinating agent, for example phosphorous (V) oxychloride or phosphorous (V) chloride, at temperatures ranging from 25 °C to reflux gives rise to compounds of formula **(XIX).**

In a particular case, intermediate nitriles of sub-formula **(XXII-a)** may be prepared by the following synthetic route as illustrated in Figure 8.

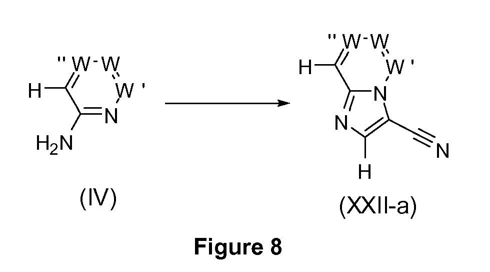

Treatment of heterocyclic amines of formula **(IV)** with 3-methoxyacrylonitrile in the presence of N-bromosuccinimide in a suitable solvent such as dioxane or water at temperatures ranging from ambient temperature to reflux furnishes intermediate nitriles of sub-formula **(XXII-a).**

In yet another particular case, intermediate nitriles of sub-formula **(XXII-b)** may be prepared by the following synthetic route as illustrated in Figure 9.

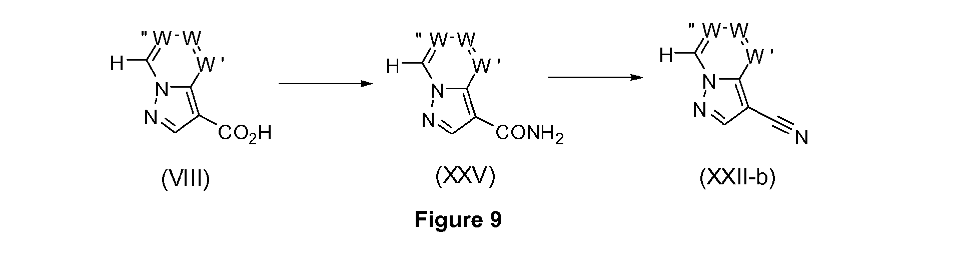

Treatment of compounds of formula **(VIII)** with a suitable chlorinating reagent such as thionyl chloride at temperatures ranging from ambient temperature to reflux furnishes intermediate acid chlorides which when treated with an ammonia source, such as aqueous ammonium hydroxide, gives rise to amides of formula **(XXV)**.
Treatment of amides of formula **(XXV)** with a suitable dehydrating reagent such as phosphoryl trichloride at temperatures ranging from ambient temperature to reflux furnishes intermediate nitriles of sub-formula **(XXII-b).**

In yet another particular case, compounds of general formula **(I-d)** may be prepared by the following synthetic route as illustrated in Figure 10.

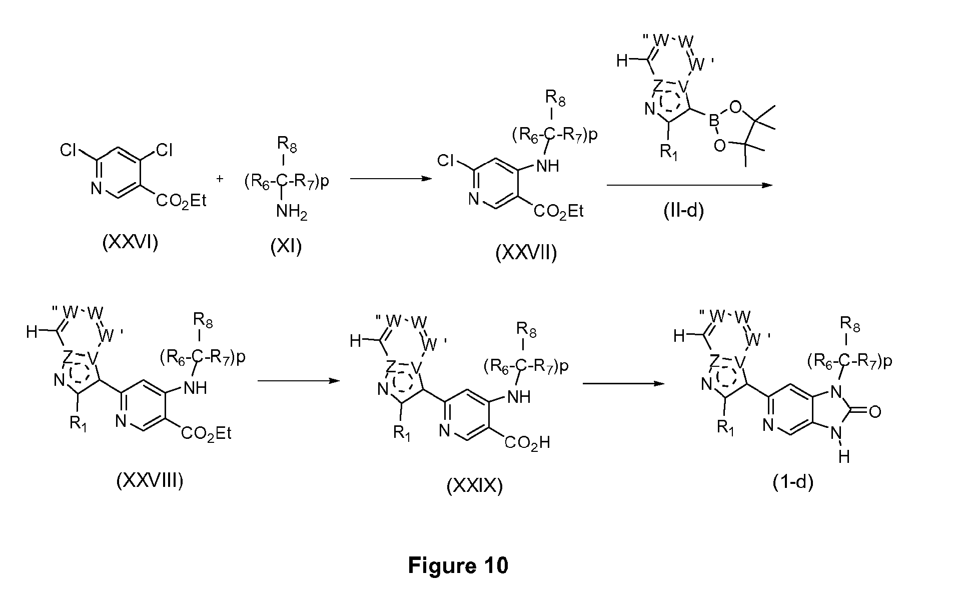

Treatment of ethyl 4,6-dichloronicotinate of formula **(XXVI)** with amines of formula **(XI)**, in the presence of a base, such as N,N-diisopropylethylamine, in a solvent such as acetonitrile or tetrahydrofuran at temperatures ranging from 0 °C to reflux furnishes compounds of formula **(XXVII)**.
Compounds of formula **(XXVII)** may be reacted with compounds of sub-formula **(II-d)** under Suzuki-Miyaura reaction conditions (Miyaura, N.; Suzuki, A. Chem. Rev. 1995, 95, 2457) to give compounds of formula **(XXVIII).** Such reactions may be catalyzed by a suitable palladium catalyst such as tetrakis(triphenylphospino)palladium (0), in a solvent such as 1,4-dioxane, in the presence of a base such as potassium carbonate or potassium acetate, at temperatures ranging from 80-120 °C with or without the use of microwave irradiation.
Treatment of compounds of formula **(XXVIII)** with a suitable base such as sodium hydroxide in a solvent such as ethanol at temperatures ranging from ambient temperature to reflux furnishes compounds of formula **(XXIX)**.
Compounds of formula **(XXIX)** may be converted into compounds of sub-formula (I-d) by treatment with a reagent such as diphenylphosphoryl azide in the presence of a base such as triethylamine in a suitable solvent such as 1,4-dioxane at temperatures ranging from ambient temperature to reflux.

In yet another synthetic pathway, compounds of general sub-formula **(I-e)** may be prepared by the following synthetic route as illustrated in Figure 11.

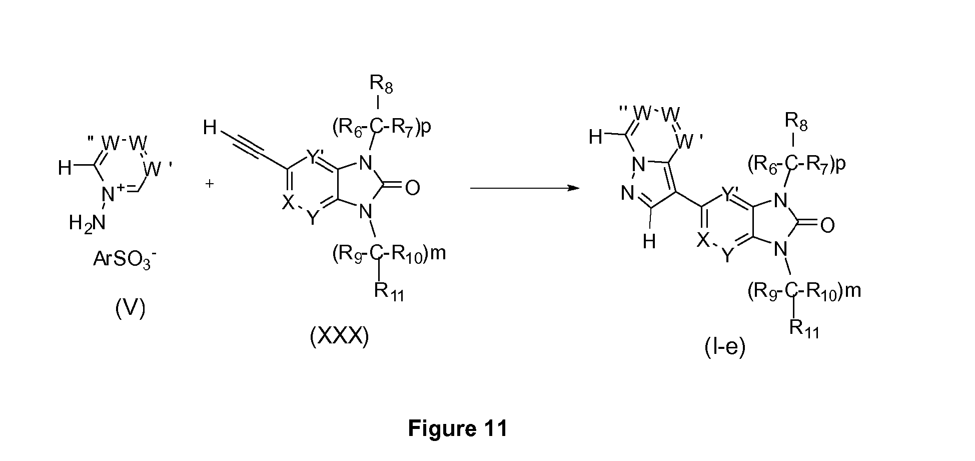

Reaction of alkynes of formula (**XXX**) with *N*-aminopyridinium (in the particular case where W = CR₃, W^{I} = CR₂ and W^{II} = CR₄) or *N*-aminopyrazinium salts (in the particular case where W = N, W^{I} = CR₂ and W^{II} = CR₄) of formula (V) in the presence of a base, for example potassium carbonate, in a solvent such as *N,N*'-dimethylformamide at temperatures ranging from 0-80 °C, furnishes compounds of formula (**1-e**).
In the particular cases of reaction with compounds of formula (**1-e**) in which m = 0 and R₁₁ represents an appropriate nitrogen protecting group such as the trimethylsilylethoxymethyl (SEM) moiety then this group may be subsequently removed under appropriate conditions *(*Greene's Protective Groups in Organic Synthesis, ISBN: 0471697540) to give compounds of formula (**I-e**) in which m = 0 and R₁₁ represents a hydrogen atom.

Intermediate compounds of general formula (**XXX**) may be prepared by the following synthetic route as illustrated in Figure 12.

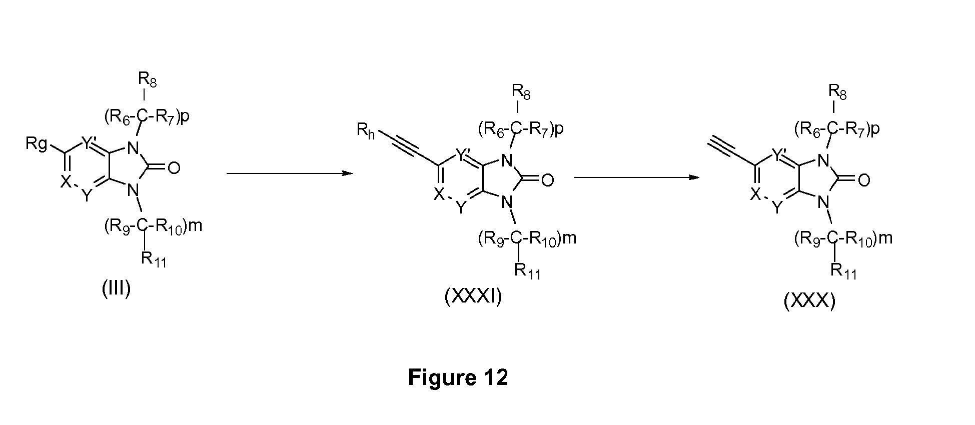

Reaction of compounds of formula (**III**) with a suitable acetylene such as ethynyltrimethylsilane or 2-methylbut-3-yn-2-ol under palladium and copper-catalyzed coupling conditions with suitable catalysts such as bis(triphenylphosphine)palladium (II) dichloride and copper(I) iodide in the presence of a base, for example triethylamine, in a solvent such as tetrahydrofuran at temperatures ranging from ambient temperature to reflux, with or without the use of microwave irradiation, furnishes compounds of formula (**XXXI**) where Rₕ = -SiMe₃ or-C(Me)₂OH.
Treatment of compounds of formula (**XXXI**) in which Rₕ = -SiMe₃ with a suitable reagent such as sodium methoxide in a solvent such as methanol at ambient temperature furnishes compounds of formula (**XXX**). Alternatively, treatment of compounds of formula (**XXXI**) in which Rₕ = - C(Me)₂OH with a suitable base such as sodium hydride in a solvent such as toluene at reflux also furnishes compounds of formula (**XXX**).

The syntheses of the compounds of the invention and of the intermediates for use therein are illustrated by the following Examples (1-93) (including Preparation Examples (Preparations 1-109)) and are given in order to provide a person skilled in the art with a sufficiently clear and complete explanation of the present invention, but should not be considered as limiting of the essential aspects of its subject, as set out in the preceding portions of this description.

### PREPARATION 1

### Imidazo[1,2-a]pyridine-6-carbonitrile

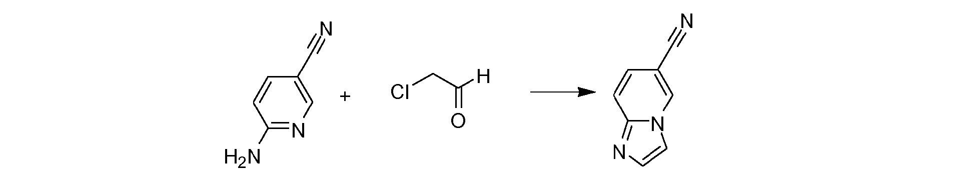

A 50% aqueous solution of 2-chloroacetaldehyde (26.40 mL, 210 mmol) was added to a solution of 2-aminonicotinonitrile (10 g, 80 mmol) in acetonitrile (300 mL) and the mixture was stirred and heated to reflux. After 20 hours, aqueous saturated sodium hydrogencarbonate solution was added, the mixture was partially concentrated and further aqueous saturated sodium hydrogencarbonate solution was added until the pH reached 7. The mixture was extracted with dichloromethane and the organic layer was dried (MgSO₄) and evaporated and the residue was triturated with diethyl ether to yield the title compound (10.75 g, 89%) as a brown solid.
LRMS (m/z): 144 (M+1)⁺.
1H NMR (300 MHz, DMSO-*d*₆) δppm 7.49 (dd, 1H), 7.72 (s, 1H), 7.75 - 7.78 (m, 1H), 8.07 (s, 1H), 9.37 (s, 1H).

### PREPARATION 2

### 2-Chloro-9-(tetrahydro-2H-pyran-4-yl)-7,9-dihydro-8H-purin-8-one

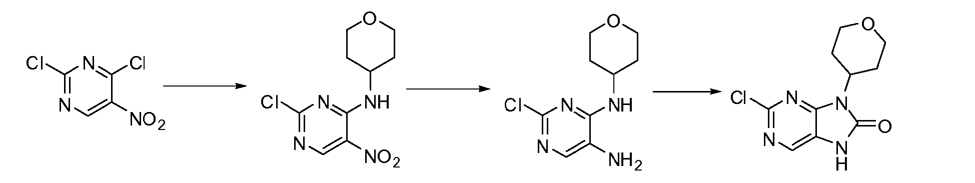

### a) 2-Chloro-5-nitro-N-(tetrahydro-2H-pyran-4-yl)pyrimidin-4-amine

Diisopropylethylamine (19.80 mL, 110 mmol) was added dropwise over 15 minutes to a stirred suspension of 2,4-dichloro-5-nitropyrimidine (11.56 g, 60 mmol) and tetrahydro-2*H*-pyran-4-amine hydrochloride (WO200424728 A2) (7.81 g, 60 mmol) in dichloromethane (400 mL) at -78 °C under a nitrogen atmosphere. The reaction mixture was stirred at -78 °C for 2 hours and then was allowed to warm to ambient temperature. The solvent was evaporated, water was added and the resultant solid was filtered, washed with water and dried to yield the title compound (13.62 g, 93%) as a yellow solid.
LRMS (m/z): 259 (M+1)⁺.
1H NMR (300 MHz, CDCl₃) δ ppm 1.62 - 1.80 (m, 2H), 2.06 (d, 2H), 3.59 (t, 2H), 4.04 (d, 2H), 4.45 (td, 1H), 8.33 (br s, 1H), 9.07 (s, 1H).

### b) 2-Chloro-N⁴-(tetrahydro-2H-pyran-4-yl)pyrimidine-4,5-diamine

Zinc bromide (2.37 g, 10.5 mmol) and 5% platinum on carbon (5.13 g, 25.7 mmol) were added to a solution of 2-chloro-5-nitro-*N*-(tetrahydro-2*H*-pyran-4-yl)pyrimidin-4-amine (**Preparation 2a**, 13.62 g, 51.0 mmol) in ethyl acetate (200 mL) and the reaction mixture was stirred at room temperature overnight under a hydrogen atmosphere. The mixture was then filtered through diatomaceous earth (Celite®) and the filter cake was washed with methanol. The combined filtrate and washings were concentrated to give the title compound (11.9 g, 100%) as a solid.
LRMS (m/z): 229 (M+1)⁺.
1H NMR (300 MHz, DMSO-*d*₆) δ ppm 1.35 - 1.62 (m, 2H), 1.85 (d, 2H), 3.40 (t, 2H), 3.87 (d, 2H), 4.03 (m, 1H), 4.96 (br s, 2H), 6.66 (d, 1H), 7.38 (s, 1H).

### c) 2-Chloro-9-(tetrahydro-2H-pyran-4-yl)-7,9-dihydro-8H-purin-8-one

A mixture of 2-chloro-*N*⁴-(tetrahydro-2*H*-pyran-4-yl)pyrimidine-4,5-diamine (**Preparation 2b**, 5.40 g, 20 mmol) and 1,1'-carbonylbis-1*H*-imidazole (5.74 g, 40 mmol) in acetonitrile (100 mL) was stirred and heated to 80 °C in a sealed tube. After 2 hours, the solvent was evaporated under reduced pressure and the residue was dissolved in 2M aqueous hydrochloric acid. 2M aqueous sodium hydroxide solution was added until the pH reached approximately 7 and the resultant precipitate was filtered and dried to give the title compound (4.80 g, 80%) as a white solid.
LRMS (m/z): 255 (M+1)⁺.
1H NMR (300 MHz, CDCl₃) δ ppm 1.75 (m, 2H), 2.68 - 2.86 (m, 2H), 3.55 (m, 2H), 4.16 (m, 2H), 4.53 - 4.68 (m, 1H), 8.16 (s, 1H).

### PREPARATION 3

### 2-Chloro-9-(tetrahydro-2H-pyran-4-yl)-7-{[2-(trimethylsilyl)ethoxy]methyl}-7,9-dihydro-8H-purin-8-one

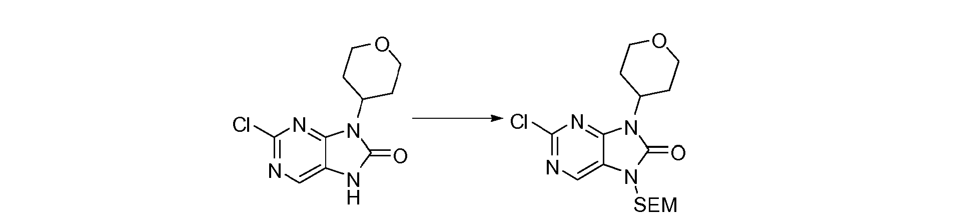

Sodium hydride (60% dispersion in mineral oil, 0.40 g, 10.0 mmol) was added portion wise to a stirred solution of 2-chloro-9-(tetrahydro-2*H*-pyran-4-yl)-7,9-dihydro-8*H*-purin-8-one (**Preparation 2**, 1.98 g, 7.8 mmol) in N,N'-dimethylformamide (30 mL) at 0 °C under an argon atmosphere. After 15 minutes, (2-(chloromethoxy)ethyl)trimethylsilane (1.53 mL, 8.6 mmol) was added and the mixture was warmed to room temperature and stirred for 4 hours. The mixture was then partitioned between water and ethyl acetate and the organic layer was washed with water and brine, dried (MgSO₄ and the solvent was evaporated under reduced pressure. The residue was purified by flash chromatography (99:1 dichloromethane/methanol) to give the title compound (2.94 g, 98%) as a pale yellow oil.
LRMS (m/z): 385 (M+1)⁺.
1H NMR (300 MHz, CDCl₃) δ ppm -0.20 - 0.08 (m, 9H), 0.92 (m, 2H), 1.73 (m, 2H), 2.62 - 2.84 (m, 2H), 3.39 - 3.71 (m, 4H), 4.15 (m, 2H), 4.48 - 4.76 (m, 1H), 5.31 (s, 2H), 8.18 (s, 1H).

### PREPARATION 4

### 3-(8-Oxo-9-(tetrahydro-2H-pyran-4-yl)-7-((2-(trimethylsilyl)ethoxy)methyl)-8,9-dihydro-7H-purin-2-yl)imidazo[1,2-a]pyridine-6-carbonitrile

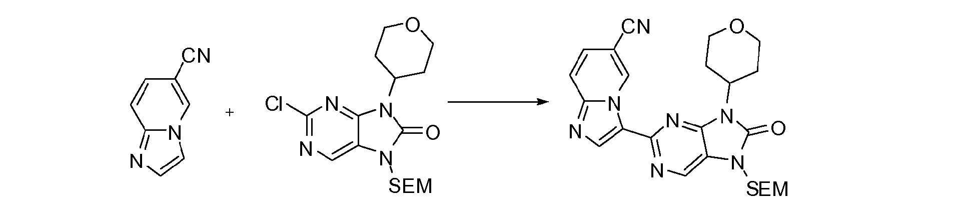

A Schlenck vessel was charged with 2-chloro-9-(tetrahydro-2*H*-pyran-4-yl)-7-{[2-(trimethylsilyl)ethoxy]methyl}-7,9-dihydro-8*H*-purin-8-one (**Preparation** 2, 1.50 g, 3.90 mmol), imidazo[1,2-a]pyridine-6-carbonitrile (**Preparation** 1, 1.12 g, 7.82 mmol) and *N,N*'-dimethylacetamide (20 mL). Potassium acetate (1.15 g, 11.72 mmol) was added and the reaction mixture was submitted to three vacuum-argon cycles. Tetrakis(triphenylphosphine)palladium(0) (0.45 g, 0.39 mmol) was then added and the mixture was further submitted to three vacuum-argon cycles. The reaction vessel was sealed and the contents were stirred and heated to 150 °C. After 2 hours, the mixture was cooled to room temperature and evaporated. Ethyl acetate was added and the organic layer was washed with water (x 3), dried (MgSO₄) and evaporated. The residue was purified by flash chromatography (98:2 dichloromethane/methanol) to give the title compound (1.73 g, 90%) as a white solid.
LRMS (m/z): 492 (M+1)⁺.
1H NMR (300 MHz, CDCl₃) δ ppm 0.01 (s, 9H), 0.99 (d, 2H), 1.83 (dd, 2H), 2.86 (m, 2H), 3.51 - 3.73 (m, 4H), 4.22 (dd, 2H), 4.59 - 4.76 (m, 1H), 5.38 (s, 2H), 7.45 (dd, 1H), 7.84 (d, 1H), 8.44 (s, 1H), 8.68 (s, 1H), 10.54 (s, 1H).

### PREPARATION 5

### 2-Chloro-9-cyclohexyl-7-{[2-(trimethylsilyl)ethoxy]methyl}-7,9-dihydro-8H-purin-8-one

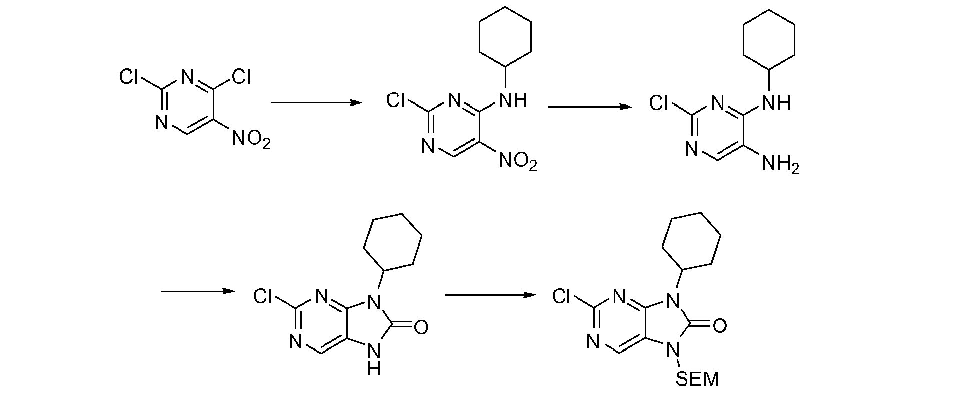

### a) 2-Chloro-N-cyclohexyl-5-nitropyrimidin-4-amine

Obtained as a white solid (100%) from 2,4-dichloro-5-nitropyrimidine and cyclohexylamine following the experimental procedure as described in **Preparation 2a**.
LRMS (m/z): 257 (M+1)⁺.
1H NMR (300 MHz, CDCl₃) δ ppm 1.22 - 1.53 (m, 4H), 1.54 - 1.74 (m, 2H), 1.75 - 1.87 (m, 2H), 1.97 - 2.11 (m, 2H), 4.02 - 4.44 (m, 1H), 8.36 (br s, 1H), 9.04 (s, 1H).

### b) 2-Chloro-N⁴-cyclohexylpyrimidine-4,5-diamine

A suspension of 2-chloro-*N*-cyclohexyl-5-nitropyrimidin-4-amine (**Preparation 5a**, 3.36 g, 13.1 mmol) and SnCl₂.2H₂O (11.81 g, 52.3 mmol) in ethanol (75 mL) was stirred and heated to 80 °C in a sealed tube. After 7 hours, the mixture was cooled and evaporated. The residue was treated with 2M aqueous sodium hydroxide solution and extracted with ethyl acetate. The organic layer was washed with water, brine, dried (MgSO₄) and evaporated to give the title compound (2.68 g, 90%) as a foam.
LRMS (m/z): 227 (M+1)⁺.
1H NMR (300 MHz, CDCl₃) δ ppm 1.09 - 1.30 (m, 2H), 1.33 - 1.53 (m, 2H), 1.60 - 1.82 (m, 4H), 1.96 - 2.10 (m, 2H), 2.96 (br s, 2H), 3.87 - 4.08 (m, 1H), 4.96 (br s, 1H), 7.59 (s, 1H).

### c) 2-Chloro-9-cyclohexyl-7,9-dihydro-8H-purin-8-one

Obtained as a solid (96%) from 2-chloro-*N*⁴-cyclohexylpyrimidine-4,5-diamine (Preparation **5b**) following the experimental procedure as described in **Preparation 2c**.
LRMS (m/z): 253 (M+1)⁺.
1H NMR (300 MHz, DMSO-*d*₆) δ ppm 1.06 - 1.49 (m, 4H), 1.60 - 1.91 (m, 4H), 2.06 - 2.26 (m, 2H), 4.05 - 4.22 (m, 1H), 8.12(s, 1H), 11.62 (s, 1H).

### d) 2-Chloro-9-cyclohexyl-7-{[2-(trimethylsilyl)ethoxy]methyl}-7,9-dihydro-8H-purin-8-one

Obtained as a white solid (65%) from 2-chloro-9-cyclohexyl-7,9-dihydro-8*H*-purin-8-one (**Preparation 5c**) following the experimental procedure as described in **Preparation 3**.
LRMS (m/z): 383 (M+1)⁺.
1H NMR (300 MHz, CDCl₃) δ ppm - 0.02 (s, 9H), 0.67 - 1.00 (m, 2H), 1.24 - 1.52 (m, 3H), 1.65 - 1.98 (m, 5H), 2.23 - 2.44 (m, 2H), 3.46 - 3.65 (m, 2H), 4.22 - 4.44 (m, 1H), 5.28 (s, 2H), 8.15 (s, 1H).

### PREPARATION 6

### 3-(9-Cyclohexyl-8-oxo-7-((2-(trimethylsilyl)ethoxy)methyl)-8,9-dihydro-7H-purin-2-yl)imidazo[1,2-a]pyridine-6-carbonitrile

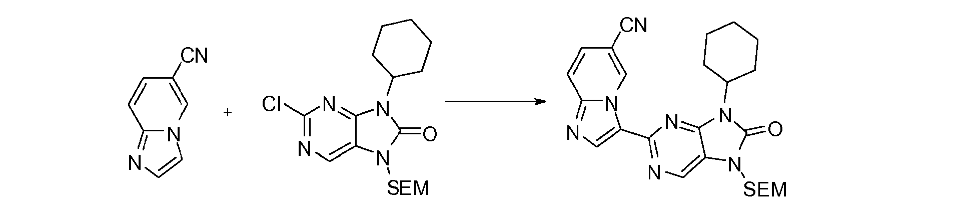

Obtained as a white solid (74%) from 2-chloro-9-cyclohexyl-7-{[2-(trimethylsilyl)ethoxy] methyl}-7,9-dihydro-8*H*-purin-8-one (**Preparation 5**) and imidazo[1,2-*a*]pyridine-6-carbonitrile (**Preparation 1**) following the experimental procedure as described in **Preparation 4**.
LRMS (m/z): 490 (M+1)⁺.
1H NMR (300 MHz, CDCl₃) δ ppm -0.01 (s, 9H), 0.95 (d, 2H), 1.38 - 1.50 (m, 4H), 1.77 - 2.08 (m, 4H), 2.31 - 2.57 (m, 2H), 3.63 (d, 2H), 4.29 - 4.53 (m, 1H), 5.37 (s, 2H), 7.43 (d, 1H), 7.83 (d, 1H), 8.41 (s, 1H), 8.65 (s, 1H), 10.51 (s, 1H).

### PREPARATION 7

### 2-Chloro-9-[(1S,2R)-2-methylcyclohexyl]-7-{[2-(trimethylsilyl)ethoxy]methyl}-7,9-dihydro-8H-purin-8-one

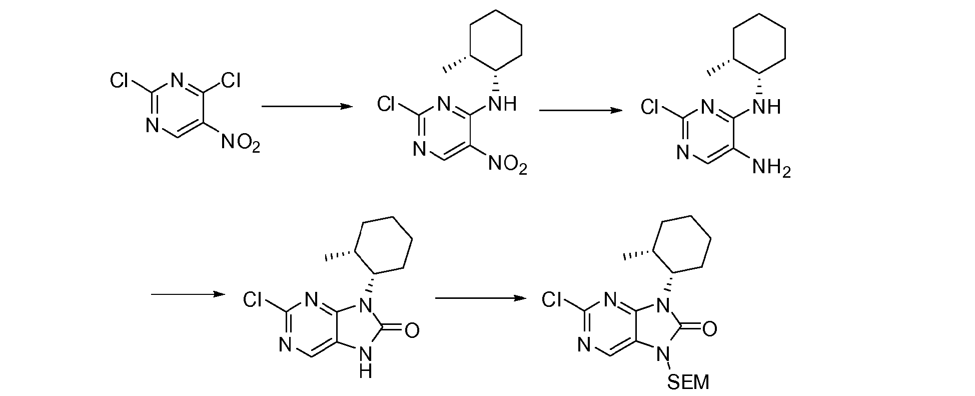

### a) 2-Chloro-N-[(1S,2R)-2-methylcyclohexyl]-5-nitropyrimidin-4-amine

Obtained as a yellow solid (78%) from 2,4-dichloro-5-nitropyrimidine and (1*S*,2*R*)-2-methylcyclohexanamine *(*Chemische Berichte, 1984, 117(6), 2076) following the experimental procedure as described in **Preparation 2a**.
LRMS (m/z): 271 (M+1)⁺.
1H NMR (300 MHz, CDCl₃) δ ppm 0.94 (d, 3H), 1.26 - 1.54 (m, 3H), 1.55 - 1.75 (m, 4H), 1.79 - 1.90 (m, 1H), 1.91 - 2.03 (m, 1H), 4.29 - 4.65 (m, 1H), 8.66 (br s, 1H), 9.05 (s, 1H).

### b) 2-Chloro-N⁴-[(1S,2R)-2-methylcyclohexyl]pyrimidine-4,5-diamine

Obtained as an off-white solid (95%) from 2-chloro-*N*-[(1*S*,2*R*)-2-methylcyclohexyl]-5-nitropyrimidin-4-amine (**Preparation 7a**) following the experimental procedure as described in **Preparation 5b**.
LRMS (m/z): 241 (M+1)⁺.
1H NMR (300 MHz, CDCl₃) δ ppm 0.88 (d, 3H), 1.20 - 1.40 (m, 2H), 1.42 - 1.53 (m, 2H), 1.53 - 1.66 (m, 3H), 1.67 - 1.80 (m, 1H), 1.94 - 2.03 (m, 1H), 3.03 (br s, 2H), 4.24 - 4.38 (m, 1H), 5.21 (d, 1H), 7.58 (s, 1H).

### c) 2-Chloro-9-[(1S,2R)-2-methylcyclohexyl]-7,9-dihydro-8H-purin-8-one

Obtained as a white solid (81%) from 2-chloro-*N*⁴-[(1*S*,2*R*)-2-methylcyclohexyl]pyrimidine-4,5-diamine (**Preparation 7b**) following the experimental procedure as described in **Preparation 2c**.
LRMS (m/z): 267 (M+1)⁺.
1H NMR (300 MHz, DMSO-*d*₆) δ ppm 0.90 (d, 3H), 1.23 - 1.55 (m, 3H), 1.55 - 1.72 (m, 3H), 1.87 (d, 1H), 2.19 (td, 1H), 2.80 (qd, 1H), 4.22 - 4.35 (m, 1H), 8.11 (s, 1H), 11.61 (s, 1H).

### d) 2-Chloro-9-[(1S,2R)-2-methylcyclohexyl]-7-{[2-(trimethylsilyl)ethoxy]methyl}-7,9-dihydro-8H-purin-8-one

Obtained as an oil (69%) from 2-chloro-9-[(1*S*,2*R*)-2-methylcyclohexyl]-7,9-dihydro-8*H-*purin-8-one (**Preparation 7c**) following the experimental procedure as described in **Preparation 3.**
LRMS (m/z): 397 (M+1)⁺.
1H NMR (300 MHz, CDCl₃) δ ppm -0.02 (s, 9H), 0.87 - 0.96 (m, 2H), 0.99 (d, 3H), 1.33 - 1.46 (m, 1H), 1.47 - 1.57 (m, 2H), 1.62 - 1.81 (m, 2H), 1.90 - 2.02 (m, 1H), 2.34 (q, 1H), 2.98 (qd,1H), 3.52 - 3.64 (m, 2H), 4.50 (dt, 1H), 5.30 (s, 2H), 8.15 (s, 1H).

### PREPARATION 8

### 3-(9-((1S,2R)-2-Methylcyclohexyl)-8-oxo-7-((2-(trimethylsilyl)ethoxy)methyl)-8,9-dihydro-7H-purin-2-yl)imidazo[1,2-a]pyridine-6-carbonitrile

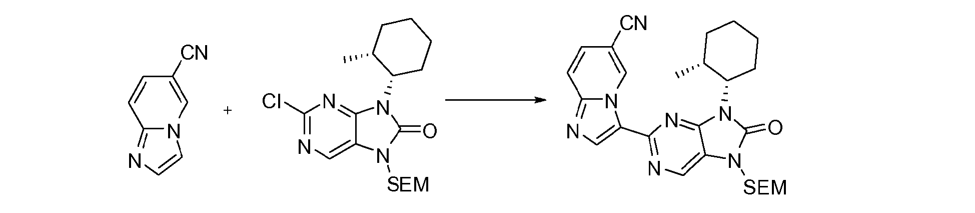

Obtained as a white solid (81%) from 2-chloro-9-[(1*S*,2*R*)-2-methylcyclohexyl]-7-{[2-(trimethylsilyl)ethoxy]methyl}-7,9-dihydro-8*H*-purin-8-one (**Preparation 7d**) and imidazo[1,2-a]pyridine-6-carbonitrile (**Preparation 1**) following the experimental procedure as described in **Preparation 4**.
LRMS (m/z): 504 (M+1)⁺.
1H NMR (300 MHz, CDCl₃) δ ppm -0.01 (s, 9H), 0.95 (d, 2H), 1.07 (d, 3H), 1.17 - 1.36 (m, 2H), 1.38 - 1.92 (m, 5H), 2.41 (dd, 1H), 2.87 - 3.20 (m, 1H), 3.64 (t, 2H), 4.49 - 4.68 (m, 1H), 5.37 (s, 2H), 7.43 (dd, 1H), 7.82 (d, 1H), 8.41 (s, 1H), 8.62 (s, 1H), 10.51 (s, 1H).

### PREPARATION 9

### 2-Chloro-9-[(4R)-8-fluoro-3,4-dihydro-2H-chromen-4-yl]-7-{[2-(trimethylsilyl) ethoxy]methyl}-7,9-dihydro-8H-purin-8-one

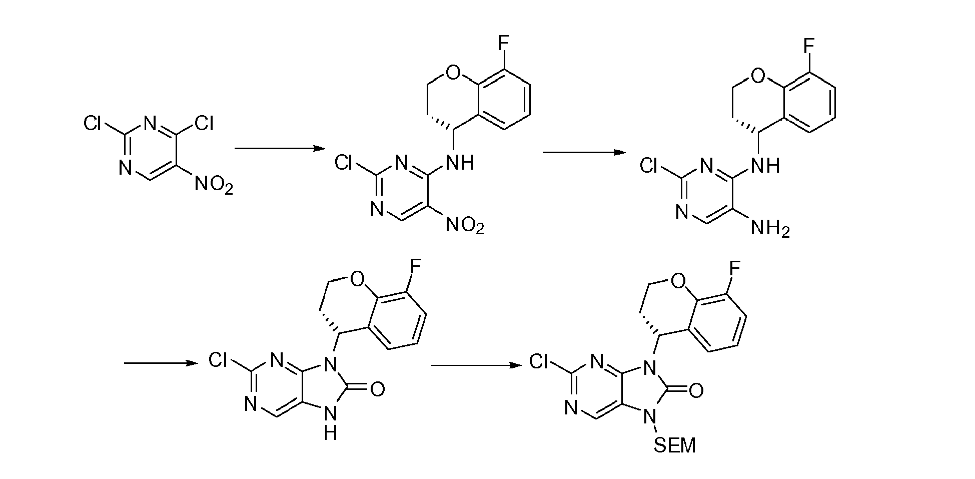

### a) 2-Chloro-N-[(4R)-8-fluoro-3,4-dihydro-2H-chromen-4-yl]-5-nitropyrimidin-4-amine

Obtained as an orange solid (100%) from 2,4-dichloro-5-nitropyrimidine and (R)-8-fluorochroman-4-amine hydrochloride (WO2006/108103 A1) following the experimental procedure as described in **Preparation 2a**.
LRMS (m/z): 323 (M-1)⁺.
1H NMR (300 MHz, CDCl₃) δ ppm 2.19 - 2.29 (m, 1H), 2.43 (ddd, 1H), 4.28 - 4.38 (m, 1H), 4.40 - 4.50 (m, 1H), 5.58 - 5.71 (m, 1H), 6.83 - 6.92 (m, 1H), 6.97 - 7.03 (m, 1H), 7.04 - 7.12 (m, 1H), 8.57 (d, 1H), 9.12 (s, 1H).

### b) 2-Chloro-N⁴-[(4R)-8-fluoro-3,4-dihydro-2H-chromen-4-yl]pyrimidine-4,5-diamine

Obtained as a yellow solid (100%) from 2-chloro-*N*-[(4R)-8-fluoro-3,4-dihydro-2*H-*chromen-4-yl]-5-nitropyrimidin-4-amine (**Preparation 9a**) following the experimental procedure as described in **Preparation 2b**.
LRMS (m/z): 293 (M-1)⁺.
1H NMR (300 MHz, CDCl₃) δ ppm 2.09 - 2.39 (m, 2H), 4.25 - 4.47 (m, 2H), 5.38 - 5.51 (m, 1H), 6.61 (br s, 1H), 6.82 (td, 1H), 6.95 - 7.07 (m, 2H), 7.64 (s, 1H).

### c) 2-Chloro-9-[(4R)-8-fluoro-3,4-dihydro-2H-chromen-4-yl]-7,9-dihydro-8H-purin-8-one

Obtained as a yellow solid (77%) from 2-chloro-*N*⁴-[(4R)-8-fluoro-3,4-dihydro-2*H-*chromen-4-yl]pyrimidine-4,5-diamine (**Preparation 9b**) following the experimental procedure as described in **Preparation 2c**.
LRMS (m/z): 321 (M+1)⁺.
1H NMR (300 MHz, CDCl₃) δ ppm 2.27 - 2.36 (m, 1H), 2.94 - 3.06 (m, 1H), 4.38 (dt, 1H), 4.65-4.72 (m, 1H), 5.89 (m, 1H), 6.56 (dd, 1H), 6.75 (m, 1H), 7.02 (m, 1H), 8.19 (s, 1H), 9.44 (s, 1H).

### d) 2-Chloro-9-[(4R)-8-fluoro-3,4-dihydro-2H-chromen-4-yl]-7-{[2-(trimethylsilyl) ethoxy]methyl}-7,9-dihydro-8H-purin-8-one

Obtained as a pale yellow solid (100%) from 2-chloro-9-[(4*R*)-8-fluoro-3,4-dihydro-2*H-*chromen-4-yl]-7,9-dihydro-8*H*-purin-8-one (**Preparation 9c**) and (2-(chloromethoxy)ethyl)trimethylsilane following the experimental procedure as described **in Preparation 3**.
LRMS (m/z): 451 (M+1)⁺.
1H NMR (300 MHz, CDCl₃) δ ppm -0.02 (s, 9H), 0.85 - 1.02 (m, 2H), 2.22 - 2.39 (m, 1H), 2.87 - 3.05 (m, 1H), 3.51 - 3.61 (m, 2H), 4.24 - 4.46 (m,1H), 4.61 - 4.75 (m, 1H), 5.31 (s, 2H), 5.78 - 5.93 (m, 1H), 6.52 (m, 1H), 6.72 (m, 1H), 7.01 (m, 1H), 8.23 (s, 1H).

### PREPARATION 10

### (R)-3-(9-(8-Fluorochroman-4-yl)-8-oxo-7-((2-(trimethylsilyl)ethoxy)methyl)-8,9-dihydro-7H-purin-2-yl)imidazo[1,2-a]pyridine-6-carbonitrile

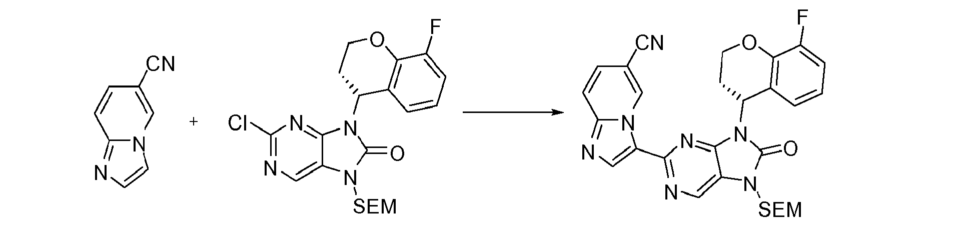

Obtained (44%) from 2-chloro-9-[(4*R*)-8-fluoro-3,4-dihydro-2*H*-chromen-4-yl]-7-{[2-(trimethylsilyl) ethoxy]methyl}-7,9-dihydro-8*H*-purin-8-one (**Preparation 9**) and imidazo[1,2-a]pyridine-6-carbonitrile (**Preparation 1**) following the experimental procedure as described in **Preparation 4**.
LRMS (m/z): 558 (M+1)⁺.
1H NMR (300 MHz, CDCl₃) δ ppm 0.02 (s, 9H), 0.98 (t, 2H), 2.39 - 2.46 (m, 1H), 2.88 - 2.99 (m , 1H), 3.66 (t, 2H), 4.45 (dt, 1H), 4.69 - 4.76 (m, 1H), 5.40 (s, 2H), 5.92 (t, 1H), 6.62 - 6.74 (m, 2H), 7.02 (t, 1H), 7.41 (dd, 1H), 7.77 (dd, 1H), 8.38 (s, 1H), 8.45 (s, 1H), 10.37 (s, 1H).

### PREPARATION 11

### 2-Chloro-7-methyl-9-(tetrahydro-2H-pyran-4-yl)-7,9-dihydro-8H-purin-8-one

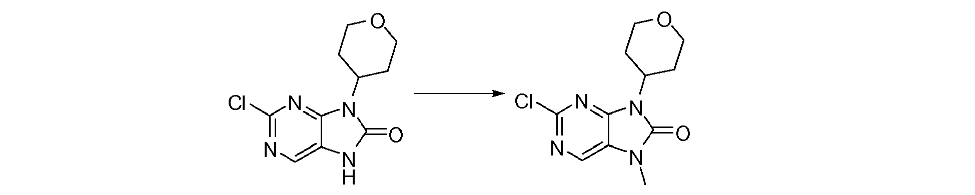

Sodium hydride (60% dispersion in mineral oil, 0.016 g, 0.40 mmol) was added portion wise to a cold (0 °C) solution of 2-chloro-9-(tetrahydro-2*H*-pyran-4-yl)-7,9-dihydro-8*H-*purin-8-one (**Preparation 2c**, 0.090 g, 0.35 mmol) in N,N'-dimethylformamide (2 mL) and the reaction mixture was stirred at 0 °C for a further 15 minutes. Methyl iodide (0.026 mL, 0.42 mmol) was then added and the mixture was stirred at 0 °C for 90 minutes. Water was added and the mixture was extracted with ethyl acetate. The organic layer was washed with water and brine, dried (MgSO₄ and evaporated to give the title compound (0.075 g, 79%) as a white solid.
LRMS (m/z): 269 (M+1)⁺.

### PREPARATION 12

### 9-Benzyl-2-chloro-7-methyl-7,9-dihydro-8H-purin-8-one

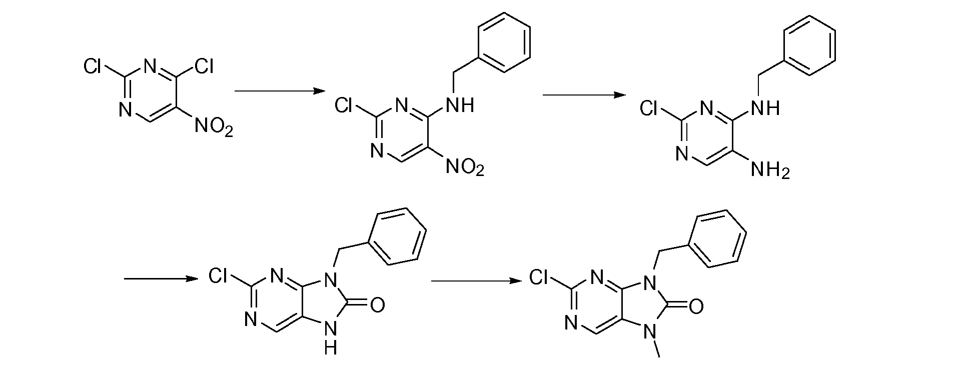

### a) N-Benzyl-2-chloro-5-nitropyrimidin-4-amine

Obtained as a pale yellow solid (79%) from 2,4-dichloro-5-nitropyrimidine and phenylmethanamine following the experimental procedure as described in **Preparation 2a**.
LRMS (m/z): 265 (M+1)⁺.

### b) N⁴-Benzyl-2-chloropyrimidine-4,5-diamine

Obtained (94%) from *N*-benzyl-2-chloro-5-nitropyrimidin-4-amine (**Preparation 12a**) following the experimental procedure as described in **Preparation 5b**.
LRMS (m/z): 235 (M+1)⁺.

### c) 9-Benzyl-2-chloro-7,9-dihydro-8H-purin-8-one

Obtained as a beige solid (90%) from *N*⁴-benzyl-2-chloropyrimidine-4,5-diamine (**Preparation 12b**) following the experimental procedure as described in **Preparation 2c**.
LRMS (m/z): 261 (M+1)⁺.

### d) 9-Benzyl-2-chloro-7-methyl-7,9-dihydro-8H-purin-8-one

Obtained as a beige solid (95%) from 9-benzyl-2-chloro-7,9-dihydro-8*H*-purin-8-one (**Preparation 12c**) and methyl iodide following the experimental procedure as described in **Preparation 11**.
LRMS (m/z): 275 (M+1)⁺.

### PREPARATION 13

### 2-Chloro-7-(2-morpholin-4-ylethyl)-9-(tetrahydro-2H-pyran-4-yl)-7,9-dihydro-8H-purin-8-one

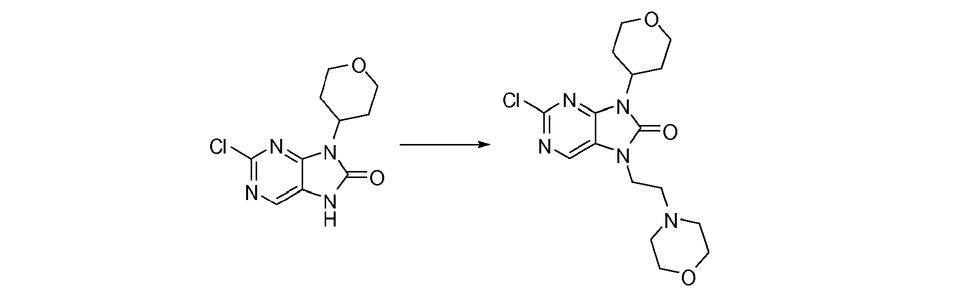

4-(2-Chloroethyl)morpholine hydrochloride (0.105 g, 0.57 mmol) and potassium carbonate (0.326 g, 2.36 mmol) were added to a stirred solution of 2-chloro-9-(tetrahydro-2*H*-pyran-4-yl)-7,9-dihydro-8*H*-purin-8-one (**Preparation 2c**, 0.120 g, 0.47 mmol) in N,N'-dimethylformamide (4 mL) and the mixture was stirred and heated to 90 °C. After 2 hours, the reaction mixture was cooled and ethyl acetate was added and the organic layer was washed with water (x3), brine, dried (MgSO₄ and the solvent was evaporated to give the title compound (0.100 g, 58%) as a pale brown solid.
LRMS (m/z): 368 (M+1)⁺.
1H NMR (300 MHz, CDCl₃) δ ppm 1.75 (m, 2H), 2.5 (m, 4H), 2.60 - 2.80 (m, 4H), 3.45-3.75 (m, 6H), 3.98 (m, 2H), 4.05 (m, 2H), 4.58 (m, 1H), 8.10 (s, 1H).

### PREPARATION 14

### 2-Chloro-7-[2-(dimethylamino)ethyl]-9-(tetrahydro-2H-pyran-4-yl)-7,9-dihydro-8H-purin-8-one

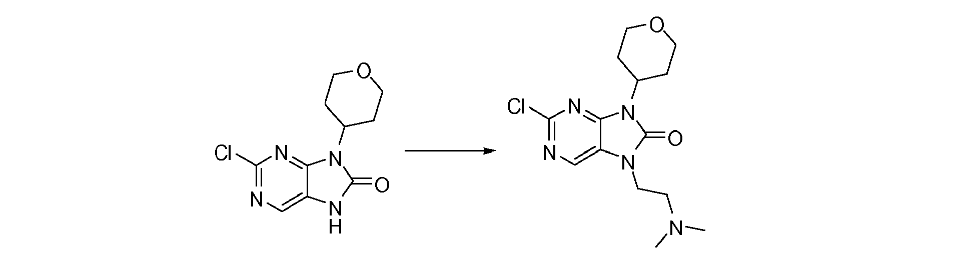

Obtained as a white solid (45%) from 2-chloro-9-(tetrahydro-2*H*-pyran-4-yl)-7,9-dihydro-8H-purin-8-one (**Preparation 2c**) and 2-chloro-*N,N*-dimethylethanamine hydrochloride following the experimental procedure as described in **Preparation 13**.
LRMS (m/z): 326 (M+1)⁺.
1H NMR (300 MHz, DMSO-*d*₆) δ ppm 1.68 (dd, 2H), 2.15 (s, 6H), 2.33 - 2.60 (m, 4H), 3.27 - 3.53 (m, 4H), 3.94 (d, 2H), 4.35 - 4.54 (m, 1H), 8.41 (s, 1H).

### PREPARATION 15

### 2-Chloro-9-(tetrahydro-2H-pyran-4-yl)-7-[2-(tetrahydro-2H-pyran-2-yloxy)ethyl]-7,9-dihydro-8H-purin-8-one

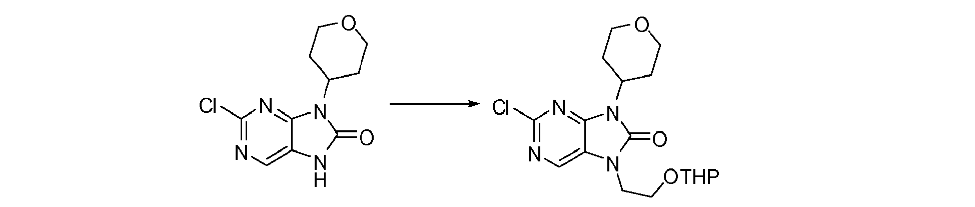

Obtained as an orange solid (90%) from 2-chloro-9-(tetrahydro-2*H*-pyran-4-yl)-7,9-dihydro-8H-purin-8-one (**Preparation 2c**) and 2-(2-bromoethoxy)tetrahydro-2H-pyran following the experimental procedure as described in **Preparation 13**.
LRMS (m/z): 383 (M+1)⁺.
1H NMR (300 MHz, DMSO-*d*₆) δ ppm 1.25 - 1.56 (m, 6H), 1.68 (d, 2H), 2.35 - 2.48 (m, 2H), 3.45 (t, 4H), 3.58 - 3.68 (m, 1H), 3.78 - 3.89 (m, 1H), 3.97 (d, 2H), 4.01 - 4.18 (m, 2H), 4.41 - 4.58 (m, 2H), 8.40 (s, 1H).

### PREPARATION 16

### 2-Chloro-7-(2-hydroxy-2-methylpropyl)-9-(tetrahydro-2H-pyran-4-yl)-7,9-dihydro-8H-purin-8-one

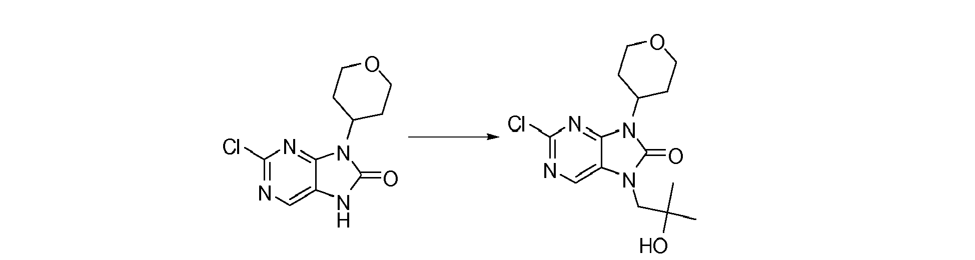

Obtained as a brown solid (70%) from 2-chloro-9-(tetrahydro-2*H*-pyran-4-yl)-7,9-dihydro-8*H*-purin-8-one (**Preparation 2c**) and 1-chloro-2-methylpropan-2-ol following the experimental procedure as described in **Preparation 13**.
LRMS (m/z): 327 (M+1)⁺.
1H NMR (300 MHz, CDCl₃) δ ppm 1.33 (s, 6H), 1.54 (s, 1H), 1.74 (dd, 2H), 2.67 - 2.86 (m, 2H), 3.46 - 3.63 (m, 2H), 3.85 (s, 2H), 4.08 - 4.19 (m, 2H), 4.53 - 4.68 (m, 1H), 8.25(s, 1H).

### PREPARATION 17

### 2-Chloro-7-{[(4S)-2,2-dimethyl-1,3-dioxolan-4-yl]methyl}-9-(tetrahydro-2H-pyran-4-yl)-7,9-dihydro-8H-purin-8-one

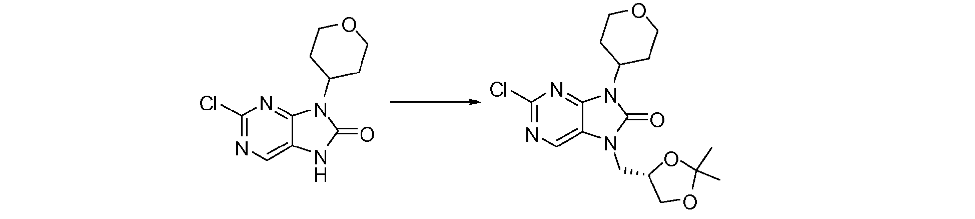

(*S*)-4-(Chloromethyl)-2,2-dimethyl-1,3-dioxolane (0.077 mL, 0.56 mmol) was added to a suspension of 2-chloro-9-(tetrahydro-2*H*-pyran-4-yl)-7,9-dihydro-8*H*-purin-8-one (**Preparation** 2c, 0.120 g, 0.47 mmol) and potassium carbonate (0.130 g, 0.94 mmol) in *N,N*'-dimethylformamide (2 mL) and the reaction mixture was stirred and heated to 90 °C in a sealed tube. After 5 hours, further (*S*)-4-(chloromethyl)-2,2-dimethyl-1,3-dioxolane (0.231 mL, 1.68 mmol) was added and the mixture was stirred at 90 °C for 3 days. Water was added and the mixture was extracted with ethyl acetate and the organic layer was dried (MgSO₄ and evaporated. The residue was treated with hexane, the hexane was discarded and the oily residue was treated with Et₂O and evaporated to give the title compound (0.090 g, 52%) as a foam.
LRMS (m/z): 369 (M+1)⁺.
1H NMR (300 MHz, CDCl₃) δ ppm 1.32 (s, 3H), 1.35 (s, 3H), 1.73 (d, 2H), 2.74 (dd, 2H), 3.41 - 3.63 (m, 2H), 3.69 - 3.81 (m, 1H), 3.83 - 3.95 (m, 1H), 4.03 - 4.21 (m, 4H), 4.37 - 4.50 (m, 1H), 4.52 - 4.70 (m, 1H), 8.25 (s, 1H).

### PREPARATION 18

### 2-Chloro-7-{[(4R)-2,2-dimethyl-1,3-dioxolan-4-yl]methyl}-9-(tetrahydro-2H-pyran-4-yl)-7,9-dihydro-8H-purin-8-one

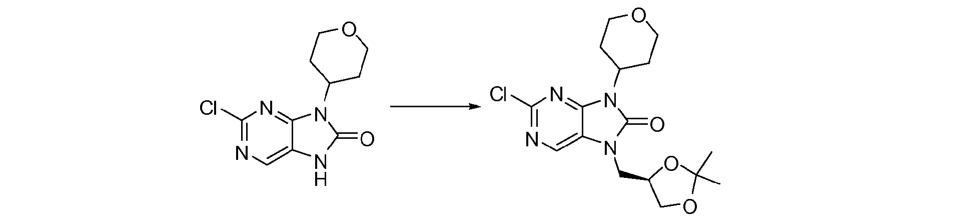

Obtained as a foam (67%) from 2-chloro-9-(tetrahydro-2*H*-pyran-4-yl)-7,9-dihydro-8*H-*purin-8-one (**Preparation 2c**) and (*R*)-4-(chloromethyl)-2,2-dimethyl-1,3-dioxolane following the experimental procedure as described in **Preparation 17**.
LRMS (m/z): 369 (M+1)⁺.
1H NMR (300 MHz, CDCl₃) δ ppm 1.32 (s, 3H), 1.35 (s, 3H), 1.73 (d, 2H), 2.74 (dd, 2H), 3.41 - 3.63 (m, 2H), 3.69 - 3.81 (m, 1H), 3.83 - 3.95 (m, 1H), 4.03 - 4.21 (m, 4H), 4.37 - 4.50 (m, 1H), 4.52 - 4.70 (m, 1H), 8.25 (s, 1H).

### PREPARATION 19

### 2-Chloro-9-(tetrahydro-2H-pyran-4-yl)-7-{2-[(2,2,2-trifluoroethyl)amino]ethyl}-7,9-dihydro-8H-purin-8-one

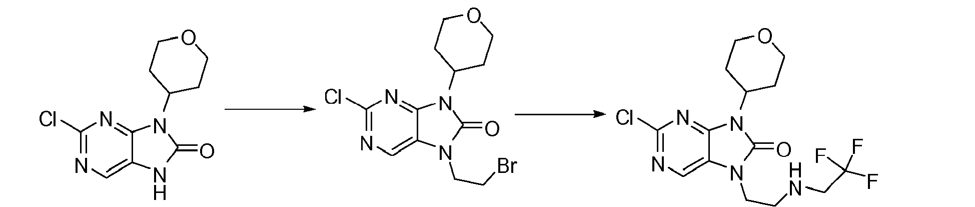

### a) 7-(2-Bromoethyl)-2-chloro-9-(tetrahydro-2H-pyran-4-yl)-7,9-dihydro-8H-purin-8-one

Obtained as a white solid (29%) from 2-chloro-9-(tetrahydro-2*H*-pyran-4-yl)-7,9-dihydro-8*H*-purin-8-one (**Preparation 2c**) and 1,2-dibromoethane following the experimental procedure as described in **Preparation 13** followed by purification of the crude product by flash chromatography (98:2 dichloromethane/methanol).
LRMS (m/z): 361/363 (M+1)⁺.
1H NMR (300 MHz, CDCl₃) δ ppm 1.73 (d, 2H), 2.73 (qd, 2H), 3.53 (t, 2H), 3.69 (t, 2H), 4.13 (dd, 2H), 4.30 (t, 2H), 4.51 - 4.68 (m, 1H), 8.16 (s, 1H).

### b) 2-Chloro-9-(tetrahydro-2H-pyran-4-yl)-7-{2-[(2,2,2-trifluoroethyl)amino]ethyl}-7,9-dihydro-8H-purin-8-one

Triethylamine (0.027 mL, 0.19 mmol) was added to a solution of 7-(2-bromoethyl)-2-chloro-9-(tetrahydro-2*H*-pyran-4-yl)-7,9-dihydro-8H-purin-8-one (**Preparation 19a**, 0.060 g, 0.17 mmol) and 2,2,2-trifluoroethanamine (0.019 mL, 0.24 mmol) in *N,N*'-dimethylformamide (1 mL) and the mixture was stirred and heated to 80 °C. After stirring overnight, further 2,2,2-trifluoroethanamine (0.038 mL, 0.48 mmol) was added and the reaction was stirred at 80 °C for a further 48 hours. Ethyl acetate was then added and the organic layer was washed with water (x 3), brine, dried (MgSO₄) and evaporated to give the title compound (0.047 g, 75%) as a pale yellow solid.
LRMS (m/z): 380 (M+1)⁺.

### PREPARATION 20

### 2-Chloro-7-(2,4-dimethoxybenzyl)-9-(tetrahydro-2H-pyran-4-yl)-7,9-dihydro-8H-purin-8-one

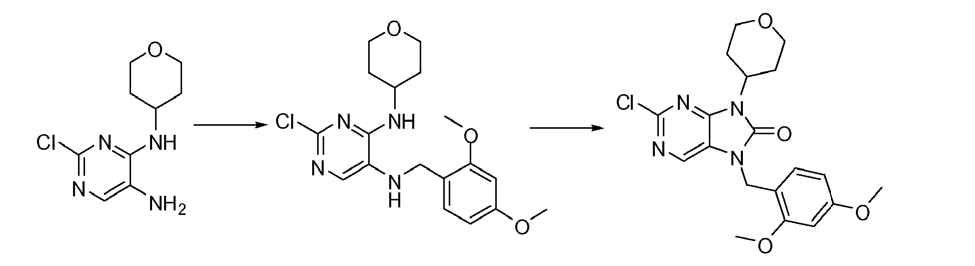

### a) 2-Chloro-N⁵-(2,4-dimethoxybenzyl)-N⁴-(tetrahydro-2H-pyran-4-yl)pyrimidine-4,5-diamine

Acetic acid (0.52 mL, 9.2 mmol) and sodium triacetoxyborohydride (1.80 g, 8.5 mmol) were added to a stirred solution of 2-chloro-*N*⁴-(tetrahydro-2*H*-pyran-4-yl)pyrimidine-4,5-diamine (**Preparation 2b**, 0.700 g, 3.1 mmol) and 2,4-dimethoxybenzaldehyde (0.61 g, 3.7 mmol) in dichloromethane (30 mL). After stirring at room temperature overnight, water and saturated aqueous potassium carbonate were added and the organic layer was separated and the aqueous layer was extracted with dichloromethane. The combined organic extract was dried (MgSO₄ and evaporated and the residue was purified by flash chromatography (98:2 dichloromethane/methanol) to give the title compound (0.82 g, 71 %) as a white solid.
LRMS (m/z): 379 (M+1)⁺.
1H NMR (300 MHz, CDCl₃) δ ppm 1.53 (dd, 2H), 2.03 (dt, 2H), 3.56 (t, 2H), 3.82 (s, 3H), 3.85 (s, 3H), 3.94 - 4.04 (m, 2H), 4.07 (d, 2H), 4.16 - 4.31 (m, 1H), 5.10 (d, 1H), 6.46 (d, 1H), 6.51 (s, 1H), 7.12 (d, 1H), 7.26 (s, 1H), 7.55 (s, 1H).

### b) 2-Chloro-7-(2,4-dimethoxybenzyl)-9-(tetrahydro-2H-pyran-4-yl)-7,9-dihydro-8H-purin-8-one

Triphosgene (0.440 g, 1.45 mmol) and triethylamine (1.30 mL, 10.15 mmol) were added to a stirred solution of 2-chloro-*N*⁵-(2,4-dimethoxybenzyl)-*N*⁴-(tetrahydro-2*H-*pyran-4-yl)pyrimidine-4,5-diamine (**Preparation 20a**, 0.550 g, 1.45 mmol) in tetrahydrofuran (20 mL). After stirring for 3 days, dichloromethane and saturated aqueous sodium hydrogencarbonate were added and the organic layer was separated, washed with brine, dried (MgSO₄) and evaporated. The residue was purified by flash chromatography (99:1 dichloromethane/methanol) to give the title compound (0.504 g, 78%) as a white solid.
LRMS (m/z): 405 (M+1)⁺.
1H NMR (300 MHz, CDCl₃) δ ppm 1.71 (d, 2H), 2.66 - 2.83 (m, 2H), 3.47 - 3.61 (m, 2H), 3.80 (s, 3H), 3.83 (s, 3H), 4.13 (d, 2H), 4.50 - 4.66 (m, 1H), 4.97 (s, 2H), 6.37 - 6.52 (m, 2H), 7.33 (d, 1H), 8.02 (s, 1H).

### PREPARATION 21

### 6-Fluoroimidazo[1,2-a]pyridine

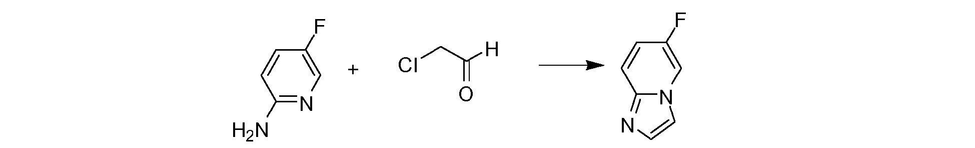

Obtained as a brown solid (100%) from 2-chloroacetaldehyde and 5-fluoropyridin-2-amine following the experimental procedure as described in **Preparation 1**.
LRMS (m/z): 137 (M+1)⁺.
1H NMR (300 MHz, CDCl₃) δ ppm 7.10 (ddd, 1H), 7.57 - 7.63 (m, 2H), 7.68 (s, 1H), 8.08(d, 1H).

### PREPARATION 22

### 2-(6-Fluoroimidazo[1,2-a]pyridin-3-yl)-9-(tetrahydro-2H-pyran-4-yl)-7-((2-(trimethylsilyl)ethoxy)methyl)-7H-purin-8(9H)-one

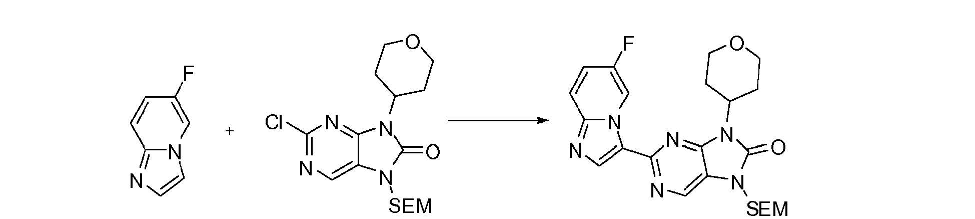

Obtained as a white solid (35%) from 2-chloro-9-(tetrahydro-2*H*-pyran-4-yl)-7-{[2-(trimethylsilyl)ethoxy]methyl}-7,9-dihydro-8H-purin-8-one (**Preparation 3**) and 6-fluoroimidazo[1,2-a]pyridine (**Preparation 21**) following the experimental procedure as described in **Preparation 4** followed by purification of the crude product by reverse phase chromatography (C-18 silica from Waters@, water/acetonitrile/methanol as eluents [0.1 % v/v formic acid buffered] 0% to 100%).
LRMS (m/z): 485 (M+1)⁺.
1H NMR (300 MHz, CDCl₃) δ ppm 0.00 (s, 9H), 0.98 (d, 2H), 1.82 (dd, 2H), 2.76 - 2.99 (m, 2H), 3.50 - 3.72 (m, 4H), 4.20 (dd, 2H), 4.57 - 4.77 (m, 1H), 5.36 (s, 2H), 7.22 - 7.33 (m, 1H), 7.73 (dd, 1H), 8.42 (s, 1H), 8.61 (s, 1H), 9.97 (dd, 1H).

### PREPARATION 23

### 9-Cyclohexyl-2-(6-fluoroimidazo[1,2-a]pyridin-3-yl)-7-((2-(trimethylsilyl)ethoxy)methyl)-7H-purin-8(9H)-one

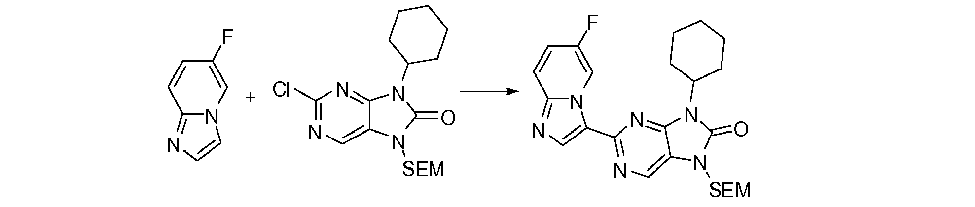

Obtained as a white solid (60%) from 2-chloro-9-cyclohexyl-7-{[2-(trimethylsilyl)ethoxy]methyl}-7,9-dihydro-8H-purin-8-one (**Preparation 5**) and 6-fluoroimidazo[1,2-a]pyridine (**Preparation 21**) following the experimental procedure as described in **Preparation 4** followed by purification of the crude product by flash chromatography (100:1 dichloromethane/methanol).
LRMS (m/z): 485 (M+1)⁺.

### PREPARATION 24

### (R)-9-(8-Fluorochroman-4-yl)-2-(6-fluoroimidazo[1,2-a]pyridin-3-yl)-7-((2-(trimethylsilyl)ethoxy)methyl)-7H-purin-8(9H)-one

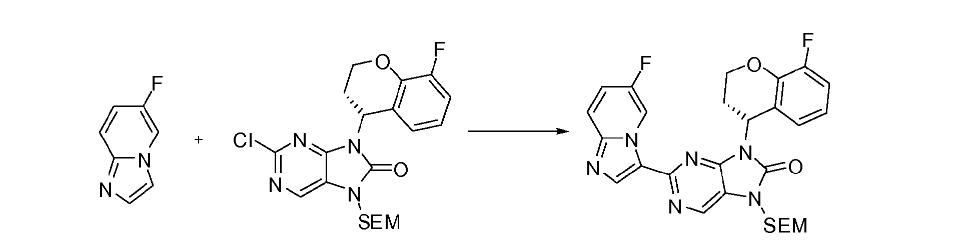

Obtained (43%) from 2-chloro-9-[(4*R*)-8-fluoro-3,4-dihydro-2*H*-chromen-4-yl]-7-{[2-(trimethylsilyl) ethoxy]methyl}-7,9-dihydro-8*H*-purin-8-one (**Preparation 9**) and 6-fluoroimidazo[1,2-a]pyridine (**Preparation 21**) following the experimental procedure as described in **Preparation 4**.
LRMS (m/z): 551 (M+1)⁺.
1H NMR (300 MHz, CDCl₃) δ ppm 0.01 (s, 9H), 1.00 (d, 2H), 1.18 - 1.34 (m, 1H), 2.37 (dt, 1H), 2.87 - 3.07 (m, 1H), 3.67 (dd, 2H), 4.37 - 4.52 (m, 1H), 4.64 - 4.81 (m, 1H), 5.41 (s, 2H), 5.94 (dd, 1H), 6.58 - 6.78 (m, 1H), 6.96 - 7.09 (m, 1H), 7.28 - 7.37 (m, 1H), 7.76 (dd, 1H), 8.40 (s, 1H), 8.46 (s, 1H), 9.42 (d, 1H).

### PREPARATION 25

### 3-(4-Chloro-5-nitropyrimidin-2-yl)-6-fluoroimidazo[1,2-a]pyridine

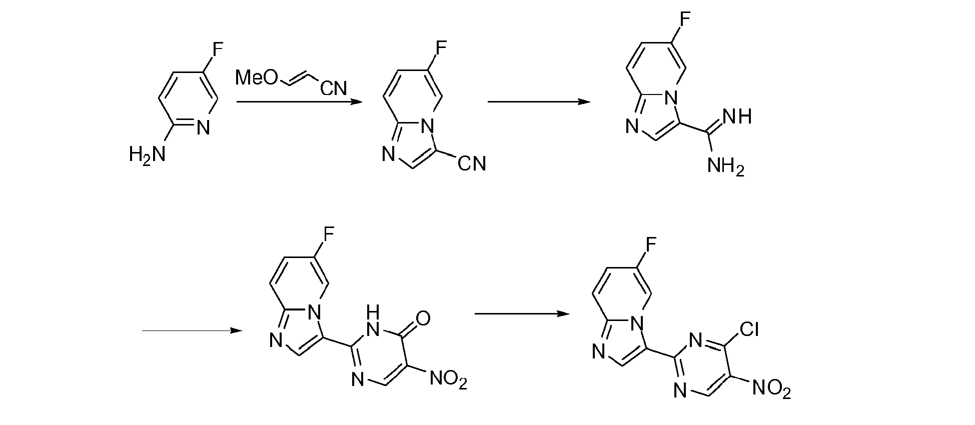

### a) 6-Fluoroimidazo[1,2-a]pyridine-3-carbonitrile

*N*-bromosuccinimide (15.88 g, 89.20 mmol) was added portion wise to a cooled (0 °C), stirred solution of methoxyacrylonitrile (7.50 mL, 89.20 mmol) in 3:1 dioxane/water (600 mL). The mixture was stirred at 0 °C for a further 30 minutes and then 2-amino-5-fluoropyridine (5.00 g, 44.60 mmol) was added. The mixture was warmed to room temperature, stirred for 2 hours and then heated to 60 °C and stirring was continued at this temperature for 16 hours. The solvent was evaporated under vacuum and saturated aqueous sodium hydrogencarbonate was added. The aqueous layer was extracted with ethyl acetate (x3) and the combined organic extract was dried (Na₂SO₄), filtered and the solvent was evaporated. The residue was dissolved in methanol and purified by flash chromatography (hexanes/ethyl acetate) to give the title compound (6.80 g, 94%) as a brown solid.
LRMS (m/z): 162 (M+1)⁺.
1H NMR (300 MHz, CDCl₃) δ ppm 7.39 (m, 1H), 7.78 (dd, 1H), 8.18 (s, 1H), 8.39 (t, 1H).

### b) 6-Fluoroimidazo[1,2-a]pyridine-3-carboximidamide

Sodium methoxide (0.53 g, 9.87 mmol) was added to a stirred suspension of 6-fluoroimidazo[1,2-*a*]pyridine-3-carbonitrile (**Preparation 25a**, 15.9 g, 98.67 mmol) in methanol (200 mL) and the resulting mixture was stirred at room temperature for 3 days. Ammonium chloride (5.8 g, 108.5 mmol) was added and the mixture was heated to reflux and stirred for 24 hours. The solvent was evaporated and the resulting solid was triturated with ethyl acetate to give the title compound (14.3 g, 81%) as a brown solid.
LRMS (m/z): 179 (M+1)⁺.
1H NMR (300 MHz, DMSO-d₆) δ ppm 7.68 (m , 1H), 7.87 (dd, 1H), 8.33 (s, 1H), 8.87 (m, 1H).

### c) 2-(6-Fluoroimidazo[1,2-a]pyridin-3-yl)-5-nitropyrimidin-4(3H)-one

A mixture of 6-fluoroimidazo[1,2-*a*]pyridine-3-carboximidamide (Preparation 25b, 1.03 g, 5.75 mmol), (Z)-ethyl 3-(dimethylamino)-2-nitroacrylate (3.0 g, 15.9 mmol) and triethylamine (4.8 mL, 34.4 mmol) in ethanol (15 mL) was stirred and heated to 90 °C in a sealed tube. After 22 hours the solvent was evaporated, water was added and the mixture was extracted with chloroform. The combined organic extract was dried (MgSO₄) and concentrated to give a semi-solid which was treated with aqueous saturated potassium carbonate solution to give a solid which was filtered, washed with water and dried to give the title compound (1.23 g, 78%) as a solid.
LRMS (m/z): 274 (M-1)⁺.
1H NMR (300 MHz, DMSO-*d*₆) δ ppm 7.48 - 7.61 (m, 2H), 7.74 - 7.88 (m, 1H), 8.39 (br s, 1H), 8.79 (br s, 1H), 10.19 (brs, 1H).

### d) 3-(4-Chloro-5-nitropyrimidin-2-yl)-6-Fluoroimidazo[1,2-a]pyridine

Phosphorous oxychloride (10 mL) was added to 2-(6-fluoroimidazo[1,2-a]pyridin-3-yl)-5-nitropyrimidin-4(3*H*)-one (**Preparation 25c,** 1.12 g, 4.07 mmol) and the suspension was stirred and heated to 90 °C in a sealed tube. After 2 hours, the mixture was evaporated and taken up in dichloromethane and then neutralized to pH 7 by vigorous stirring with an aqueous sodium hydrogencarbonate solution. The organic layer was separated and the aqueous layer was extracted with further dichloromethane. The combined organic extract was dried (MgSO₄) and evaporated to give the title compound (0.81 g, 68%) as a yellow solid.
LRMS (m/z): 294 (M+1)⁺.
1H NMR (300 MHz, CDCl₃) δ ppm 7.47 (ddd, 1H), 7.84 (ddd, 1H), 8.84 (s, 1H), 9.35 (s, 1H), 9.83 (ddd, 1H).

### PREPARATION 26

### (S)-2-(6-Fluoroimidazo[1,2-a]pyridin-3-yl)-N⁴-(1-phenylethyl)pyrimidine-4,5-diamine

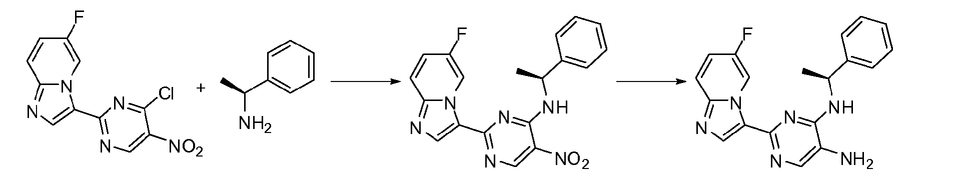

### a) (S)-2-(6-Fluoroimidazo[1,2-a]pyridin-3-yl)-5-nitro-N-(1-phenylethyl)pyrimidin-4-amine

Diisopropylethylamine (0.071 mL, 0.41 mmol) and (*S*)-1-phenylethanamine (0.046 mL, 0.36 mmol) were added to a stirred suspension of 3-(4-chloro-5-nitropyrimidin-2-yl)-6-fluoroimidazo[1,2-a]pyridine (Preparation 25, 0.10 g, 0.34 mmol) in tetrahydrofuran (1 mL) and the mixture was stirred at room temperature overnight. The solvent was evaporated and water was added. The mixture was filtered to give the title compound (0.101 g, 78%) as a yellow solid.
LRMS (m/z): 379 (M+1)⁺.
1H NMR (300 MHz, DMSO*-d*₆) δ ppm 1.68 (d, 3H), 5.46 - 5.66 (m, 1H), 7.23 (d, 1H), 7.33 (t, 2H), 7.54 (d, 2H), 7.59 - 7.71 (m, 1H), 7.87 (dd, 1H), 8.56 (s, 1H), 8.95 (d, 1H), 9.22 (s, 1H), 9.50 - 9.60 (m, 1H).

### b) (S)-2-(6-Fluoroimidazo[1,2-a]pyridin-3-yl)-N⁴-(1-phenylethyl)pyrimidine-4,5-diamine

Obtained as a pale brown solid (87%) from (S)-2-(6-fluoroimidazo[1,2-a]pyridin-3-yl)-5-nitro-N-(1-phenylethyl)pyrimidin-4-amine (**Preparation 26a**) following the experimental procedure as described in **Preparation 5b**.
LRMS (m/z): 349 (M+1)⁺.
1H NMR (300 MHz, CDCl₃) δ ppm 1.68 (d, 3H), 3.13 (br s, 2H), 5.31 (d, 1H), 5.34 - 5.45 (m, 1H), 7.11 - 7.21 (m, 1H), 7.23 - 7.30 (m, 1H), 7.32 - 7.42 (m, 2H), 7.43 - 7.52 (m, 2H), 7.56 - 7.68 (m, 1H), 7.90 (s, 1H), 8.37 (s, 1H), 9.68 - 9.77 (m, 1H).

### PREPARATION 27

### (R)-2-(6-Fluoroimidazo[1,2-a]pyridin-3-yl)-N4-(1-phenylethyl)pyrimidine-4,5-diamine

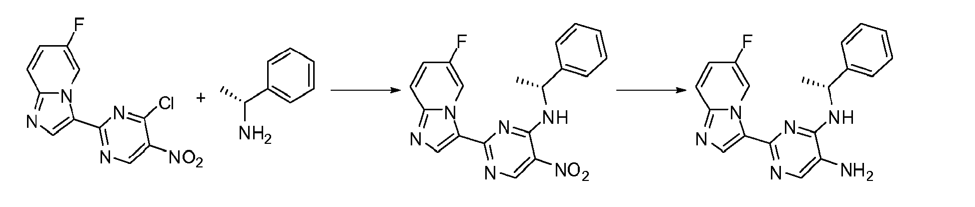

### a) (R)-2-(6-Fluoroimidazo[1,2-a]pyridin-3-yl)-5-nitro-N-(1-phenylethyl)pyrimidin-4-amine

Obtained as a yellow solid (87%) from 3-(4-chloro-5-nitropyrimidin-2-yl)-6-fluoroimidazo[1,2-a]pyridine (**Preparation 25**) and (*R*)-1-phenylethanamine following the experimental procedure as described in **Preparation 26a**.
LRMS (m/z): 379 (M+1)⁺.
1H NMR (300 MHz, DMSO-*d*₆) δ ppm 1.69 (d, 3H), 5.55 (m, 1H), 7.24 (d, 1H), 7.34 (t, 2H), 7.54 (d, 2H), 7.65 (ddd, 1H), 7.87 (dd, 1H), 8.56 (s, 1H), 8.95 (d, 1H), 9.22 (s, 1H), 9.53(dd, 1H).

### b) (R)-2-(6-Fluoroimidazo[1,2-a]pyridin-3-yl)-N4-(1-phenylethyl)pyrimidine-4,5-diamine

Obtained as a pale brown solid (91%) from (R)-2-(6-fluoroimidazo[1,2-a]pyridin-3-yl)-5-nitro-N-(1-phenylethyl)pyrimidin-4-amine (**Preparation 27a**) following the experimental procedure as described in **Preparation 5b**.
LRMS (m/z): 349 (M+1)⁺.
1H NMR (300 MHz, CDCl₃) δ ppm 1.67 (d, 3H), 3.13 (br s, 2H), 5.26 - 5.44 (m, 2H), 7.15 (ddd, 1H), 7.22 - 7.29 (m, 1H), 7.32 - 7.40 (m, 2H), 7.42 - 7.50 (m, 2H), 7.61 (dd, 1H), 7.89 (s, 1H), 8.36 (s, 1H), 9.71 (m, 1H).

### PREPARATION 28

### 2-(6-Fluoroimidazo[1,2-a]pyridin-3-yl)-N⁴-(pyridin-3-ylmethyl)pyrimidine-4,5-diamine

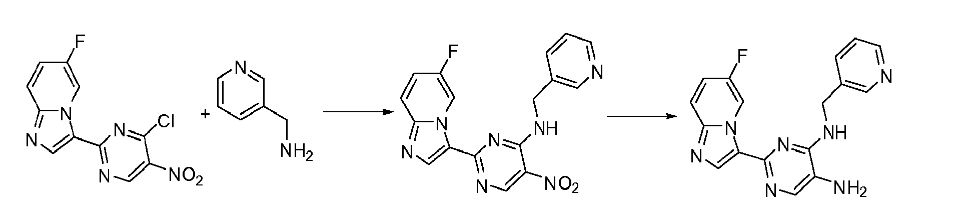

### a) 2-(6-Fluoroimidazo[1,2-a]pyridin-3-yl)-5-nitro-N-(pyridin-3-ylmethyl)pyrimidin-4-amine

Obtained as a yellow solid (75%) from 3-(4-chloro-5-nitropyrimidin-2-yl)-6-fluoroimidazo[1,2-a]pyridine (**Preparation 25**) and pyridin-3-ylmethanamine following the experimental procedure as described in **Preparation 26a**.
LRMS (m/z): 364 (M-1)⁺.
1H NMR (300 MHz, DMSO*-d*₆) δ ppm 4.96 (d, 2H), 7.33 (dd, 1H), 7.64 (dd, 1H), 7.80 - 7.94 (m, 2H), 8.42 (dd, 1H), 8.59 (s, 1H), 8.69 (s, 1H), 9.25 (s, 1H), 9.55 (dd, 1H), 9.65(m, 1H).

### b) 2-(6-Fluoroimidazo[1,2-a]pyridin-3-yl)-N⁴-(pyridin-3-ylmethyl)pyrimidine-4,5-diamine

Obtained as a pale yellow solid (96%) from 2-(6-fluoroimidazo[1,2-a]pyridin-3-yl)-5-nitro-*N*-(pyridin-3-ylmethyl)pyrimidin-4-amine (**Preparation 28a**) following the experimental procedure as described in **Preparation 5b**.
LRMS (m/z): 336 (M+1)⁺.

### PREPARATION 29

### 2-(6-Fluoroimidazo[1,2-a]pyridin-3-yl)-N⁴-(5,6,7,8-tetrahydroquinolin-5-yl)pyrimidine-4,5-diamine

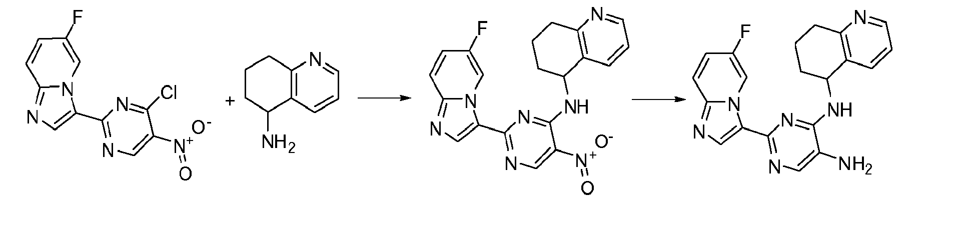

### a) N-(2-(6-Fluoroimidazo[1,2-a]pyridin-3-yl)-5-nitropyrimidin-4-yl)-5,6,7,8-tetrahydroquinolin-5-amine

Obtained as a yellow solid (51%) from 3-(4-chloro-5-nitropyrimidin-2-yl)-6-fluoroimidazo[1,2-a]pyridine (**Preparation 25**) and 5,6,7,8-tetrahydroquinolin-5-amine following the experimental procedure as described in **Preparation 26a**.
LRMS (m/z): 406 (M+1)⁺.

### b) 2-(6-Fluoroimidazo[1,2-a]pyridin-3-yl)-N⁴-(5,6,7,8-tetrahydroquinolin-5-yl)pyrimidine-4,5-diamine

Obtained as a pale green solid (94%) from N-(2-(6-fluoroimidazo[1,2-a]pyridin-3-yl)-5-nitropyrimidin-4-yl)-5,6,7,8-tetrahydroquinolin-5-amine (**Preparation 29a**) following the experimental procedure as described in **Preparation 5b**.
LRMS (m/z): 376 (M+1)⁺.
1H NMR (300 MHz, DMSO-*d*₆) δ ppm 1.90 - 2.16 (m, 4H), 2.93 (m, 2H), 5.08 (br s, 2H), 5.58 (dd, 1H), 6.99 (d, 1H), 7.20 (dd, 1H), 7.42 (ddd, 1H), 7.67 (d, 1H), 7.74 (dd, 1H), 7.76 (s, 1H), 8.22 (s, 1H), 8.41 (dd, 1H), 9.91 (dd, 1H).

### PREPARATION 30

### 2-(6-Fluoroimidazo[1,2-a]pyridin-3-yl)-N⁴-(pyridin-2-ylmethyl)pyrimidine-4,5-diamine

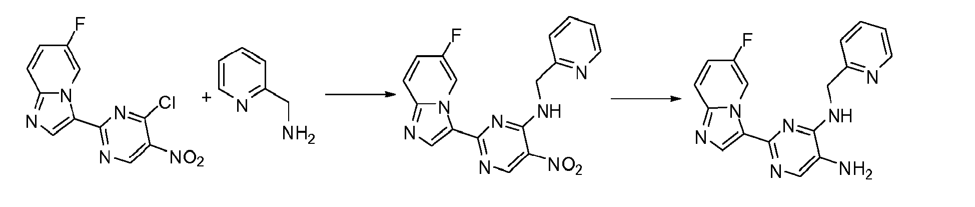

### a) 2-(6-Fluoroimidazo[1,2-a]pyridin-3-yl)-5-nitro-N-(pyridin-2-ylmethyl)pyrimidin-4-amine

Obtained as a yellow solid (80%) from 3-(4-chloro-5-nitropyrimidin-2-yl)-6-fluoroimidazo[1,2-a]pyridine (Preparation 25) and pyridin-2-ylmethanamine following the experimental procedure as described in Preparation 26a.
LRMS (m/z): 364 (M-1)⁻.

### b) 2-(6-Fluoroimidazo[1,2-a]pyridin-3-yl)-N⁴-(pyridin-2-ylmethyl)pyrimidine-4,5-diamine

10% Palladium on carbon (0.048 g, 0.05 mmol) was added to a suspension of 2-(6-fluoroimidazo[1,2-a]pyridin-3-yl)-5-nitro-N-(pyridin-2-ylmethyl)pyrimidin-4-amine (**Preparation 30a**, 0.100 g, 0.27 mmol) in ethanol (15 mL) and the mixture was stirred under a hydrogen atmosphere at ambient temperature. After 2 hours, the mixture was filtered through diatomaceous earth (Celite®) and the filter cake was washed with ethanol. The combined filtrate and washings were evaporated to give the title compound (0.088 g, 96%) as a pale green solid.
LRMS (m/z): 336 (M+1)⁺.

### PREPARATION 31

### 2-(6-Fluoroimidazo[1,2-a]pyridin-3-yl)-N⁴-((5-fluoropyridin-2-yl)methyl)pyrimidine-4,5-diamine

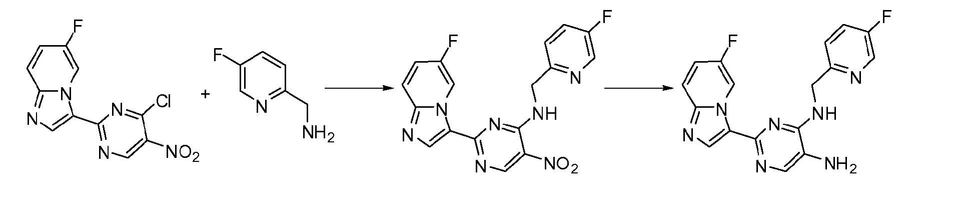

### a) 2-(6-Fluoroimidazo[1,2-a]pyridin-3-yl)-N-((5-fluoropyridin-2-yl)methyl)-5-nitropyrimidin-4-amine

Obtained as a yellow solid (83%) from 3-(4-chloro-5-nitropyrimidin-2-yl)-6-fluoroimidazo[1,2-a]pyridine (**Preparation 25**) and (5-fluoropyridin-2-yl)methanamine following the experimental procedure as described in **Preparation 26a**.
LRMS (m/z): 384 (M+H)⁺.
1H NMR (300 MHz, DMSO*-d*₆) ppm 5.01 (d, 2H), 7.55 - 7.74 (m, 3H), 7.87 (dd, 1H), 8.54 (d, 1H), 8.56 (s, 1H), 9.25 (s, 1H), 9.47 (dd, 1H), 9.73 (t, 1H).

### b) 2-(6-Fluoroimidazo[1,2-a]pyridin-3-yl)-N⁴-((5-fluoropyridin-2-yl)methyl)pyrimidine-4,5-diamine:

Obtained as a pale green solid (96%) from 2-(6-fluoroimidazo[1,2-a]pyridin-3-yl)-N-((5-fluoropyridin-2-yl)methyl)-5-nitropyrimidin-4-amine (**Preparation 31a**) following the experimental procedure as described in **Preparation 30b**.
LRMS (m/z): 354 (M+1)⁺.
1H NMR (300 MHz, DMSO*-d*₆) ppm 4.80 (d, 2H), 5.09 (br s, 2H), 7.39 (dd, 1H), 7.46 - 7.50 (m, 2H), 7.64 - 7.72 (m, 2H), 7.76 (s, 1H), 8.11 (s, 1H), 8.53 (d, 1H), 9.62 (dd, 1H).

### PREPARATION 32

### (R)-1-(5-Fluoropyridin-2-yl)ethanamine hydrochloride

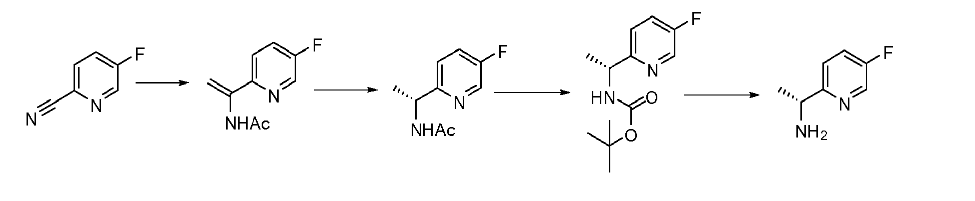

### a) N-(1-(5-Fluoropyridin-2-yl)vinyl)acetamide:

5-Fluoropicolinonitrile (9.30 g, 76.2 mmol) in tetrahydrofuran (40 mL) was added dropwise to a cold (0 °C), stirred solution of methylmagnesium bromide (3M in diethylether, 30.47 mL, 91.4 mmol) in tetrahydrofuran (40 mL). The mixture was stirred for 30 minutes at 0 °C then diluted with dichloromethane (30 mL) and then acetic anhydride (8.64 mL, 91.4 mmol) in dichloromethane (2 mL) was added dropwise at 0 °C. The mixture was warmed to room temperature and stirred overnight. 4% Aqueous sodium hydrogen carbonate solution was added and the mixture was extracted with ethyl acetate. The organic layer was dried (MgSO4), evaporated and the residue was purified by flash chromatography (3:1 hexanes/ethyl acetate) to give the title compound (4.30 g, 31%) as a yellow solid.
LRMS (m/z): 181 (M+1)⁺.
1H NMR (300 MHz, CDCl₃) δ ppm 2.21 (s, 3H), 5.47 (s, 1H), 6.47 (s, 1H), 7.48 (m, 1H), 7.78 (m, 1H), 8.37 (m, 1H), 9.07 (br s, 1H).

### b) (R)-N-(1-(5-Fluoropyridin-2-yl)ethyl)acetamide:

A solution of N-(1-(5-fluoropyridin-2-yl)vinyl)acetamide (**Preparation 32a**, 2.00 g, 11.1 mmol) and 1,2-Bis[(2R, 5R)-2,5-diethylphospholano]benzene(1,5-cyclooctadiene)rhodium(I)trifluoromethanesulphonate (0.08 g, 0.11 mmol) in methanol (15 mL) was hydrogenated at 130 psi for 4 hours. The mixture was then concentrated in vacuo and the residue was purified by flash chromatography (3:1 to 1:1 hexanes/ethyl acetate) to give the title compound (1.91 g, 92%) as a pale yellow oil. The enantiomeric excess of the product was determined to be 96% (Chiralpak IA, 4:1 heptane/isopropyl alcohol).
LRMS (m/z): 183 (M+1)⁺.
1H NMR (300 MHz, DMSO-d₆) δ ppm 1.35 (d, 3H), 1.82 (s, 3H), 4.98 (m, 1H), 7.40 (m, 1H), 7.72 (m, 1H), 8.38 (d, 1H), 8.45 (d, 1H).

### c) (R)-tert-Butyl 1-(5-fluoropyridin-2-yl)ethylcarbamate:

A solution of (R)-N-(1-(5-fluoropyridin-2-yl)ethyl)acetamide (**Preparation 32b**, 1.85 g, 10.15 mmol), N,N-dimethylpyridin-4-amine (0.24 g, 1.96 mmol) and di-tert-butyl dicarbonate (4.46 g, 20.44 mmol) in tetrahydrofuran (15 mL) was stirred and heated to 50 °C. After 20 hours, the mixture was cooled and a solution of lithium hydroxide monohydrate (0.89 g, 21.21 mmol) in water (18 mL) was added and stirring was continued for 5 hours at room temperature. Diethyl ether (100 mL) was then added and the organic layer was washed with brine, dried (MgSO₄ and concentrated and the residue was purified by flash chromatography (4:1 hexanes/ethyl acetate) to give the title compound (1.65 g, 68%) as a yellow solid.
LRMS (m/z): 241 (M+1)⁺.
1H NMR (300 MHz, DMSO-d₆) δ ppm 1.35 (d, 3H), 1.40 (s, 9H), 4.87 (m, 1H), 7.41 (m, 2H), 7.78 (m, 1H), 8.57 (d, 1H).

### d) (R)-1-(5-Fluoropyridin-2-yl)ethanamine hydrochloride:

A solution of hydrogen chloride in dioxane (4M, 13 mL) was added to a solution of (R)-tert-butyl 1-(5-fluoropyridin-2-yl)ethylcarbamate (**Preparation 32c**, 1.65 g, 6.87 mmol) in dichloromethane (12 mL). After stirring at room temperature for 2.5 hours the mixture was concentrated in vacuo to give the title compound (1.30 g, 100%) as a white solid.
LRMS (m/z): 141 (M+1)⁺.
1H NMR (300 MHz, DMSO-d₆) δ ppm 1.45 (d, 3H), 4.62 (m, 1H), 7.62 (m, 1H), 7.92 (m, 1H), 8.60 (br s, 4H).

### PREPARATION 33

### (R)-2-(6-Fluoroimidazo[1,2-a]pyridin-3-yl)-N⁴-(1-(5-fluoropyridin-2-yl)ethyl)pyrimidine-4,5-diamine

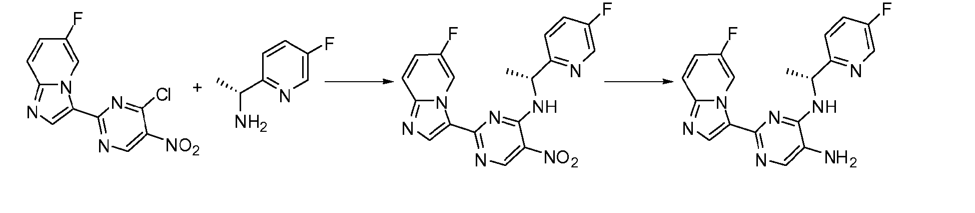

### a) (R)-2-(6-Fluoroimidazo[1,2-a]pyridin-3-yl)-N-(1-(5-fluoropyridin-2-yl)ethyl)-5-nitropyrimidin-4-amine

Obtained as a yellow solid (92%) from 3-(4-chloro-5-nitropyrimidin-2-yl)-6-fluoroimidazo[1,2-a]pyridine (**Preparation 25**) and (R)-1-(5-fluoropyridin-2-yl)ethanamine hydrochloride (**Preparation 32**) following the experimental procedure as described in **Preparation 26a**.
LRMS (m/z): 398 (M+H)⁺.
1H NMR (300 MHz, DMSO-d₆) δ ppm 1.68 (d, 3H), 5.68 (m, 1H), 7.72 (m, 3H), 7.91 (m, 1H), 8.60 (d, 1H), 8.64 (s, 1H), 9.27 (s, 1H), 9.37 (d, 1H), 9.68 (m, 1H).

### b) (R)-2-(6-Fluoroimidazo[1,2-a]pyridin-3-yl)-N4-(1-(5-fluoropyridin-2-yl)ethyl)pyrimidine-4,5-diamine

Obtained as a pale brown solid (79%) from (R)-2-(6-fluoroimidazo[1,2-a]pyridin-3-yl)-N-(1-(5-fluoropyridin-2-yl)ethyl)-5-nitropyrimidin-4-amine (**Preparation 33a**) following the experimental procedure as described in **Preparation 5b** followed by purification of the crude product by flash chromatography (95:5 to 9:1 dichloromethane/methanol).
LRMS (m/z): 368 (M+1)⁺.
1H NMR (300 MHz, DMSO-d₆) δ ppm 1.58 (d, 3H), 5.21 (br s, 2H), 5.35 (m, 1H), 7.11 (br s, 1H), 7.49 (m, 1H), 7.53 (m, 1H), 7.66 (m, 2H), 7.73 (s, 1H), 8.07 (s, 1H), 8.52(d, 1H), 9.60 (m, 1H).

### PREPARATION 34

### 1-(5-fluoropyridin-2-yl)-2-methoxyethanamine

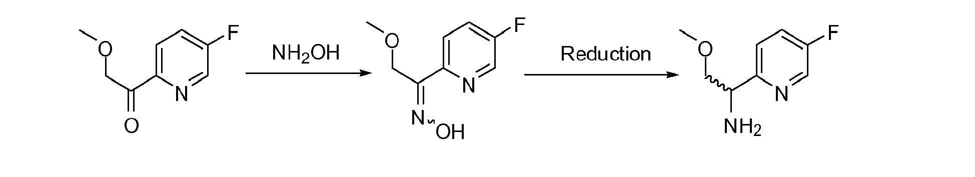

### a) 1-(5-fluoropyridin-2-yl)-2-methoxyethanone oxime

Hydroxylamine hydrochloride (0.82 g, 11.8 mmol) and triethylamine (1.85 mL, 13.3 mmol) were added sequentially to a solution of 1-(5-fluoropyridin-2-yl)-2-methoxyethanone (WO2009/007753, 1.50 g, 8.9 mmol) in ethanol (15 mL) and the mixture was stirred and heated to 50 °C. After 60 minutes, the mixture was concentrated in vacuo and partitioned between ethyl acetate and water. The organic extract was washed with brine, dried (MgSO₄ and concentrated and the residue was purified by flash chromatography (hexanes/ethyl acetate) to give the title compound (1.60 g, 98%) as a 3:1 mixture of isomers.
LRMS (m/z): 185 (M+1)⁺.

### b) 1-(5-fluoropyridin-2-yl)-2-methoxyethanamine

Palladium on carbon (10%, 0.300 g) was added to a solution of 1-(5-fluoropyridin-2-yl)-2-methoxyethanone oxime (**Preparation 34a,** 2.00 g, 10.9 mmol) in ethyl acetate (30 mL) and the mixture was stirred and heated to 40 °C under a hydrogen atmosphere (5 bar). After 10 hours, the mixture was filtered through diatomaceous earth (Celite®) and the filter cake was washed with ethyl acetate. The combined filtrate and washings were evaporated and the residue was purified by flash chromatography (0 - 10% methanol in dichloromethane) to give the title compound (0.90 g, 49%) as a yellow oil.
LRMS (m/z): 171 (M+1)⁺.
1H NMR (300 MHz, CDCl₃) δ ppm 3.37 (s, 3H), 3.47 (dd, 1H), 3.61 (dd, 1H), 4.22 (dd, 1H), 7.34 - 7.43 (m, 2H), 8.41 (d, 1H).

### PREPARATION 35

### 2-(6-fluoroimidazo[1,2-a]pyridin-3-yl)-N⁴-(1-(5-fluoropyridin-2-yl)-2-methoxyethyl)pyrimidine-4,5-diamine

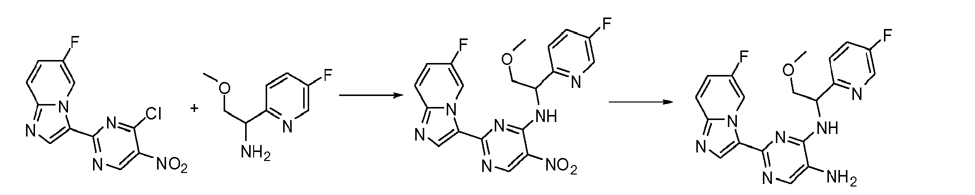

### a) 2-(6-fluoroimidazo[1,2-a]pyridin-3-yl)-N-(1-(5-fluoropyridin-2-yl)-2-methoxyethyl)-5-nitropyrimidin-4-amine

Obtained as a yellow solid (85%) from 3-(4-chloro-5-nitropyrimidin-2-yl)-6-fluoroimidazo[1,2-a]pyridine (**Preparation 25**) and 1-(5-fluoropyridin-2-yl)-2-methoxyethanamine (**Preparation 34**) following the experimental procedure as described in **Preparation 26a**.
LRMS (m/z): 428 (M+1)⁺.
1H NMR (300 MHz, DMSO-*d*₆) δ ppm 3.34 (s, 3H), 3.85 (dd, 1H), 3.98 (dd, 1H), 5.72 (m, 1H), 7.60 - 7.79 (m, 3H), 7.90 (m, 1H), 8.62 (s, 1H), 9.27 (m, 1H), 9.38 (s, 1H), 9.55(m, 1H).

### b) 2-(6-fluoroimidazo[1,2-a]pyridin-3-yl)-N⁴-(1-(5-fluoropyridin-2-yl)-2-methoxyethyl)pyrimidine-4,5-diamine

Obtained as a pale brown solid (87%) from 2-(6-fluoroimidazo[1,2-a]pyridin-3-yl)-N-(1-(5-fluoropyridin-2-yl)-2-methoxyethyl)-5-nitropyrimidin-4-amine (**Preparation 35a**) following the experimental procedure as described in **Preparation 30b** followed by purification of the crude product by flash chromatography (1 - 5 % methanol in dichloromethane).
LRMS (m/z): 398 (M+1)⁺.
1H NMR (300 MHz, DMSO-*d*₆) δ ppm 3.35 (s, 3H), 3.82 (m, 2H), 5.26 (br s, 2H), 5.53 (m, 1H), 7.11 (d, 1H), 7.39 (m, 1H), 7.56 (dd, 1H), 7.69 (m, 1H), 7.74 (s, 1H), 8.10 (s, 1H), 8.55(d, 1H), 9.63(dd, 1H).

### PREPARATION 36

### (R)-1-(Pyridin-2-yl)ethanamine dihydrochloride

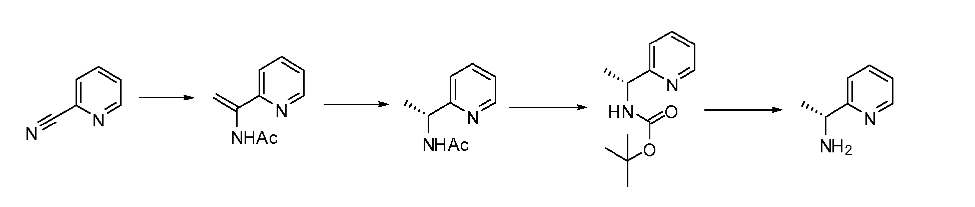

### a) N-(1-(Pyridin-2-yl)vinyl)acetamide:

Obtained as a pale yellow oil (23%) from picolinonitrile following the experimental procedure as described in **Preparation 32a**.
LRMS (m/z): 163 (M+1)⁺.
1H NMR (300 MHz, CDCl₃) δ ppm 2.21 (s, 3H), 5.45 (s, 1H), 6.43 (s, 1H), 7.22 (m, 1H), 7.78 (m, 2H), 8.56 (m, 1H), 9.21 (br s, 1H).

### b) (R)-N-(1-(Pyridin-2-yl)ethyl)acetamide:

Obtained as a pale yellow solid (89%) from N-(1-(pyridin-2-yl)vinyl)acetamide (**Preparation 36a**) following the experimental procedure as described in **Preparation 32b**. The enantiomeric excess of the product was determined to be 86% (Chiralpak IA, 1:1 heptane/tetrahydrofuran).
LRMS (m/z): 165 (M+1)⁺.
1H NMR (300 MHz, CDCl₃) δ ppm 1.42 (d, 3H), 2.02 (s, 3H), 5.18 (m, 1H), 6.95 (br s, 1H), 7.21 (m, 2H), 7.66 (m, 1H), 8.56 (d, 1H).

### c) (R)-tert-Butyl 1-(pyridin-2-yl)ethylcarbamate:

Obtained as a pale yellow oil (71%) from (R)-N-(1-(pyridin-2-yl)ethyl)acetamide (**Preparation 36b**) following the experimental procedure as described in **Preparation 32c**.
LRMS (m/z): 223 (M+1)⁺.
1H NMR (300 MHz, CDCl₃) δ ppm 1.41 (m, 12H), 4.95 (m, 1H), 5.72 (br s, 1H), 7.18 (m, 1H), 7.22 (m, 1H), 7.62 (m, 1H), 8.59 (d, 1H).

### d) (R)-1-(Pyridin-2-yl)ethanamine dihydrochloride:

Obtained as a white solid (95%) from (R)-tert-butyl 1-(pyridin-2-yl)ethylcarbamate (**Preparation 36c**) following the experimental procedure as described in **Preparation 32d**.
LRMS (m/z): 123 (M+1)⁺.
1H NMR (300 MHz, DMSO-d₆) δ ppm 1.45 (d, 3H), 4.57 (m, 1H), 7.49 (m, 1H), 7.65 (d, 1H), 7.98 (m, 1H), 8.60 (br s, 4H).

### PREPARATION 37

### (R)-2-(6-Fluoroimidazo[1,2-a]pyridin-3-yl)-N⁴-(1-(pyridin-2-yl)ethyl)pyrimidine-4,5-diamine

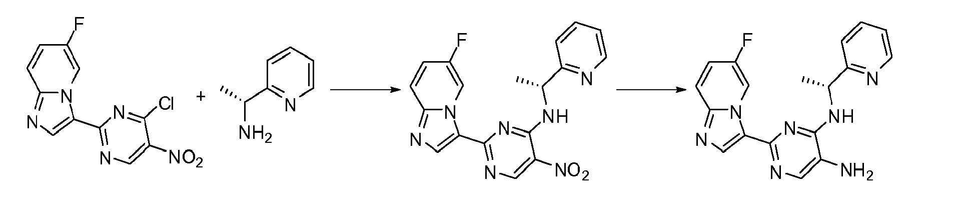

### a) (R)-2-(6-Fluoroimidazo[1,2-a]pyridin-3-yl)-5-nitro-N-(1-(pyridin-2-yl)ethyl)pyrimidin-4-amine:

Obtained as a yellow solid (80%) from 3-(4-chloro-5-nitropyrimidin-2-yl)-6-fluoroimidazo[1,2-a]pyridine (**Preparation 25**) and (R)-1-(pyridin-2-yl)ethanamine dihydrochloride (Preparation 36) following the experimental procedure as described in **Preparation 26a**.
LRMS (m/z): 380 (M+H)⁺.
1H NMR (300 MHz, DMSO-d₆) δ ppm 1.63 (d, 3H), 5.64 (m, 1H), 7.34 (m, 1H), 7.62 (d, 1H), 7.70 (m, 1H), 7.83 (m, 1H), 7.92 (m, 1H), 8.63 (m, 1H), 8.66 (s, 1H), 9.28 (s, 1H), 9.55(d, 1H), 9.75(m, 1H).

### b) (R)-2-(6-Fluoroimidazo[1,2-a]pyridin-3-yl)-N⁴-(1-(pyridin-2-yl)ethyl)pyrimidine-4,5-diamine

Obtained as a pale brown solid (54%) from (R)-2-(6-fluoroimidazo[1,2-a]pyridin-3-yl)-5-nitro-N-(1-(pyridin-2-yl)ethyl)pyrimidin-4-amine (**Preparation 37a**) following the experimental procedure as described in **Preparation 5b** followed by purification of the crude product by flash chromatography (95:5 to 9:1 dichloromethane/methanol).
LRMS (m/z): 350 (M+1)⁺.
1H NMR (300 MHz, DMSO-d₆) δ ppm 1.59 (d, 3H), 5.21 (br s, 2H), 5.34 (m, 1H), 7.10 (d, 1H), 7.20 (m, 1H), 7.40 (m, 2H), 7.66-7.75 (m, 3H), 8.06 (s, 1H), 8.53 (d, 1H), 9.64(m, 1H).

### PREPARATION 38

### (R)-1-(5-Fluoropyrimidin-2-yl)ethanamine hydrochloride

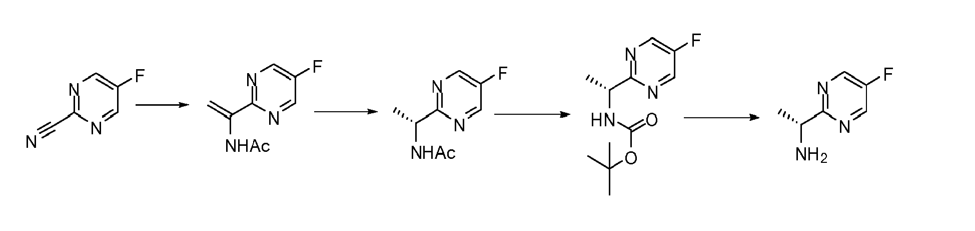

### a) N-(1-(5-Fluoropyrimidin-2-yl)vinyl)acetamide:

Obtained as a yellow solid (23%) from 5-fluoropyrimidine-2-carbonitrile following the experimental procedure as described in **Preparation 32a**.
LRMS (m/z): 182 (M+1)⁺.
1H NMR (300 MHz, CDCl₃) δ ppm 2.20 (s, 3H), 6.32 (s, 1H), 6.60 (s, 1H), 8.58 (s, 2H), 8.8 (br s, 1H).

### b) (R)-N-(1-(5-Fluoropyrimidin-2-yl)ethyl)acetamide:

Obtained as a yellow solid (80%) from N-(1-(5-fluoropyrimidin-2-yl)vinyl)acetamide (**Preparation 38a**) following the experimental procedure as described in **Preparation** 32b. The enantiomeric excess of the product was determined to be 99% (Chiralpak IA, 9:1 heptane/ethanol).
LRMS (m/z): 184 (M+1)⁺.
1H NMR (300 MHz, CDCl₃) δ ppm 1.45 (d, 3H), 2.10 (s, 3H), 5.32 (m, 1H), 6.68 (br s, 1H), 8.59 (s, 2H).

### c) (R)-tert-Butyl 1-(5-fluoropyrimidin-2-yl)ethylcarbamate:

Obtained as a colourless oil (74%) from (R)-N-(1-(5-fluoropyrimidin-2-yl)ethyl)acetamide (**Preparation 38b**) following the experimental procedure as described in **Preparation 32**c.
LRMS (m/z): 242 (M+1)⁺.
1H NMR (300 MHz, CDCl₃) δ ppm 1.32-1.49 (m, 12H), 5.02 (m, 1H), 5.60 (br s, 1H), 8.58(s,2H).

### d) (R)-1-(5-Fluoropyrimidin-2-yl)ethanamine hydrochloride:

Obtained as a white solid (88%) from (R)-tert-butyl 1-(5-fluoropyrimidin-2-yl)ethylcarbamate (**Preparation 38c**) following the experimental procedure as described in **Preparation 32d**.
LRMS (m/z): 142 (M+1)⁺.
1H NMR (300 MHz, DMSO-d₆) δ ppm 1.49 (d, 3H), 4.59 (m, 1H), 8.60 (br s, 3H), 9.02 (m, 1H).

### PREPARATION 39

### (R)-2-(6-Fluoroimidazo[1,2-a]pyridin-3-yl)-N⁴-(1-(5-fluoropyrimidin-2-yl)ethyl)pyrimidine-4,5-diamine

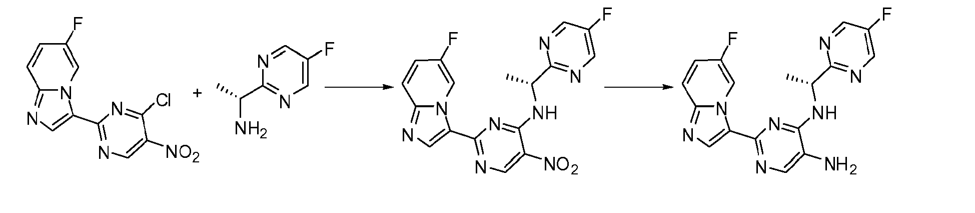

### a) (R)-2-(6-Fluoroimidazo[1,2-a]pyridin-3-yl)-N-(1-(5-fluoropyrimidin-2-yl)ethyl)-5-nitropyrimidin-4-amine:

Obtained as a yellow solid (85%) from 3-(4-chloro-5-nitropyrimidin-2-yl)-6-fluoroimidazo[1,2-a]pyridine (**Preparation 25**) and (R)-1-(5-fluoropyrimidin-2-yl)ethanamine hydrochloride (**Preparation 38**) following the experimental procedure as described in **Preparation 26a**.
LRMS (m/z): 399 (M+H)⁺.
1H NMR (300 MHz, DMSO-d₆) δ ppm 1.77 (d, 3H), 5.69 (m, 1H), 7.72 (m, 1H), 7.92 (m, 1H), 8.60 (s, 1H), 8.99 (s, 2H), 9.28 (s, 1H), 9.45 (m, 1H), 9.73 (d, 1H).

### b) (R)-2-(6-Fluoroimidazo[1,2-a]pyridin-3-yl)-N⁴-(1-(5-fluoropyrimidin-2-yl)ethyl)pyrimidine-4,5-diamine:

Obtained as a pale brown solid (84%) from (R)-2-(6-fluoroimidazo[1,2-a]pyridin-3-yl)-N-(1-(5-fluoropyrimidin-2-yl)ethyl)-5-nitropyrimidin-4-amine (**Preparation 39a**) following the experimental procedure as described in **Preparation 5b** followed by purification of the crude product by flash chromatography (95:5 dichloromethane/methanol).
LRMS (m/z): 369 (M+1)⁺.
1H NMR (300 MHz, DMSO-d₆) δ ppm 1.62 (d, 3H), 5.23 (br s, 2H), 5.38 (m, 1H), 7.18 (m, 1H), 7.39 (m, 1H), 7.66 (m, 1H), 7.71 (s, 1H), 7.96 (s, 1H), 8.85 (s, 2H), 9.66 (m, 1H).

### PREPARATION 40

### (S)-2-(6-Fluoroimidazo[1,2-a]pyridin-3-yl)-N⁴-(1-(5-fluoropyridin-2-yl)ethyl)pyrimidine-4,5-diamine

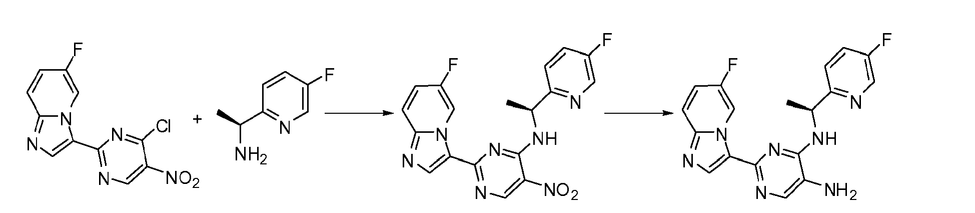

### a) (S)-2-(6-Fluoroimidazo[1,2-a]pyridin-3-yl)-N-(1-(5-fluoropyridin-2-yl)ethyl)-5-nitropyrimidin-4-amine:

Obtained as a yellow solid (92%) from 3-(4-chloro-5-nitropyrimidin-2-yl)-6-fluoroimidazo[1,2-a]pyridine (**Preparation 25**) and (S)-1-(5-fluoropyridin-2-yl)ethanamine hydrochloride (WO2008/135785 A1) following the experimental procedure as described in **Preparation 26a**.
LRMS (m/z): 398 (M+H)⁺.
1H NMR (300 MHz, DMSO-d₆) δ ppm 1.72 (d, 3H), 5.70 (m, 1H), 7.69-7.85 (m, 3H), 7.94 (m, 1H), 8.64 (d, 1H), 8.66 (s, 1H), 9.30 (s, 1H), 9.40 (d, 1H), 9.70 (m, 1H).

### b) (S)-2-(6-Fluoroimidazo[1,2-a]pyridin-3-yl)-N⁴-(1-(5-fluoropyridin-2-yl)ethyl)pyrimidine-4,5-diamine:

Obtained as a pale brown solid (83%) from (S)-2-(6-fluoroimidazo[1,2-a]pyridin-3-yl)-N-(1-(5-fluoropyridin-2-yl)ethyl)-5-nitropyrimidin-4-amine (**Preparation 40a**) following the experimental procedure as described in **Preparation 5b** followed by purification of the crude product by flash chromatography (95:5 to 9:1 dichloromethane/methanol).
LRMS (m/z): 368 (M+1)⁺.
1H NMR (300 MHz, DMSO-d₆) δ ppm 1.62 (d, 3H), 5.24 (br s, 2H), 5.39 (m, 1H), 7.14 (d, 1H), 7.45 (m, 1H), 7.55 (m, 1H), 7.63-7.65 (m, 2H), 7.77 (s, 1H), 8.11 (s, 1H), 8.55(m, 1H), 9.64 (m, 1H).

### PREPARATION 41

### (S)-2-Amino-2-(5-fluoropyridin-2-yl)ethanol hydrochloride

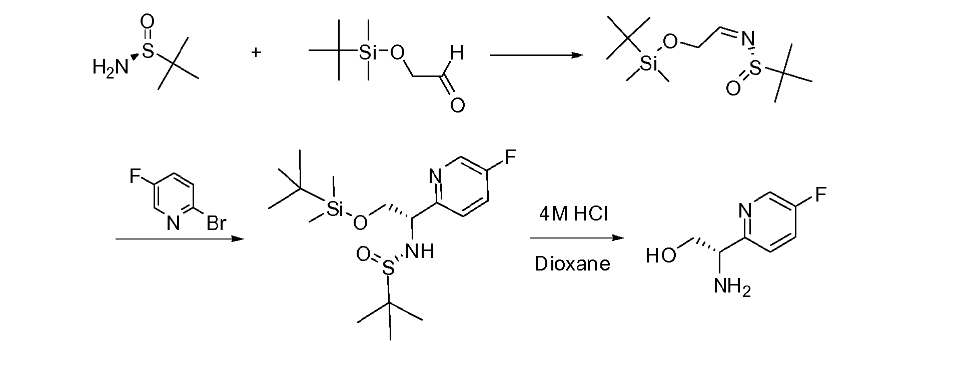

### a) (S,Z)-N-(2-(tert-Butyldimethylsilyloxy)ethylidene)-2-methylpropane-2-sulfinamide:

Copper(II)sulphate (7.23 g, 32.4 mmol) was added to a solution of (S)-2-methylpropane-2-sulfinamide (2.50 g, 20.6 mmol) and 2-(tert-butyldimethylsilyloxy)acetaldehyde (4.32 mL, 22.7 mmol) in dichloromethane (30 mL) and the mixture was stirred overnight. The mixture was then filtered through diatomaceous earth (Celite®) and the filter cake was washed with dichloromethane. The combined filtrate and washings were concentrated in vacuo and the residue was purified by flash chromatography (5:1 hexanes/ethyl acetate) to give the title compound (5.10 g, 89%) as a yellow oil.
1H NMR (300 MHz, CDCl₃) δ ppm 0.3 (s, 6H), 0.85 (s, 9H), 1.14 (s, 9H), 4.48 (m, 2H), 7.99(m, 1H).

### b) (S)-N-((S)-2-(tert-Butyldimethylsilyloxy)-1-(5-fluoropyridin-2-yl)ethyl)-2-methylpropane-2-sulfinamide:

A solution of tert-butyl lithium in pentane (1.7 M, 8.5 mL) was added dropwise to a cooled (-78 °C), stirred, solution of 2-bromo-5-fluoropyridine (1.30 g, 7.4 mmol) in diethyl ether (8 mL). After stirring for a further 15 minutes at -78 °C, a solution of (S,Z)-N-(2-(tert-butyldimethylsilyloxy)ethylidene)-2-methylpropane-2-sulfinamide (**Preparation 41a**, 1.00 g, 3.8 mmol) in diethyl ether (24 mL) was added dropwise via cannula over 15 minutes. After stirring for a further 3 hours at -78 °C, the reaction was quenched with saturated ammonium chloride solution and diluted with ethyl acetate. The organic layer was dried (MgSO₄ and concentrated and the residue was purified by flash chromatography (3:1 hexanes/ethyl acetate) to give the title compound (0.910 g, 64%) as an oil.
LRMS (m/z): 375 (M+1)⁺.
1H NMR (300 MHz, CDCl₃) δ ppm 0.0 (s, 3H), 0.3 (s, 3H), 0.89 (s, 9H), 1.30 (s, 9H), 3.99 (m, 2H), 4.51 (d, 1H), 4.68 (m, 1H),7.42 (d, 2H), 8.49 (s, 1H).

### c) (S)-2-Amino-2-(5-fluoropyridin-2-yl)ethanol hydrochloride:

Obtained as a hygroscopic brown solid (80%) from (S)-N-((S)-2-(tert-butyldimethylsilyloxy)-1-(5-fluoropyridin-2-yl)ethyl)-2-methylpropane-2-sulfinamide (**Preparation 41b**) following the experimental procedure as described in **Preparation 32d** followed by trituration of the crude product with hexanes.
LRMS (m/z): 157 (M+1)⁺.
1H NMR (300 MHz, DMSO-d₆) δ ppm 3.81 (m, 2H), 4.47 (m, 1H), 7.70 (m, 1H), 7.89 (m, 1H), 8.59 (s, 2H), 8.66 (s, 1H).

### PREPARATION 42

### (S)-9-(2-(tert-Butyldiphenylsilyloxy)-1-(5-fluoropyridin-2-yl)ethyl)-2-(6-fluoroimidazo[1,2-a]pyridin-3-yl)-7H-purin-8(9H)-one

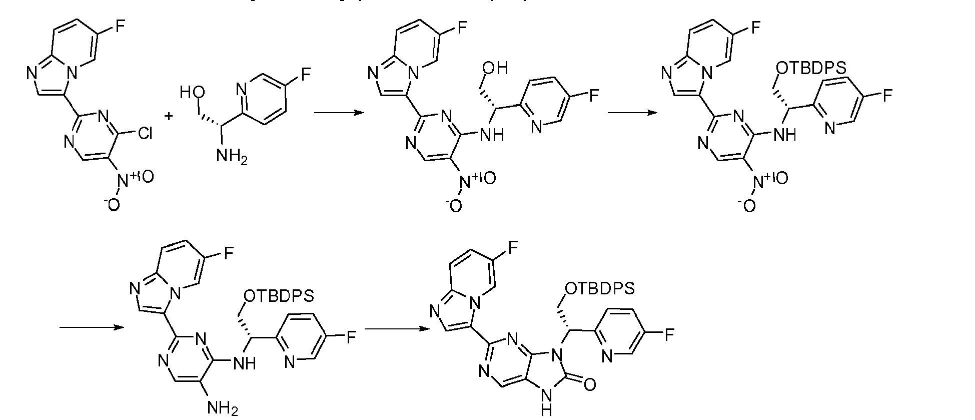

### a) (S)-2-(2-(6-Fluoroimidazo[1,2-a]pyridin-3-yl)-5-nitropyrimidin-4-ylamino)-2-(5-fluoropyridin-2-yl)ethanol:

Obtained as a yellow solid (85%) from 3-(4-chloro-5-nitropyrimidin-2-yl)-6-fluoroimidazo[1,2-a]pyridine (**Preparation 25**) and (S)-2-amino-2-(5-fluoropyridin-2-yl)ethanol hydrochloride (**Preparation 41**) following the experimental procedure as described in **Preparation 26a**.
LRMS (m/z): 414 (M+H)⁺.
1H NMR (300 MHz, DMSO-d₆) δ ppm 4.00 (m, 2H), 5.42 (t, 1H), 5.56 (m, 1H), 7.68 - 7.76 (m, 3H), 7.91 (m, 1H), 8.64 (s, 1H), 9.31 (s, 1H), 9.46 (d, 1H), 9.53 (s, 1H).

### b) (S)-N-(2-(tert-Butyldiphenylsilyloxy)-1-(5-fluoropyridin-2-yl)ethyl)-2-(6-fluoroimidazo[1,2-a]pyridin-3-yl)-5-nitropyrimidin-4-amine:

Imidazole (0.070 g, 1.02 mmol) and tert-butylchlorodiphenylsilane (0.307 mL, 1.18 mmol) were added sequentially to a solution of (S)-2-(2-(6-fluoroimidazo[1,2-a]pyridin-3-yl)-5-nitropyrimidin-4-ylamino)-2-(5-fluoropyridin-2-yl)ethanol (**Preparation 42a**, 0.195 g, 0.47 mmol) in N,N-dimethylformamide (4 mL) and the mixture was stirred overnight. Water was added and the mixture was extracted with ethyl acetate and the organic layer was dried (MgSO₄ and evaporated. The residue was purified by flash chromatography (3:1 hexanes/ethyl acetate) to give the title compound (0.245 g, 80%) as a yellow solid.
LRMS (m/z): 652 (M+H)⁺.
1H NMR (300 MHz, DMSO-d₆) δ ppm 0.92 (s, 9H), 4.21 (m, 2H), 5.73 (m, 1H), 7.23 - 7.41 (m, 6H), 7.47 - 7.50 (m, 3H), 7.64 - 7.79 (m, 4H), 7.90 (m, 1H), 8.60 (s, 1H), 8.62 (s, 1H), 9.30 (s, 1H), 9.45 (br s, 1H), 9.32 (d, 1H).

### c) (S)-N⁴-(2-(tert-Butyldiphenylsilyloxy)-1-(5-fluoropyridin-2-yl)ethyl)-2-(6-fluoroimidazo[1,2-a]pyridin-3-yl)pyrimidine-4,5-diamine:

Obtained as a yellow solid (98%) from (S)-N-(2-(tert-butyldiphenylsilyloxy)-1-(5-fluoropyridin-2-yl)ethyl)-2-(6-fluoroimidazo[1,2-a]pyridin-3-yl)-5-nitropyrimidin-4-amine (**Preparation 42b**) following the experimental procedure as described in **Preparation 30b.**
LRMS (m/z): 622 (M+1)⁺.
1H NMR (300 MHz, DMSO-d₆) δ ppm 0.89 (s, 9H), 4.09 (m, 2H), 5.18 (s, 2H), 5.59 (m, 1H), 7.01 (d, 1H), 7.32-7.55 (m, 12H), 7.67 (m, 1H), 7.71-7.76 (m, 2H), 8.17 (s, 1H), 8.55(d, 1H), 9.76 (m, 1H).

### d) (S)-9-(2-(tert-Butyldiphenylsilyloxy)-1-(5-fluoropyridin-2-yl)ethyl)-2-(6-fluoroimidazo[1,2-a]pyridin-3-yl)-7H-purin-8(9H)-one:

1,1'-Carbonylbis-1*H*-imidazole (0.120 g, 0.74 mmol) was added to a solution of (S)-N⁴-(2-(tert-butyldiphenylsilyloxy)-1-(5-fluoropyridin-2-yl)ethyl)-2-(6-fluoroimidazo[1,2-a]pyridin-3-yl)pyrimidine-4,5-diamine (**Preparation 42c**, 0.230 g, 0.37 mmol) in acetonitrile (6 mL) and the mixture was stirred and heated to 80 °C in a sealed tube. After 3 hours, the mixture was concentrated in vacuo and water was added. The resultant suspension was stirred for 1 hour at room temperature and then was filtered to give the title compound (0.201 g, 84%) as an off white solid.
LRMS (m/z): 648 (M+1)⁺.
1H NMR (300 MHz, DMSO-d6) δ ppm 0.77 (s, 9H), 4.62 (dd, 1H), 4.78 (t, 1H), 5.98 (dd, 1H), 6.95 (m, 2H), 7.05 (m, 1H), 7.23-7.36 (m, 5H), 7.45-7.52 (m, 3H), 7.59 (dd, 1H), 7.72 (dt, 1H), 7.80 (dd, 1H), 8.30 (s, 1H), 8.44 (s, 1H), 8.51 (d, 1H), 9.36 (m, 1H), 11.75(s, 1H).

### PREPARATION 43

### 1-(5-Amino-2-(6-fluoroimidazo[1,2-a]pyridin-3-yl)pyrimidin-4-ylamino)cyclohexanecarbonitrile

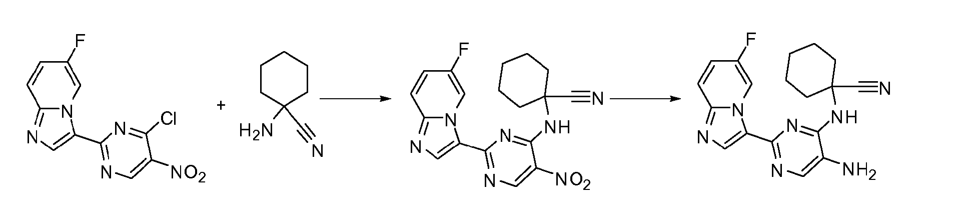

### a) 1-(2-(6-Fluoroimidazo[1,2-a]pyridin-3-yl)-5-nitropyrimidin-4-ylamino)cyclohexanecarbonitrile

Obtained as a yellow solid (42%) from 3-(4-chloro-5-nitropyrimidin-2-yl)-6-fluoroimidazo[1,2-a]pyridine (**Preparation 25**) and 1-aminocyclohexanecarbonitrile (WO2006/28545) following the experimental procedure as described in **Preparation 26a** followed by purification of the crude product by flash chromatography (99:1 dichloromethane/methanol).
LRMS (m/z): 382 (M+1)⁺.
1H NMR (300 MHz, CDCl₃) δ ppm 1.39 - 1.52 (m, 1H), 1.74 - 2.01 (m, 7H), 2.60 - 2.73 (m, 2H), 7.40 (m, 1H), 7.78 (dd, 1H), 8.53 (br s, 1H), 8.82 (s, 1H), 9.35 (s, 1H), 9.99(dd, 1H).

### b) 1-(5-Amino-2-(6-fluoroimidazo[1,2-a]pyridin-3-yl)pyrimidin-4-ylamino)cyclohexanecarbonitrile

Obtained as a pale brown solid (100%) from 1-(2-(6-fluoroimidazo[1,2-a]pyridin-3-yl)-5-nitropyrimidin-4-ylamino)cyclohexanecarbonitrile (**Preparation 43a**) following the experimental procedure as described in **Preparation 30b**.
LRMS (m/z): 352 (M+1)⁺.
1H NMR (300 MHz, CDCl₃) δ ppm 1.30 - 1.44 (m, 1H), 1.74 - 1.91 (m, 7H), 2.62 - 2.76 (m, 2H), 3.13 (br s, 2H), 5.09 (br s, 1H), 7.21 (m, 1H), 7.67 (dd, 1H), 8.00 (s, 1H), 8.51 (s, 1H), 9.99 (dd, 1H).

### PREPARATION 44

### (1s,4s)-ethyl 4-(5-amino-2-(6-fluoroimidazo[1,2-a]pyridin-3-yl)pyrimidin-4-ylamino)cyclohexanecarboxylate

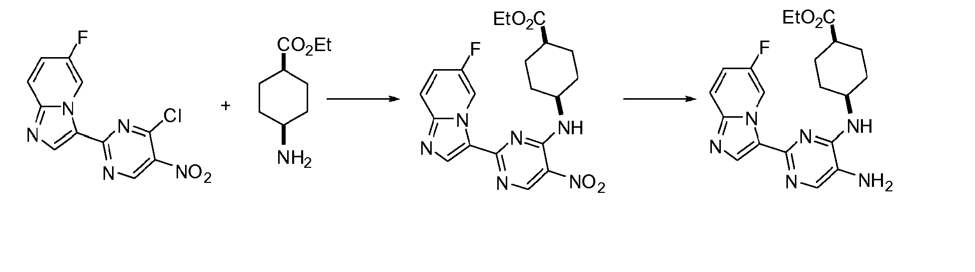

### a) (1s,4s)-ethyl 4-(2-(6-fluoroimidazo[1,2-a]pyridin-3-yl)-5-nitropyrimidin-4-ylamino)cyclohexanecarboxylate

Obtained as a yellow solid (71%) from 3-(4-chloro-5-nitropyrimidin-2-yl)-6-fluoroimidazo[1,2-a]pyridine (**Preparation 25**) and (1s,4s)-ethyl 4-aminocyclohexanecarboxylate following the experimental procedure as described in **Preparation 26a.**
LRMS (m/z): 429 (M+1)⁺.
1H NMR (300 MHz, DMSO-*d*₆) δ ppm 1.21 (t, 3H), 1.71 - 2.03 (m, 8H), 2.67 (m, 1H), 4.11 (q, 2H), 4.37 (m, 1H), 7.71 (m, 1H), 7.93 (dd, 1H), 8.58 (d, 1H), 8.64 (s, 1H), 9.22 (s, 1H), 9.80 (m, 1H).

### b) (1s,4s)-ethyl 4-(5-amino-2-(6-fluoroimidazo[1,2-a]pyridin-3-yl)pyrimidin-4-ylamino)cyclohexanecarboxylate

Obtained as a green solid (100%) from (1s,4s)-ethyl 4-(2-(6-fluoroimidazo[1,2-a]pyridin-3-yl)-5-nitropyrimidin-4-ylamino)cyclohexanecarboxylate (Preparation **44a**) following the experimental procedure as described in **Preparation 30b**.
LRMS (m/z): 399 (M+1)⁺.
1H NMR (300 MHz, CDCl₃) δ ppm 1.29 (t, 3H), 1.74 - 2.09 (m, 8H), 2.61 (m, 1H), 3.07 (br s, 2H), 4.17 (q, 2H), 4.27 (m, 1H), 4.97 (d, 1H), 7.20 (m, 1H), 7.65 (dd, 1H), 7.88 (s, 1H), 8.43 (s, 1H), 9.96 (dd, 1H).

### PREPARATION 45

### (1 r,4r)-ethyl 4-(5-amino-2-(6-fluoroimidazo[1,2-a]pyridin-3-yl)pyrimidin-4-ylamino)cyclohexanecarboxylate

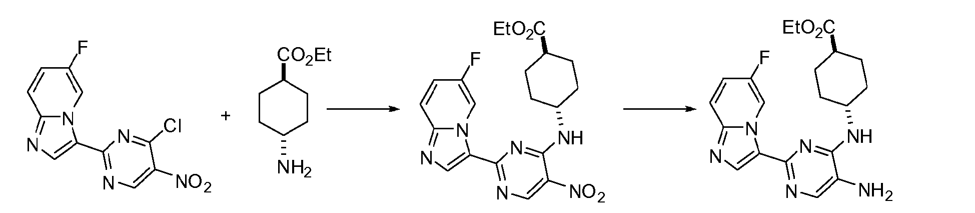

### a) (1r,4r)-ethyl 4-(2-(6-fluoroimidazo[1,2-a]pyridin-3-yl)-5-nitropyrimidin-4-ylamino)cyclohexanecarboxylate

Obtained as a yellow solid (78%) from 3-(4-chloro-5-nitropyrimidin-2-yl)-6-fluoroimidazo[1,2-a]pyridine (**Preparation 25**) and (1r,4r)-ethyl 4-aminocyclohexanecarboxylate following the experimental procedure as described in **Preparation 26a.**
LRMS (m/z): 429 (M+1)⁺.
1H NMR (300 MHz, DMSO-*d*₆) δ ppm 1.20 (t, 3H), 1.52 - 1.73 (m, 4H), 1.93 - 2.20 (m, 4H), 2.36 (m, 1H), 4.08 (q, 2H), 4.23 (m, 1H), 7.70 (m, 1H), 7.93 (dd, 1H), 8.53 (d, 1H), 8.65 (s, 1H), 9.20 (s, 1H), 9.78 (m, 1H).

### b) (1r,4r)-ethyl 4-(5-amino-2-(6-fluoroimidazo[1,2-a]pyridin-3-yl)pyrimidin-4-ylamino)cyclohexanecarboxylate

Obtained as a green solid (86%) from (1r,4r)-ethyl 4-(2-(6-fluoroimidazo[1,2-a]pyridin-3-yl)-5-nitropyrimidin-4-ylamino)cyclohexanecarboxylate (**Preparation 45a**) following the experimental procedure as described in **Preparation 30b.**
LRMS (m/z): 399 (M+1)⁺.
1H NMR (300 MHz, CDCl₃) δ ppm 1.29 (t, 3H), 1.72 - 2.37 (m, 9H), 3.03 (br s, 2H), 4.09 (m, 1H), 4.16 (q, 2H), 4.83 (s, 1H), 7.19 (m, 1H), 7.66 (dd, 1H), 7.88 (s, 1H), 8.43 (s, 1H), 9.95 (m, 1H).

### PREPARATION 46

### N⁴-(2,2-Dimethyltetrahydro-2H-pyran-4-yl)-2-(6-fluoroimidazo[1,2-a]pyridin-3-yl)pyrimidine-4,5-diamine

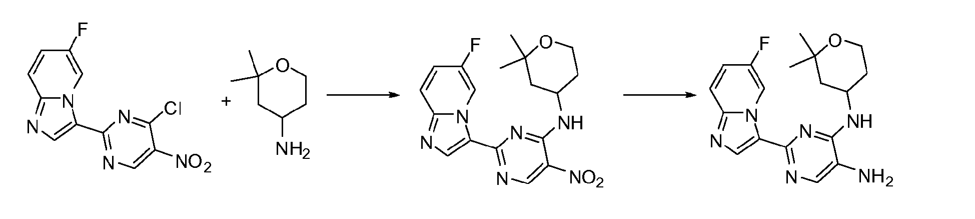

### a) N-(2,2-Dimethyltetrahydro-2H-pyran-4-yl)-2-(6-fluoroimidazo[1,2-a]pyridin-3-yl)-5-nitropyrimidin-4-amine

Obtained as a yellow solid (72%) from 3-(4-chloro-5-nitropyrimidin-2-yl)-6-fluoroimidazo[1,2-a]pyridine (**Preparation 25**) and 2,2-dimethyltetrahydro-2H-pyran-4-amine hydrochloride following the experimental procedure as described in **Preparation 26a.**
LRMS (m/z): 387 (M+1)⁺.
1H NMR (300 MHz, DMSO-*d*₆) δ ppm 1.19 (s, 3H), 1.33 (s, 3H), 1.50 - 1.78 (m, 2H), 1.84 - 2.03 (m, 2H), 3.73 - 3.83 (m, 2H), 4.57 - 4.84 (m, 1H), 7.70 (ddd, 1H), 7.93 (dd, 1H), 8.53 (d, 1H), 8.63 (s, 1H), 9.22 (s, 1H), 9.78 (dd, 1H).

### b) N⁴-(2,2-Dimethyltetrahydro-2H-pyran-4-yl)-2-(6-fluoroimidazo[1,2-a]pyridin-3-yl)pyrimidine-4,5-diamine

Obtained as a pale brown solid (97%) from *N*-(2,2-dimethyltetrahydro-2*H*-pyran-4-yl)-2-(6-fluoroimidazo[1,2-a]pyridin-3-yl)-5-nitropyrimidin-4-amine (**Preparation 46a**) following the experimental procedure as described in **Preparation 5b**.
LRMS (m/z): 357 (M+1)⁺.

### PREPARATION 47

### 2,2-dimethyl-3,4-dihydro-2H-pyrano[2,3-b]pyridin-4-amine

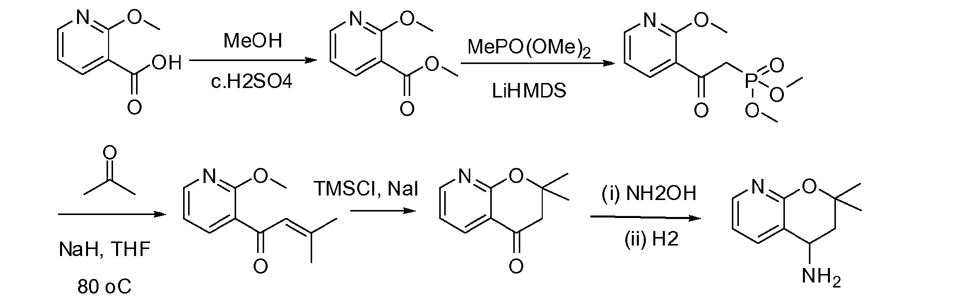

### a) methyl 2-methoxynicotinate

Concentrated aqueous sulphuric acid (0.1 mL) was added to a solution of 2-methoxynicotinic acid (2.00 g, 13.1 mmol) in methanol (20 mL) and the mixture was stirred and heated to reflux. After 6 hours, a saturated aqueous solution of sodium hydrogen carbonate was added and the mixture was extracted with ethyl acetate. The organic layer was dried (MgSO₄ and concentrated to give the title compound (1.85 g, 85%) as a colourless oil.
LRMS (m/z): 168 (M+1)⁺.
1H NMR (300 MHz, DMSO-*d*₆) δ ppm 3.81 (s, 3H), 3.92 (s, 3H), 7.11 (dd, 1H), 8.13 (dd, 1H), 8.38(dd, 1H).

### b) dimethyl 2-(2-methoxypyridin-3-yl)-2-oxoethylphosphonate

A solution of lithium hexamethyldisilazane (1 M in toluene, 12.6 mL, 12.6 mmol) was added dropwise to a stirred, cooled (-78 °C) solution of dimethyl methylphosphonate (1.28 mL, 12.0 mmol) in tetrahydrofuran (30 mL). After stirring for 30 minutes at the same temperature, the mixture was warmed to -60 °C then a solution of methyl 2-methoxynicotinate (**Preparation 47a,** 1.00 g, 6.0 mmol) in tetrahydrofuran (6 mL) was added dropwise and the mixture was stirred for 60 minutes at -60 °C and a further 60 minutes at 0 °C. The mixture was diluted with saturated aqueous ammonium chloride solution and extracted with dichloromethane. The organic extract was dried (MgSO₄) evaporated and the residue was purified by flash chromatography (98:2 dichloromethane/methanol) to give the title compound (1.90 g, >100%) as a pale yellow oil. The crude product was used without further purification in the next synthetic step.
LRMS (m/z): 260 (M+1)⁺.
1H NMR (300 MHz, DMSO-*d₆*) δ ppm 3.60 (d, 6H), 3.87 (d, 2H), 3.99 (s, 3H), 7.14 (dd, 1H), 8.04 (dd, 1H), 8.39 (dd, 1H).

### c) 1-(2-methoxypyridin-3-yl)-3-methylbut-2-en-1-one

Sodium hydride (60% dispersion in mineral oil, 0.32 g, 8.0 mmol) was added portion wise to a stirred solution of crude dimethyl 2-(2-methoxypyridin-3-yl)-2-oxoethylphosphonate **(Preparation 47b,** 1.90 g, ca. 7.0 mmol) in tetrahydrofuran (30 mL) under an argon atmosphere. After stirring 30 minutes at room temperature, the mixture was cooled in an ice-water bath and acetone (25 mL) was added and then the mixture was heated to 80 °C. After 48 hours, the mixture was concentrated in vacuo and partitioned between ethyl acetate and water. The organic extract was washed with brine, dried (MgSO₄) and concentrated to give the title compound (1.80 g, >100%) as a yellow oil. The crude product was used without further purification in the next synthetic step.
LRMS (m/z): 192 (M+1)⁺.
1H NMR (300 MHz, DMSO-*d₆*) δ ppm 1.95 (s, 3H), 2.14 (s, 3H), 3.93 (s, 3H), 6.65 (s, 1H), 7.09 (m, 1H), 7.88 (dd, 1H), 8.31 (dd, 1H).

### d) 2,2-dimethyl-2H-pyrano[2,3-b]pyridin-4(3H)-one

Trimethylsilyl chloride and sodium iodide were added to a stirred solution of 1-(2-methoxypyridin-3-yl)-3-methylbut-2-en-1-one (Preparation 47c, 1.30 g, 6.80 mmol) in acetonitrile (25 mL). After 16 hours, water was added and the mixture was extracted with dichloromethane. The organic extract was dried (MgSO₄) concentrated and the residue was purified by flash chromatography (98:2 dichloromethane/methanol) to give the title compound (0.62 g, 52%) as a yellow solid.
LRMS (m/z): 178 (M+1)⁺.
1H NMR (300 MHz, DMSO-*d₆*) δ ppm 1.41 (s, 6H), 2.86 (s, 2H), 7.14 (m, 1H), 8.13 (dd, 1H), 8.46 (dd, 1H).

### e) (E)-2,2-dimethyl-2H-pyrano[2,3-b]pyridin-4(3H)-one oxime

Hydroxylamine hydrochloride (0.130 g, 1.87 mmol) and sodium acetate (0.152 g, 1.85 mmol) were added to a solution of 2,2-dimethyl-2H-pyrano[2,3-b]pyridin-4(3H)-one **(Preparation 47d,** 0.300 g, 1.69 mmol) in ethanol (15 mL) and the mixture was stirred and heated to reflux. After 16 hours, the mixture was concentrated in vacuo and partitioned between ethyl acetate and water. The organic extract was washed with water, brine, dried (MgSO₄) and concentrated to give the title compound (0.320 g, 98%) as a white solid.
LRMS (m/z): 193 (M+1)⁺.

### f) 2,2-dimethyl-3,4-dihydro-2H-pyrano[2,3-b]pyridin-4-amine

Raney-Ni (50% suspension in water, 1.0 g) was added to a stirred solution of (E)-2,2-dimethyl-2H-pyrano[2,3-b]pyridin-4(3H)-one oxime **(Preparation 47e,** 0.310 g, 1.61 mmol) in methanol (15 mL) and the mixture was hydrogenated at 50 psi. After 24 hours, the mixture was filtered through Celite® and the filter cake was washed with methanol. The combined filtrate and washings were evaporated to give the title compound (0.255 g, 89%) as a yellow oil.
LRMS (m/z): 179 (M+1)⁺.
1H NMR (300 MHz, DMSO-*d₆*) δ ppm 1.23 (s, 3H), 1.39 (s, 3H), 1.53 (t, 1H), 2.02 (m, 1H), 3.88 (m, 1H), 6.93 (dd, 1H), 7.97 (m, 2H).

### PREPARATION 48

### N⁴-(2,2-dimethyl-3,4-dihydro-2H-pyrano[2,3-b]pyridin-4-yl)-2-(6-fluoroimidazo[1,2-a]pyridin-3-yl)pyrimidine-4,5-diamine

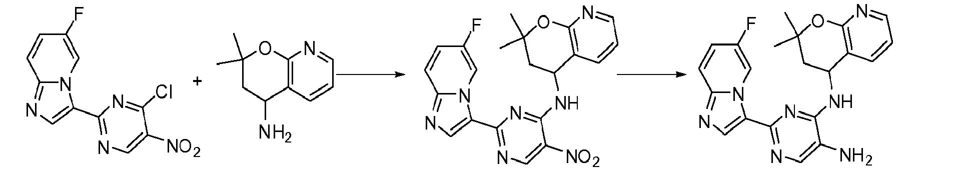

### a) N-(2-(6-fluoroimidazo[1,2-a]pyridin-3-yl)-5-nitropyrimidin-4-yl)-2,2-dimethyl-3,4-dihydro-2H-pyrano[2,3-b]pyridin-4-amine

Obtained as a yellow solid (83%) from 3-(4-chloro-5-nitropyrimidin-2-yl)-6-fluoroimidazo[1,2-a]pyridine **(Preparation 25)** and 2,2-dimethyl-3,4-dihydro-2H-pyrano[2,3-b]pyridin-4-amine **(Preparation 47)** following the experimental procedure as described in **Preparation 26a.**
LRMS (m/z): 436 (M+1)⁺.
1H NMR (300 MHz, CDCl₃) δ ppm 1.58 (s, 6H), 2.05 (dd, 1H), 2.49 (dd, 1H), 5.95 (m, 1H), 6.96 (dd, 1H), 7.39 (m, 1H), 7.69 (m, 1H), 7.79 (dd, 1H), 8.26 (m, 1H), 8.67 (d, 1H), 8.70 (s, 1H), 9.37 (s, 1H), 9.86 (dd, 1H).

### b) N⁴-(2,2-dimethyl-3,4-dihydro-2H-pyrano[2,3-b]pyridin-4-yl)-2-(6-fluoroimidazo[1,2-a]pyridin-3-yl)pyrimidine-4,5-diamine

Obtained as a green solid (92%) from N-(2-(6-fluoroimidazo[1,2-a]pyridin-3-yl)-5-nitropyrimidin-4-yl)-2,2-dimethyl-3,4-dihydro-2H-pyrano[2,3-b]pyridin-4-amine **(Preparation 48a)** following the experimental procedure as described in **Preparation 5b.**
LRMS (m/z): 406 (M+1)⁺.
1H NMR (300 MHz, DMSO-*d₆*) δ ppm 1.43 (s, 3H), 1.47 (s, 3H), 1.90 (t, 1H), 2.34 (dd, 1H), 5.12 (br s, 2H), 5.74 (m, 1H), 6.95 (m, 1H), 7.14 (d, 1H), 7.40 (m, 1H), 7.67 - 7.76 (m, 2H), 7.79 (s, 1H), 8.08 (d, 1H), 8.19 (s, 1H), 9.87 (m, 1H).

### PREPARATION 49

### 2-(6-Fluoroimidazo[1,2-a]pyridin-3-yl)-N4-(1,4-dioxaspiro[4.5]decan-8-yl)pyrimidine-4,5-diamine

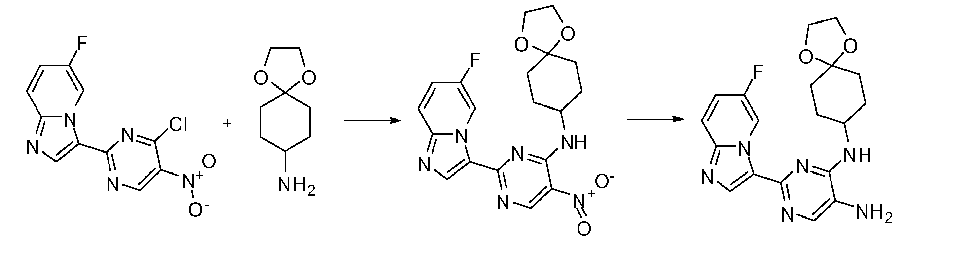

### a) 2-(6-Fluoroimidazo[1,2-a]pyridin-3-yl)-5-nitro-N-(1,4-dioxaspiro[4.5]decan-8-yl)pyrimidin-4-amine:

Obtained as a yellow solid (72%) from 3-(4-chloro-5-nitropyrimidin-2-yl)-6-fluoroimidazo[1,2-a]pyridine **(Preparation 25)** and 1,4-dioxaspiro[4.5]decan-8-amine following the experimental procedure as described in **Preparation 26a.**
LRMS (m/z): 415 (M+1)⁺.
1H NMR (300 MHz, CDCl₃) δ ppm 1.72 - 1.95 (m, 6H), 2.14 - 2.28 (m, 2H), 4.01 (s, 4H), 4.34 (m, 1H), 7.38 (m, 1H), 7.77 (m, 1H), 8.47 (m, 1H), 8.72 (s, 1H), 9.27 (s, 1H), 9.87 (m, 1H).

### b) 2-(6-Fluoroimidazo[1,2-a]pyridin-3-yl)-N4-(1,4-dioxaspiro[4.5]decan-8-yl)pyrimidine-4,5-diamine:

Obtained as a pink solid (93%) from 2-(6-fluoroimidazo[1,2-a]pyridin-3-yl)-5-nitro-N-(1,4-dioxaspiro[4.5]decan-8-yl)pyrimidin-4-amine **(Preparation 49a)** following the experimental procedure as described in **Preparation 30b.**
LRMS (m/z): 385 (M+1)⁺.
1H NMR (300 MHz, CDCl₃) δ ppm 1.52 - 2.10 (m, 8H), 3.90 (s, 4H), 4.09 (m, 1H), 5.06 (br s, 2H), 6.50 (d, 1H), 7.42 (dt, 1H), 7.69 (s, 1H), 7.74 (dd, 1H), 8.22 (s, 1H), 9.95 (dd, 1H).

### PREPARATION 50

### 2-((1r,4r)-4-Aminocyclohexyl)acetonitrile

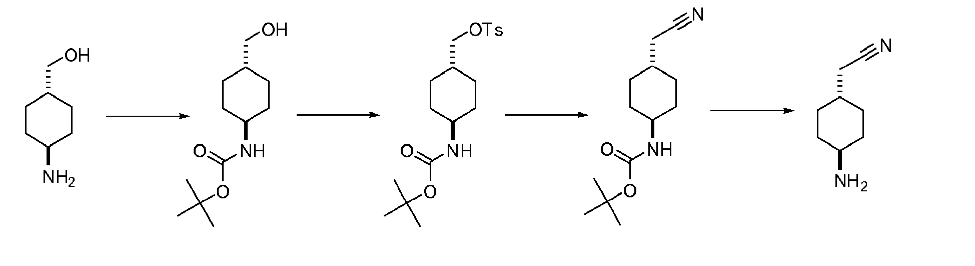

### a) tert-Butyl (1 r,4r)-4-(hydroxymethyl)cyclohexylcarbamate

Di-tert-butyl dicarbonate (3.04 g, 13.9 mmol) was added to a stirred solution of ((1r,4r)-4-aminocyclohexyl)methanol (1.50 g, 11.6 mmol) in tetrahydrofuran (20 mL). After stirring overnight at room temperature, the mixture was evaporated and partitioned between ethyl acetate and water. The organic layer was washed with water, brine, dried (MgSO₄) and evaporated. The residue was treated with hexanes and the suspension was filtered to give the title compound (2.11 g, 79%) as a white solid.
LRMS (m/z): 228 (M-H)⁺.
1H NMR (300 MHz, DMSO-*d₆*) δ ppm 0.84 - 0.95 (m, 2H), 1.05 - 1.18 (m, 2H), 1.20 - 1.29 (m, 2H), 1.40 (s, 9H), 1.71 -1.80 (m, 3H), 3.14 (m, 1H), 3.21 (t, 2H), 4.41 (t, 1H), 6.73 (d, 1H).

### b) ((1r,4r)-4-(tert-Butoxycarbonylamino)cyclohexyl)methyl 4-methylbenzene-sulfonate

A solution of 4-methylbenzene-1-sulfonyl chloride (2.28 g, 11.96 mmol) in dichloromethane was added to a solution of *tert*-butyl (1r,4r)-4-(hydroxymethyl)cyclohexylcarbamate **(Preparation 50a,** 2.11 g, 9.2 mmol) and triethylamine (1.59 mL, 11.4 mmol) in dichloromethane (50 mL) and the mixture was stirred overnight at room temperature. The mixture was washed with 1 M aqueous sodium hydroxide solution and the organic layer was dried (MgSO₄) evaporated and the residue was purified by flash chromatography (diethyl ether/hexanes) to give the title compound (2.91 g, 83%) as a white solid.
LRMS (m/z): 382 (M-H)⁺.
1 H NMR (300 MHz, CDCl₃) δ ppm 0.90 - 1.12 (m, 4H), 1.43 (s, 3H), 1.78 (dd, 2H), 1.99 (d, 2H), 3.34 (m, 1H), 3.46 (t, 1H), 3.81 (d, 2H), 4.37 (m, 1H), 7.34 (d, 2H), 7.77 (d, 2H).

### c) tert-Butyl (1 r,4r)-4-(cyanomethyl)cyclohexylcarbamate

Sodium cyanide (0.38 g, 7.8 mmol) was added to a solution of ((1r,4r)-4-(tert-butoxycarbonylamino)cyclohexyl)methyl 4-methylbenzene-sulfonate **(Preparation 50b,** 1.00 g, 2.6 mmol) in dimethylsulphoxide (10 mL) and the mixture was stirred and heated to 55 °C. After stirring for 20 hours, the mixture was diluted with ethyl acetate and washed with aqueous potassium carbonate solution, water, brine, dried (MgSO₄) and evaporated. The residue was purified by flash chromatography (100% dichloromethane to 95:5 dichloromethane/methanol) to give the title compound (0.450 g, 72%) as a white solid.
LRMS (m/z): 239 (M+H)⁺.
1H NMR (300 MHz, CDCl₃) δ ppm 1.06 - 1.25 (m, 4H), 1.44 (s, 9H), 1.65 (m, 1H), 1.90 (d, 2H), 2.06 (d, 2H), 2.25 (d, 2H), 3.39 (m, 1H), 4.38 (m, 1H).

### d) 2-((1r,4r)-4-Aminocyclohexyl)acetonitrile hydrochloride

A solution of tert-butyl (1r,4r)-4-(cyanomethyl)cyclohexylcarbamate **(Preparation 50c,** 0.348 g, 1.46 mmol) in 4M hydrogen chloride in dioxane (3.65 mL) was stirred overnight at room temperature. The mixture was evaporated in vacuo and treated with diethyl ether and the resultant suspension was filtered to give the hydrochloride salt of the title compound (0.226 g, 89%) as a white solid.
LRMS (m/z): 139 (M+H)⁺.
1H NMR (300 MHz, DMSO-*d₆*) δ ppm 1.14 (ddd, 2H), 1.37 (ddd, 2H), 1.60 (m, 1H), 1.83 (d, 2H), 1.99 (d, 2H), 2.50 (d, 2H), 2.94 (m, 1H), 8.08 (br s, 2H).

### PREPARATION 51

### 2-((1r,4r)-4-(5-Amino-2-(6-fluoroimidazo[1,2-a]pyridin-3-yl)pyrimidin-4-ylamino)cyclohexyl)acetonitrile

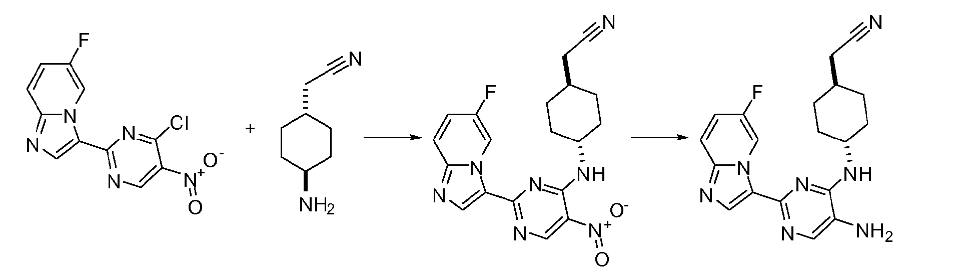

### a) 2-((1r,4r)-4-(2-(6-Fluoroimidazo[1,2-a]pyridin-3-yl)-5-nitropyrimidin-4-ylamino)cyclohexyl)acetonitrile

Obtained as a yellow solid (81%) from 3-(4-chloro-5-nitropyrimidin-2-yl)-6-fluoroimidazo[1,2-a]pyridine **(Preparation 25)** and 2-((1r,4r)-4-aminocyclohexyl)acetonitrile hydrochloride **(Preparation 50)** following the experimental procedure as described in **Preparation 26a.**
LRMS (m/z): 396 (M+1)⁺.
1H NMR (300 MHz, CDCl₃) δ ppm 1.27 (d, 1H), 1.37 - 1.61 (m, 4H), 1.85 (m, 1H), 2.10 (d, 2H), 2.39 (m, 3H), 4.25 (m, 1H), 7.39 (br t, 1H), 7.78 (dd, 1H), 8.41 (d, 1H), 8.72 (s, 1H), 9.27 (s, 1H), 9.88 (m, 1H).

### b) 2-((1r,4r)-4-(5-Amino-2-(6-fluoroimidazo[1,2-a]pyridin-3-yl)pyrimidin-4-ylamino)cyclohexyl)acetonitrile

Obtained as a pale green solid (92%) from 2-((1r,4r)-4-(2-(6-fluoroimidazo[1,2-a]pyridin-3-yl)-5-nitropyrimidin-4-ylamino)cyclohexyl)acetonitrile **(Preparation 51a)** following the experimental procedure as described in **Preparation 30b.**
LRMS (m/z): 366 (M+1)⁺.
1H NMR (300 MHz, CDCl₃) δ ppm 1.26 - 1.47 (m, 4H), 1.79 (m, 1H), 2.05 (m, 2H), 2.36 (d, 4H), 3.06 (br s, 2H), 4.06 (m, 1H), 4.85 (d, 1H), 7.21 (ddd, 1H), 7.66 (dd, 1H), 7.89 (s, 1H), 8.43 (s, 1H), 9.95 (dd, 1H).

### PREPARATION 52

### (1 r,4r)-4-((tert-Butyldiphenylsilyloxy)methyl)cyclohexanamine

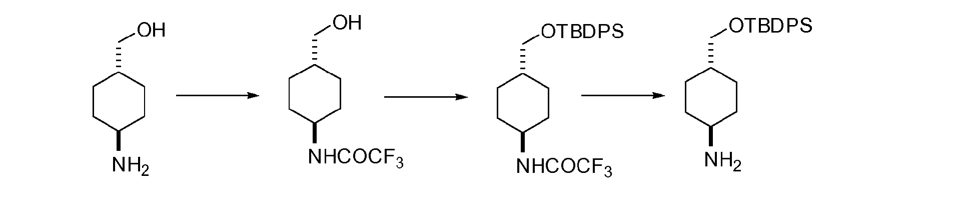

### a) 2,2,2-Trifluoro-N-((1r,4r)-4-(hydroxymethyl)cyclohexyl)acetamide

Ethyl 2,2,2-trifluoroacetate (2.0 mL, 16.8 mmol) was added to a solution of ((1r,4r)-4-aminocyclohexyl)methanol (1.50 g, 11.6 mmol) in 1,4-dioxane (20 mL) and the mixture was stirred overnight at room temperature. Further ethyl 2,2,2-trifluoroacetate (0.69 mL, 5.8 mmol) was added and the mixture was stirred for a further 30 minutes then evaporated and 0.1 M aqueous hydrochloric acid solution was added. The mixture was extracted with dichloromethane and the organic layer was washed with brine, dried (MgSO₄) and evaporated and the residue was purified by flash chromatography to give the title compound (1.64 g, 63%) as a white solid.
LRMS (m/z): 224 (M-1)⁺.
1H NMR (300 MHz, CDCl₃) δ ppm 1.04 - 1.18 (m, 2H), 1.19 - 1.28 (m, 2H), 1.34 (m, 1H), 1.49 (m, 1H), 1.90 (d, 2H), 2.09 (dd, 2H), 3.49 (t, 2H), 3.78 (m, 1H), 6.09 (br s, 1H).

### b) N-((1r,4r)-4-((tert-Butyldiphenylsilyloxy)methyl)cyclohexyl)-2,2,2-trifluoroacetamide:

Obtained as a white solid (76%) from 2,2,2-trifluoro-N-((1r,4r)-4-(hydroxymethyl)cyclohexyl)acetamide **(Preparation 52a)** following the experimental procedure as described in **Preparation 42b** followed by purification of the crude product by flash chromatography (100% dichloromethane to 5% methanol in dichloromethane).
LRMS (m/z): 462 (M-1)⁺.
1H NMR (300 MHz, CDCl₃) δ ppm 1.05 (s, 9H), 1.10 - 1.28 (m, 4H), 1.46 - 1.53 (m, 1H), 1.87 (d, 2H), 2.06 (d, 2H), 3.48 (d, 2H), 3.75 (m, 1H), 6.05 (d, 1H), 7.35 - 7.43 (m, 6H), 7.63 - 7.66 (m, 2H), 7.70 - 7.73 (m, 2H).

### c) (1r,4r)-4-((tert-Butyldiphenylsilyloxy)methyl)cyclohexanamine:

Aqueous sodium hydroxide solution (2M, 10 mL) was added to a solution of N-((1r,4r)-4-((tert-butyldiphenylsilyloxy)methyl)cyclohexyl)-2,2,2-trifluoroacetamide **(Preparation 52b,** 1.00 g, 2.2 mmol) in methanol (20 mL) and the mixture was stirred at room temperature overnight. The methanol was evaporated and the residue was partitioned between diethyl ether and water. The organic layer was washed with brine, dried (MgSO₄) and evaporated to give the title compound (0.800 g, 100%) as a colourless oil.
LRMS (m/z): 368 (M+1)⁺.
1H NMR (300 MHz, CDCl₃) δ ppm 1.10 (s, 9H), 1.61 (m, 5H), 1.89 (m, 4H), 2.64 (m, 1H), 3.52 (m, 2H), 7.45 (m, 6H), 7.71 (m, 4H).

### PREPARATION 53

### 9-((1r,4r)-4-((tert-Butyldiphenylsilyloxy)methyl)cyclohexyl)-2-(6-fluoroimidazo[1,2-a]pyridin-3-yl)-7H-purin-8(9H)-one

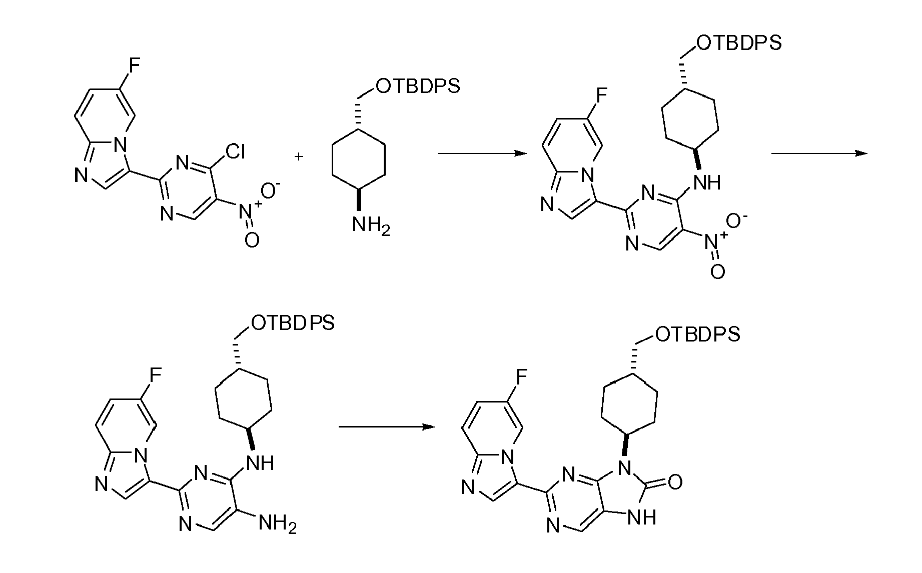

### a) N-((1r,4r)-4-((tert-Butyldiphenylsilyloxy)methyl)cyclohexyl)-2-(6-fluoroimidazo[1,2-a]pyridin-3-yl)-5-nitropyrimidin-4-amine

Obtained as a yellow solid (86%) from 3-(4-chloro-5-nitropyrimidin-2-yl)-6-fluoroimidazo[1,2-a]pyridine **(Preparation 25)** and (1r,4r)-4-((tert-butyldiphenylsilyloxy)methyl)cyclohexanamine **(Preparation 52)** following the experimental procedure as described in **Preparation 26a.**
LRMS (m/z): 625 (M+1)⁺.
1H NMR (300 MHz, CDCl₃) δ ppm 1.08 (s, 9H), 1.26 - 1.53 (m, 4H), 1.65 (m, 1H), 2.01 (d, 2H), 2.31 (d, 2H), 3.56 (br s, 2H), 4.19 (m, 1H), 7.42 - 7.43 (m, 6H), 7.68 (m, 4H), 7.77 (m, 1H), 8.41 (br s, 1H), 8.72 (s, 1H), 9.27 (s, 1H), 9.87 (br s, 1H).

### b) N4-((1r,4r)-4-((tert-Butyldiphenylsilyloxy)methyl)cyclohexyl)-2-(6-fluoroimidazo[1,2-a]pyridin-3-yl)pyrimidine-4,5-diamine

Obtained as a pale green solid (94%) from N-((1r,4r)-4-((tert-butyldiphenylsilyloxy)methyl)cyclohexyl)-2-(6-fluoroimidazo[1,2-a]pyridin-3-yl)-5-nitropyrimidin-4-amine **(Preparation 53a)** following the experimental procedure as described in **Preparation 30b.**
LRMS (m/z): 595 (M+1)⁺.
1H NMR (300 MHz, CDCl₃) δ ppm 1.09 (s, 9H), 1.26 - 1.33 (m, 4H), 1.62 (m, 3H), 1.99 (m, 2H), 2.30 (br s, 1H), 3.04 (br s, 1H), 3.56 (d, 2H), 4.03 (m, 1H), 4.84 (d, 1H), 7.20 (ddd, 1H), 7.38 - 7.48 (m, 6H), 7.64 - 7.71 (m, 5H), 7.88 (s, 1H), 8.44 (s, 1H), 9.97 (dd, 1H).

### c) 9-((1r,4r)-4-((tert-Butyidiphenylsilyloxy)methyl)cyclohexyl)-2-(6-fluoroimidazo[1,2-a]pyridin-3-yl)-7H-purin-8(9H)-one

Obtained as a beige solid (91%) from N4-((1r,4r)-4-((tert-butyldiphenylsilyloxy)methyl)cyclohexyl)-2-(6-fluoroimidazo[1,2-a]pyridin-3-yl)pyrimidine-4,5-diamine **(Preparation 53b)** following the experimental procedure as described in **Preparation 42d.**
LRMS (m/z): 621 (M+1)⁺.
1H NMR (300 MHz, DMSO-*d₆*) δ ppm 1.06 (s, 9H), 1.18 - 1.34 (m, 2H), 1.76 (m, 1H), 1.90 (d, 2H), 1.99 (d, 2H), 2.46 (dd, 2H), 3.59 (d, 2H), 4.27 (br t, 1H), 7.41 (ddd, 1H), 7.49 - 7.51 (m, 6H), 7.58 (ddd, 1H), 7.66 - 7.70 (m, 4H), 7.88 (dd, 1H), 8.40 (s, 1H), 8.47 (s, 1H), 9.96 (dd, 1H).

### PREPARATION 54

### (1r,4r)-4-(tert-Butyldiphenylsilyloxy)cyclohexanamine

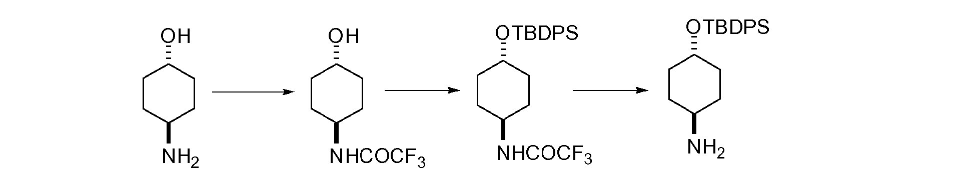

### a) 2,2,2-Trifluoro-N-((1r,4r)-4-hydroxycyclohexyl)acetamide

Obtained as a white solid (72%) from (1r,4r)-4-aminocyclohexanol following the experimental procedure as described in **Preparation 52a.**
LRMS (m/z): 210 (M-1)⁺.
1H NMR (300 MHz, CDCl₃) δ ppm 1.26 (m, 4H), 2.05 (m, 4H), 3.65 (m, 1H), 3.81 (m, 1H), 6.06 (m, 1H).

### b) N-((1r,4r)-4-(tert-Butyldiphenylsilyloxy)cyclohexyl)-2,2,2-trifluoroacetamide

Obtained as a white solid (71%) from 2,2,2-trifluoro-N-((1r,4r)-4-hydroxycyclohexyl)acetamide **(Preparation 54a)** following the experimental procedure as described in **Preparation 52b.**
LRMS (m/z): 448 (M-1)⁺.
1H NMR (300 MHz, CDCl₃) δ ppm 1.05 (s, 9H), 1.11 (ddd, 2H), 1.51 (ddd, 2H), 1.81 (dd, 2H), 1.95 (dd, 2H), 3.60 (m, 1H), 3.78 (m, 1H), 5.95 (d, 1H), 7.34 - 7.46 (m, 6H), 7.64 (m, 2H), 7.67 (m, 2H).

### c) (1 r,4r)-4-(tert-Butyldiphenylsilyloxy)cyclohexanamine

Obtained as a white solid (97%) from N-((1r,4r)-4-(tert-butyldiphenylsilyloxy)cyclohexyl)-2,2,2-trifluoroacetamide **(Preparation 54b)** following the experimental procedure as described in **Preparation 52c.**
LRMS (m/z): 354 (M+1)⁺.
1H NMR (300 MHz CDCl₃) δ ppm 1.06 (s, 9H), 1.42 (m, 4H), 1.78 (m, 4H), 2.65 (m, 1H), 3.60 (m, 1H), 7.35- 7.44 (m, 6H), 7.67-70 (m, 4H).

### PREPARATION 55

### 9-((1r,4r)-4-(tert-Butyldiphenylsilyloxy)cyclohexyl)-2-(6-fluoroimidazo[1,2-a]pyridin-3-yl)-7H-purin-8(9H)-one

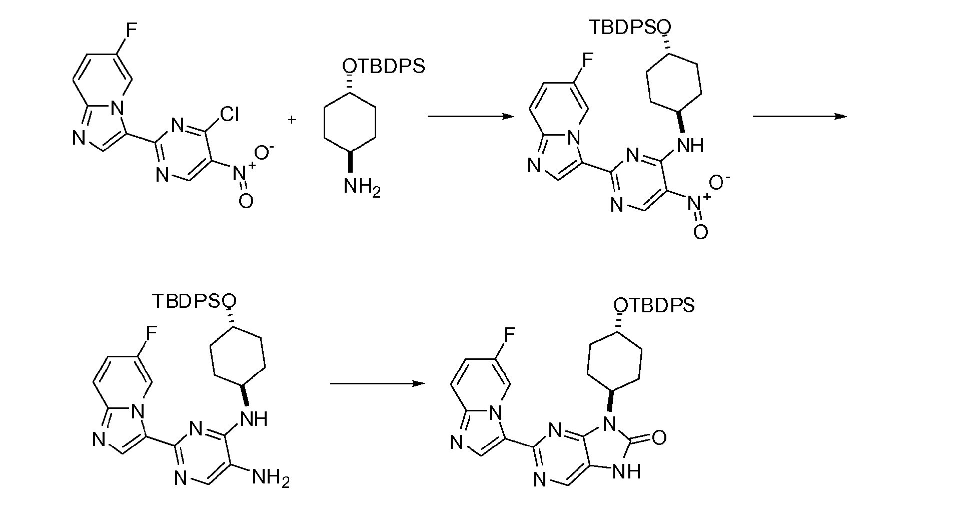

### a) N-((1r,4r)-4-(tert-Butyldiphenylsilyloxy)cyclohexyl)-2-(6-fluoroimidazo[1,2-a]pyridin-3-yl)-5-nitropyrimidin-4-amine

Obtained as a yellow solid (81%) from 3-(4-chloro-5-nitropyrimidin-2-yl)-6-fluoroimidazo[1,2-a]pyridine **(Preparation 25)** and (1r,4r)-4-(tert-butyldiphenylsilyloxy)cyclohexanamine **(Preparation 54)** following the experimental procedure as described in **Preparation 26a.**
LRMS (m/z): 611 (M+1)⁺.
1H NMR (300 MHz CDCl₃) δ ppm 1.09 (s, 9H), 1.67-1.92 (m, 8H), 3.04 (m, 1H), 4.23 (m, 1H), 7.40- 7.42 (m, 6H), 7.69-7.79 (m, 5H), 8.33 (m, 1H), 8.71 (s, 1H), 9.24 (s, 1H), 9.87 (s, 1H).

### b) N⁴-((1r,4r)-4-(tert-Butyldiphenylsilyloxy)cyclohexyl)-2-(6-fluoroimidazo[1,2-a]pyridin-3-yl)pyrimidine-4,5-diamine

Obtained as an off white solid (82%) from N-((1r,4r)-4-(tert-butyldiphenylsilyloxy)cyclohexyl)-2-(6-fluoroimidazo[1,2-a]pyridin-3-yl)-5-nitropyrimidin-4-amine **(Preparation 55a)** following the experimental procedure as described in **Preparation 30b.**
LRMS (m/z): 581 (M+1)⁺.
1H NMR (300 MHz CDCl₃) δ ppm 1.08 (s, 9H), 1.15-1.31 (m, 2H), 1.60-1.72 (m, 2H), 1.89-1.94 (m, 2H), 2.17-2.18 (m, 2H), 3.68 (m, 1H), 4.04 (m, 1H), 7.23 (m, 1H), 7.36-7.44 (m, 6H), 7.63-7.72 (m, 5H), 7.83 (s, 1H), 8.42 (s, 1H), 9.94 (m, 1H).

### c) 9-((1r,4r)-4-(tert-Butyidiphenyisilyloxy)cyclohexyl)-2-(6-fluoroimidazo[1,2-a]pyridin-3-yl)-7H-purin-8(9H)-one

Obtained as a beige solid (91%) from N⁴-((1r,4r)-4-(tert-butyldiphenylsilyloxy)cyclohexyl)-2-(6-fluoroimidazo[1,2-a]pyridin-3-yl)pyrimidine-4,5-diamine **(Preparation 55b)** following the experimental procedure as described in **Preparation 42d.**
LRMS (m/z): 607 (M+1)⁺.
1H NMR (300 MHz CDCl₃) δ ppm 1.09 (s, 9H), 1.64 (m, 2H), 1.80 (m, 2H), 2.02 (m, 2H), 2.35 (m, 2H), 3.85 (m, 1H), 4.40 (m, 1H), 7.24 (m, 1H), 7.41-7.48 (m, 6H), 7.71-7.74 (m, 5H), 8.30 (s, 1H), 8.43 (s, 1H), 8.96 (m, 1H), 9.90 (s, 1H).

### PREPARATION 56

### 9-((1R,4R)-4-(tert-butyidiphenylsilyloxy)-1,2,3,4-tetrahydronaphthalen-1-yl)-2-(6-fluoroimidazo[1,2-a]pyridin-3-yl)-7H-purin-8(9H)-one

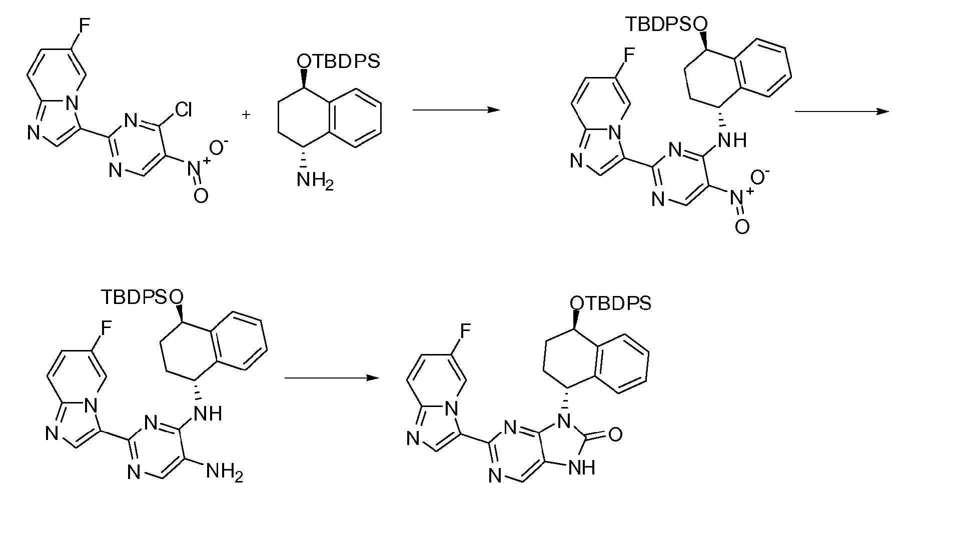

### a) N-((1R,4R)-4-(tert-butyldiphenylsilyloxy)-1,2,3,4-tetrahydronaphthalen-1-yl)-2-(6-fluoroimidazo[1,2-a]pyridin-3-yl)-5-nitropyrimidin-4-amine

Obtained as a yellow solid (87%) from 3-(4-chloro-5-nitropyrimidin-2-yl)-6-fluoroimidazo[1,2-a]pyridine **(Preparation 25)** and (1R,4R)-4-(tert-butyldiphenylsilyloxy)-1,2,3,4-tetrahydronaphthalen-1-amine (WO2009/48474) following the experimental procedure as described in **Preparation 26a** followed by purification of the crude product by flash chromatography (1:1 hexanes/ethyl acetate).
LRMS (m/z): 659 (M+1)⁺.
1H NMR (300 MHz, CDCl₃) δ ppm 1.11 (s, 9H), 1.59 - 1.70 (m, 1H), 1.98 (m, 2H), 2.63 (m, 1H), 4.93 (m, 1H), 5.79 (m, 1H), 7.23 - 7.31 (m, 6H), 7.34 - 7.46 (m, 5H), 7.67 - 7.81 (m, 4H), 8.57 (d, 1H), 8.73 (s, 1H), 9.30 (s, 1H), 9.92 (s, 1H).

### b) N⁴-((1R,4R)-4-(tert-butyidiphenylsilyloxy)-1,2,3,4-tetrahydronaphthalen-1-yl)-2-(6-fluoroimidazo[1,2-a]pyridin-3-yl)pyrimidine-4,5-diamine

Obtained as a green solid (75%) from N-((1R,4R)-4-(tert-butyldiphenylsilyloxy)-1,2,3,4-tetrahydronaphthalen-1-yl)-2-(6-fluoroimidazo[1,2-a]pyridin-3-yl)-5-nitropyrimidin-4-amine **(Preparation 56a)** following the experimental procedure as described in **Preparation 30b** followed by purification of the crude product by flash chromatography (0 - 5% methanol in dichloromethane).
LRMS (m/z): 629 (M+1)⁺.
1H NMR (300 MHz, CDCl₃) δ ppm 1.09 (s, 9H), 1.96 (m, 2H), 2.56 (m, 1H), 2.97 (m, 1H), 4.86 (m, 1H), 5.05 (br s, 1H), 5.08 (br s, 1H), 5.61 (m, 1H), 7.16 - 7.27 (m, 6H), 7.30 - 7.45 (m, 5H), 7.63 - 7.79 (m, 5H), 7.89 (s, 1H), 8.45 (s, 1H), 9.96 (s, 1H).

### c) 9-((1R,4R)-4-(tert-butyidiphenylsilyloxy)-1,2,3,4-tetrahydronaphthalen-1-yl)-2-(6-fluoroimidazo[1,2-a]pyridin-3-yl)-7H-purin-8(9H)-one

Obtained as a pale pink solid (67%) from N⁴-((1R,4R)-4-(tert-butyldiphenylsilyloxy)-1,2,3,4-tetrahydronaphthalen-1-yl)-2-(6-fluoroimidazo[1,2-a]pyridin-3-yl)pyrimidine-4,5-diamine **(Preparation 56b)** following the experimental procedure as described in **Preparation 42d** followed by purification of the crude product by flash chromatography (55 - 100% ethyl acetate in hexanes).
LRMS (m/z): 655 (M+1)⁺.
1H NMR (300 MHz, CDCl₃) δ ppm 1.16 (s, 9H), 2.01 (m, 1H), 2.19 (m, 2H), 2.35 (m, 1H), 5.22 (m, 1H), 5.87 (m, 1H), 6.90 (d, 1H), 7.09 - 7.23 (m, 6H), 7.37 - 7.45 (m, 6H), 7.63 - 7.82 (m, 4H), 8.32 (s, 1H), 9.08 (br s, 1H), 9.49 (s, 1H).

### PREPARATION 57

### (1r,4r)-4-(5-Amino-2-(6-fluoroimidazo[1,2-a]pyridin-3-yl)pyrimidin-4-ylamino)-1-methylcyclohexanol

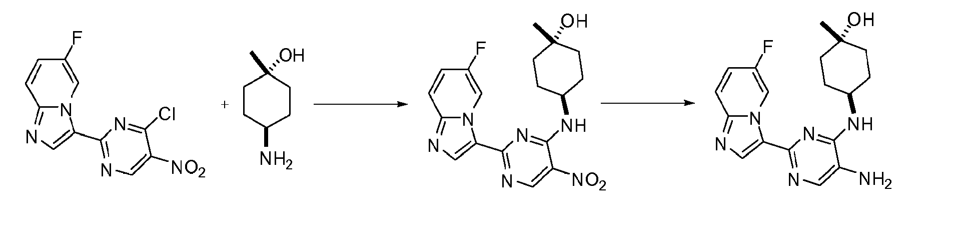

### a) (1r,4r)-4-(2-(6-Fluoroimidazo[1,2-a]pyridin-3-yl)-5-nitropyrimidin-4-ylamino)-1-methylcyclohexanol:

Obtained as a yellow solid (55%) from 3-(4-chloro-5-nitropyrimidin-2-yl)-6-fluoroimidazo[1,2-a]pyridine **(Preparation 25)** and (1r,4r)-4-amino-1-methylcyclohexanol hydrochloride (WO2005/9966 A1) following the experimental procedure as described in **Preparation 26a.**
LRMS (m/z): 387 (M+1)⁺.
1H NMR (300 MHz, DMSO-*d₆*) δ ppm 1.26 (br s, 4H), 1.64 (br s, 3H), 1.78 (m, 1H), 2.01 (m, 2H), 4.31 (m, 1H), 4.48 (s, 1H), 7.75 (m, 1H), 7.97 (m, 1H), 8.67 (s, 2H), 9.24 (s, 1H), 9.82 (m, 1H).

### b) (1r,4r)-4-(5-Amino-2-(6-fluoroimidazo[1,2-a]pyridin-3-yl)pyrimidin-4-ylamino)-1-methylcyclohexanol

Obtained as an off white solid (51%) from (1r,4r)-4-(2-(6-fluoroimidazo[1,2-a]pyridin-3-yl)-5-nitropyrimidin-4-ylamino)-1-methylcyclohexanol **(Preparation 57a)** following the experimental procedure as described in **Preparation 30b.**
LRMS (m/z): 357 (M+1)⁺.

### PREPARATION 58

### tert-butyl ((1r,4r)-4-(2-(6-fluoroimidazo[1,2-a]pyridin-3-yl)-8-oxo-7H-purin-9(8H)-yl)cyclohexyl)methylcarbamate

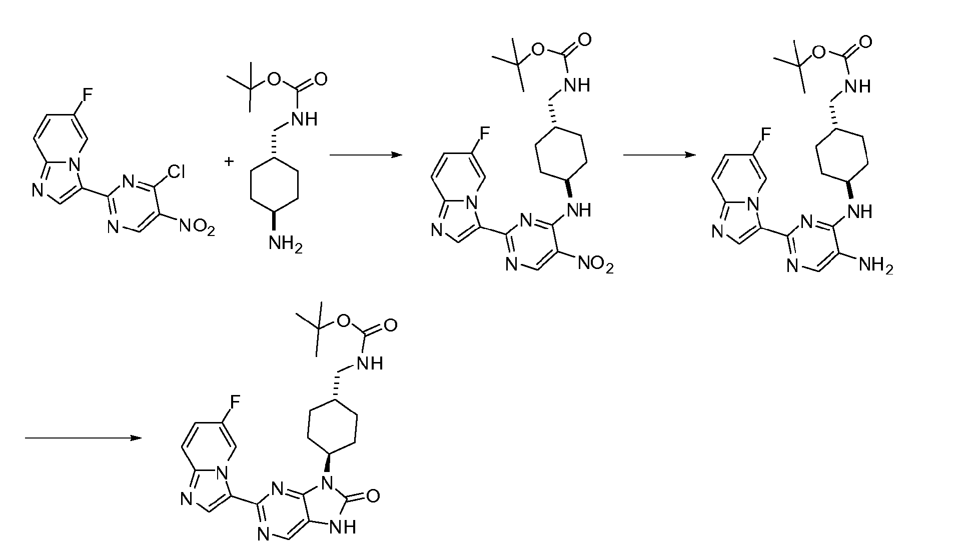

### a) tert-butyl ((1r,4r)-4-(2-(6-fluoroimidazo[1,2-a]pyridin-3-yl)-5-nitropyrimidin-4-ylamino)cyclohexyl)methylcarbamate

Obtained as a yellow solid (77%) from 3-(4-chloro-5-nitropyrimidin-2-yl)-6-fluoroimidazo[1,2-a]pyridine **(Preparation 25)** and tert-butyl ((1r,4r)-4-aminocyclohexyl)methylcarbamate (US2006/281712) following the experimental procedure as described in **Preparation 26a.**
LRMS (m/z): 486 (M+1)⁺.
1H NMR (300 MHz, CDCl₃) δ ppm 1.16 - 1.32 (m, 2H), 1.37 - 1.79 (m, 12H), 1.97 (d, 2H), 2.32 (d, 2H), 3.09 (m, 2H), 4.20 (m, 1H), 4.67 (br s, 1H), 7.38 (m, 1H), 7.78 (dd, 1H), 8.40 (d, 1H), 8.72 (s, 1H), 9.27 (s, 1H), 9.88 (dd, 1H).

### b) tert-butyl ((1r,4r)-4-(5-amino-2-(6-fluoroimidazo[1,2-a]pyridin-3-yl)pyrimidin-4-ylamino)cyclohexyl)methylcarbamate

Obtained as a pale brown solid (95%) from tert-butyl ((1r,4r)-4-(2-(6-fluoroimidazo[1,2-a]pyridin-3-yl)-5-nitropyrimidin-4-ylamino)cyclohexyl)methylcarbamate **(Preparation 58a)** following the experimental procedure as described in **Preparation 30b.**
LRMS (m/z): 456 (M+1)⁺.
1H NMR (300 MHz, CDCl₃) δ ppm 1.12 - 1.34 (m, 4H), 1.39 - 1.81 (m, 10H), 1.92 (d, 2H), 2.30 (m, 2H), 2.90 - 3.12 (m, 4H), 4.03 (m, 1H), 4.69 (br s, 1H), 7.19 (m, 1H), 7.66 (dd, 1H), 7.87 (s, 1H), 8.43 (s, 1H), 9.97 (dd, 1H).

### c) tert-butyl ((1r,4r)-4-(2-(6-fluoroimidazo[1,2-a]pyridin-3-yl)-8-oxo-7H-purin-9(8H)-yl)cyclohexyl)methylcarbamate

Obtained as a beige solid (77%) from tert-butyl ((1r,4r)-4-(5-amino-2-(6-fluoroimidazo[1,2-a]pyridin-3-yl)pyrimidin-4-ylamino)cyclohexyl)methylcarbamate **(Preparation 58b)** following the experimental procedure as described in **Preparation 42d.**
LRMS (m/z): 482 (M+1)⁺.
1H NMR (300 MHz, DMSO-*d₆*) δ ppm 0.97 - 1.61 (m, 12H), 1.74 - 1.92 (m, 4H), 2.37 (m, 2H), 2.87 (m, 2H), 4.23 (m, 2H), 6.90 (t, 1H), 7.54 (m, 1H), 7.84 (dd, 1H), 8.37 (s, 1H), 8.43 (s, 1H), 9.92 (dd, 1H), 11.50 (br s, 1H).

### PREPARATION 59

### tert-butyl (1r,4r)-4-(2-(6-fluoroimidazo[1,2-a]pyridin-3-yl)-8-oxo-7H-purin-9(8H)-yl)cyclohexylcarbamate

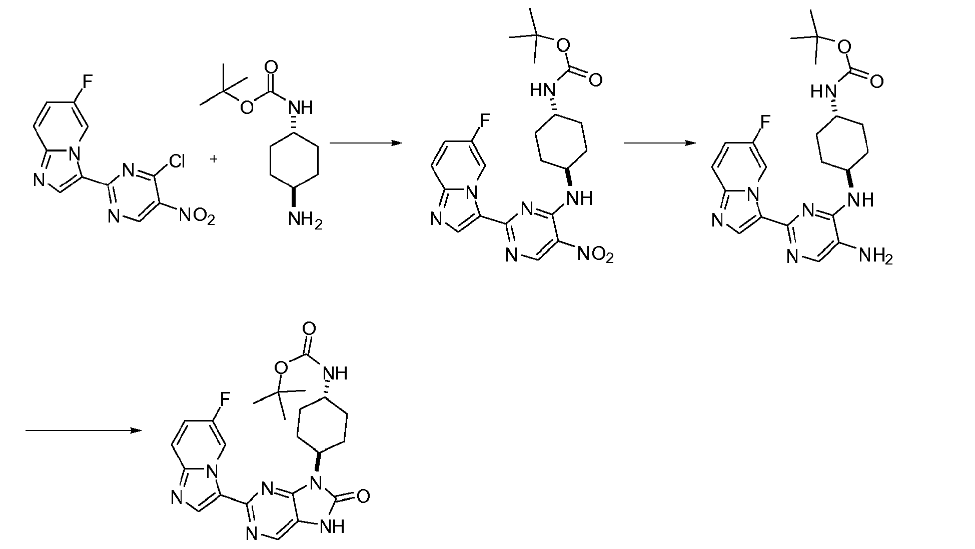

### a) tert-butyl (1r,4r)-4-(2-(6-fluoroimidazo[1,2-a]pyridin-3-yl)-5-nitropyrimidin-4-ylamino)cyclohexylcarbamate

Obtained as a yellow solid (64%) from 3-(4-chloro-5-nitropyrimidin-2-yl)-6-fluoroimidazo[1,2-a]pyridine **(Preparation 25)** and tert-butyl (1r,4r)-4-aminocyclohexylcarbamate following the experimental procedure as described in **Preparation 26a.**
LRMS (m/z): 472 (M+1)⁺.
1H NMR (300 MHz, DMSO-*d₆*) δ ppm 1.11 - 1.49 (m, 11H), 1.66 (q, 2H), 1.90 (m, 2H), 2.10 (m, 2H), 4.14 (m, 1H), 6.93 (d, 1H), 7.72 (m, 1H), 7.94 (dd, 1H), 8.53 (d, 1H), 8.63 (s, 1H), 9.21 (s, 1H), 9.78 (m, 1H).

### b) tert-butyl (1r,4r)-4-(5-amino-2-(6-fluoroimidazo[1,2-a]pyridin-3-yl)pyrimidin-4-ylamino)cyclohexylcarbamate

Obtained as a white solid (92%) from tert-butyl (1r,4r)-4-(2-(6-fluoroimidazo[1,2-a]pyridin-3-yl)-5-nitropyrimidin-4-ylamino)cyclohexylcarbamate **(Preparation 59a)** following the experimental procedure as described in **Preparation 30b.**
LRMS (m/z): 442 (M+1)⁺.
1H NMR (300 MHz, CDCl₃) δ ppm 1.32 - 1.51 (m, 13H), 2.14 (m, 2H), 2.28 (m, 2H), 3.10 (br s, 2H), 3.52 (m, 1H), 4.04 (m, 1H), 4.50 (br s, 1H), 7.20(m, 1H), 7.66 (dd, 1H), 7.87 (s, 1H), 8.43 (s, 1H), 9.95 (dd, 1H).

### c) tert-butyl (1r,4r)-4-(2-(6-fluoroimidazo[1,2-a]pyridin-3-yl)-8-oxo-7H-purin-9(8H)-yl)cyclohexylcarbamate

Obtained as a pale pink solid (82%) from tert-butyl (1r,4r)-4-(5-amino-2-(6-fluoroimidazo[1,2-a]pyridin-3-yl)pyrimidin-4-ylamino)cyclohexylcarbamate **(Preparation 59b)** following the experimental procedure as described in **Preparation 42d.**
LRMS (m/z): 468 (M+1)⁺.
1H NMR (300 MHz, DMSO-*d₆*) δ ppm 1.31 - 1.46 (m, 11H), 1.82 (m, 2H), 1.97 (m, 2H), 2.43 (m, 2H), 3.42 (m, 1H), 4.20 (m, 1H), 6.84 (d, 1H), 7.56 (m, 1H), 7.84 (dd, 1H), 8.36 (s, 1H), 8.45 (s, 1H), 9.92 (m, 1H), 11.50 (s, 1H).

### PREPARATION 60

### N⁴-Cyclobutyl-2-(6-fluoroimidazo[1,2-a]pyridin-3-yl)pyrimidine-4,5-diamine

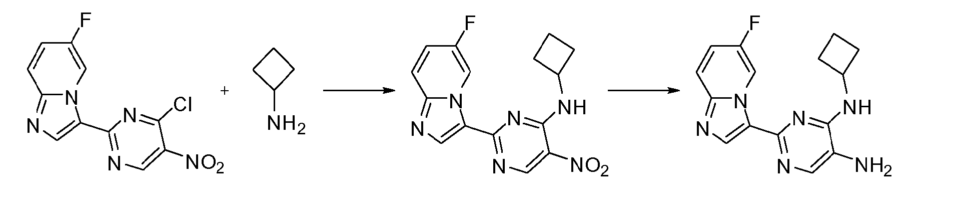

### a) N-Cyclobutyl-2-(6-fluoroimidazo[1,2-a]pyridin-3-yl)-5-nitropyrimidin-4-amine:

Obtained as a yellow solid (73%) from 3-(4-chloro-5-nitropyrimidin-2-yl)-6-fluoroimidazo[1,2-a]pyridine **(Preparation 25)** and cyclobutylamine following the experimental procedure as described in **Preparation 26a.**
LRMS (m/z): 329 (M+1)⁺.

### b) N⁴-Cyclobutyl-2-(6-fluoroimidazo[1,2-a]pyridin-3-yl)pyrimidine-4,5-diamine:

Obtained as an off white solid (99%) from N-cyclobutyl-2-(6-fluoroimidazo[1,2-a]pyridin-3-yl)-5-nitropyrimidin-4-amine **(Preparation 60a)** following the experimental procedure as described in **Preparation 30b.**
LRMS (m/z): 299 (M+1)⁺.

### PREPARATION 61

### (R)-2-(6-Fluoroimidazo[1,2-a]pyridin-3-yl)-N⁴-(3-methylbutan-2-yl)pyrimidine-4,5-diamine

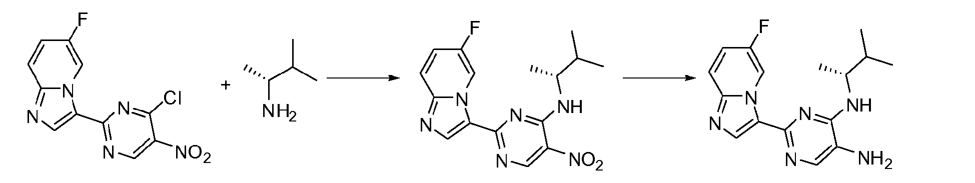

### a) (R)-2-(6-fluoroimidazo[1,2-a]pyridin-3-yl)-N-(3-methylbutan-2-yl)-5-nitropyrimidin-4-amine

Obtained as a yellow solid (85%) from 3-(4-chloro-5-nitropyrimidin-2-yl)-6-fluoroimidazo[1,2-a]pyridine **(Preparation 25)** and (R)-3-methylbutan-2-amine following the experimental procedure as described in **Preparation 26a.**
LRMS (m/z): 345 (M+1)⁺.

### b) (R)-2-(6-Fluoroimidazo[1,2-a]pyridin-3-yl)-N⁴-(3-methylbutan-2-yl)pyrimidine-4,5-diamine

Obtained as a beige solid (99%) from (R)-2-(6-fluoroimidazo[1,2-a]pyridin-3-yl)-N-(3-methylbutan-2-yl)-5-nitropyrimidin-4-amine **(Preparation 61a)** following the experimental procedure as described in **Preparation 30b.**
LRMS (m/z): 315 (M+1)⁺.
1H NMR (300 MHz, DMSO-*d₆*) δ ppm 0.92 (d, 3H), 0.98 (d, 3H), 1.17 (d, 3H), 1.98 (m, 1H), 4.17 (m, 1H), 5.07 (br s, 2H), 6.34 (d, 1H), 7.42 (m, 1H), 7.67 (s, 1H), 7.74 (dd, 1H), 8.17 (s, 1H), 9.93 (dd, 1H).

### PREPARATION 62

### 2-(6-fluoroimidazo[1,2-a]pyridin-3-yl)-N4-(1-methoxypropan-2-yl)pyrimidine-4,5-diamine

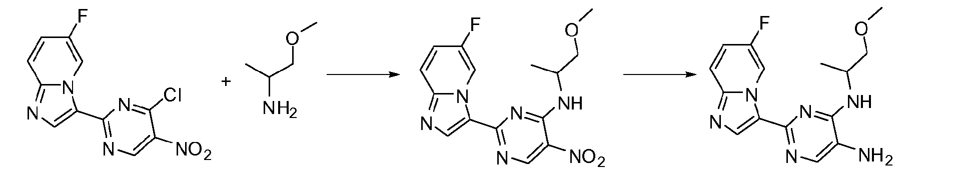

### a) 2-(6-fluoroimidazo[1,2-a]pyridin-3-yl)-N-(1-methoxypropan-2-y1)-5-nitropyrimidin-4-amine

Obtained as a yellow solid (81%) from 3-(4-chloro-5-nitropyrimidin-2-yl)-6-fluoroimidazo[1,2-a]pyridine **(Preparation 25)** and 1-methoxypropan-2-amine following the experimental procedure as described in **Preparation 26a.**
LRMS (m/z): 347 (M+1)⁺.

### b) 2-(6-fiuoroimidazo[1,2-a]pyridin-3-yl)-N⁴-(1-methoxypropan-2-yi)pyrimidine-4,5-diamine

Obtained as a white solid (100%) from 2-(6-fluoroimidazo[1,2-a]pyridin-3-yl)-N-(1-methoxypropan-2-yl)-5-nitropyrimidin-4-amine **(Preparation 62a)** following the experimental procedure as described in **Preparation 30b.**
LRMS (m/z): 317 (M+1)⁺.
1H NMR (300 MHz, DMSO-*d₆*) δ ppm 1.27 (d, 3H), 3.31 - 3.40 (m, 4H), 3.54 (dd, 1H), 4.45 (m, 1H), 5.06 (br s, 2H), 6.45 (d, 1H), 7.42 (m, 1H), 7.70 (s, 1H), 7.73 (dd, 1H), 8.19 (s, 1H), 9.91 (dd, 1H).

### PREPARATION 63

### (R)-tert-butyl 3-(5-amino-2-(6-fluoroimidazo[1,2-a]pyridin-3-yl)pyrimidin-4-ylamino)butanoate

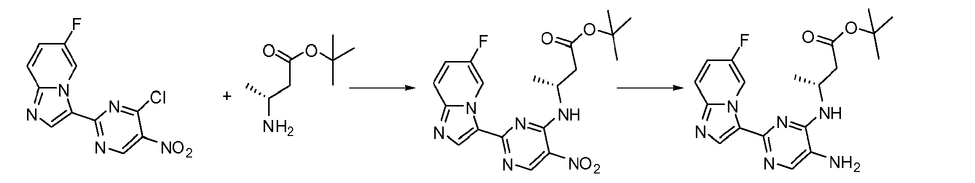

### a) (R)-tert-butyl 3-(2-(6-fluoroimidazo[1,2-a]pyridin-3-yl)-5-nitropyrimidin-4-ylamino)butanoate

Obtained as a yellow solid (82%) from 3-(4-chloro-5-nitropyrimidin-2-yl)-6-fluoroimidazo[1,2-a]pyridine **(Preparation 25)** and (R)-tert-butyl 3-aminobutanoate following the experimental procedure as described in **Preparation 26a.**
LRMS (m/z): 417 (M+1)⁺.
1H NMR (300 MHz, DMSO-*d₆*) δ ppm 1.19 - 1.45 (m, 12H), 2.67 (dd, 1H), 2.79 (dd, 1H), 4.88 (m, 1H), 7.69 (m, 1H), 7.91 (dd, 1H), 8.61 (s, 1H), 8.85 (d, 1H), 9.22 (s, 1H), 9.77 (m, 1H).

### b) (R)-tert-butyl 3-(5-amino-2-(6-fluoroimidazo[1,2-a]pyridin-3-yl)pyrimidin-4-ylamino)butanoate

Obtained as a grey solid (92%) from (R)-tert-butyl 3-(2-(6-fluoroimidazo[1,2-a]pyridin-3-yl)-5-nitropyrimidin-4-ylamino)butanoate (**Preparation 63a**) following the experimental procedure as described in **Preparation 30b.**
LRMS (m/z): 387 (M+1)⁺.
1H NMR (300 MHz, DMSO-*d₆*) δ ppm 1.30 (d, 3H), 1.35 (s, 9H), 2.40 (dd, 1H), 2.68 (dd, 1H), 4.63 (m, 1H), 5.01 (br s, 2H), 6.54 (d, 1H), 7.42 (m, 1H), 7.70 (s, 1H), 7.73 (dd, 1H), 8.20 (s, 1H), 9.90 (dd, 1H).

### PREPARATION 64

### N⁴-(1-(2,2-Difluoroethyl)piperidin-4-yl)-2-(6-fluoroimidazo[1,2-a]pyridin-3-yl)pyrimidine-4,5-diamine

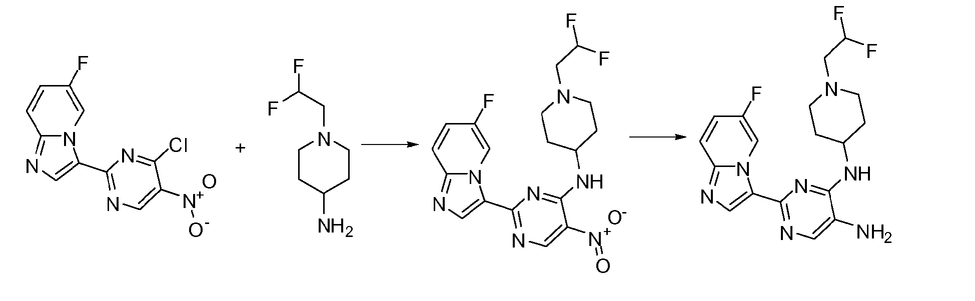

### a) N-(1-(2,2-Difluoroethyl)piperidin-4-yl)-2-(6-fluoroimidazo[1,2-a]pyridin-3-yl)-5-nitropyrimidin-4-amine

Obtained as a yellow solid (68%) from 3-(4-chloro-5-nitropyrimidin-2-yl)-6-fluoroimidazo[1,2-a]pyridine (**Preparation 25**) and 1-(2,2-difluoroethyl)piperidin-4-amine following the experimental procedure as described in **Preparation 26a.**
LRMS (m/z): 422 (M+1)⁺.
1H NMR (300 MHz, DMSO-d₆) δ ppm 1.82 (m, 2H), 1.89 (m, 2H), 2.42 (m, 2H), 2.78 (m, 2H), 3.00 (m, 2H), 4.23 (m, 1H), 6.18 (tt, 1H), 7.68-7.74 (m, 1H), 7.91-7.97 (dd, 1H), 8.58 (d, 1H), 8.63 (s, 1H), 9.21 (s, 1H), 9.76 (m, 1H).

### b) N⁴-(1-(2,2-Difluoroethyl)piperidin-4-yl)-2-(6-fluoroimidazo[1,2-a]pyridin-3-yl)pyrimidine-4,5-diamine

Obtained as an off white solid (88%) from N-(1-(2,2-difluoroethyl)piperidin-4-yl)-2-(6-fluoroimidazo[1,2-a]pyridin-3-yl)-5-nitropyrimidin-4-amine (**Preparation 64a**) following the experimental procedure as described in **Preparation 30b.**
LRMS (m/z): 392 (M+1)⁺.
1H NMR (300 MHz, CDCl₃) δ ppm 1.71 (m, 2H), 2.17 (m, 2H), 2.49 (m, 2H), 2.82 (m, 2H), 3.03 (m, 2H), 4.15 (m, 1H), 5.20 (d, 1H), 5.93 (tt, 1H), 7.21 (m, 1H), 7.63-7.68 (dd, 1H), 7.84 (s, 1H), 8.38 (s, 1H), 9.93 (m, 1H).

### PREPARATION 65

### N⁴-(4,4-Difluorocyclohexyl)-2-(6-fluoroimidazo[1,2-a]pyridin-3-yl)pyrimidine-4,5-diamine

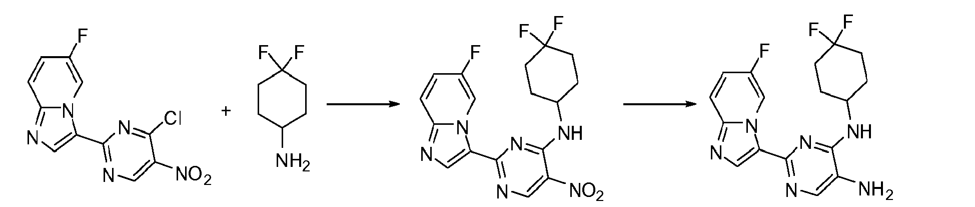

### a) N-(4,4-Difluorocyclohexyl)-2-(6-fluoroimidazo[1,2-a]pyridin-3-yl)-5-nitropyrimidin-4-amine

Obtained as a yellow solid (80%) from 3-(4-chloro-5-nitropyrimidin-2-yl)-6-fluoroimidazo[1,2-a]pyridine (**Preparation 25**) and 4,4-difluorocyclohexanamine following the experimental procedure as described in **Preparation 26a.**
LRMS (m/z): 393 (M+1)⁺.
1H NMR (300 MHz, DMSO-*d₆*) δ ppm 1.81 - 2.22 (m, 8H), 4.51 (td, 1H), 7.71 (ddd, 1H), 7.94 (dd, 1H), 8.62 (d, 1H), 8.71 (s, 1H), 9.24 (s, 1H), 9.83 (dd, 1H).

### b) N⁴-(4,4-Difluorocyclohexyl)-2-(6-fluoroimidazo[1,2-a]pyridin-3-yl)pyrimidine-4,5-diamine

Obtained as a pale brown solid (78%) from *N*-(4,4-difluorocyclohexyl)-2-(6-fluoroimidazo[1,2-a]pyridin-3-yl)-5-nitropyrimidin-4-amine (**Preparation 65a**) following the experimental procedure as described in **Preparation 5b.**
LRMS (m/z): 363 (M+1)⁺.
1H NMR (300 MHz, CDCl₃) δ ppm 1.53 - 1.72 (m, 2H), 1.74 - 1.99 (m, 2H), 2.01 - 2.18 (m, 4H), 3.91 (br s, 2H), 4.05 - 4.20 (m, 1H), 5.86 (d, 1H), 7.04 (ddd, 1H), 7.49 (dd, 1H), 7.65 (s, 1H), 8.20 (s, 1H), 9.82 (dd, 1H).

### PREPARATION 66

### tert-Butyl (3R)-3-(2-chloro-8-oxo-7-{[2-(trimethylsilyl)ethoxy]methyl}-7,8-dihydro-9H-purin-9-yl)piperidine-1-carboxylate

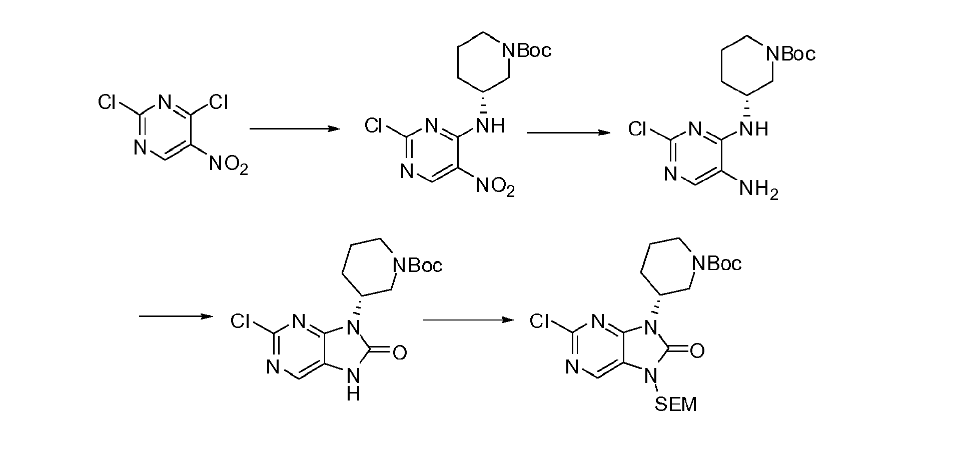

### a) tert-Butyl (3R)-3-[(2-chloro-5-nitropyrimidin-4-yl)amino]piperidine-1-carboxylate

Obtained (84%) from 2,4-dichloro-5-nitropyrimidine and *tert*-butyl (3*R*)-3-aminopiperidine-1-carboxylate following the experimental procedure as described in **Preparation 2a.**
LRMS (m/z): 358 (M+1)⁺.
1H NMR (300 MHz, CDCl₃) δ ppm 1.46 (s, 9H), 1.61 - 1.78 (m, 2H), 1.79 - 2.03 (m, 2H), 3.17 - 3.89 (m, 4H), 4.23 - 4.61 (m, 1H), 8.50 (br s, 1H), 9.07 (s, 1H).

### b) tert-Butyl (3R)-3-[(5-amino-2-chloropyrimidin-4-yl)amino]piperidine-1-carboxylate

Obtained as an oil (78%) from *tert*-butyl (3*R*)-3-[(2-chloro-5-nitropyrimidin-4-yl)amino]piperidine-1-carboxylate (**Preparation 66a**) following the experimental procedure as described in **Preparation 2b.**
LRMS (m/z): 328 (M+1)⁺.
1H NMR (300 MHz, DMSO-d₆) δ ppm 1.32-1.38 (m, 11H), 1.76-1.91 (m, 2H), 3.12 (m, 2H), 3.81 (m, 3H), 5.02 (s, 2H), 6.53 (s, 1H), 7.41 (s, 1H).

### c) tert-Butyl (3R)-3-(2-chloro-8-oxo-7,8-dihydro-9H-purin-9-yl)piperidine-1-carboxylate

Obtained as an orange oil (76%) from *tert*-butyl (3*R*)-3-[(5-amino-2-chloropyrimidin-4-yl)amino]piperidine-1-carboxylate (**Preparation 66b**) following the experimental procedure as described in **Preparation 42d.**
LRMS (m/z): 354 (M+1)⁺.
1H NMR (300 MHz, CDCl₃) δ ppm 1.48 (m, 9H), 1.86 (m, 2H), 2.84 (m, 2H), 3.63 (m, 2H), 4.17 (m, 2H), 4.40 (m, 1H), 8.14 (s, 1H).

### d) tert-Butyl (3R)-3-(2-chloro-8-oxo-7-{[2-(trimethylsilyl)ethoxy]methyl}-7,8-dihydro-9H-purin-9-yl)piperidine-1-carboxylate

Obtained as an orange oil (82%) from *tert*-butyl (3*R*)-3-(2-chloro-8-oxo-7,8-dihydro-9*H-*purin-9-yl)piperidine-l-carboxylate **(Preparation 66c)** following the experimental procedure as described in **Preparation 3.**
LRMS (m/z): 485 (M+1)⁺.
1H NMR (300 MHz, CDCl₃) ppm -0.01 (s, 9H), 0.89 - 1.04 (m, 2H), 1.47 (s, 9H), 1.78 - 1.99 (m, 2H), 2.31 - 2.58 (m, 1H), 2.68 - 2.87 (m, 1H), 3.51 - 3.69 (m, 4H), 4.01 - 4.28 (m, 2H), 4.33 - 4.49 (m, 1H), 5.30 (s, 2H), 8.19 (s, 1H).

### PREPARATION 67

### (R)-tert-Butyl 3-(2-(6-fluoroimidazo[1,2-a]pyridin-3-yl)-8-oxo-7-((2-(trimethylsilyl)ethoxy)methyl)-7H-purin-9(8H)-yl)piperidine-1-carboxylate

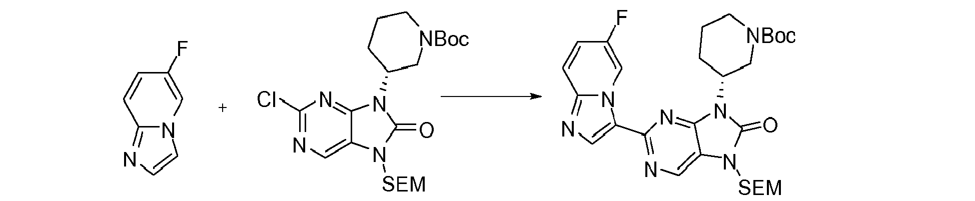

Obtained (57%) from *tert*-butyl (3*R*)-3-(2-chloro-8-oxo-7-{[2-(trimethylsilyl)ethoxy]methyl}-7,8-dihydro-9*H-*purin-9-yl)piperidine-1-carboxylate **(Preparation 66d)** and 6-fluoroimidazo[1,2-a]pyridine (**Preparation 21**) following the experimental procedure as described in **Preparation 4.**
LRMS (m/z): 584 (M+1)⁺.
1H NMR (300 MHz, CDCl₃) δ ppm 0.02 (m, 9H), 0.98 (m, 2H), 1.49 (s, 9H), 1.72 (m, 1H), 1.92 (m, 1H), 2.62 (m, 1H), 3.00 (m, 2H), 3.64 - 3.80 (m, 3H), 4.24 (m, 2H), 4.52 (m, 1H), 5.36 (s, 2H), 7.49 (m, 1H), 8.10 (d, 1H), 8.43 (s, 1H), 8.57 (s, 1H), 9.96 (s, 1H).

### PREPARATION 68

### (R)-2-(6-Fluoroimidazo[1,2-a]pyridin-3-yl)-9-(1-(methylsulfonyl)piperidin-3-yl)-7-((2-(trimethylsilyl)ethoxy)methyl)-7H-purin-8(9H)-one

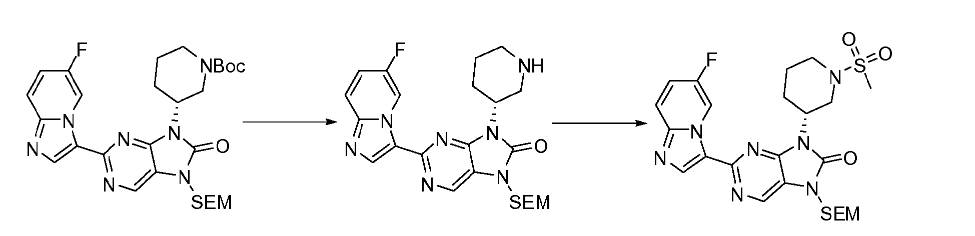

### a) (R)-2-(6-Fluoroimidazo[1,2-a]pyridin-3-yl)-9-(piperidin-3-yl)-7-((2-(trimethylsilyl)ethoxy)methyl)-7H-purin-8(9H)-one

A suspension of (R)-*tert*-butyl 3-(2-(6-fluoroimidazo[1,2-a]pyridin-3-yl)-8-oxo-7-((2-(trimethylsilyl)ethoxy)methyl)-7H-purin-9(8H)-yl)piperidine-1-carboxylate **(Preparation 67,** 3.38 g, 5.8 mmol) in trifluoroacetic acid (2.7 mL) and dichloromethane was stirred and heated to 50 °C in a sealed tube. After 2 hours, water was added and then the mixture was neutralized with aqueous sodium hydrogencarbonate solution. The mixture was extracted with ethyl acetate and the organic layer was dried (MgSO₄) and concentrated. The residue was purified by reverse phase chromatography (C-18 silica from Waters©, water/acetonitrile/methanol as eluents [0.1% v/v formic acid buffered] 0% to 100%) to give the title compound (0.73 g, 26%) as an oil.
LRMS (m/z): 484 (M+1)⁺.
1H NMR (300 MHz, CDCl₃) δ ppm 0.00 (s, 9H), 0.96 (t, 2H), 1.82 - 2.14 (m, 4H), 2.53 - 2.69 (m, 1H), 2.86 - 3.03 (m, 1H), 3.21 - 3.49 (m, 2H), 3.64 (t, 2H), 3.69 - 3.82 (m, 1H), 4.69 - 4.90 (m, 1H), 5.32 (s, 2H), 7.67 - 7.76 (m, 1H), 8.33 - 8.40 (m, 2H), 8.57 (s, 1H), 9.91 (br s, 1H).

### b) (R)-2-(6-fluoroimidazo[1,2-a]pyridin-3-yl)-9-(1-(methylsulfonyl)piperidin-3-yl)-7-((2-(trimethylsilyl)ethoxy)methyl)-7H-purin-8(9H)-one

Methanesulfonyl chloride (0.024 mL, 0.31 mmol) was added to a solution of (R)-2-(6-fluoroimidazo[1,2-a]pyridin-3-yl)-9-(piperidin-3-yl)-7-((2-(trimethylsilyl)ethoxy)methyl)-7H-purin-8(9H)-one **(Preparation 68a,** 0.100 g, 0.21 mmol) in anhydrous dichloromethane (1.5 mL) and the mixture was stirred at room temperature for 1 hour. The mixture was then partitioned between water and ethyl acetate and the organic layer was washed with water, dried (MgSO₄) and concentrated to give the title compound (0.115 g, 99%) as a white solid.
LRMS (m/z): 562 (M+1)⁺.
1H NMR (400 MHz, CDCl₃) δ ppm 0.01 (s, 9H), 0.98 (d, 2H), 1.42 (t, 2H), 2.18 (s, 3H), 2.82 - 2.91 (m, 1H), 3.07 - 3.20 (m, 1H), 3.56 - 3.71 (m, 4H), 3.96 (t, 2H), 4.62 - 4.80 (m, 1H), 5.36 (s, 2H), 7.35 - 7.45 (m, 1H), 7.90 (dd, 1H), 8.45 (s, 1H), 8.59 (s, 1H), 10.03 (br s, 1H).

### PREPARATION 69

### (R)-9-(1-Acetylpiperidin-3-yl)-2-(6-fluoroimidazo[1,2-a]pyridin-3-yl)-7-((2-(trimethylsilyl)ethoxy)methyl)-7H-purin-8(9H)-one

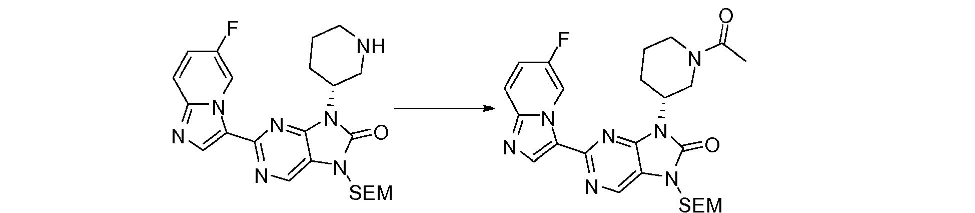

Triethylamine (0.048 mL, 0.34 mmol) and acetyl chloride (0.025 mL, 0.35 mmol) were added sequentially to a solution of (R)-2-(6-fluoroimidazo[1,2-a]pyridin-3-yl)-9-(piperidin-3-yl)-7-((2-(trimethylsilyl)ethoxy)methyl)-7H-purin-8(9H)-one (**Preparation 68a,** 0.130 g, 0.27 mmol) in dichloromethane (2 mL) and the mixture was stirred at room temperature for 10 minutes. The mixture was diluted with ethyl acetate was added and the organic solution was washed with water, brine, dried (MgSO₄) and concentrated to give the title compound (0.132 g, 93%) as a white solid.
LRMS (m/z): 526 (M+1)⁺.
1H NMR (300 MHz, CDCl₃) δ ppm 0.01 (s, 9H), 0.96 (dd, 2H), 1.54 - 1.89 (m, 4H), 1.93 - 2.28 (m, 4H), 2.55 - 2.85 (m, 1H), 3.51 - 3.73 (m, 2H), 3.87 - 4.06 (m, 1H), 4.50 (td, 1H), 4.80 (t, 1H), 5.35 (s, 2H), 7.19 - 7.37 (m, 1H), 7.68 - 7.81 (m, 1H), 8.39 - 8.48 (m, 1H), 8.56 (s, 1H), 9.86 - 10.03 (m, 1H).

### PREPARATION 70

### 5-Chloro-3-(tetrahydro-2H-pyran-4-yl)-1-{[2-(trimethylsilyl)ethoxy]methyl}-1,3-dihydro-2H-imidazo[4,5-b]pyridin-2-one

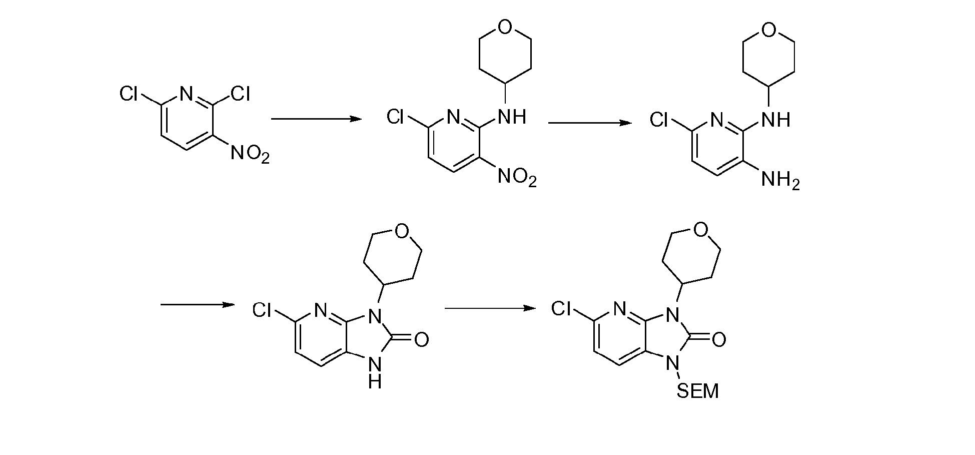

### a) 6-Chloro-3-nitro-N-(tetrahydro-2H-pyran-4-yl)pyridin-2-amine

Tetrahydro-2*H-*pyran-4-amine (3.00 g, 21.8 mmol) and triethylamine (5.50 mL, 39.9 mmol) were added to a suspension of 2,6-dichloro-3-nitropyridine (3.50 g, 18.1 mmol) in chloroform (60 mL) and the reaction mixture was stirred at room temperature overnight and then at 50 °C for 24 hours. The mixture was then cooled to room temperature, washed with water, dried (MgSO₄) and evaporated. The residue was purified by flash chromatography (98:2 dichloromethane/methanol) to give the title compound (4.20 g, 90%) as a yellow solid.
LRMS (m/z): 258 (M+1)⁺.
1H NMR (300 MHz, CDCl₃) δ ppm 1.60 - 1.75 (m, 2H), 2.08 (dd, 2H), 3.60 (td, 2H), 4.03 (ddd, 2H), 4.31 - 4.50 (m, 1H), 6.64 (d, 1H), 8.30 (br s, 1H), 8.37 (d, 1H).

### b) 6-Chloro-N²-(tetrahydro-2H-pyran-4-yl)pyridine-2,3-diamine

Obtained as a brown solid (100%) from 6-chloro-3-nitro*-N-*(tetrahydro-2*H-*pyran-4-yl)pyridin-2-amine (**Preparation 70a**) following the experimental procedure as described in **Preparation 2b.**
LRMS (m/z): 228 (M+1)⁺.
1H NMR (300 MHz, DMSO-*d₆*) δ ppm 1.43 (qd, 2H), 1.82 - 1.94 (m, 2H), 3.38 - 3.49 (m, 2H), 3.79 - 4.11 (m, 3H), 4.87 (s, 2H), 5.69 (d, 1H), 6.33 (d, 1H), 6.67 (d, 1H).

### c) 5-Chloro-3-(tetrahydro-2H-pyran-4-yl)-1,3-dihydro-2H-imidazo[4,5-b]pyridin-2-one

Obtained as a pale purple solid (75%) from 6-chloro-*N²*-(tetrahydro-2*H-*pyran-4-yl)pyridine-2,3-diamine **(Preparation 70b)** following the experimental procedure as described in **Preparation 42d.**
LRMS (m/z): 254 (M+1)⁺.
1H NMR (300 MHz, CDCl3) δ ppm 1.74 (d, 2H), 2.72 - 2.94 (m, 2H), 3.56 (t, 2H), 4.15 (dd, 2H), 4.54 - 4.71 (m, 1H), 7.03 (d, 1H), 7.25 (d, 1H), 9.23 (br s, 1H).

### d) 5-Chloro-3-(tetrahydro-2H-pyran-4-yl)-1-{[2-(trimethylsilyl)ethoxy]methyl}-1,3-dihydro-2H-imidazo[4,5-b]pyridin-2-one

Obtained as a yellow solid (89%) from 5-chloro-3-(tetrahydro-2*H-*pyran-4-yl)-1,3-dihydro-2*H-*imidazo[4,5*-b*]pyridin-2-one **(Preparation 70c)** following the experimental procedure as described in **Preparation 3.**
LRMS (m/z): 384 (M+1)⁺.
1H NMR (300 MHz, CDCl₃) δ ppm 0.00 (s, 9H), 0.94 (m, 2H), 1.75 (m, 2H), 2.84 (m, 2H), 3.59 (m, 4H), 4.15 (m, 2H), 4.65 (m, 1H), 5.32 (m, 2H), 7.08 (d, 1H), 7.30 (d, 1H).

### PREPARATION 71

### 3-(2-Oxo-3-(tetrahydro-2H-pyran-4-yl)-1-((2-(trimethylsilyl)ethoxy)methyl)-2,3-dihydro-1H-imidazo[4,5-b]pyridin-5-yl)imidazo[1,2-a]pyridine-6-carbonitrile

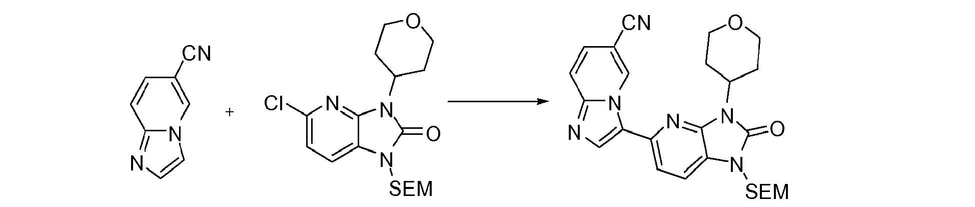

Obtained (32%) from 5-chloro-3-(tetrahydro-2*H-*pyran-4-yl)-1-{[2-(trimethylsilyl)ethoxy]methyl}-1,3-dihydro-2*H-*imidazo[4,5*-b*]pyridin-2-one (**Preparation 70**) and imidazo[1,2-a]pyridine-6-carbonitrile (**Preparation 1**) following the experimental procedure as described in **Preparation 4.**
LRMS (m/z): 491 (M+1)⁺.
1H NMR (300 MHz, CDCl₃) δ ppm 0.00 (s, 9H), 0.87 - 1.01 (m, 2H), 1.80 - 2.00 (m, 2H), 2.84 (dd, 2H), 3.65 (dd, 4H), 4.25 (dd, 2H), 4.62 - 4.87 (m, 1H), 5.37 (s, 2H), 7.39 (dd, 1H), 7.52 (m, 2H), 7.79 (dd, 1H), 8.18 (s, 1H), 10.25 (s, 1H).

### PREPARATION 72

### 5-Chloro-1-methyl-3-(tetrahydro-2H-pyran-4-yl)-1,3-dihydro-2H-imidazo[4,5-b]pyridin-2-one

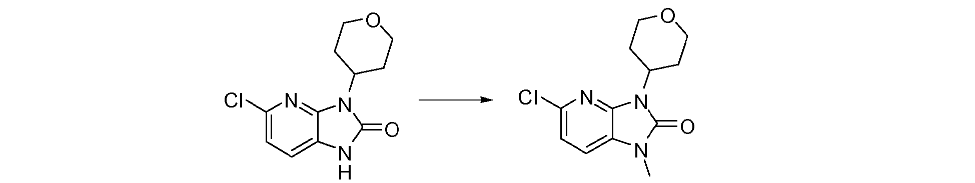

Obtained as a brown solid (100%) from 5-chloro-3-(tetrahydro-2*H-*pyran-4-yl)-1,3-dihydro-2*H-*imidazo[4,5*-b*]pyridin-2-one (**Preparation 70c**) following the experimental procedure as described in **Preparation 11.**
LRMS (m/z): 268 (M+1)⁺.
1H NMR (300 MHz, CDCl₃) δ ppm 1.71 (d, 2H), 2.80 (m, 2H), 3.42 (s, 3H), 3.55 (t, 2H), 4.13 (dd, 2H), 4.62 (m, 1H), 7.03 (d, 1H), 7.11 (d, 1H).

### PREPARATION 73

### 5-(6-Fluoroimidazo[1,2-a]pyridin-3-yl)-3-(tetrahydro-2H-pyran-4-yl)-1-((2-(trimethylsilyl)ethoxy)methyl)-1H-imidazo[4,5-b]pyridin-2(3H)-one

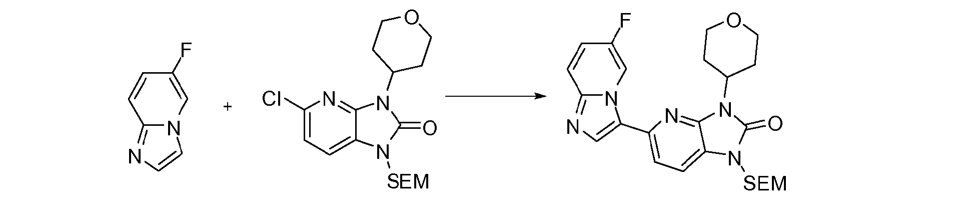

Obtained (48%) from 5-chloro-3-(tetrahydro-2*H-*pyran-4-yl)-1-{[2-(trimethylsilyl)ethoxy]methyl}-1,3-dihydro-2*H-*imidazo[4,5*-b*]pyridin-2-one **(Preparation 70)** and 6-fluoroimidazo[1,2-a]pyridine **(Preparation 21)** following the experimental procedure as described in **Preparation 4.**
LRMS (m/z): 484 (M+1)⁺.
1H NMR (300 MHz, CDCl₃) δ ppm -0.08 - 0.11 (m, 9H), 0.94 (d, 2H), 1.86 (dd, 2H), 2.87 (dd, 2H), 3.51 - 3.74 (m, 4H), 4.21 (dd, 2H), 4.72 (m, 1H), 5.37 (s, 2H), 7.50 (d, 2H), 7.77 (dd, 1H), 8.13 (s, 2H), 9.72 (dd, 1H).

### PREPARATION 74

### 5-Chloro-3-(tetrahydro-2H-pyran-4-yl)-1-[2-(tetrahydro-2H-pyran-2-yloxy)ethyl]-1,3-dihydro-2H-imidazo[4,5-b]pyridin-2-one

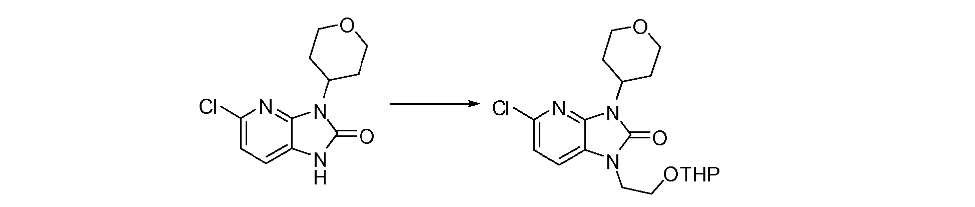

Obtained as a brown oil (100%) from 5-chloro-3-(tetrahydro-2*H-*pyran-4-yl)-1,3-dihydro-2*H-*imidazo[4,5*-b*]pyridin-2-one **(Preparation 70c)** and 2-(2-bromoethoxy)tetrahydro-2*H-*pyran following the experimental procedure as described in **Preparation 15.**
LRMS (m/z): 382 (M+1)⁺.
1H NMR (300 MHz, CDCl₃) δ ppm 1.46-1.72 (m, 8H), 2.79 (m, 2H), 3.42-3.68 (m, 6H), 3.99-4.15 (m, 4H), 4.54 (m, 1H), 4.62 (m, 1H), 7.00 (d, 1H), 7.35 (d, 1H).

### PREPARATION 75

### tert-Butyl 4-(5-chloro-2-oxo-1-{[2-(trimethylsilyl)ethoxy]methyl}-1,2-dihydro-3H-imidazo[4,5-b]pyridin-3-yl)piperidine-1-carboxylate

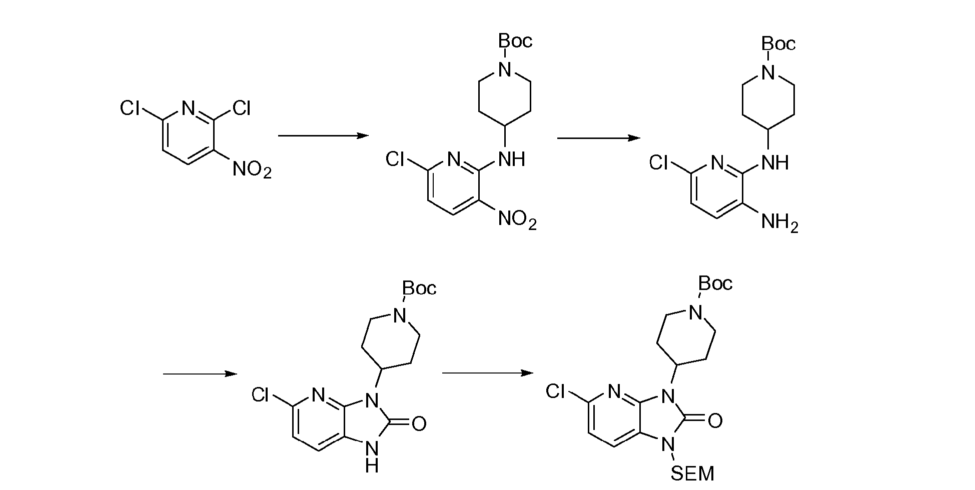

### a) tert-Butyl 4-[(6-chloro-3-nitropyridin-2-yl)amino]piperidine-1-carboxylate

Obtained as a yellow solid (74%) from 2,6-dichloro-3-nitropyridine and *tert*-butyl 4-aminopiperidine-1-carboxylate following the experimental procedure as described in **Preparation 70a.**
LRMS (m/z): 374 (M+18)⁺.
1H NMR (300 MHz, CDCl₃) δ ppm 1.48 (s, 9H), 1.52 (d, 2H), 2.06 (d, 2H), 3.02 (t, 2H), 4.07 (d, 2H), 4.25 - 4.42 (m, 1H), 6.64 (d, 1H), 8.29 (d, 1H), 8.36 (d, 1H).

### b) tert-Butyl 4-[(3-amino-6-chloropyridin-2-yl)amino]piperidine-1-carboxylate

Obtained as a brown solid (84%) from *tert*-butyl 4-[(6-chloro-3-nitropyridin-2-yl)amino]piperidine-1-carboxylate **(Preparation 75a)** following the experimental procedure as described in **Preparation 2b.**
LRMS (m/z): 327 (M+1)⁺.
1H NMR (300 MHz, DMSO-d₆) δ ppm 1.35 (m, 2H), 1.45 (s, 9H), 1.93 (m, 2H), 2.94 (m, 2H), 3.95 (m, 3H), 4.90 (s, 2H), 5.72 (d, 1H), 6.40 (d, 1H), 6.72 (d, 1H).

### c) tert-Butyl 4-(5-chloro-2-oxo-1,2-dihydro-3H-imidazo[4,5-b]pyridin-3-yl)piperidine-1-carboxylate

Obtained as a purple solid (51%) from *tert*-butyl 4-[(3-amino-6-chloropyridin-2-yl)amino]piperidine-1-carboxylate **(Preparation 75b)** following the experimental procedure as described in **Preparation 42d.**
LRMS (m/z): 353 (M+1)⁺.
1H NMR (300 MHz, CDCl₃) δ ppm 1.52 (s, 9H), 1.77 (m, 2H), 2.63 (m, 2H), 2.87 (m, 2H), 4.30 (m, 2H), 4.55 (m, 1H), 7.05 (d, 1H), 7.28 (d, 1H), 10.12 (br s, 1H).

### d) tert-Butyl 4-(5-chloro-2-oxo-1-{[2-(trimethylsilyl)ethoxy]methyl}-1,2-dihydro-3H-imidazo[4,5-b]pyridin-3-yl)piperidine-1-carboxylate

Obtained as an oil (95%) from *tert*-butyl 4-(5-chloro-2-oxo-1,2-dihydro-3*H-*imidazo[4,5-b]pyridin-3-yl)piperidine-1-carboxylate **(Preparation 75c)** and (2-(chloromethoxy)ethyl)trimethylsilane following the experimental procedure as described in **Preparation 3.**
LRMS (m/z): 484 (M+1)⁺.
1H NMR (300 MHz, CDCl₃) δ ppm 0.03 (s, 9H), 0.96 (m, 2H), 1.54 (s, 9H), 1.81 (m, 2H), 2.66 (m, 2H), 2.89 (m, 2H), 3.63 (m, 2H), 4.34 (m, 2H), 4.57 (m, 1H), 5.32 (s, 2H), 7.08 (d, 1H), 7.33 (d, 1H).

### PREPARATION 76

### tert-Butyl 4-(5-(6-fluoroimidazo[1,2-a]pyridin-3-yl)-2-oxo-1-((2-(trimethylsilyl)ethoxy)methyl)-1H-imidazo[4,5-b]pyridin-3(2H)-yl)piperidine-1-carboxylate

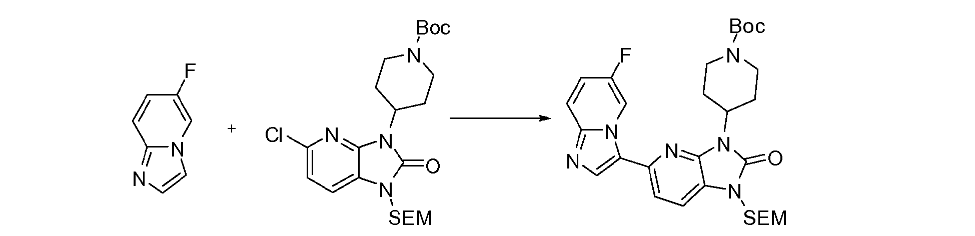

Obtained as a white solid (58%) from *tert*-butyl 4-(5-chloro-2-oxo-1-{[2-(trimethylsilyl)ethoxy]methyl}-1,2-dihydro-3*H-*imidazo[4,5*-b*]pyridin-3-yl)piperidine-1-carboxylate **(Preparation 75)** and 6-fluoroimidazo[1,2-*a*]pyridine **(Preparation 21)** following the experimental procedure as described in **Preparation 4.**
LRMS (m/z): 583 (M+1)⁺.
1H NMR (300 MHz, CDCl₃) δ ppm -0.01 (s, 9H), 0.88 - 1.02 (m, 2H), 1.50 (br s, 9H), 1.92 (d, 2H), 2.66 (br s, 2H), 2.90 (br s, 2H), 3.64 (d, 2H), 4.39 (br s, 2H), 4.51 - 4.70 (m, 1H), 5.45 (br s, 2H), 7.29 - 7.37 (m, 1 H), 7.51 (s, 2H), 7.87 (dd, 1H), 8.15 (s, 1H), 9.64 (dd, 1H).

### PREPARATION 77

### 5-(6-Fluoroimidazo[1,2-a]pyridin-3-yl)-3-(1-(methylsulfonyl)piperidin-4-yl)-1-((2-(trimethylsilyl)ethoxy)methyl)-1H-imidazo[4,5-b]pyridin-2(3H)-one

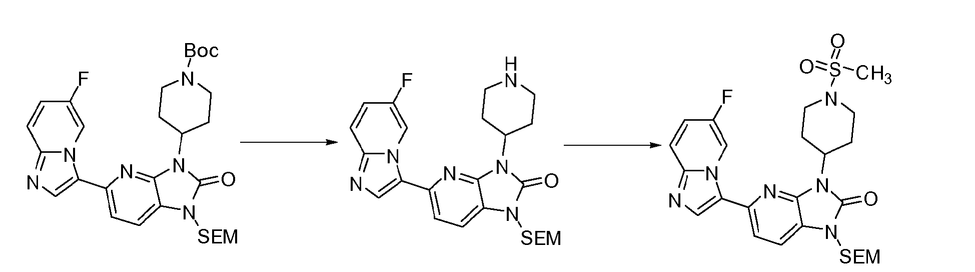

### a) 5-(6-Fluoroimidazo[1,2-a]pyridin-3-yl)-3-(piperidin-4-yl)-1-((2-(trimethylsilyl)ethoxy)methyl)-1H-imidazo[4,5-b]pyridin-2(3H)-one

Obtained as a white solid (92%) from *tert*-butyl 4-(5-(7-fluoroimidazo[1,2-a]pyridin-3-yl)-2-oxo-1-{[2-(trimethylsilyl)ethoxy]methyl}-1,2-dihydro-3*H-*imidazo[4,5-b]pyridin-3-yl)piperidine-1-carboxylate **(Preparation 76)** following the experimental procedure as described in **Preparation 68a.**
LRMS (m/z): 483 (M+1)⁺.
1H NMR (300 MHz, CDCl₃) δ ppm 0.00 (s, 9H), 0.96 (m, 2H), 2.10 (m, 2H), 2.88 (m, 2H), 3.06 (m, 2H), 3.57-3.68 (m, 4H), 4.70 (m, 1H), 5.36 (s, 2H), 7.18-7.29 (m, 2H), 7.50 (d, 1H), 7.68 (m, 1H), 8.09 (s, 1H), 9.51 (m, 1H).

### b) 5-(6-Fluoroimidazo[1,2-a]pyridin-3-yl)-3-(1-(methylsulfonyl)piperidin-4-yl)-1-((2-(trimethylsilyl)ethoxy)methyl)-1H-imidazo[4,5-b]pyridin-2(3H)-one

Obtained as a white solid (73%) from 5-(6-fluoroimidazo[1,2-a]pyridin-3-yl)-3-(piperidin-4-yl)-1-((2-(trimethylsilyl)ethoxy)methyl)-1H-imidazo[4,5-b]pyridin-2(3H)-one **(Preparation 77a)** following the experimental procedure as described in **Preparation 68b.**
LRMS (m/z): 561 (M+1)⁺.
1H NMR (300 MHz, CDCl₃) δ ppm 0.00 (s, 9H), 0.87 - 1.04 (m, 2H), 1.88 - 2.13 (m, 4H), 2.84 - 2.98 (m, 5H), 3.65 (dd, 2H), 4.08 (d, 2H), 4.47 - 4.68 (m, 1H), 5.36 (s, 2H), 7.22 - 7.35 (m, 1H), 7.52 (d, 2H), 7.78 (dd, 1H), 8.14 (s, 1H), 9.67 (dd, 1H).

### PREPARATION 78

### 3-(1-Acetyipiperidin-4-yl)-5-(6-fluoroimidazo[1,2-a]pyridin-3-yl)-1-((2-(trimethylsilyl)ethoxy)methyl)-1H-imidazo[4,5-b]pyridin-2(3H)-one

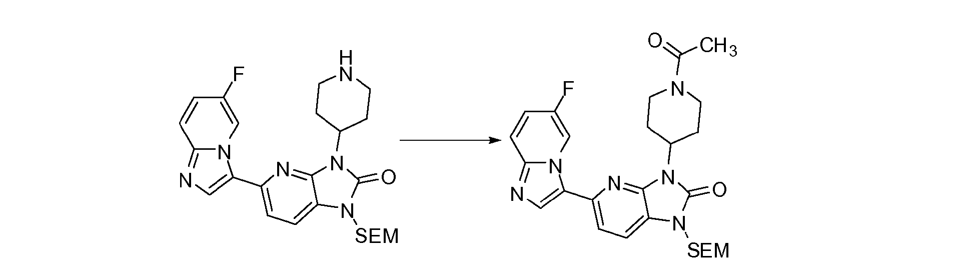

Obtained as a white solid (73%) from 5-(6-fluoroimidazo[1,2-a]pyridin-3-yl)-3-(piperidin-4-yl)-1-((2-(trimethylsilyl)ethoxy)methyl)-1H-imidazo[4,5-b]pyridin-2(3H)-one **(Preparation 77a)** and acetyl chloride following the experimental procedure as described in **Preparation 69.**
LRMS (m/z): 525 (M+1)⁺.
1H NMR (300 MHz, CDCl₃) δ ppm 0.01 (s, 9H), 0.96 (m, 2H), 2.03 (m, 2H), 2.20 (s, 3H), 2.53-2.86 (m, 3H), 3.28 (m, 1H), 3.65 (m, 2H), 4.09 (m, 1H), 4.71 (m, 1H), 4.92 (m, 1H), 5.37 (s, 2H), 7.24 (m, 1H), 7.48-7.55 (m, 2H), 7.70 (m, 1H), 8.13 (s, 1H), 9.64 (m, 1H).

### PREPARATION 79

### 5-Chloro-6-fluoro-3-(tetrahydro-2H-pyran-4-yl)-1-{[2-(trimethylsilyl)ethoxy] methyl}-1,3-dihydro-2H-imidazo[4,5-b]pyridin-2-one

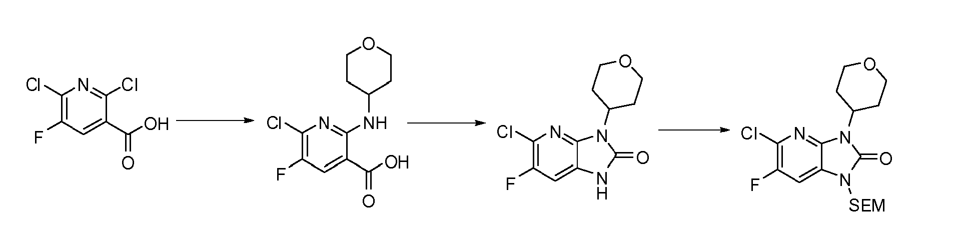

### a) 6-Chloro-5-fluoro-2-(tetrahydro-2H-pyran-4-ylamino)nicotinic acid

A mixture of 2,6-dichloro-5-fluoronicotinic acid (1.01 g, 4.8 mmol), diisopropylethylamine (11 mL, 62.5 mmol) and tetrahydro-2H-pyran-4-amine hydrochloride (3.30 g, 24 mmol) in acetonitrile (5 mL) was stirred and heated under microwave irradiation ("Initiator sixty" from Biotage^{®}) at 130 °C for 21 hours. The mixture was then cooled and dichloromethane was added and the organic layer was washed with 5% aqueous citric acid, water, brine, dried (MgSO₄) and the solvent was evaporated. The residue was purified by reverse phase chromatography (C-18 silica from Waters©, water/acetonitrile/methanol as eluents [0.1% v/v formic acid buffered] 0% to 100%) to give the title compound (0.51 g, 39%) of as a white solid.
LRMS (m/z): 273 (M-1)⁺.
1H NMR (300 MHz, CDCl₃) δ ppm 1.48 - 1.69 (m, 2H), 1.97 - 2.14 (m, 2H), 3.58 (t, 2H), 4.03 (dd, 2H), 4.29 (ddd, 1H), 5.04 (d, 1H), 7.84 (d, 1H).

### b) 5-Chloro-6-fluoro-3-(tetrahydro-2H-pyran-4-yl)-1,3-dihydro-2H-imidazo[4,5-b]pyridin-2-one

Triethylamine (0.20 mL, 1.43 mmol) and diphenylphosphoryl azide (0.19 mL, 0.88 mmol) were added to a solution of 6-chloro-5-fluoro-2-(tetrahydro-2*H-*pyran-4-ylamino)nicotinic acid **(Preparation 79a,** 0.200 g, 0.74 mmol) in 1,4-dioxane (5 mL) and the mixture was stirred and heated to 110 °C. After 2 hours, the solvent was evaporated and the residue was partitioned between water and ethyl acetate and the organic layer was washed with 4% aqueous sodium hydrogencarbonate solution and dried (MgSO₄). The solvent was evaporated and the residue was triturated with diethyl ether to give a solid which was filtered and dried to give the title compound (0.092 g, 46%) as a white solid.
LRMS (m/z): 270 (M-1)⁺.
1H NMR (300 MHz, DMSO-d₆) ppm 1.63 (d, 2H), 2.49 (ddd, 2H), 3.55 - 3.48 (m, 3H), 3.97 (dd, 2H), 4.41 (tt, 1H), 7.57 (d, 1H).

### c) 5-Chloro-6-fluoro-3-(tetrahydro-2H-pyran-4-yl)-1-{[2-(trimethylsilyl)ethoxy] methyl}-1,3-dihydro-2H-imidazo[4,5-b]pyridin-2-one

Obtained as a white solid (70%) from 5-chloro-6-fluoro-3-(tetrahydro-2*H-*pyran-4-yl)-1,3-dihydro-2*H-*imidazo[4,5*-b*]pyridin-2-one **(Preparation 79b)** and (2-(chloromethoxy) ethyl)trimethylsilane following the experimental procedure as described in **Preparation** 3.
LRMS (m/z): 402 (M+1)⁺.
1H NMR (300 MHz, CDCl₃) δ ppm -0.01 (s, 9H), 0.80 - 1.05 (m, 2H), 1.64 - 1.77 (m, 2H), 2.78 (m, 2H), 3.47 - 3.65 (m, 4H), 4.14 (dd, 2H), 4.51 - 4.67 (m, 1H), 5.28 (s, 2H), 7.27 (s, 1H).

### PREPARATION 80

### 6-Fluoro-5-(6-fluoroimidazo[1,2-a]pyridin-3-yl)-3-(tetrahydro-2H-pyran-4-yl)-1-((2-(trimethylsilyl)ethoxy)methyl)-1H-imidazo[4,5-b]pyridin-2(3H)-one

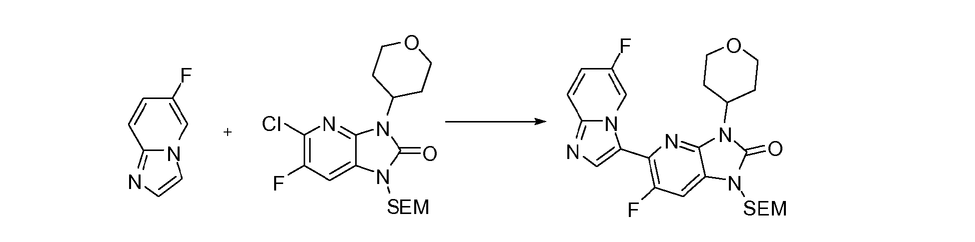

Obtained (57%) from 5-chloro-6-fluoro-3-(tetrahydro-2*H-*pyran-4-yl)-1-{[2-(trimethylsilyl)ethoxy] methyl}-1,3-dihydro-2*H-*imidazo[4,5*-b*]pyridin-2-one **(Preparation 79)** and 6-fluoroimidazo[1,2-a]pyridine (Preparation **21**) following the experimental procedure as described in **Preparation 4.**
LRMS (m/z): 502 (M+1)⁺.
1H NMR (300 MHz, CDCl₃) δ ppm -0.01 (s, 9H), 0.86 - 1.10 (m, 2H), 1.80 - 2.07 (m, 2H), 2.83 (dd, 2H), 3.52 - 3.76 (m, 4H), 4.20 (dd, 2H), 4.60 - 4.81 (m, 1H), 5.35 (s, 2H), 7.38 (d, 1H), 7.79 (dd, 1H), 8.09 (s, 1H), 8.31 (d, 1H), 9.74 (dd, 1H).

### PREPARATION 81

### 5-Bromo-3-(tetrahydro-2H-pyran-4-yl)-1-{[2-(trimethylsilyl)ethoxy]methyl}-1,3-dihydro-2H-imidazo[4,5-b]pyrazin-2-one

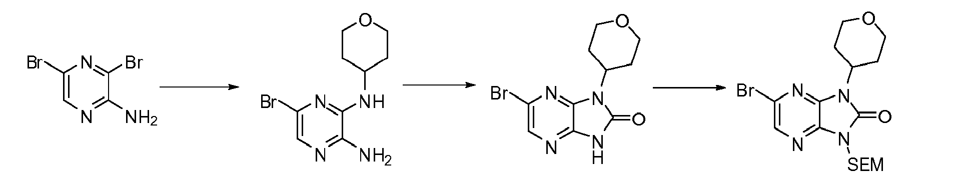

### a) 5-Bromo-N³-(tetrahydro-2H-pyran-4-yl)pyrazine-2,3-diamine

A mixture of 3,5-dibromopyrazin-2-amine (0.400 g, 1.6 mmol), tetrahydro-2H-pyran-4-amine hydrochloride (0.441 g, 3.20 mmol) and N,N-diisopropylethylamine (0.83 mL, 4.8 mmol) in n-butanol (3 mL) was stirred and heated under microwave irradiation ("Initiator sixty" from Biotage^{®}) at 150 °C. After 13 hours, the mixture was cooled and the mixture was partitioned between ethyl acetate and water. The organic layer was dried (MgSO₄) and concentrated and the residue was purified by flash chromatography (2:1 hexanes/ethyl acetate) to give the title compound (0.34 g, 78%) as a white solid.
LRMS (m/z): 273, 275 (M+1)⁺.
1H NMR (300 MHz, CDCl₃) δ ppm 1.54 (dd, 2H), 1.97 - 2.18 (m, 2H), 3.57 (t, 2H), 3.93 - 4.21 (m, 5H), 7.26 - 7.28 (m, 1H), 7.47 (s, 1H).

### b) 6-Bromo-1-(tetrahydro-2H-pyran-4-yl)-1,3-dihydro-2H-imidazo[4,5-b]pyrazin-2-one

A mixture of 5-bromo-*N³*-(tetrahydro-2*H-*pyran-4-yl)pyrazine-2,3-diamine **(Preparation 81a,** 0.33 g, 1.2 mmol) and 1,1'-carbonylbis-1*H-*imidazole (0.27 g, 1.5 mmol) in tetrahydrofuran (3 mL) was stirred and heated under microwave irradiation ("Initiator sixty" from Biotage^{®}) at 130 °C for 4 hours. The solvent was concentrated, the residue was dissolved in the minimum amount of *N,N*'-dimethylformamide and water was added. The resulting precipitate was filtered, washed with water and dried to give the title compound (0.23 g, 64%) as a white solid.
LRMS (m/z): 299, 301 (M+1)⁺.
1H NMR (300 MHz, DMSO-*d₆*) δ ppm 1.64 - 1.84 (m, 2H), 2.73 (qd, 2H), 3.54 (t, 2H), 4.15 (dd, 2H), 4.60 (tt, 1H), 8.05 (s, 1H), 9.52 (br s, 1H).

### c) 5-Bromo-3-(tetrahydro-2H-pyran-4-yl)-1-{[2-(trimethylsilyl)ethoxy]methyl}-1,3-dihydro-2H-imidazo[4,5-b]pyrazin-2-one

Obtained as a white solid (81%) from 6-bromo-1-(tetrahydro-2*H-*pyran-4-yl)-1,3-dihydro-2*H-*imidazo[4,5*-b*]pyrazin-2-one **(Preparation 81b)** and (2-(chloromethoxy)ethyl)trimethylsilane following the experimental procedure as described in **Preparation 3.**
LRMS (m/z): 429, 431 (M+1)⁺.
1H NMR (300 MHz, CDCl₃) δ ppm 0.00 (s, 9H), 0.95 (t, 2H), 1.67 - 1.83 (m, 2H), 2.63 - 2.84 (m, 2H), 3.48 - 3.61 (m, 2H), 3.64 - 3.76 (m, 2H), 4.15 (dd, 2H), 4.49 - 4.68 (m, 1H), 5.38 (s, 2H), 8.06 (s, 1H).

### PREPARATION 82

### 5-(6-Fluoroimidazo[1,2-a]pyridin-3-yl)-3-(tetrahydro-2H-pyran-4-yl)-1-((2-(trimethylsilyl)ethoxy)methyl)-1H-imidazo[4,5-b]pyrazin-2(3H)-one

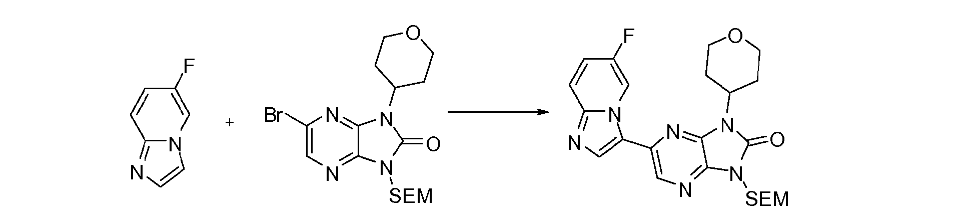

Obtained (34%) from 5-bromo-3-(tetrahydro-2*H-*pyran-4-yl)-1-{[2-(trimethylsilyl) ethoxy]methyl}-1,3-dihydro-2*H-*imidazo[4,5*-b*]pyrazin-2-one **(Preparation 81)** and 6-fluoroimidazo[1,2-a]pyridine **(Preparation 21)** following the experimental procedure as described in **Preparation 4.**
LRMS (m/z): 485 (M+1)⁺.
1H NMR (300 MHz, CDCl₃) δ ppm 0.01 (s, 9H), 0.95 - 1.05 (m, 2H), 1.82 - 1.95 (m, 2H), 2.78 (dd, 2H), 3.60 (t, 2H), 3.71 - 3.82 (m, 2H), 4.20 (dd, 2H), 4.58 - 4.81 (m, 1H), 5.45 (s, 2H), 7.21 - 7.33 (m, 1H), 7.73 (dd, 1H), 8.14 (s, 1H), 8.45 (s, 1H), 9.38 (dd, 1H).

### PREPARATION 83

### 2,2,2-Trifluoro-N-(6-fluoroimidazo[1,2-a]pyridin-2-yl)acetamide

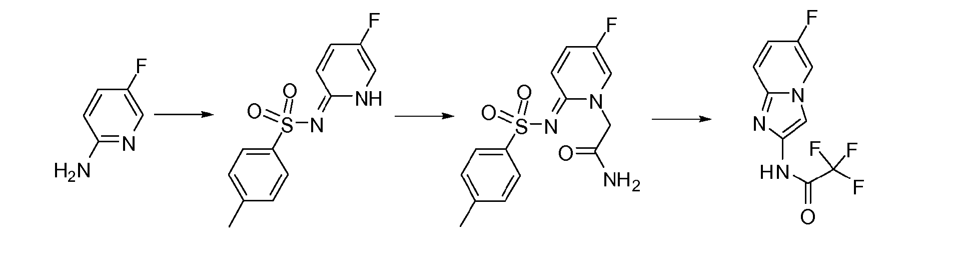

### a) (E)-N-(5-Fluoropyridin-2(1H)-ylidene)-4-methylbenzenesulfonamide:

4-Methylbenzene-1-sulfonyl chloride (9.35 g, 49.0 mmol) was added to a solution of 5-fluoropyridin-2-amine (5.0 g, 44.6 mmol) in anhydrous pyridine (34 mL) and the mixture was stirred and heated to 90 °C under an atmosphere of argon. After 18 hours, the mixture was evaporated to dryness and water was added to the residue. After stirring for 1.5 hours, the precipitate was filtered, washed with water and diethyl ether and dried to give the title compound (10.44 g, 88%) as a white solid.
LRMS (m/z): 265 (M-1)⁺.
1H NMR (300 MHz, DMSO-*d₆*) δ ppm 2.35 (s, 3H), 7.11 (dd, 1H), 7.37 (d, 2H), 7.66 (ddd, 1H), 7.77 (d, 2H), 8.17 (d, 1H), 11.08 (br s, 1H).

### b) (E)-2-(5-Fluoro-2-(tosylimino)pyridin-1(2H)-yl)acetamide:

Diisopropylethylamine (7.30 mL, 41.8 mmol) and 2-iodoacetamide (7.70 g, 41.6 mmol) were added to a stirred solution of (E)-N-(5-fluoropyridin-2(1H)-ylidene)-4-methylbenzenesulfonamide **(Preparation 83a,** 10.07 g, 37.8 mmol) in N,N'-dimethylformamide (65 mL). After stirring for 20 hours at ambient temperature further diisopropylethylamine (1.8 mL) and 2-iodoacetamide (1.93 g) were added and stirring was continued for a further 18 hours. The mixture was evaporated to dryness and water was added to the residue. After stirring for 1 hour, the precipitate was filtered, washed with water and ethyl acetate and dried to give the title compound (8.25 g, 68%) as a white solid.
LRMS (m/z): 324 (M+1)⁺.
1H NMR (300 MHz, DMSO-*d₆*) δ ppm 2.34 (s, 3H), 4.77 (s, 2H), 7.27 (d, 2H), 7.36 (dd, 1H), 7.41 (br s, 1H), 7.66 (d, 2H), 7.79 (br s, 1H), 7.88 (ddd, 1H), 8.33 (dd, 1H).

### c) 2,2,2-Trifluoro-N-(6-fluoroimidazo[1,2-a]pyridin-2-yl)acetamide:

Trifluoroacetic anhydride (9.85 mL, 70.8 mmol) was added to a suspension of (E)-2-(5-fluoro-2-(tosylimino)pyridin-1 (2H)-yl)acetamide **(Preparation 83b,** 11.45 g, 35.4 mmol) in dichloromethane (170 mL) and the mixture was stirred and heated to reflux. After 3 hours, the mixture was evaporated to dryness and the residue was partitioned between ethyl acetate and 4% aqueous sodium hydrogencarbonate solution. The organic layer was washed with further 4% aqueous sodium hydrogencarbonate solution, brine, dried (MgSO₄) and evaporated and the residue was treated with diethyl ether. The resultant precipitate was filtered and dried to give the title compound (4.84 g, 55%) as a white solid.
LRMS (m/z): 248 (M+1)⁺.
1H NMR (300 MHz, DMSO-*d₆*) ppm 7.38 (ddd, 1H), 7.58 (dd, 1H), 8.26 (s, 1H), 8.83 (dd, 1H), 12.51 (br s, 1H).

### PREPARATION 84

### 2-(2-Amino-6-fluoroimidazo[1,2-a]pyridin-3-yl)-9-(tetrahydro-2H-pyran-4-yl)-7-((2-(trimethylsilyl)ethoxy)methyl)-7H-purin-8(9H)-one

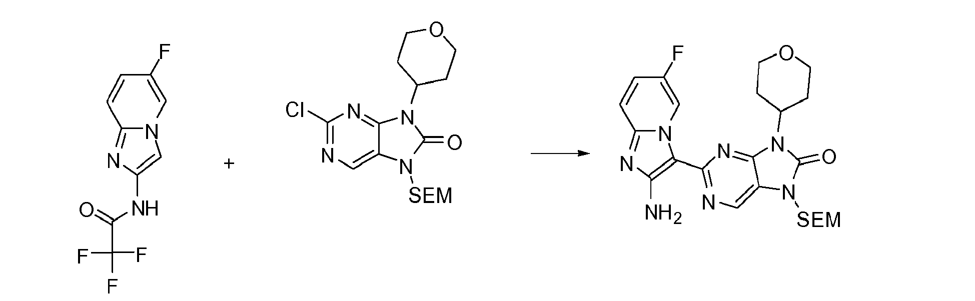

Obtained (78%) from 2-chloro-9-(tetrahydro-2H-pyran-4-yl)-7-((2-(trimethylsilyl)ethoxy)methyl)-7H-purin-8(9H)-one **(Preparation 3)** and 2,2,2-trifluoro-N-(6-fluoroimidazo[1,2-a]pyridin-2-yl)acetamide **(Preparation 83)** following the experimental procedure as described in **Preparation 4.**
LRMS (m/z): 500 (M+1)⁺.
1H NMR (300 MHz, DMSO-d6) δ ppm 0.00 (s, 9H), 0.93 (t, 2H), 1.88 (m, 2H), 2.60 (m, 2H), 3.52-3.69 (m, 4H), 4.07 (m, 2H), 4.63 (m, 1H), 5.37 (s, 2H), 6.75 (s, 1H), 7.46 (d, 1H), 8.86 (s, 1H), 9.92 (d, 1H).

### PREPARATION 85

### 2-(Imidazo[1,2-a]pyrazin-3-yl)-9-(tetrahydro-2H-pyran-4-yl)-7-((2-(trimethylsilyl)ethoxy)methyl)-7H-purin-8(9H)-one

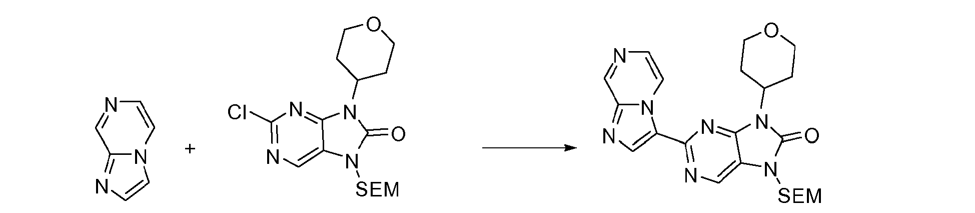

Obtained (82%) from 2-chloro-9-(tetrahydro-2H-pyran-4-yl)-7-((2-(trimethylsilyl)ethoxy)methyl)-7H-purin-8(9H)-one **(Preparation 3)** and imidazo[1,2-a]pyrazine following the experimental procedure as described in **Preparation 4.**
LRMS (m/z): 468 (M+1)⁺.
1H NMR (300 MHz, CDCl₃) δ ppm 0.00 (s, 9H), 0.96 (t, 2H), 1.82 (dd, 2H), 2.86 (ddd, 2H), 3.56 - 3.68 (m, 4H), 4.21 (dd, 2H), 4.68 (tt, 1H), 5.37 (s, 2H), 8.10 (d, 1H), 8.45 (s, 1H), 8.69 (s, 1H), 9.23 (d, 1H), 9.75 (dd, 1H).

### PREPARATION 86

### 1-Aminopyrazin-1-ium 2,4,6-trimethylbenzenesulfonate

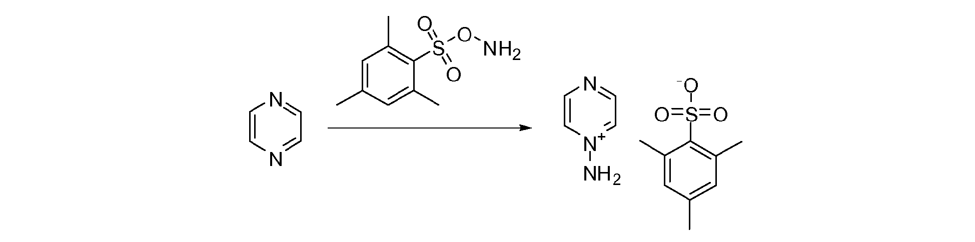

O-(Mesitylsulfonyl)hydroxylamine (2.17 g, 10.1 mmol) in anhydrous dichloromethane (21 mL) was added dropwise to a stirred solution of pyrazine (0.80 g, 10.1 mmol) in anhydrous dichloromethane (10 mL) at 0°C and the mixture was stirred at room temperature. After 1 hour and 30 minutes, diethyl ether was added to the mixture and the precipitate that formed was collected by filtration, washed with diethyl ether and dried in vacuo to give the title compound (2.23 g, 75%) as a white solid.
1H NMR (300 MHz, DMSO-*d*₆) ppm 2.18 (s, 3H), 2.50 (s, 6H), 6.75 (s, 2H), 8.73 (d, 2H), 9.16 (d, 2H), 9.55 (s, 2H).

### PREPARATION 87

### 2-(Pyrazolo[1,5-a]pyrazin-3-yl)-9-(tetrahydro-2H-pyran-4-yl)-7-((2-(trimethylsilyl)ethoxy)methyl)-7H-purin-8(9H)-one

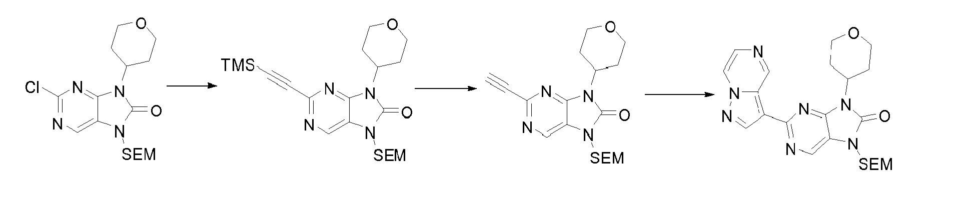

### a) 9-(Tetrahydro-2H-pyran-4-yl)-7-{[2-(trimethylsilyl)ethoxy]methyl}-2-[(trimethylsilyl)ethynyl]-7,9-dihydro-8H-purin-8-one

Triethylamine (5.60 mL, 40.0 mmol) was added to a mixture of 2-chloro-9-(tetrahydro-2*H-*pyran-4-yl)-7-{[2-(trimethylsilyl)ethoxy]methyl}-7,9-dihydro-8*H-*purin-8-one **(Preparation 3,** 4.40 g, 11.4 mmol) and ethynyltrimethylsilane (3.21 mL, 22.9 mmol) in N,N'-dimethylformamide (20 mL) contained in a Schlenck vessel. The mixture was submitted to three vacuum-argon cycles and bis(triphenylphosphine)palladium(II) dichloride (0.80 g, 1.14 mmol) and copper (I) iodide (0.11 g, 0.58 mmol) were added. The mixture was further submitted to three vacuum-argon cycles and sealed and then was stirred and heated to 120 °C. After 30 minutes, the reaction mixture was cooled, partitioned between water and ethyl acetate and the organic layer was washed with water, dried (MgSO₄) and evaporated. The residue was dissolved in ethyl acetate, filtered through Celite® and evaporated. The residue was triturated with a 2:1 mixture of hexane/ethyl acetate and filtered and the filtrate was concentrated to give the title compound (5.10 g, 100%) as an oil.
LRMS (m/z): 447 (M+1)⁺.
1H NMR (300 MHz, CDCl₃) δ ppm 0.01 (s, 9H), 0.05 (s, 9H), 0.98 (m, 2H), 1.68 (m, 2H), 2.81 (m, 2H), 3.53-3.70 (m, 4H), 4.16 (m, 2H), 4.68 (m, 1H), 5.33 (s, 2H), 8.32 (s, 1H).

### b) 2-Ethynyl-9-(tetrahydro-2H-pyran-4-yl)-7-{[2-(trimethylsilyl)ethoxy]methyl}-7,9-dihydro-8H-purin-8-one

Potassium carbonate (0.19 g, 1.4 mmol) was added to a solution of 9-(tetrahydro-2*H-*pyran-4-yl)-7-{[2-(trimethylsilyl)ethoxy]methyl}-2-[(trimethylsilyl)ethynyl]-7,9-dihydro-8*H-*purin-8-one **(Preparation 87a,** 6.00 g, 10.8 mmol) in methanol (20 mL) under an argon atmosphere. After stirring for 30 minutes at room temperature, the mixture was concentrated, partitioned between water and ethyl acetate and the organic layer was washed with brine, dried (MgSO₄) and evaporated. The residue was purified by flash chromatography (99:1 dichloromethane/methanol) to give the title compound (1.30 g, 32%) as an orange solid.
LRMS (m/z): 375 (M+1)⁺.
1H NMR (300 MHz, CDCl₃) δ ppm -0.02 (s, 9H), 0.67 - 0.85 (m, 2H), 1.72 (d, 2H), 2.64 - 2.88 (m, 2H), 3.08 (s, 1H), 3.46 - 3.65 (m, 4H), 4.13 (dd, 2H), 4.58 - 4.73 (m, 1H), 5.44 (s, 2H), 8.33 (s, 1H).

### c) 2-(Pyrazolo[1,5-a]pyrazin-3-yl)-9-(tetrahydro-2H-pyran-4-yl)-7-((2-(trimethylsilyl)ethoxy)methyl)-7H-purin-8(9H)-one

1-Aminopyrazin-1-ium 2,4,6-trimethylbenzenesulfonate **(Preparation 86,** 0.47 g, 1.60 mmol) and potassium carbonate (0.22 g, 1.60 mmol) were added to a solution of 2-ethynyl-9-(tetrahydro-2*H-*pyran-4-yl)-7-{[2-(trimethylsilyl)ethoxy]methyl}-7,9-dihydro-8*H-*purin-8-one **(Preparation 87b,** 0.65 g, 1.74 mmol) in *N,N*'-dimethylformamide (10 mL) in a sealed vessel and the reaction mixture was stirred and heated to 55 °C. After 16 hours, further 1-aminopyrazin-1-ium 2,4,6-trimethylbenzenesulfonate (1.41 g, 4.80 mmol) and potassium carbonate (0.66 g, 4.80 mmol) were added and heating and stirring was continued at 55 °C. The same quantities of these two reactants were added again after 3 hours and again after a further 6 hours and then the mixture was stirred at 55 °C for a further 24 hours. The mixture was then cooled to room temperature, partitioned between dichloromethane and water and the organic layer was dried (MgSO₄) and evaporated. The residue was purified by flash chromatography (98:2 dichloromethane/methanol) to give the title compound (0.26 g, 36%) as a yellow solid.
LRMS (m/z): 468 (M+1)⁺.
1H NMR (300 MHz, CDCl₃) ppm 0.00 (s, 9H), 0.98 (d, 2H), 1.82 (d, 2H), 2.79 - 2.99 (m, 2H), 3.52 - 3.72 (m, 4H), 4.22 (d, 2H), 4.59 - 4.77 (m, 1H), 5.36 (s, 2H), 8.01 (d, 1H), 8.43 (m, 2H), 8.77 (s, 1H), 10.03 (s, 1H).

### PREPARATION 88

### 2-Chloro-9-[(1R)-1-phenylethyl]-7-{[2-(trimethylsilyl)ethoxy]methyl}-7,9-dihydro-8H-purin-8-one

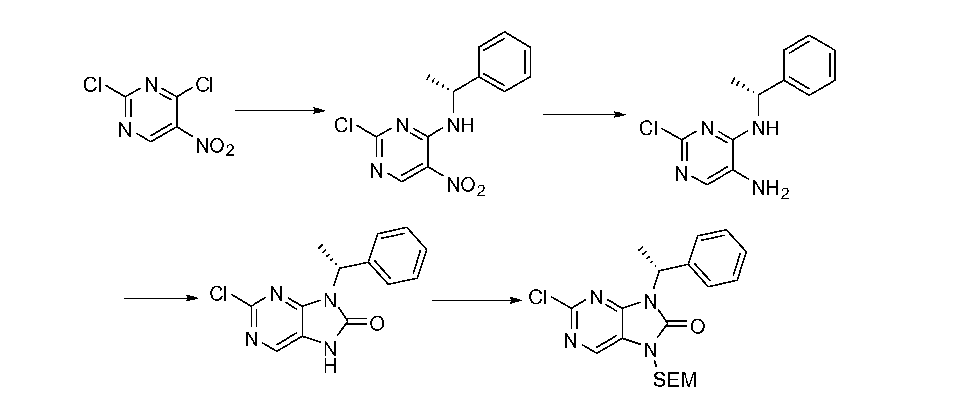

### a) 2-Chloro-5-nitro-N-[(1R)-1-phenylethyl]pyrimidin-4-amine

Obtained as a yellow solid (98%) from 2,4-dichloro-5-nitropyrimidine and (1*R*)-1-phenylethanamine following the experimental procedure as described in Preparation 2a.
LRMS (m/z): 279 (M+1)⁺.
1H NMR (300 MHz, CDCl₃) δ ppm 1.68 (d, 3H), 5.56 (qd, 1H), 7.39 (m, 5H), 8.67 (br s, 1H), 9.05 (s, 1H).

### b) 2-Chloro-N⁴-[(1R)-1-phenylethyl]pyrimidine-4,5-diamine

Obtained as a solid (93%) from 2-chloro-5-nitro-N-[(1*R*)-1-phenylethyl]pyrimidin-4-amine **(Preparation 88a)** following the experimental procedure as described in **Preparation 5b.**
LRMS (m/z): 249 (M+1)⁺.
1H NMR (300 MHz, CDCl₃) δ ppm 1.53 (d, 3H), 5.20 - 5.42 (m, 1H), 7.17 - 7.35 (m, 5H), 7.51 (s, 1H).

### c) 2-Chloro-9-[(1R)-1-phenylethyl]-7,9-dihydro-8H-purin-8-one

Obtained as a solid (57%) from 2-chloro-N-[(1*R*)-1-phenylethyl]pyrimidine-4,5-diamine **(Preparation 88b)** following the experimental procedure as described in **Preparation 42d.**
LRMS (m/z): 275 (M+1)⁺.
1H NMR (300 MHz, CDCl₃) δ ppm 2.06 (d, 3H), 5.79 (q, 1H), 7.28 - 7.41 (m, 3H), 7.57 (d, 2H), 8.11 (s, 1H).

### d) 2-Chloro-9-[(1 R)-1-phenylethyl]-7-{[2-(trimethylsilyl)ethoxy]methyl}-7,9-dihydro-8H-purin-8-one

Obtained (86%) from 2-chloro-9-[(1*R*)-1-phenylethyl]-7,9-dihydro-8*H-*purin-8-one **(Preparation 88c)** following the experimental procedure as described in **Preparation 3.**
LRMS (m/z): 405 (M+1)⁺.
1H NMR (300 MHz, CDCl₃) δ ppm 0.00 (s, 9H), 0.94 (t, 2H), 2.10 (d, 3H), 3.58 (t, 2H), 5.31 (s, 2H), 5.83 (q, 1H), 7.33 -7.42 (m, 3H), 7.59 (d, 1H), 7.62 (d, 1H), 8.18 (s, 1H).

### PREPARATION 89

### (R)-9-(1-Phenylethyl)-2-(pyrazolo[1,5-a]pyrazin-3-yl)-7-((2-(trimethylsilyl)ethoxy)methyl)-7H-purin-8(9H)-one

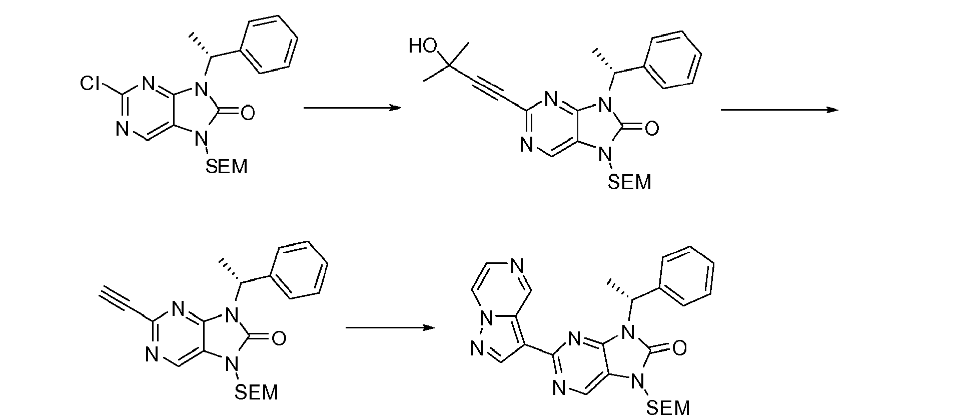

### a) 2-(3-Hydroxy-3-methylbut-1-yn-1-yl)-9-[(1R)-1-phenylethyl]-7-{[2-(trimethylsilyl) ethoxy]methyl}-7,9-dihydro-8H-purin-8-one

Obtained (66%) from 2-chloro-9-[(1*R*)-1-phenylethyl]-7-{[2-(trimethylsilyl)ethoxy] methyl}-7,9-dihydro-8*H-*purin-8-one **(Preparation 88)** and 2-methylbut-3-yn-2-ol following the experimental procedure as described in **Preparation 87a** followed by purification of the crude product by flash chromatography (5.1 to 2:1 hexanes/ethyl acetate).
LRMS (m/z): 453 (M+1)⁺.
1H NMR (300 MHz, CDCl₃) δ ppm -0.05 (s, 9H), 0.83 - 0.95 (m, 2H), 1.67 (s, 6H), 2.05 (d, 3H), 3.40 - 3.63 (m, 2H), 5.28 (s, 2H), 5.85 (q, 1H), 7.28 - 7.39 (m, 3H), 7.56 (m, 2H), 8.27 (s, 1H).

### b) 2-Ethynyl-9-[(1R)-1-phenylethyl]-7-{[2-(trimethylsilyl)ethoxy]methyl}-7,9-dihydro -8H-purin-8-one

Sodium hydride (60% dispersion in mineral oil, 0.014 g, 0.35 mmol) was added to a solution of 2-(3-hydroxy-3-methylbut-1-yn-1-yl)-9-[(1*R*)-1-phenylethyl]-7-([2-(trimethylsilyl) ethoxy]methyl}-7,9-dihydro-8*H-*purin-8-one **(Preparation 89a,** 0.370 g, 0.82 mmol) in toluene (6 mL) and the reaction mixture was stirred and heated to 120 °C in a flask fitted with an open-air condenser. After 90 minutes, the reaction mixture was cooled and applied to a plug of silica and then the product was eluted with 4:1 hexanes/ethyl acetate to yield the title compound (0.110 g, 34%) as a solid.
LRMS (m/z): 395 (M+1)⁺.
1H NMR (300 MHz, CDCl₃) δ ppm -0.03 (s, 9H), 0.83 - 1.00 (m, 2H), 1.72 (s, 1H), 2.07 (d, 3H), 3.47 - 3.64 (m, 2H), 5.30 (s, 2H), 5.86 (q, 1H), 7.27 - 7.40 (m, 3H), 7.58 (d, 2H), 8.30 (s, 1H).

### c) (R)-9-(1-Phenylethyl)-2-(pyrazolo[1,5-a]pyrazin-3-yl)-7-((2-(trimethylsilyl)ethoxy)methyl)-7H-purin-8(9H)-one

Obtained as a white solid (67%) from 2-ethynyl-9-[(1*R*)-1-phenylethyl]-7-{[2-(trimethylsilyl)ethoxy]methyl}-7,9-dihydro -8*H-*purin-8-one **(Preparation 89b)** and 1-aminopyrazin-1-ium 2,4,6-trimethylbenzenesulfonate **(Preparation 86)** following the experimental procedure as described in **Preparation 87c.**
LRMS (m/z): 488 (M+1)⁺.
1H NMR (300 MHz, CDCl₃) δ ppm 0.01 (s, 9H), 0.97 (m, 2H), 1.63 (d, 3H), 3.63 (m, 2H), 5.37 (s, 2H), 5.93 (m, 1H), 7.39 (m, 3H), 7.61 (m, 2H), 8.01 (s, 1H), 8.40 (m, 2H), 8.73 (s, 1H), 9.88 (s, 1H).

### PREPARATION 90

### 3-(4-Chloro-5-nitropyrimidin-2-yl)pyrazolo[1,5-a]pyrazine

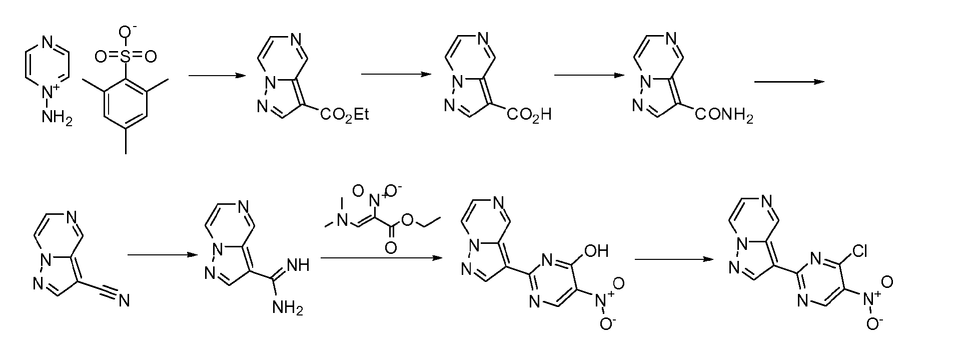

### a) Ethyl pyrazolo[1,5-a]pyrazine-3-carboxylate

Ethyl propiolate (7.43 mL, 73.3 mmol) was added to a suspension of potassium carbonate (12.68 g, 91.8 mmol) in N,N'-dimethylformamide (180 mL). Then 1-aminopyrazin-1-ium 2,4,6-trimethylbenzenesulfonate **(Preparation 86,** 19.50 g, 66.0 mmol) was added in small portions over 90 minutes to the reaction mixture. After stirring at room temperature for 3 hours, the reaction mixture was partitioned between ethyl acetate and water and the aqueous phase was extracted with further ethyl acetate. The combined organic extract was dried (MgSO₄) and evaporated and the residue was purified by flash chromatography (10:1 to 3:1 hexanes/ethyl acetate) to give the title compound (3.66 g, 30%) as an orange solid.
LRMS (m/z): 192 (M+1)⁺.
1H NMR (250 MHz, DMSO-*d₆*) δ ppm 1.37 (t, 3H), 4.36 (q, 2H), 8.19 (d, 1H), 8.66 (s, 1H), 9.00 (dd, 1H), 9.49 (s, 1H).

### b) Pyrazolo[1,5-a]pyrazine-3-carboxylic acid

An aqueous solution of sodium hydroxide (2.5 M, 43.5 mL) was added to a solution of ethyl pyrazolo[1,5-a]pyrazine-3-carboxylate **(Preparation 90a,** 5.20 g, 27.2 mmol) in ethanol (145 mL) and the mixture was stirred and heated to reflux. After 1 hour, the mixture was concentrated to dryness and 10% aqueous hydrogen chloride solution (20 mL) was added. The precipitate was filtered and dried to give the title compound (3.92 g, 88%) as a pink solid.
LRMS (m/z): 162 (M-1)⁺.
1H NMR (300 MHz, DMSO-*d₆*) ppm 8.16 (d, 1H), 8.55 (s, 1H), 8.96 (d, 1H), 9.46 (s, 1H).

### c) Pyrazolo[1,5-a]pyrazine-3-carboxamide:

A suspension of pyrazolo[1,5-a]pyrazine-3-carboxylic acid **(Preparation 90b,** 6.70 g, 41.1 mmol) in thionyl chloride (50 mL) was stirred and heated to reflux. After 7 hours, the mixture was concentrated in vacuo and the residue was azeotroped with toluene (2 x 30 mL). The resultant solid was suspended in 25% aqueous ammonium hydroxide solution (80 mL) and the mixture was stirred for 16 hours at ambient temperature. The mixture was concentrated to dryness to give the crude title compound (10.0 g, >100%) as a beige solid which was used without further purification.
LRMS (m/z): 163 (M+1)⁺.
1H NMR (300 MHz, DMSO-*d₆*) δ ppm 8.06 (d, 1H), 8.68 (s, 1H), 8.86 (d, 1H), 9.55 (s, 1H).

### d) Pyrazolo[1,5-a]pyrazine-3-carbonitrile:

A suspension of crude pyrazolo[1,5-a]pyrazine-3-carboxamide **(Preparation 90c,** 6.66 g) in phosphoryl trichloride (80 mL) was stirred and heated to reflux. After 2.5 hours, the mixture was poured onto a saturated aqueous sodium hydrogen carbonate solution (200 mL) and then the pH was adjusted to 7-8 with a 10% aqueous sodium hydroxide solution. The mixture was extracted with ethyl acetate, the organic layer was dried (MgSO₄) and evaporated and the residue was purified by flash chromatography (1:1 hexanes/ethyl acetate) to give the title compound (3.10 g, 52%) as a yellow solid.
LRMS (m/z): 145 (M+1)⁺.
1H NMR (300 MHz, DMSO-*d₆*) δ ppm 8.25 (d, 1H), 8.84 (s, 1H), 9.05 (d, 1H), 9.48 (s, 1H).

### e) Pyrazolo[1,5-a]pyrazine-3-carboximidamide hydrochloride

Freshly prepared sodium methoxide (0.44 g, 8.1 mmol) was added to a suspension of pyrazolo[1,5-a]pyrazine-3-carbonitrile **(Preparation 90d,** 6.09 g, 42.3 mmol) in anhydrous methanol (350 mL) and the mixture was stirred at ambient temperature. After 20 hours, further sodium methoxide (0.44 g, 8.1 mmol) was added and the reaction mixture was stirred for a further 48 hours. Ammonium chloride (3.91 g, 73.1 mmol) was added and the mixture was stirred and heated to 70 °C in a sealed tube. After 3 days, the mixture was concentrated to dryness to give a solid which was suspended in ethyl acetate and stirred overnight. The precipitate was filtered and dried in vacuo to give the crude title compound (8.50 g, >100%) as a white solid which was used without further purification.
LRMS (m/z): 162 (M+1)⁺.
1H NMR (300 MHz, DMSO-d₆) δ ppm 8.24 (d, 1H), 8.78 (s, 1H), 9.04 (d, 1H), 9.47 (s, 1H).

### f) 5-Nitro-2-(pyrazolo[1,5-a]pyrazin-3-yl)pyrimidin-4-ol

A mixture of crude pyrazolo[1,5-a]pyrazine-3-carboximidamide hydrochloride **(Preparation 90e,** 4.88 g), (Z)-ethyl 3-(dimethylamino)-2-nitroacrylate (8.04 g, 42.7 mmol) and triethylamine (6.25 mL, 44.8 mmol) in ethanol (165 mL) was stirred and heated to 90 °C in a sealed tube. After 22 hours, the reaction mixture was cooled and the precipitate was filtered, washed with ethanol and diethyl ether and dried to give the title compound (3.12 g, 66%) as a yellow solid.
LRMS (m/z): 257 (M-1)⁺.
1H NMR (300 MHz, DMSO-d₆) δ ppm 8.06 (d, 1H), 8.65 (s, 1H), 8.82 (s, 1H), 8.90 (d, 1H), 9.94 (s, 1H).

### g) 3-(4-Chloro-5-nitropyrimidin-2-yl)pyrazolo[1,5-a]pyrazine

A suspension of 5-nitro-2-(pyrazolo[1,5-a]pyrazin-3-yl)pyrimidin-4-ol **(Preparation 90f,** 1.50 g, 5.8 mmol) in phosphoryl trichloride (12 mL) was stirred and heated to 90 °C in a sealed tube. After 2 hours, the mixture was concentrated in vacuo and the residue was azeotroped with toluene. The resultant solid was treated with saturated aqueous sodium hydrogen carbonate solution and, upon scratching, a solid formed which was filtered, washed with water (40 mL) and dried to give the title compound (1.38 g, 86%) as a yellow solid.
LRMS (m/z): 277 (M+1)⁺.
1H NMR (300 MHz, DMSO-d₆) δ ppm 8.30 (d, 1H), 8.97 (s, 1H), 9.10 (d, 1H), 9.57 (s, 1H), 9.88 (s, 1H).

### PREPARATION 91

### (R)-N⁴-(1-(5-Fluoropyridin-2-yl)ethyl)-2-(pyrazolo[1,5-a]pyrazin-3-yl)pyrimidine-4,5-diamine

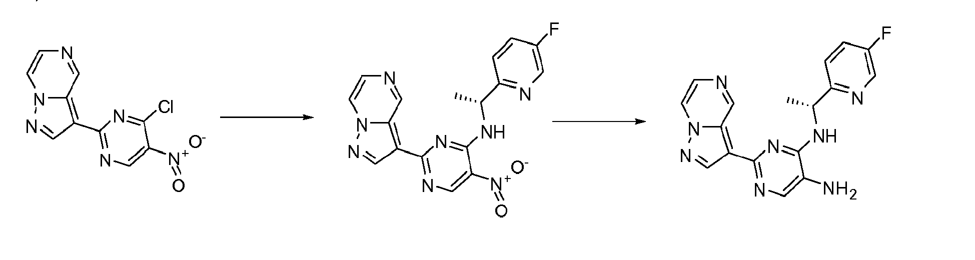

### a) (R)-N-(1-(5-Fluoropyridin-2-yl)ethyl)-5-nitro-2-(pyrazolo[1,5-a]pyrazin-3-yl)pyrimidin-4-amine

Obtained as a yellow solid (86%) from 3-(4-chloro-5-nitropyrimidin-2-yl)pyrazolo[1,5-a]pyrazine **(Preparation 90)** and (R)-1-(5-fluoropyridin-2-yl)ethanamine hydrochloride (Preparation **31d)** following the experimental procedure as described in **Preparation 26a.**
LRMS (m/z): 381 (M+1)⁺.
1H NMR (300 MHz, DMSO-d₆) δ ppm 1.68 (d, 3H), 5.77 (m, 1H), 7.76-7.79 (m, 2H), 8.20 (d, 1H), 8.64 (d, 1H), 8.86 (s, 1H), 8.99 (m, 1H), 9.26 (s, 1H), 9.38 (d, 1H), 9.77 (s, 1H).

### b) (R)-N⁴-(1 -(5-Fluoropyridin-2-yl)ethyl)-2-(pyrazolo[1,5-a]pyrazin-3-yl)pyrimidine-4,5-diamine

Obtained as an off white solid (97%) from (R)-N-(1-(5-fluoropyridin-2-yl)ethyl)-5-nitro-2-(pyrazolo[1,5-a]pyrazin-3-yl)pyrimidin-4-amine **(Preparation 91a)** following the experimental procedure as described in **Preparation 5b.**
LRMS (m/z): 351 (M+1)⁺.
1H NMR (300 MHz, DMSO-d₆) δ ppm 1.59 (d, 3H), 5.11 (s, 2H), 5.39 (m, 1H), 6.99 (d, 1H), 7.49 (m, 1H), 7.65 (m, 1H), 7.69 (s, 1H), 7.90 (d, 1H), 8.40 (s, 1H), 8.53 (d, 1H), 8.72 (m, 1H), 9.46 (s, 1H).

### PREPARATION 92

### 2-(pyrazolo[1,5-a]pyrazin-3-yl)-N⁴-((tetrahydro-2H-pyran-4-yl)methyl)pyrimidine-4,5-diamine

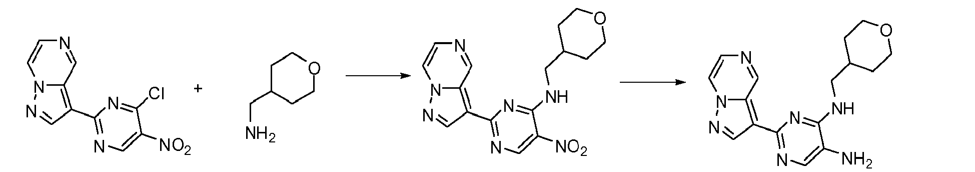

### a) 5-nitro-2-(pyrazolo[1,5-a]pyrazin-3-yl)-N-((tetrahydro-2H-pyran-4-yl)methyl)pyrimidin-4-amine

Obtained as a yellow solid (98%) from 3-(4-chloro-5-nitropyrimidin-2-yl)pyrazolo[1,5-a]pyrazine **(Preparation 90)** and (tetrahydro-2H-pyran-4-yl)methanamine following the experimental procedure as described in **Preparation 26a.**
LRMS (m/z): 356 (M+1)⁺.
1H NMR (300 MHz, DMSO-*d₆*) δ ppm 1.33 (dq, 2H), 1.67 (d, 2H), 2.08 (m, 1H), 3.25 (t, 2H), 3.66 (m, 2H), 3.86 (dd, 2H), 8.19 (d, 1H), 8.86 (s, 1H), 9.02 (dd, 1H), 9.05 (m, 1H), 9.21 (s, 1H), 9.87 (d, 1H).

### b) 2-(pyrazolo[1,5-a]pyrazin-3-yl)-N⁴-((tetrahydro-2H-pyran-4-yl)methyl)pyrimidine-4,5-diamine

Obtained as a pale brown solid (91%) from 5-nitro-2-(pyrazolo[1,5-a]pyrazin-3-yl)-N-((tetrahydro-2H-pyran-4-yl)methyl)pyrimidin-4-amine **(Preparation 92a)** following the experimental procedure as described in **Preparation 30b.**
LRMS (m/z): 326 (M+1)⁺.
1H NMR (300 MHz, CDCl₃) δ ppm 1.46 (dq, 2H), 1.76 (d, 2H), 2.04 (m, 1H), 3.01 (br s, 2H), 3.41 (t, 2H), 3.57 (m, 2H), 4.02 (dd, 2H), 5.09 (br s, 1H), 7.88 (s, 1H), 7.93 (d, 1H), 8.40 (dd, 1H), 8.65 (s, 1H), 9.97 (d, 1H).

### PREPARATION 93

### N4-(4,4-Difluorocyclohexyl)-2-(pyrazolo[1,5-a]pyrazin-3-yl)pyrimidine-4,5-diamine

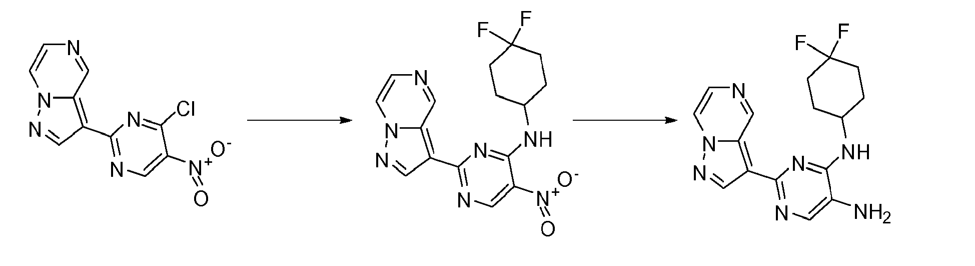

### a) N-(4,4-Difluorocyclohexyl)-5-nitro-2-(pyrazolo[1,5-a]pyrazin-3-yl)pyrimidin-4-amine

Obtained as a yellow solid (86%) from 3-(4-chloro-5-nitropyrimidin-2-yl)pyrazolo[1,5-a]pyrazine **(Preparation 90)** and 4,4-difluorocyclohexanamine following the experimental procedure as described in **Preparation 26a.**
LRMS (m/z): 376 (M+1)⁺.
1H NMR (300 MHz, DMSO-d₆) δ ppm 1.88-2.17 (m, 8H), 4.58 (m, 1H), 8.20 (d, 1H), 8.54 (d, 1H), 8.95 (s, 1H), 9.02 (d, 1H), 9.23 (s, 1H), 9.86 (s, 1H).

### b) N⁴-(4,4-Difluorocyclohexyl)-2-(pyrazolo[1,5-a]pyrazin-3-yl)pyrimidine-4,5-diamine

Obtained as an off white solid (87%) from N-(4,4-difluorocyclohexyl)-5-nitro-2-(pyrazolo[1,5-a]pyrazin-3-yl)pyrimidin-4-amine **(Preparation 93a)** following the experimental procedure as described in **Preparation 5b.**
LRMS (m/z): 346 (M+1)⁺.
1H NMR (300 MHz, DMSO-d₆) δ ppm 1.66 (m, 2H), 2.03-2.18 (m, 6H), 4.32 (m, 1H), 4.99 (s, 2H), 6.44 (d, 1H), 7.71 (s, 1H), 7.96 (d, 1H), 8.60 (s, 1H), 8.84 (d, 1H), 9.84 (s, 1H).

### PREPARATION 94

### N-(2,2-Dimethyltetrahydro-2H-pyran-4-yl)-5-nitro-2-(pyrazolo[1,5-a]pyrazin-3-yl)pyrimidin-4-amine

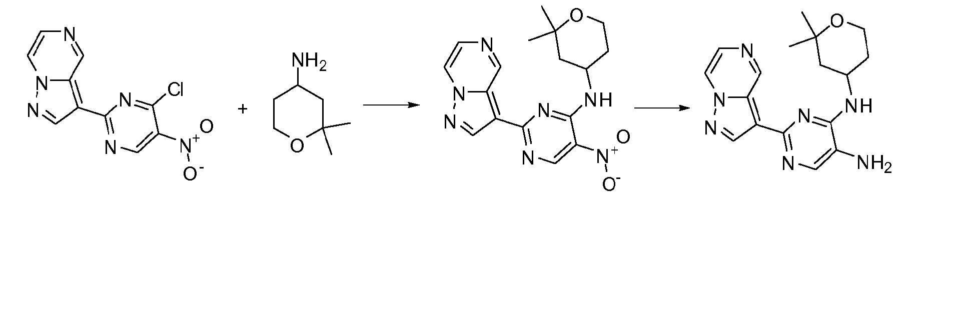

### a) N-(2,2-Dimethyltetrahydro-2H-pyran-4-yl)-5-nitro-2-(pyrazolo[1,5-a]pyrazin-3-yl)pyrimidin-4-amine

Obtained as a yellow solid (59%) from 3-(4-chloro-5-nitropyrimidin-2-yl)pyrazolo[1,5-a]pyrazine **(Preparation 90)** and 2,2-dimethyltetrahydro-2H-pyran-4-amine following the experimental procedure as described in **Preparation 26a.**
LRMS (m/z): 370 (M+1)⁺.
1H NMR (300 MHz, DMSO-d₆) δ ppm 1.22 (s, 3H), 1.42 (s, 3H), 1.60 (m, 1H), 1.77 (m, 1H), 1.97 (m, 2H), 3.80 (m, 2H), 4.81 (m, 1H), 8.22 (d, 1H), 8.47 (d, 1H), 8.87 (s, 1H), 9.04 (d, 1H), 9.24 (s, 1H), 9.89 (s, 1H).

### b) N⁴-(2,2-Dimethyltetrahydro-2H-pyran-4-yl)-2-(pyrazolo[1,5-a]pyrazin-3-yl)pyrimidine-4,5-diamine

Obtained as a yellow solid (87%) from N-(2,2-dimethyltetrahydro-2H-pyran-4-yl)-5-nitro-2-(pyrazolo[1,5-a]pyrazin-3-yl)pyrimidin-4-amine **(Preparation 94a)** following the experimental procedure as described in **Preparation 30b.**
LRMS (m/z): 340 (M+1)⁺.
1H NMR (300 MHz, DMSO-d₆) δ ppm 1.20 (s, 3H), 1.29 (m, 1H), 1.39 (s, 3H), 1.50 (m, 1H), 2.0 (m, 2H), 3.77 (m, 2H), 4.51 (m, 1H), 4.96 (s, 2H), 6.36 (d, 1H), 7.67 (s, 1H), 7.95 (d, 1H), 8.53 (s, 1H), 8.80 (d, 1H), 9.87 (s, 1H).

### PREPARATION 95

### (R)-N⁴-(8-Fluorochroman-4-yl)-2-(pyrazolo[1,5-a]pyrazin-3-yl)pyrimidine-4,5-diamine

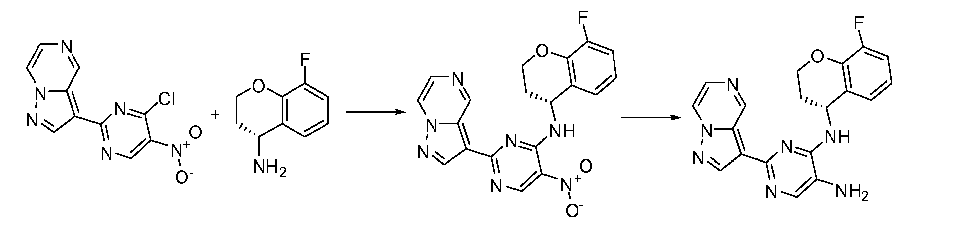

### a) (R)-N-(8-Fluorochroman-4-yl)-5-nitro-2-(pyrazolo[1,5-a]pyrazin-3-yl)pyrimidin-4-amine

Obtained as a yellow solid (94%) from 3-(4-chloro-5-nitropyrimidin-2-yl)pyrazolo[1,5-a]pyrazine **(Preparation 90)** and (*R*)-8-fluorochroman-4-amine hydrochloride following the experimental procedure as described in **Preparation 26a.**
LRMS (m/z): 408 (M+1)⁺.
1H NMR (300 MHz, DMSO-d₆) δ ppm 2.36 (m, 2H), 4.47 (m, 2H), 5.97 (d, 1H), 6.69 (m, 1H), 7.17 (m, 2H), 8.20 (d, 1H), 8.94 (s, 1H), 8.96 (d, 1H), 9.02 (d, 1H), 9.31 (s, 1H), 9.84 (s, 1H).

### b) (R)-N⁴-(8-Fluorochroman-4-yl)-2-(pyrazolo[1,5-a]pyrazin-3-yl)pyrimidine-4,5-diamine

Obtained as a yellow solid (95%) from (R)-N-(8-fluorochroman-4-yl)-5-nitro-2-(pyrazolo[1,5-a]pyrazin-3-yl)pyrimidin-4-amine **(Preparation 95a)** following the experimental procedure as described in **Preparation 30b.**
LRMS (m/z): 378 (M+1)⁺.
1H N MR (300 MHz, CDCl₃) δ ppm 2.41 (m, 2H), 3.08 (s, 2H), 4.30-4.49 (m, 2H), 5.20 (d, 1H), 5.56 (d, 1H), 6.83 (m, 1H), 7.09 (m, 2H), 7.94 (m, 2H), 8.40 (d, 1H), 8.67 (s, 1H), 9.94 (s, 1H).

### PREPARATION 96

### 2-((1r,4r)-4-(5-Amino-2-(pyrazolo[1,5-a]pyrazin-3-yl)pyrimidin-4-ylamino)cyclohexyl)acetonitrile

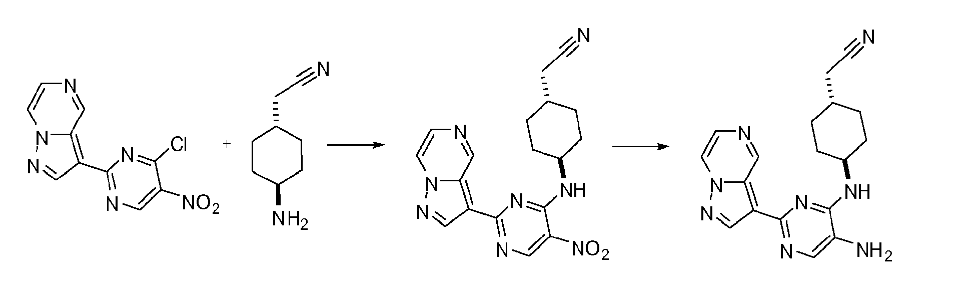

### a) 2-((1r,4r)-4-(5-nitro-2-(pyrazolo[1,5-a]pyrazin-3-yl)pyrimidin-4-ylamino)cyclohexyl)acetonitrile

Obtained as a yellow solid (90%) from 3-(4-chloro-5-nitropyrimidin-2-yl)pyrazolo[1,5-a]pyrazine **(Preparation 90)** and 2-((1r,4r)-4-aminocyclohexyl)acetonitrile hydrochloride (Preparation **42d)** following the experimental procedure as described in **Preparation 26a.**
LRMS (m/z): 379 (M+1)⁺.
1H NMR (300 MHz, DMSO-d₆) δ ppm 1.28-1.44 (m, 3H), 1.59 -1.78 (m, 4H), 1.94 (m, 2H), 2.15 (m, 2H), 4.31 (m, 1H), 8.24 (d, 1H), 8.50 (d, 1H), 8.90 (s, 1H), 9.06 (d, 1H), 9.26 (s, 1H), 9.87 (s, 1H).

### b) 2-((1r,4r)-4-(5-Amino-2-(pyrazolo[1,5-a]pyrazin-3-yl)pyrimidin-4-ylamino)cyclohexyl)acetonitrile

Obtained as a pale yellow solid (78%) from 2-((1r,4r)-4-(5-nitro-2-(pyrazolo[1,5-a]pyrazin-3-yl)pyrimidin-4-ylamino)cyclohexyl)acetonitrile **(Preparation 96a)** following the experimental procedure as described in **Preparation 30b.**
LRMS (m/z): 349 (M+1)⁺.
1H NMR (300 MHz, DMSO-d₆) δ ppm 1.32-1.38 (m, 4H), 1.75 (m, 1H), 1.95 (m, 2H), 2.21 (m, 2H), 4.02 (m, 1H), 4.97 (s, 2H), 6.41 (d, 1H), 7.67 (s, 1H), 7.96 (d, 1H), 8.54 (s, 1H), 8.83 (d, 1H), 9.84 (s, 1H).

### PREPARATION 97

### 3-(Tributylstannyl)pyrazolo[1,5-a]pyrazine

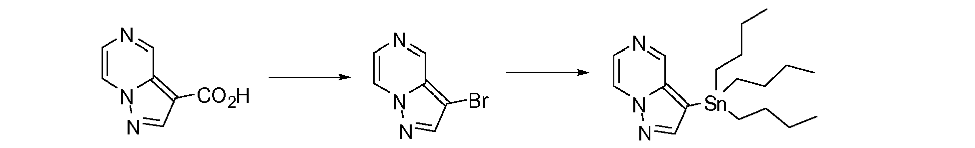

### a) 3-Bromopyrazolo[1,5-a]pyrazine

Sodium hydrogen carbonate (6.06 g, 72.1 mmol) and N-bromosuccinimide (4.28 g, 24.0 mmol) were added sequentially to a suspension of pyrazolo[1,5-a]pyrazine-3-carboxylic acid **(Preparation 90b,** 3.92 g, 24.0 mmol) in N,N'-dimethylformamide (67 mL) and the mixture was stirred at room temperature. After 6 hours, the mixture was partitioned between ethyl acetate and water and the aqueous phase was extracted with further ethyl acetate. The combined organic extract was dried (MgSO₄) and evaporated and the residue was purified by flash chromatography (4:1 hexanes/ethyl acetate) to give the title compound (3.60 g, 76%) as a cream coloured solid.
LRMS (m/z): 198/200 (M+1)⁺.
H NMR (250 MHz, DMSO-d₆) δ ppm 8.00 (d, 1H), 8.35 (s, 1H), 8.83 (dd, 1H), 9.11 (d, 1H).

### b) 3-(Tributylstannyl)pyrazolo[1,5-a]pyrazine

A mixture of 3-bromopyrazolo[1,5-a]pyrazine **(Preparation 97a,** 2.00 g, 10.1 mmol), 1,1,1,2,2,2-hexabutyldistannane (21.0 mL, 41.3 mmol) and tetrakis(triphenylphosphine)palladium(0) (1.17 g, 1.0 mmol) in 1,4-dioxane (20 mL) was stirred and heated under microwave irradiation ("Initiator sixty" from Biotage^{®}) at 130 °C under an atmosphere of argon. After 1 hour, the reaction mixture was diluted with ethyl acetate and filtered through diatomaceous earth (Celite^{®}) and the filtrate was concentrated. The residue was purified by flash chromatography (hexanes/ethyl acetate) to give the title compound (1.39 g, 34%) as an oil.
LRMS (m/z): 409 (M+1)⁺.
1H NMR (300 MHz, CDCl₃) δ ppm 0.86-0.92 (m, 9H), 1.15-1.21 (m, 6H), 1.32-1.39 (m, 6H), 1.52-1.59 (m, 6H), 7.85 (d, 1H), 7.94 (s 1H), 8.46 (d, 1H), 8.97 (s, 1H).

### PREPARATION 98

### 5-(Pyrazolo[1,5-a]pyrazin-3-yl)-3-(tetrahydro-2H-pyran-4-yl)-1-((2-(trimethylsilyl)ethoxy)methyl)-1-imidazo[4,5-b]pyridin-2(3H)-one

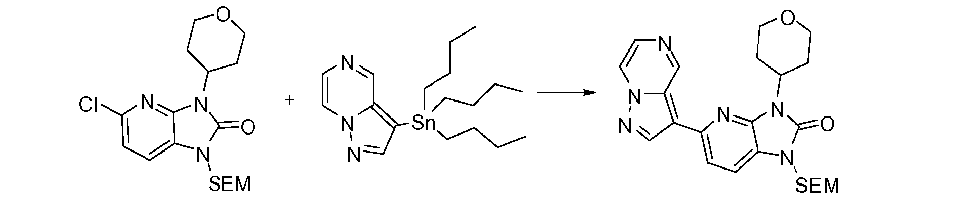

A mixture of 5-chloro-3-(tetrahydro-2*H-*pyran-4-yl)-1-{[2-(trimethylsilyl)ethoxy]methyl}-1,3-dihydro-2*H-*imidazo[4,5*-b*]pyridin-2-one **(Preparation 70,** 0.877 g, 2.15 mmol) and 3-(tributylstannyl)pyrazolo[1,5-a]pyrazine (Preparation **97,** 0.550 g, 1.43 mmol) in 1,4-dioxane contained in a Schlenck vessel was submitted to three vacuum-argon cycles and tetrakis(triphenylphosphine)palladium(0) (0.165 g, 0.14 mmol) was then added. The mixture was further submitted to three vacuum-argon cycles, sealed and then was stirred and heated to 100 °C. After 20 hours, the reaction mixture was cooled, diluted with methanol, filtered through diatomaceous earth (Celite®) and evaporated. The residue was purified by flash chromatography (98:2 dichloromethane/methanol) to give the title compound (0.444 g, 66%) as a yellow solid.
LRMS (m/z): 467 (M+1)⁺.
1H NMR (300 MHz, CDCl₃) δ ppm 0.03 (s, 9H), 0.97 (m, 2H), 1.84 (m, 2H), 2.97 (m, 2H), 3.59-3.69 (m, 4H), 4.22 (m, 2H), 4.75 (m, 1H), 5.38 (s, 2H), 7.48 (m, 2H), 7.98 (d, 1H), 8.42 (d, 1H), 8.44 (s, 1H), 9.98 (d, 1H).

### PREPARATION 99

### 6-Fluoro-5-(pyrazolo[1,5-a]pyrazin-3-yl)-3-(tetrahydro-2H-pyran-4-yl)-1-((2-(trimethylsilyl)ethoxy)methyl)-1H-imidazo[4,5-b]pyridin-2(3H)-one

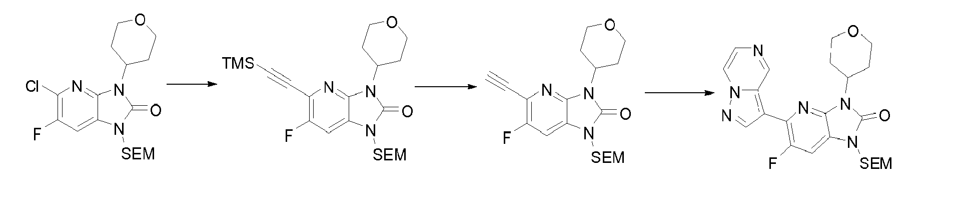

### a) 6-Fluoro-3-(tetrahydro-2H-pyran-4-yl)-1-{[2-(trimethylsilyl)ethoxy]methyl}-5-[(trimethylsilyl)ethynyl]-1,3-dihydro-2H-imidazo[4,5-b]pyridin-2-one

Obtained as a brown oil (68%) from 5-chloro-6-fluoro-3-(tetrahydro-2*H-*pyran-4-yl)-1-{[2-(thmethylsilyl)ethoxy]methyl}-1,3-dihydro-2*H-*imidazo[4,5*-b*]pyridin-2-one **(Preparation 79)** and ethynyltrimethylsilane following the experimental procedure as described in **Preparation 87a.**
LRMS (m/z): 464 (M+1)⁺.

### b) 5-Ethynyl-6-fluoro-3-(tetrahydro-2H-pyran-4-yl)-1-{[2-(trimethylsilyl)ethoxy] methyl}-1,3-dihydro-2H-imidazo[4,5-b]pyridin-2-one

Obtained as a yellow solid (50%) from 6-fluoro-3-(tetrahydro-2*H-*pyran-4-yl)-1-{[2-(trimethylsilyl)ethoxy]methyl}-5-[(trimethylsilyl)ethynyl]-1,3-dihydro-2*H-*imidazo[4,5-b]pyridin-2-one **(Preparation 99a)** following the experimental procedure as described in **Preparation 87b.**
LRMS (m/z): 392 (M+1)⁺.
1H NMR (300 MHz, CDCl₃) δ ppm 0.02 (s, 9H), 0.94 (m, 2H), 1.74 (m, 2H), 2.82 (m, 2H), 3.47-3.64 (m, 5H), 4.15 (m, 2H), 4.65 (m, 1H), 5.30 (s, 2H), 7.20 (d, 1H).

### c) 6-Fluoro-5-(pyrazoio[1,5-a]pyrazin-3-yl)-3-(tetrahydro-2H-pyran-4-yl)-1-((2-(trimethylsilyl)ethoxy)methyl)-1H-imidazo[4,5-b]pyridin-2(3H)-one

Obtained as a yellow solid (51%) from 5-ethynyl-6-fluoro-3-(tetrahydro-2*H-*pyran-4-yl)-1-{[2-(trimethylsilyl)ethoxy] methyl}-1,3-dihydro-2*H-*imidazo[4,5*-b*]pyridin-2-one **(Preparation 99b)** and 1-aminopyrazin-1-ium 2,4,6-trimethylbenzenesulfonate **(Preparation 86)** following the experimental procedure as described in **Preparation 87c.**
LRMS (m/z): 485 (M+1)⁺.
1H NMR (300 MHz, CDCl₃) δ ppm 0.00 (s, 9H), 0.76 - 1.05 (m, 2H), 1.82 (dd, 2H), 2.91 (dd, 2H), 3.52 - 3.71 (m, 4H), 4.22 (dd, 2H), 4.63 - 4.80 (m, 1H), 5.34 (s, 2H), 7.33 (dd, 1H), 8.01 (dd, 1H), 8.45 (dt, 1H), 8.59 (dd, 1H), 10.03 (s, 1H).

### PREPARATION 100

### 1-Aminopyridinium 2,4,6-trimethylbenzenesulfonate

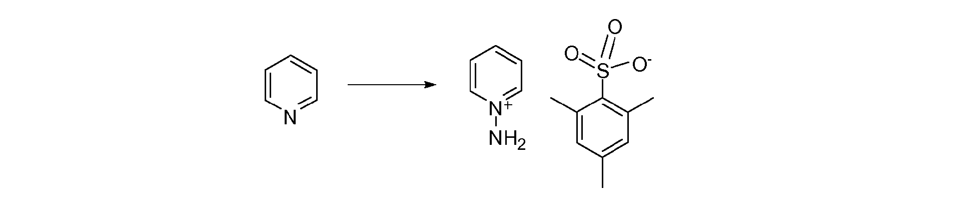

Obtained as a white solid (65%) from O-(mesitylsulfonyl)hydroxylamine and pyridine following the experimental procedure as described in **Preparation 86.**
1H NMR (300 MHz, CDCl₃) δ ppm 2.23 (s, 3H), 2.61 (s, 6H), 6.82 (s, 2H), 7.63 (t, 2H), 7.85 (t, 1H), 8.96 (d, 2H).

### PREPARATION 101

### 2-(Pyrazolo[1,5-a]pyridin-3-yl)-9-(tetrahydro-2H-pyran-4-yl)-7-((2-(trimethylsilyl)ethoxy)methyl)-7H-purin-8(9H)-one

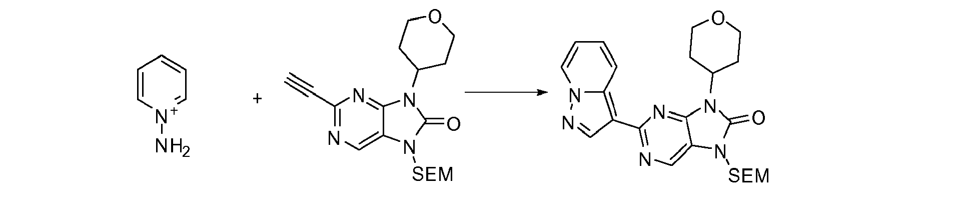

Obtained as an orange oil (41%) from 1-aminopyridinium 2,4,6-trimethylbenzenesulfonate **(Preparation 100)** and 2-ethynyl-9-(tetrahydro-2*H-*pyran-4-yl)-7-{[2-(trimethylsilyl)ethoxy]methyl}-7,9-dihydro-8*H-*purin-8-one **(Preparation 87b)** following the experimental procedure as described in **Preparation 87c.**
LRMS (m/z): 467 (M+1)⁺.
1H NMR (300 MHz, CDCl₃) δ ppm 0.01 (s, 9H), 0.96 (t, 2H), 1.85 (d, 2H), 2.94 (dq, 2H), 3.58-3.68 (m, 4H), 4.20 (m, 2H), 4.67 (m, 1H), 5.35 (s, 2H), 6.91 (t, 1H), 7.39 (t, 1H), 8.39 (s, 1H), 8.57 (d, 1H), 8.61 (d, 1H), 8.73 (s, 1H).

### PREPARATION 102

### Ethyl 6-fluoropyrazolo[1,5-a]pyridine-3-carboxylate and ethyl 4-fluoropyrazolo[1,5-a]pyridine-3-carboxylate

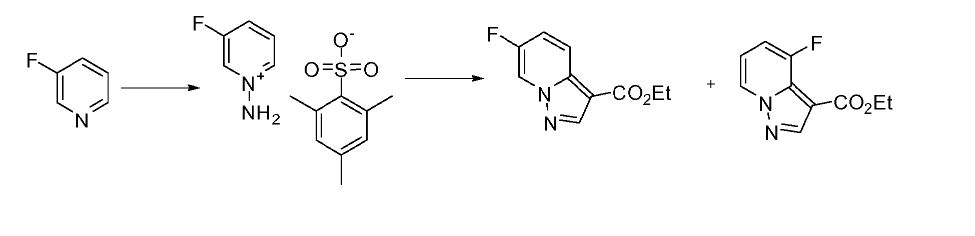

### a) 1-Amino-3-fluoropyridinium 2,4,6-trimethylbenzenesulfonate

Obtained as a white solid (60%) from O-(mesitylsulfonyl)hydroxylamine and 3-fluoropyridine following the experimental procedure as described in **Preparation 86.**
1H NMR (300 MHz, CD₃OD) δ ppm 2.23 (s, 3H), 2.61 (s, 6H), 6.86 (s, 2H), 8.00 (m, 1H), 8.15 (m, 1H), 8.64 (m, 1H), 8.92 (s, 1H).

### b) Ethyl 6-fluoropyrazolo[1,5-a]pyridine-3-carboxylate and ethyl 4-fluoropyrazolo[1,5-a]pyridine-3-carboxylate

Obtained as an isomeric mixture from 1-amino-3-fluoropyridinium 2,4,6-trimethylbenzenesulfonate **(Preparation 102a)** and ethyl propiolate following the experimental procedure as described in **Preparation 90a.** After stirring at room temperature for 3 days, the reaction mixture was partitioned between ethyl acetate and water and the aqueous phase was extracted with ethyl acetate. The combined organic extract was dried (MgSO₄) and evaporated and the residue was purified by flash chromatography (20:1 to 10:1 hexanes/ethyl acetate) to give ethyl 6-fluoropyrazolo[1,5-a]pyridine-3-carboxylate (0.450 g, 17%) as a white solid
LRMS (m/z): 209 (M+1)⁺.
1H NMR (300 MHz CDCl₃) δ ppm 1.42 (t, 3H), 4.40 (q, 2H), 7.35 (m, 1H), 8.16 (m, 1H), 8.40 (s, 1H), 8.48 (s, 1H).
and ethyl 4-fluoropyrazolo[1,5-a]pyridine-3-carboxylate (1.02 g, 38%) as a white solid.
LRMS (m/z): 209 (M+1)⁺.
1H NMR (300 MHz CDCl₃) δ ppm 1.41 (t, 3H), 4.40 (q, 2H), 6.90 (m, 1H), 7.10 (m, 1H), 8.40 (d, 1H), 8.44 (s, 1H).

### PREPARATION 103

### 6-Fluoro-3-(4,4,5,5-tetramethyl-1,3,2-dioxaborolan-2-yl)pyrazolo[1,5-a]pyridine

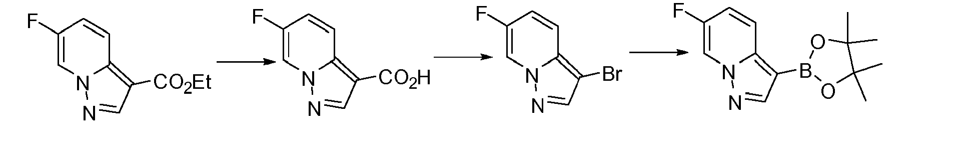

### a) 6-Fluoropyrazolo[1,5-a]pyridine-3-carboxylic acid

Obtained as a white solid (86%) from ethyl 6-fluoropyrazolo[1,5-a]pyridine-3-carboxylate **(Preparation 102)** following the experimental procedure as described in **Preparation 90b.**
LRMS (m/z): 179 (M-1)⁺.
1H NMR (300 MHz, DMSO-d₆) δ ppm 7.69 (m, 1H), 8.10 (dd, 1H), 8.44 (s, 1H), 9.23 (m, 1H).

### b) 3-Bromo-6-fluoropyrazolo[1,5-a]pyridine

Obtained as a white solid (87%) from 6-fluoropyrazolo[1,5-a]pyridine-3-carboxylic acid **(Preparation 103a)** following the experimental procedure as described in **Preparation 97a.**
LRMS (m/z): 215/217 (M+1)⁺.
1H NMR (300 MHz, CDCl₃) δ ppm 7.16 (m, 1H), 7.50 (m, 1H), 7.92 (s, 1H), 8.41 (s, 1H).

### c) 6-Fluoro-3-(4,4,5,5-tetramethyl-1,3,2-dioxaborolan-2-yl)pyrazolo[1,5-a]pyridine

A mixture of 3-bromo-6-fluoropyrazolo[1,5-a]pyridine (Preparation 103b, 0.300 g, 1.4 mmol), potassium acetate (0.492 g, 5.0 mmol) and bis(pinacolato)diboron (2.77 g, 10.9 mmol) in 1,4-dioxane (5 mL) contained in a Schlenck vessel was submitted to three vacuum-argon cycles and tetrakis(triphenylphosphine)palladium(0) (0.380 g, 0.33 mmol) was then added. The mixture was further submitted to three vacuum-argon cycles, sealed and then was stirred and heated to 100 °C. After 20 hours, the reaction mixture was cooled, evaporated and then taken up in pentane and filtered through diatomaceous earth (Celite®) and the filter cake was washed with a mixture of ethyl acetate/ether (3:2). The combined filtrate and washings were evaporated and the residue was purified by reverse phase chromatography (C-18 silica from Waters©, water/acetonitrile/methanol as eluents [0.1% v/v formic acid buffered] 0% to 100%) to give the title compound (0.130 g, 36%) as a yellow solid.
LRMS (m/z): 263 (M+1)⁺.
1H NMR (300 MHz, CDCl₃) δ ppm (two sets of peaks are seen in the NMR due to the presence of both the boronate and boronic acid): NMR of boronate: 1.21 (s, 12H), 7.56 (m, 1H), 8.02 (m, 1H), 8.36 (s, 1H), 9.16 (m, 1H).

### PREPARATION 104

### 2-(6-Fluoropyrazolo[1,5-a]pyridin-3-yl)-9-(tetrahydro-2H-pyran-4-yl)-7-((2-(trimethylsilyl)ethoxy)methyl)-7H-purin-8(9H)-one

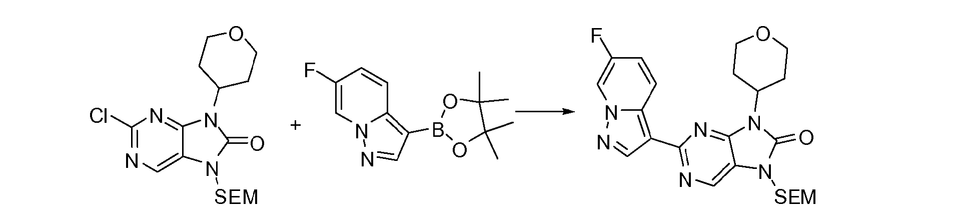

A mixture of 2-chloro-9-(tetrahydro-2*H-*pyran-4-yl)-7-{[2-(trimethylsilyl)ethoxy]methyl}-7,9-dihydro-8*H-*purin-8-one **(Preparation 3,** 0.150 g, 0.39 mmol), 6-fluoro-3-(4,4,5,5-tetramethyl-1,3,2-dioxaborolan-2-yl)pyrazolo[1,5-a]pyridine **(Preparation 103,** 0.183 g, 0.70 mmol) and potassium acetate (0.134 g, 1.37 mmol) in 1,4-dioxane (5 mL) and water (1.5 mL) contained in a Schlenck vessel was submitted to three vacuum-argon cycles and tetrakis(triphenylphosphine)palladium(0) (0.040 g, 0.03 mmol) was then added. The mixture was further submitted to three vacuum-argon cycles, sealed and then was stirred and heated under microwave irradiation ("Initiator sixty" from Biotage^{®}) at 120 °C under an atmosphere of argon. After 40 minutes, further 2-chloro-9-(tetrahydro-2*H-*pyran-4-yl)-7-{[2-(trimethylsilyl)ethoxy]methyl}-7,9-dihydro-8*H-*purin-8-one (0.060 g, 0.16 mmol) and tetrakis(triphenylphosphine)palladium(0) (0.020 g, 0.017 mmol) were added and heating was continued for 90 minutes. The reaction mixture was evaporated and then was partitioned between ethyl acetate and water and the organic layer was dried (MgSO₄) and concentrated. The residue was purified by flash chromatography (3:1 to 2:1 hexanes/ethyl acetate) to give the title compound (0.096 g, 50%) as pale yellow solid.
LRMS (m/z): 485 (M+1)⁺.
1H NMR (300 MHz, CDCl₃) δ ppm 0.06 (s, 9H), 1.00 (t, 2H), 1.80 (m, 2H), 2.91 (dq, 2H), 3.49 (s, 2H), 3.68 (m, 2H), 4.22 (m, 2H), 4.63 (m, 1H), 5.25 (s, 2H), 7.38 (t, 1H), 8.40 (s, 1H), 8.52 (m, 1H), 8.65 (dd, 1H), 8.78 (s, 1H).

### PREPARATION 105

### 4-Fluoro-3-(4,4,5,5-tetramethyl-1,3,2-dioxaborolan-2-yl)pyrazolo[1,5-a]pyridine

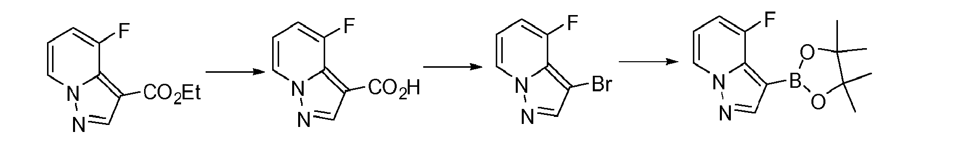

### a) 4-Fluoropyrazolo[1,5-a]pyridine-3-carboxylic acid

Obtained as a white solid (100%) from ethyl 4-fluoropyrazolo[1,5-a]pyridine-3-carboxylate **(Preparation 102)** following the experimental procedure as described in **Preparation 90b.**
LRMS (m/z): 179 (M-1)⁺.
1H NMR (300 MHz, DMSO-d₆) δ ppm 7.15 (m, 1H), 7.42 (dd, 1H), 8.45 (s, 1H), 8.75 (d, 1H).

### b) 3-Bromo-4-fluoropyrazolo[1,5-a]pyridine

Obtained as a white solid (70%) from 4-fluoropyrazolo[1,5-a]pyridine-3-carboxylic acid **(Preparation 105a)** following the experimental procedure as described in **Preparation 97a.**
LRMS (m/z): 215/217 (M+1)⁺.
1H NMR (300 MHz, DMSO-d₆) δ ppm 7.02 (m, 1H), 7.22 (dd, 1H), 8.21 (s, 1 H), 8.65 (d, 1H).

### c) 4-Fluoro-3-(4,4,5,5-tetramethyl-1,3,2-dioxaborolan-2-yl)pyrazolo[1,5-a]pyridine

Obtained as a solid (100%) from 3-bromo-4-fluoropyrazolo[1,5-a]pyridine **(Preparation 105b)** and bis(pinacolato)diboron following the experimental procedure as described in **Preparation 103c.**
LRMS (m/z): 263 (M+1)⁺.
1H NMR (300 MHz, DMSO-d₆) δ ppm 1.15 (s, 12H), 7.02 (m, 1H), 7.23 (dd, 1H), 8.18 (s, 1H), 8.69 (d, 1H).

### PREPARATION 106

### 2-(4-Fluoropyrazolo[1,5-a]pyridin-3-yl)-9-(tetrahydro-2H-pyran-4-yl)-7-((2-(trimethylsilyl)ethoxy)methyl)-7H-purin-8(9H)-one

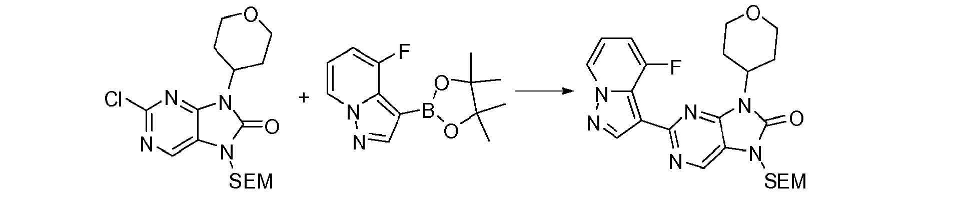

Obtained as an oil from 2-chloro-9-(tetrahydro-2*H-*pyran-4-yl)-7-{[2-(trimethylsilyl)ethoxy]methyl}-7,9-dihydro-8*H-*purin-8-one **(Preparation 3)** and 4-fluoro-3-(4,4,5,5-tetramethyl-1,3,2-dioxaborolan-2-yl)pyrazolo[1,5-a]pyridine **(Preparation 105c)** following the experimental procedure as described in **Preparation 104.** The crude product was used without further purification.
LRMS (m/z): 485 (M+1)⁺.

### PREPARATION 107

### 3-(4,4,5,5-Tetramethyl-1,3,2-dioxaborolan-2-yl)pyrazolo[1,5-a]pyrimidine

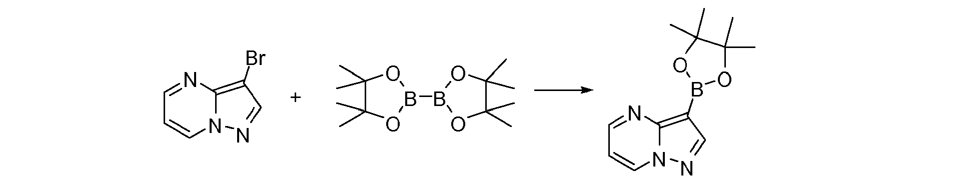

A mixture of 3-bromopyrazolo[1,5-a]pyrimidine (1.00 g, 5.1 mmol), potassium acetate (1.78 g, 18.1 mmol) and bis(pinacolato)diboron (5.77 g, 22.7 mmol) in 1,4-dioxane (20 mL) contained in a Schlenck vessel was submitted to three vacuum-argon cycles and bis(triphenylphosphine)palladium(II) dichloride (0.180 g, 0.26 mmol) was then added. The mixture was further submitted to three vacuum-argon cycles, sealed and then was stirred and heated to 100 °C. After 20 hours, the reaction mixture was cooled, evaporated and then taken up in pentane and filtered through diatomaceous earth (Celite®) and the filter cake was washed with a mixture of ethyl acetate/ether (3:2). The combined filtrate and washings were evaporated and the residue was stirred with n-pentane (15 mL) at -40 °C for 30 minutes. The solid was filtered, washed with cold pentane and dried in vacuo to give the title compound (1.66 g, >100%) as a solid which was used without further purification.
LRMS (m/z): 246 (M+1)⁺.
1H NMR (300 MHz, CDCl₃) δ ppm 1.26 (s, 12H), 6.89 (dd, 1H), 8.44 (s, 1H), 8.63 - 8.83 (m, 2H).

### PREPARATION 108

### 2-(Pyrazolo[1,5-a]pyrimidin-3-yl)-9-(tetrahydro-2H-pyran-4-yl)-7-((2-(trimethylsilyl)ethoxy)methyl)-7H-purin-8(9H)-one

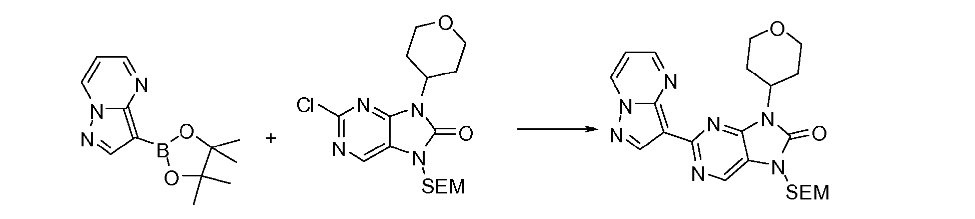

Obtained as a pale yellow solid (43%) from 2-chloro-9-(tetrahydro-2*H-*pyran-4-yl)-7-{[2-(trimethylsilyl)ethoxy]methyl}-7,9-dihydro-8*H-*purin-8-one **(Preparation 3)** and 3-(4,4,5,5-tetramethyl-1,3,2-dioxaborolan-2-yl)pyrazolo[1,5-a]pyrimidine **(Preparation 107)** following the experimental procedure as described in **Preparation 104.** The crude product was purified by flash chromatography (98:2 to 97:3 dichloromethane/methanol).
LRMS (m/z): 468 (M+1)⁺.
1H NMR (300 MHz, CDCl₃) δ ppm 0.16 (s, 9H), 0.96 (t, 2H), 1.89 (m, 2H), 2.93 (dq, 2H), 3.62 (m, 4H), 4.19 (dd, 2H), 4.88 (m, 1H), 5.36 (s, 2H), 7.02 (m, 2H), 8.58 (s, 2H), 8.74 - 8.86 (m, 2H), 8.95 (s, 1H).

### PREPARATION 109

### 6-(Pyrazolo[1,5-a]pyrimidin-3-yl)-4-(tetrahydro-2H-pyran-4-ylamino)nicotinic acid

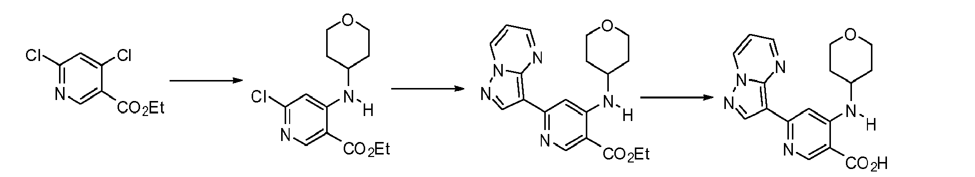

### a) Ethyl 6-chloro-4-(tetrahydro-2H-pyran-4-ylamino)nicotinate

N,N-diisopropylethylamine (3.48 mL, 20.0 mmol) was added to a cooled, stirred solution of ethyl 4,6-dichloronicotinate (EP1364950A1(2003), 2.00 g, 9.1 mmol) in acetonitrile (15 mL). Tetrahydro-2H-pyran-4-amine acetate (1.76 g, 10.9 mmol) was added portion wise and the mixture was stirred and heated to 70°C in a sealed tube. After 24 hours, the mixture was cooled and concentrated in vacuo and the residue was partitioned between ethyl acetate and water. The organic extract was dried (MgSO₄) and evaporated and the residue was purified by flash chromatography (5:1 to 3:1 hexanes/ethyl acetate) to give the title compound (2.01 g, 78%) as a white solid.
LRMS (m/z): 285 (M+1)⁺.
1H NMR (300 MHz, CDCl₃) δ ppm 1.40 (t, 3H), 1.57-1.70 (m, 2H), 2.01 (m, 2H), 3.53-3.66 (m, 3H), 4.01 (dt, 2H), 4.35 (q, 2H), 6.56 (s, 1H), 8.28 (br d, 1H), 8.69 (s, 1H).

### b) Ethyl 6-(pyrazolo[1,5-a]pyrimidin-3-yl)-4-(tetrahydro-2H-pyran-4-ylamino)nicotinate

Obtained as a pale yellow solid (19%) from ethyl 6-chloro-4-(tetrahydro-2H-pyran-4-ylamino)nicotinate **(Preparation 109a)** and 3-(4,4,5,5-tetramethyl-1,3,2-dioxaborolan-2-yl)pyrazolo[1,5-a]pyrimidine **(Preparation 107)** following the experimental procedure as described in **Preparation 104.** The crude product was purified by reverse phase chromatography (C-18 silica from Waters©, water/acetonitrile/methanol as eluents [0.1 % v/v formic acid buffered] 0% to 100%).
LRMS (m/z): 368 (M+1)⁺.
1H NMR (300 MHz, CDCl₃) δ ppm 1.43 (t, 3H), 1.72 (m, 2H), 2.16 (d, 2H), 3.65 (m, 2H), 3.89 (m, 1H), 4.07 (d, 2H), 4.38 (q, 2H), 6.99 (dd, 1H), 8.00 (s, 1H), 8.47 (d, 1H), 8.67 (m, 1H), 8.80 (d, 1H), 9.01 (s, 1H), 9.06 (s, 1H).

### c) 6-(Pyrazolo[1,5-a]pyrimidin-3-yl)-4-(tetrahydro-2H-pyran-4-ylamino)nicotinic acid

2M Aqueous sodium hydroxide solution (0.15 mL) was added to a suspension of ethyl 6-(pyrazolo[1,5-a]pyrimidin-3-yl)-4-(tetrahydro-2H-pyran-4-ylamino)nicotinate **(Preparation 109b,** 0.055 g, 0.15 mmol) in ethanol (1.5 mL) and the mixture was stirred and heated to 50 °C in a sealed vial. After 5h, the mixture was concentrated in vacuo, taken up in water and the pH was adjusted to ca. 6 with 2M aqueous hydrogen chloride solution. The mixture was evaporated to dryness to give the crude title compound which was used as such in a subsequent experiment.
LRMS (m/z): 340 (M+1)⁺.

### EXAMPLE 1

### 3-[8-Oxo-9-(tetrahydro-2H-pyran-4-yl)-8,9-dihydro-7H-purin-2-yl]imidazo[1,2-a]pyridine-6-carbonitrile

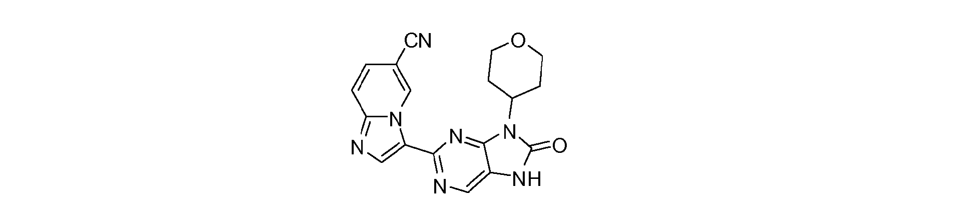

A suspension of 3-(8-oxo-9-(tetrahydro-2H-pyran-4-yl)-7-((2-(trimethylsilyl)ethoxy)methyl)-8,9-dihydro-7H-purin-2-yl)imidazo[1,2-a]pyridine-6-carbonitrile **(Preparation 4,** 1.00 g, 2.03 mmol) in 4N hydrochloric acid (25 mL) was stirred and heated to 70 °C in a sealed tube. After 1 hour, the mixture was cooled to room temperature and washed with ethyl acetate. The aqueous layer was neutralized by the addition of solid sodium hydrogen carbonate and the resulting precipitate was filtered, washed with water and dried in vacuo to yield the title compound (0.54 g, 78%) as a white solid.
LRMS (m/z): 362 (M+1)⁺.
1H NMR (300 MHz, CDCl₃) δ ppm 1.85 (d, 2H), 2.87 (dd, 2H), 3.50 - 3.72 (m, 2H), 4.22 (dd, 2H), 4.54 - 4.80 (m, 1H), 7.44 (dd, 1H), 7.83 (d, 1H), 8.37 (s, 1H), 8.68 (s, 1H), 10.52 (s, 1H).

### EXAMPLE 2

### 3-(9-Cyclohexyl-8-oxo-8,9-dihydro-7H-purin-2-yl)imidazo[1,2-a]pyridine-6-carbonitrile

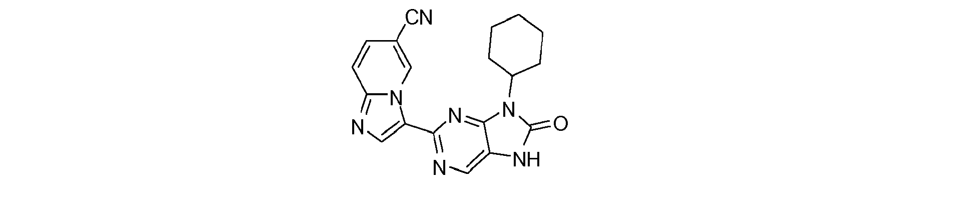

Obtained (66%) from 3-(9-cyclohexyl-8-oxo-7-((2-(trimethylsilyl)ethoxy)methyl)-8,9-dihydro-7H-purin-2-yl)imidazo[1,2-a]pyridine-6-carbonitrile **(Preparation 6)** following the experimental procedure as described in **Example 1.**
LRMS (m/z): 360 (M+1)⁺.
1H NMR (300 MHz, DMSO-*d₆*) δ ppm 1.35 - 1.52 (m, 3H), 1.70 - 1.96 (m, 5H), 2.27 - 2.43 (m, 2H), 4.26 (m, 1H), 7.72 (m, 1H), 7.92 (dd, 1H), 8.41 (s, 1H), 8.53 (s, 1H), 10.38 (d, 1H).

### EXAMPLE 3

### 3-[3-(2-Methylcyclohexyl)-2-oxo-2,3-dihydro-1H-imidazo[4,5-b]pyridin-5-yl]imidazo[1,2-a]pyridine-6-carbonitrile

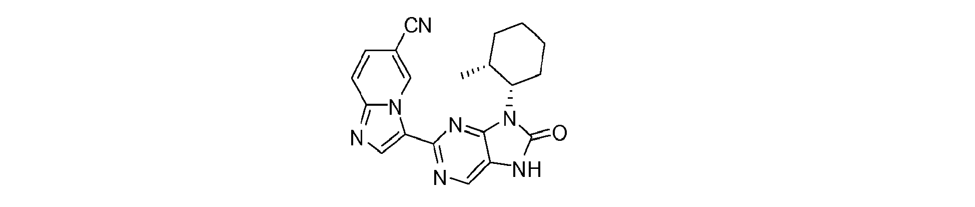

Obtained as a white solid (86%) from 3-(9-((1S,2R)-2-methylcyclohexyl)-8-oxo-7-''2-(trimethylsilyl)ethoxy)methyl)-8,9-dihydro-7H-purin-2-yl)imidazo[1,2-a]pyridine-6-carbonitrile **(Preparation 8)** following the experimental procedure as described in **Example 1.**
LRMS (m/z): 374 (M+1)⁺.
1H NMR (300 MHz, DMSO-*d₆*) δ ppm 0.99 (d, 3H), 1.30 - 2.03 (m, 7H), 2.27 (br s, 1H), 2.89 - 3.11 (m, 1H), 4.12 - 4.49 (m, 1H), 7.81 (d, 1H), 7.97 (d, 1H), 8.40 (s, 1H), 8.60 (s, 1H), 10.45 (s, 1H).

### EXAMPLE 4

### 3-{9-[(4R)-8-Fluoro-3,4-dihydro-2H-chromen-4-yl]-8-oxo-8,9-dihydro-7H-purin-2-yl}imidazo[1,2-a]pyridine-6-carbonitrile

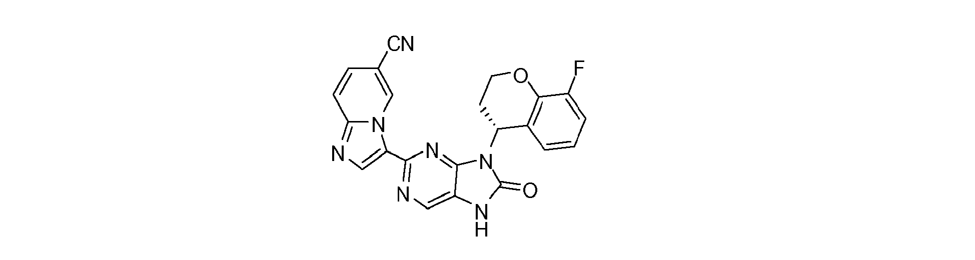

Obtained (41%) from (R)-3-(9-(8-fluorochroman-4-yl)-8-oxo-7-((2-(trimethylsilyl)ethoxy)methyl)-8,9-dihydro-7H-purin-2-yl)imidazo[1,2-a]pyridine-6-carbonitrile **(Preparation 10)** following the experimental procedure as described in **Example 1.**
LRMS (m/z): 428 (M+1)⁺.
1H NMR (300 MHz, DMSO-*d₆*) δ ppm 2.34 (dt, 1H), 2.70 - 2.92 (m, 1H), 4.44 (t, 1H), 4.54 - 4.67 (m, 1H), 5.83 (t, 1H), 6.65 - 6.81 (m, 2H), 7.04 - 7.18 (m, 1H), 7.67 (dd, 1H), 7.88 (d, 1H), 8.20 (s, 1H), 8.44 (s, 1H), 10.26 (s, 1H).

### EXAMPLE 5

### 3-[7-Methyl-8-oxo-9-(tetrahydro-2H-pyran-4-yl)-8,9-dihydro-7H-purin-2-yl]imidazo[1,2-a]pyridine-6-carbonitrile

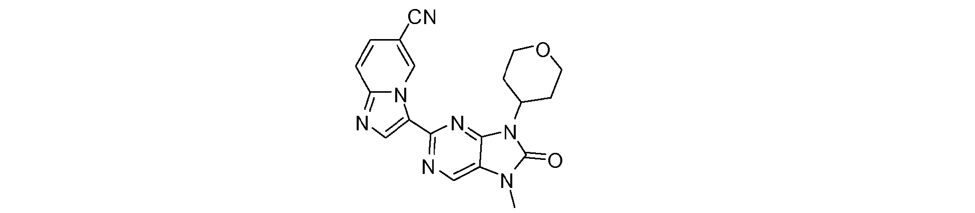

Obtained as a pale yellow solid (20%) from 2-chloro-7-methyl-9-(tetrahydro-2*H-*pyran-4-yl)-7,9-dihydro-8*H-*purin-8-one **(Preparation 11)** and imidazo[1,2*-a*]pyridine-6-carbonitrile **(Preparation 1)** following the experimental procedure as described in **Preparation 4.**
LRMS (m/z): 376 (M+1)⁺.
1H NMR (300 MHz, CDCl₃) δ ppm 1.81 (d, 2H), 2.85 (dd, 2H), 3.52 (s, 3H), 3.60 (t, 2H), 4.20 (dd, 2H), 4.56 - 4.76 (m, 1H), 7.44 (d, 1H), 7.83 (d, 1H), 8.27 (s, 1H), 8.69 (br s, 1H), 10.54 (br s, 1H).

### EXAMPLE 6

### 3-(9-Benzyl-7-methyl-8-oxo-8,9-dihydro-7H-purin-2-yl)imidazo[1,2-a]pyridine-6-carbonitrile

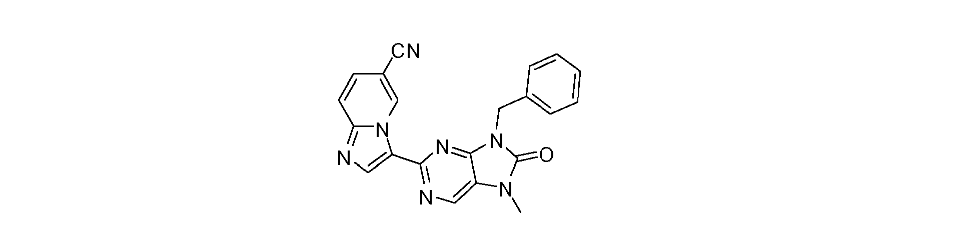

Obtained as a white solid (50%) from 9-benzyl-2-chloro-7-methyl-7,9-dihydro-8*H-*purin-8-one **(Preparation 12)** and imidazo[1,2*-a*]pyridine-6-carbonitrile **(Preparation 1)** following the experimental procedure as described in **Preparation 4.**
LRMS (m/z): 382 (M+1)⁺.
1H NMR (300 MHz, DMSO-d₆) δ ppm 3.46 (s, 3H), 5.17 (s, 2H), 7.23 - 7.42 (m, 3H), 7.47 - 7.55 (m, 2H), 7.67 - 7.77 (m, 1H), 7.92 (d, 1H), 8.55 (s, 1H), 8.62 (s, 1H), 10.36 (s, 1H).

### EXAMPLE 7

### 3-[7-(2-Morpholin-4-ylethyl)-8-oxo-9-(tetrahydro-2H-pyran-4-yl)-8,9-dihydro-7H-purin-2-yl]imidazo[1,2-a]pyridine-6-carbonitrile

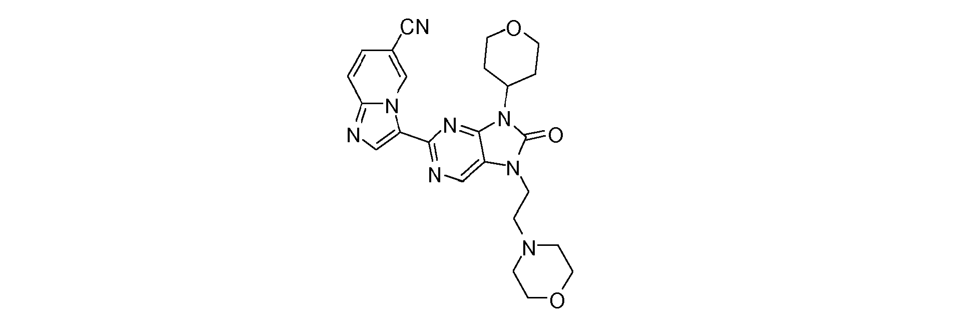

Obtained as a white solid (11 %) from 2-chloro-7-(2-morpholin-4-ylethyl)-9-(tetrahydro-2*H-*pyran-4-yl)-7,9-dihydro-8*H-*purin-8-one **(Preparation 13)** and imidazo[1,2-a]pyridine-6-carbonitrile **(Preparation 1)** following the experimental procedure as described in **Preparation 4.**
LRMS (m/z): 475 (M+1)⁺.
1H NMR (300 MHz, CDCl₃) δ ppm 1.77 - 1.90 (m, 2H), 2.55 (d, 4H), 2.69 - 2.98 (m, 4H), 3.53 - 3.74 (m, 6H), 4.02 - 4.13 (m, 2H), 4.16 - 4.27 (m, 2H), 4.55 - 4.76 (m, 1H), 7.39 - 7.50 (m, 1H), 7.83 (d, 1H), 8.33 (s, 1H), 8.66 (s, 1H), 10.53 (s, 1H).

### EXAMPLE 8

### 3-[7-[2-(Dimethylamino)ethyl]-8-oxo-9-(tetrahydro-2H-pyran-4-yl)-8,9-dihydro-7H-purin-2-yl]imidazo[1,2-a]pyridine-6-carbonitrile

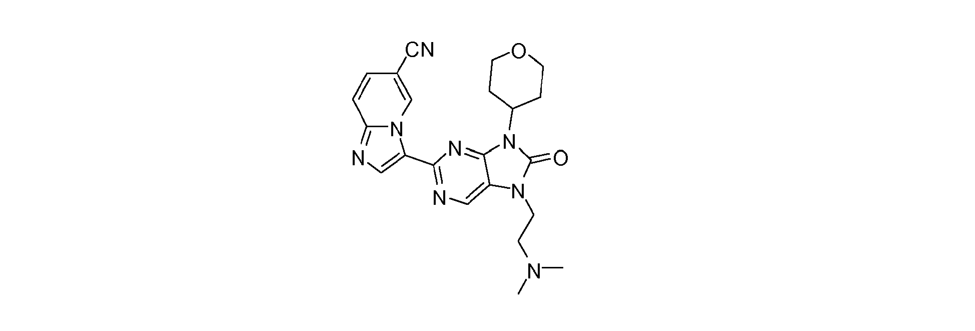

Obtained as a pale yellow solid (41%) from 2-chloro-7-[2-(dimethylamino)ethyl]-9-(tetrahydro-2*H-*pyran-4-yl)-7,9-dihydro-8*H-*purin-8-one **(Preparation 14)** and imidazo[1,2*-a*]pyridine-6-carbonitrile **(Preparation 1)** following the experimental procedure as described in **Preparation 4.**
LRMS (m/z): 433 (M+1)⁺.
1H NMR (300 MHz, DMSO-d₆) δ ppm 1.80 (d, 2H), 2.26 (s, 6H), 2.55 - 2.75 (m, 4H), 3.40-3.60 (m, 4H), 4.03 (m, 2H), 4.58 (m, 1H), 7.71 (d, 1H), 7.93 (d, 1H), 8.53 (s, 1H), 8.68 (s, 1H), 10.40 (s, 1H).

### EXAMPLE 9

### 3-[7-(2-Hydroxyethyl)-8-oxo-9-(tetrahydro-2H-pyran-4-yl)-8,9-dihydro-7H-purin-2-yl]imidazo[1,2-a]pyridine-6-carbonitrile

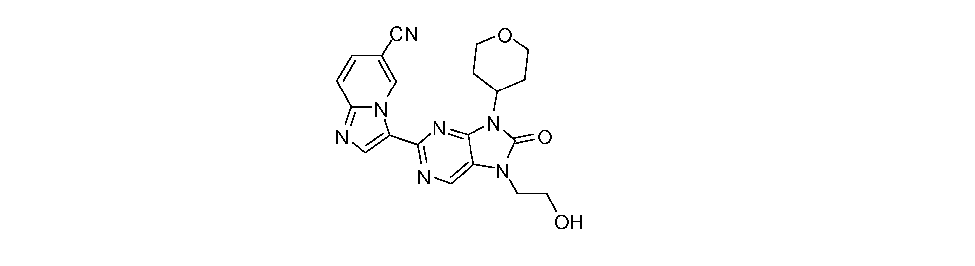

Obtained from 2-chloro-9-(tetrahydro-2*H-*pyran-4-yl)-7-[2-(tetrahydro-2*H-*pyran-2-yloxy)ethyl]-7,9-dihydro-8*H-*purin-8-one **(Preparation 15,** 0.087 g, 0.23 mmol) and imidazo[1,2*-a*]pyridine-6-carbonitrile **(Preparation 1,** 0.066 g, 0.46 mmol) following the experimental procedure as described in **Preparation 4.** The crude product was taken up in dioxane (2 mL) and 2M aqueous hydrogen chloride solution (2 mL) was added and the mixture was stirred overnight. The mixture was evaporated and taken up in 2M aqueous hydrogen chloride solution (5 mL) and a small amount of an insoluble black solid was removed by filtration. The filtrate was neutralized with solid sodium hydrogen carbonate to give a precipitate which was filtered, washed with water and dried in vacuo to give the title compound (0.071 g, 77%) as a white solid.
LRMS (m/z): 406 (M+1)⁺.
1H NMR (300 MHz, DMSO-*d₆*) δ ppm 1.66 - 1.87 (m, 2H), 2.54 - 2.70 (m, 2H), 3.50 (t, 2H), 3.66 - 3.78 (m, 2H), 3.90 - 4.14 (m, 4H), 4.51 - 4.69 (m, 1H), 5.01 (br s, 1H), 7.69 (d, 1H), 7.91 (d, 1H), 8.51 (s, 1H), 8.61 (s, 1H), 10.38 (s, 1H).

### EXAMPLE 10

### 3-[7-(2-Hydroxy-2-methylpropyl)-8-oxo-9-(tetrahydro-2H-pyran-4-yl)-8,9-dihydro-7H-purin-2-yl]imidazo[1,2-a]pyridine-6-carbonitrile

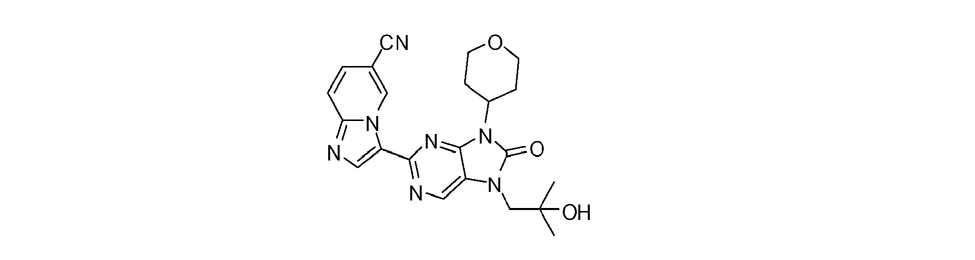

Obtained (38%) from 2-chloro-7-(2-hydroxy-2-methylpropyl)-9-(tetrahydro-2*H-*pyran-4-yl)-7,9-dihydro-8*H-*purin-8-one **(Preparation 16)** and imidazo[1,2*-a*]pyridine-6-carbonitrile **(Preparation 1)** following the experimental procedure as described in **Preparation 4.**
LRMS (m/z): 434 (M+1)⁺.
1H NMR (300 MHz, DMSO-*d₆*) δ ppm 1.17 (s, 6H), 1.80 (d, 2H), 2.53 - 2.73 (m, 2H), 3.50 (t, 2H), 3.79 (s, 2H), 4.04 (d, 2H), 4.43 - 4.70 (m, 1H), 7.67 - 7.77 (m, 1H), 7.88 - 7.99 (m, 1H), 8.53 (s, 1H), 8.61 (s, 1H), 10.40 (s, 1H).

### EXAMPLE 11

### 3-[7-[(2R)-2,3-Dihydroxypropyl]-8-oxo-9-(tetrahydro-2H-pyran-4-yl)-8,9-dihydro-7H-purin-2-yl]imidazo[1,2-a]pyridine-6-carbonitrile

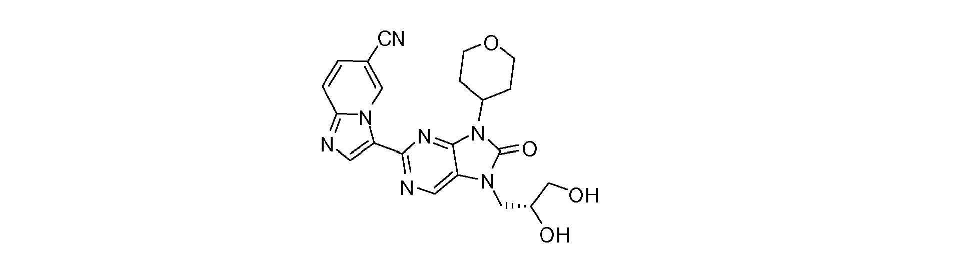

Obtained as a solid (47%) from 2-chloro-7-{[(4R)-2,2-dimethyl-1,3-dioxolan-4-yl]methyl}-9-(tetrahydro-2H-pyran-4-yl)-7,9-dihydro-8H-purin-8-one **(Preparation 17)** and imidazo[1,2*-a*]pyridine-6-carbonitrile **(Preparation 1)** following the experimental procedure as described in **Example 9.**
LRMS (m/z): 436 (M+1)⁺.
1H NMR (300 MHz, DMSO-*d₆*) δ ppm 1.60 - 1.86 (m, 2H), 2.61 (qd, 2H), 3.39 - 3.58 (m, 4H), 3.74 - 3.87 (m, 2H), 3.91 - 4.12 (m, 3H), 4.48 - 4.67 (m, 1H), 4.81 (t, 1H), 5.12 (d, 1H), 7.70 (dd, 1H), 7.93 (d, 1H), 8.52 (s, 1H), 8.59 (s, 1H), 10.39 (d, 1H).

### EXAMPLE 12

### 3-[7-[(2S)-2,3-Dihydroxypropyl]-8-oxo-9-(tetrahydro-2H-pyran-4-yl)-8,9-dihydro-7H-purin-2-yl]imidazo[1,2-a]pyridine-6-carbonitrile

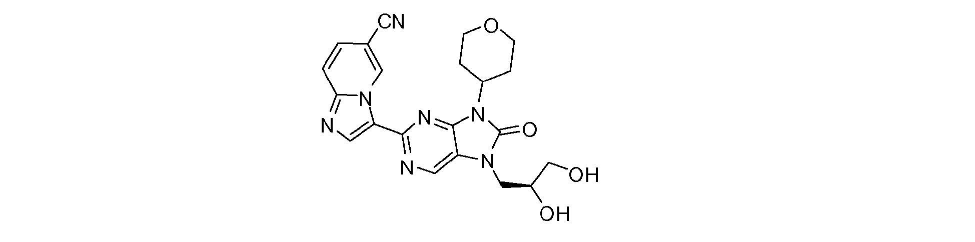

Obtained as a solid (43%) from 2-chloro-7-{[(4S)-2,2-dimethyl-1,3-dioxolan-4-yl]methyl}-9-(tetrahydro-2H-pyran-4-yl)-7,9-dihydro-8H-purin-8-one **(Preparation 18)** and imidazo[1,2*-a*]pyridine-6-carbonitrile **(Preparation 1)** following the experimental procedure as described in **Example 9.**
LRMS (m/z): 436 (M+1)⁺.
1H NMR (300 MHz, DMSO-*d₆*) δ ppm 1.79 (d, 2H), 2.60 (dd, 2H), 3.37 - 3.57 (m, 2H), 3.74 - 3.89 (m, 2H), 3.95 - 4.13 (m, 3H), 4.58 (td, 1H), 4.81 (t, 2H), 5.12 (d, 2H), 7.70 (dd, 1H), 7.92 (d, 1H), 8.52 (s, 1H), 8.59 (s, 1H), 10.39 (s, 1H).

### EXAMPLE 13

### 3-[7-[(2S)-3-(Dimethylamino)-2-hydroxypropyl]-8-oxo-9-(tetrahydro-2H-pyran-4-yl)-8,9-dihydro-7H-purin-2-yl]imidazo[1,2-a]pyridine-6-carbonitrile

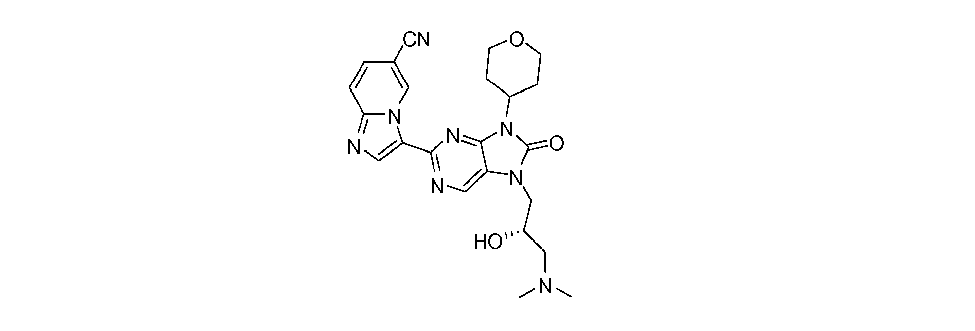

### a) 3-[7-[(2R)-Oxiran-2-ylmethyl]-8-oxo-9-(tetrahydro-2H-pyran-4-yl)-8,9-dihydro-7H-purin-2-yl]imidazo[1,2-a]pyridine-6-carbonitrile

Sodium hydride (60% dispersion in mineral oil, 0.008 g, 0.20 mmol) was added to a stirred suspension of 3-[8-oxo-9-(tetrahydro-2*H-*pyran-4-yl)-8,9-dihydro-7*H-*purin-2-yl]imidazo[1,2*-a*]pyridine-6-carbonitrile **(Example 1,** 0.060 g, 0.17 mmol) in N,N'-dimethylformamide (1.5 mL) at 0 °C. The mixture was stirred at 0 °C for 30 minutes and then (S)-2-(chloromethyl)oxirane (0.030 mL, 0.38 mmol) was added and the mixture was stirred and heated to 60 °C. After 20 hours, the mixture was cooled to room temperature and partitioned between ethyl acetate and water and the organic layer was washed with water, dried (MgSO₄) and evaporated to give the title compound (0.050 g, 72%) as a beige solid.
LRMS (m/z): 418 (M+1)⁺.
1H NMR (300 MHz, CDCl₃) δ ppm 1.83 (m, 2H), 2.73-2.98 (m, 4H), 3.30 (m, 1H), 3.62 (m, 4H), 4.22 (m, 2H), 4.66 (m, 1H), 7.46 (d, 1H), 7.82 (d, 1H), 8.52 (s, 1H), 8.68 (s, 1H), 10.55 (s, 1H).

### b) 3-[7-[(2S)-3-(Dimethylamino)-2-hydroxypropyl]-8-oxo-9-(tetrahydro-2H-pyran-4-yl)-8,9-dihydro-7H-purin-2-yl]imidazo[1,2-a]pyridine-6-carbonitrile

2M Dimethylamine in methanol (0.42 mL, 0.84 mmol) was added to a suspension of 3-[7-[(2*R*)-oxiran-2-ylmethyl]-8-oxo-9-(tetrahydro-2*H-*pyran-4-yl)-8,9-dihydro-7*H-*purin-2-yl]imidazo[1,2*-a*]pyridine-6-carbonitrile **(Example 13a,** 0.045 g, 0.11 mmol) in ethanol (1.0 mL) and the mixture was stirred and heated to 40 °C. After 4 hours, the mixture was concentrated and the residue was purified by flash chromatography (100:8:1 dichloromethane/methanol/c. NH₄OH) to give the title compound (0.024 g, 44%) as a white solid.
LRMS (m/z): 463 (M+1)⁺.
1H NMR (300 MHz, CDCl₃) δ ppm 1.83 (d, 2H), 2.32 (s, 6H), 2.37 - 2.45 (m, 2H), 2.75 - 2.97 (m, 2H), 3.60 (t, 2H), 3.80 (dd, 1H), 3.98 - 4.29 (m, 4H), 4.58 - 4.76 (m, 1H), 7.42 (d, 1H), 7.82 (d, 1H), 8.58 (s, 1H), 8.65 (s, 1H), 10.55 (s, 1H).

### EXAMPLE 14

### 3-[7-(2-Methoxyethyl)-8-oxo-9-(tetrahydro-2H-pyran-4-yl)-8,9-dihydro-7H-purin-2-yl]imidazo[1,2-a]pyridine-6-carbonitrile

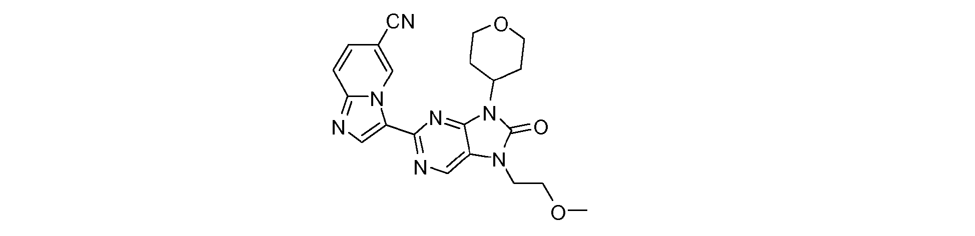

Obtained as a white solid (60%) from 3-[8-oxo-9-(tetrahydro-2*H-*pyran-4-yl)-8,9-dihydro-7*H-*purin-2-yl]imidazo[1,2*-a*]pyridine-6-carbonitrile **(Example 1)** and 1-bromo-2-methoxyethane following the experimental procedure as described in **Preparation 13** with the exception that the reaction was performed at ambient temperature.
LRMS (m/z): 420 (M+1)⁺.
1H NMR (300 MHz, CDCl₃) δ ppm 1.83 (dd, 1H), 2.86 (qd, 2H), 2.90 - 2.91 (m, 1H), 3.41 (s, 3H), 3.60 (t, 2H), 3.73 (t, 2H), 4.13 (t, 2H), 4.21 (dd, 2H), 4.58 - 4.77 (m, 1H), 7.43 (dd, 1H), 7.82 (d, 1H), 8.45 (s, 1H), 8.65 (s, 1H), 10.56 (s, 1H).

### EXAMPLE 15

### 3-[7-(2-Aminoethyl)-8-oxo-9-(tetrahydro-2H-pyran-4-yl)-8,9-dihydro-7H-purin-2-yl]imidazo[1,2-a]pyridine-6-carbonitrile

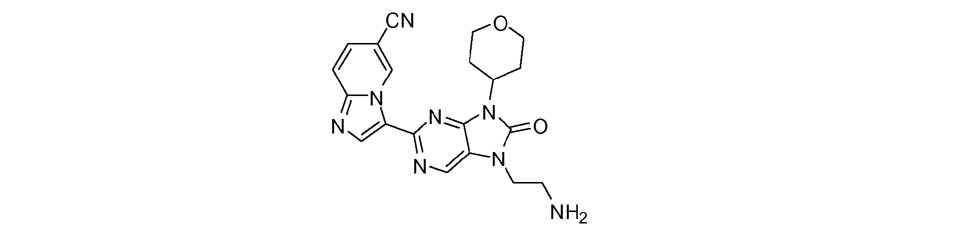

### a) tert-Butyl {2-[2-(6-cyanoimidazo[1,2-a]pyridin-3-yl)-8-oxo-9-(tetrahydro-2H-pyran-4-yl)-8,9-dihydro-7H-purin-7-yl]ethyl}carbamate

Potassium carbonate (0.034 g, 0.25 mmol) and *tert*-butyl 2-bromoethylcarbamate (0.041 g, 0.18 mmol) were added to a solution of 3-[8-oxo-9-(tetrahydro-2*H-*pyran-4-yl)-8,9-dihydro-7*H-*purin-2-yl]imidazo[1,2*-a*]pyridine-6-carbonitrile **(Example 1,** 0.045 g, 0.12 mmol) in N,N'-dimethylformamide (1.0 mL) and the mixture was stirred at room temperature overnight. The mixture was partitioned between water and ethyl acetate and the organic layer was washed with water, dried (MgSO₄) and evaporated to give the title compound (0.063 g, 100%) as a pale yellow solid.
LRMS (m/z): 505 (M+1)⁺.
1H NMR (300 MHz, CDCl₃) δ ppm 1.40 (s, 9H), 1.82 (m, 2H), 2.84 (m, 4H), 3.56 (m, 4H), 4.15 (m, 2H), 4.86 (m, 1H), 7.46 (d, 1H), 7.86 (d, 1H), 8.05 (s, 1H), 8.40 (s, 1H), 8.67 (s, 1H), 10.53 (s, 1H).

### b) 3-[7-(2-Aminoethyl)-8-oxo-9-(tetrahydro-2H-pyran-4-yl)-8,9-dihydro-7H-purin-2-yl]imidazo[1,2-a]pyridine-6-carbonitrile

Trifluoroacetic acid (0.045 mL, 0.58 mmol) was added to a solution of *tert*-butyl {2-[2-(6-cyanoimidazo[1,2*-a*]pyridin-3-yl)-8-oxo-9-(tetrahydro-2*H-*pyran-4-yl)-8,9-dihydro-7*H-*purin-7-yl]ethyl}carbamate **(Example 15a,** 0.060 g, 0.12 mmol) in dichloromethane (2 mL) and the mixture was stirred at room temperature overnight. The mixture was then concentrated and the residue was taken up in water and neutralized with solid sodium hydrogencarbonate. The aqueous mixture was extracted with chloroform and the combined organic extract was washed with water, dried (MgSO₄) and evaporated. The residue was purified by flash chromatography (100:8:1 dichloromethane/ethanol/c. NH₄OH) to give the title compound (0.013 g, 27%) as a yellow solid.
LRMS (m/z): 405 (M+1)⁺.
1H NMR (300 MHz, CDCl₃) δ ppm 1.83 (d, 2H), 2.86 (qd, 2H), 3.17 (t, 2H), 3.50 (s, 2H), 3.60 (t, 2H), 4.01 (t, 2H), 4.21 (dd, 2H), 4.59 - 4.77 (m, 1H), 7.43 (dd, 1H), 7.82 (d, 1H), 8.39 (s, 1H), 8.66 (s, 1H), 10.53 (s, 1H).

### EXAMPLE 16

### 3-(8-Oxo-9-(tetrahydro-2H-pyran-4-yl)-7-{2-[(2,2,2-trifluoroethyl)amino]ethyl}-8,9-dihydro-7H-purin-2-yl)imidazo[1,2-a]pyridine-6-carbonitrile

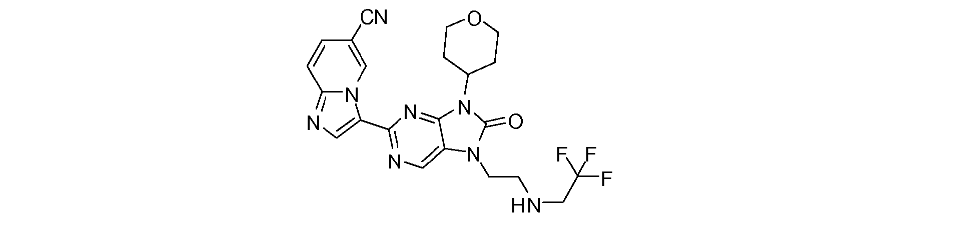

Obtained as a white solid (35%) from 2-chloro-9-(tetrahydro-2*H-*pyran-4-yl)-7-{2-[(2,2,2-trifluoroethyl)amino]ethyl}-7,9-dihydro-8*H-*purin-8-one **(Preparation 19b)** and imidazo[1,2*-a*]pyridine-6-carbonitrile **(Preparation 1)** following the experimental procedure as described in **Preparation 4.**
LRMS (m/z): 487 (M+1)⁺.
1H NMR (300 MHz, DMSO-*d₆*) δ ppm 1.78 (d, 2H), 2.54 - 2.76 (m, 2H), 2.98 (d, 2H), 3.19 - 3.31 (m, 2H), 3.50 (t, 2H), 3.87 - 4.12 (m, 4H), 4.50 - 4.70 (m, 1H), 7.71 (d, 1H), 7.93 (d, 1H), 8.53 (s, 1H), 8.66 (s, 1H), 10.40 (s, 1H).

### EXAMPLE 17

### 2-(2-(6-Cyanoimidazo[1,2-a]pyridin-3-yl)-8-oxo-9-(tetrahydro-2H-pyran-4-yl)-8,9-dihydro-7H-purin-7-yl)acetic acid

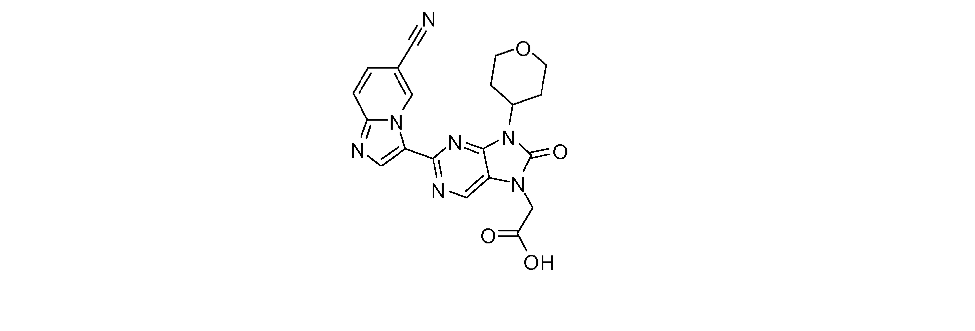

Potassium carbonate (0.038 g, 0.27 mmol) and methyl 2-bromoacetate (0.031 g, 0.20 mmol) were added to a stirred solution of 3-(8-oxo-9-(tetrahydro-2H-pyran-4-yl)-8,9-dihydro-7H-purin-2-yl)imidazo[1,2-a]pyridine-6-carbonitrile **(Example 1,** 0.050 g, 0.14 mmol) in N,N-dimethylformamide (4.0 mL). After 5 hours, aqueous sodium hydroxide solution (8M, 0.107 mL) was added and stirring was continued for a further 60 minutes. The mixture was taken to ca. pH 4-5 with 2M aqueous hydrogen chloride solution and extracted with chloroform. The organic layer was dried (MgSO₄), evaporated and the residue was purified by flash chromatography (10% methanol in dichloromethane) to give the title compound (0.015 g, 26%) as a white solid.
LRMS (m/z): 420 (M+1)⁺.
1H NMR (300 MHz, DMSO-*d₆*) δ ppm 1.81 (m, 2H), 2.60 (dq, 2H), 3.51 (t, 2H), 4.04 (dd, 2H), 4.62 (m, 3H), 7.70 (m, 1H), 7.92 (d, 1H), 8.53 (s, 1H), 8.60 (s, 1H), 10.37 (m, 1H).

### EXAMPLE 18

### 3-[7-(2,4-Dimethoxybenzyl)-8-oxo-9-(tetrahydro-2H-pyran-4-yl)-8,9-dihydro-7H-purin-2-yl]imidazo[1,2-a]pyridine-6-carbonitrile

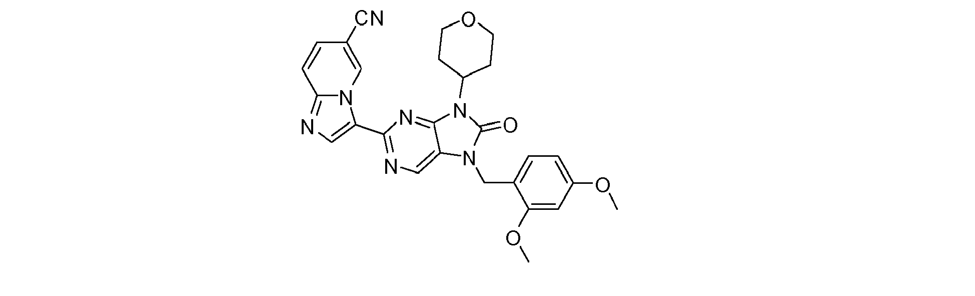

Obtained as a beige solid (60%) from 2-chloro-7-(2,4-dimethoxybenzyl)-9-(tetrahydro-2*H-*pyran-4-yl)-7,9-dihydro-8*H-*purin-8-one **(Preparation 20b)** and imidazo[1,2-a]pyridine-6-carbonitrile **(Preparation 1)** following the experimental procedure as described in **Preparation 4.**
LRMS (m/z): 512 (M+1)⁺.
1H NMR (300 MHz, CDCl₃) δ ppm 1.81 (dd, 2H), 2.85 (dd, 2H), 3.52 - 3.67 (m, 2H), 3.82 (s, 3H), 3.92 (s, 3H), 4.20 (dd, 2H), 4.67 (tt, 1 H), 5.05 (s, 2H), 6.47 - 6.57 (m, 2H), 7.33 - 7.47 (m, 2H), 7.80 (dd, 1H), 8.24 (s, 1H), 8.62 (s, 1H), 10.52 (s, 1H).

### EXAMPLE 19

### 2-(6-Fluoroimidazo[1,2-a]pyridin-3-yl)-9-(tetrahydro-2H-pyran-4-yl)-7,9-dihydro-8H-purin-8-one

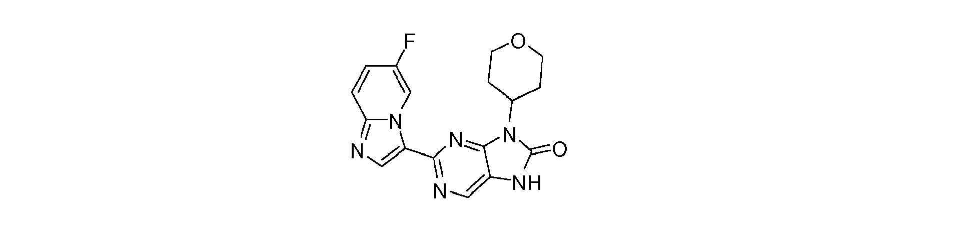

Tetrabutylammonium fluoride (1 M in tetrahydrofuran, 1.5 mL, 1.5 mmol) was added to a solution of 2-(6-fluoroimidazo[1,2-a]pyridin-3-yl)-9-(tetrahydro-2H-pyran-4-yl)-7-((2-(trimethylsilyl)ethoxy)methyl)-7H-purin-8(9H)-one **(Preparation 22,** 0.18 g, 0.37 mmol) in tetrahydrofuran (4 mL) and the mixture was stirred and heated to 80 °C in a sealed tube. After 24 hours, the mixture was concentrated and the mixture was partitioned between dichloromethane and water and the organic layer was dried (MgSO₄) and evaporated to yield the title compound (0.100 g, 76%) as a white solid. The solid was dissolved in methanol and treated with a solution of hydrogen chloride in 1,4-dioxane (4M, 0.5 mL) and concentrated to give the hydrochloride salt of the title compound (0.105 g) as a white solid.
LRMS (m/z): 355 (M+1)⁺.
1H NMR (300 MHz, DMSO-d₆) δ ppm 1.79 (m, 2H), 2.65 (m, 2H), 3.53 (m, 2H), 4.07 (m, 2H), 4.55 (m, 1H), 7.57 (m, 1H), 7.87 (m, 1H), 8.34 (s, 1H), 8.44 (s, 1H), 10.01 (m, 1H).

### EXAMPLE 20

### 3-[8-Oxo-9-(tetrahydro-2H-pyran-4-yl)-8,9-dihydro-7H-purin-2-yl]imidazo[1,2-a]pyridine-6-carbonitrile

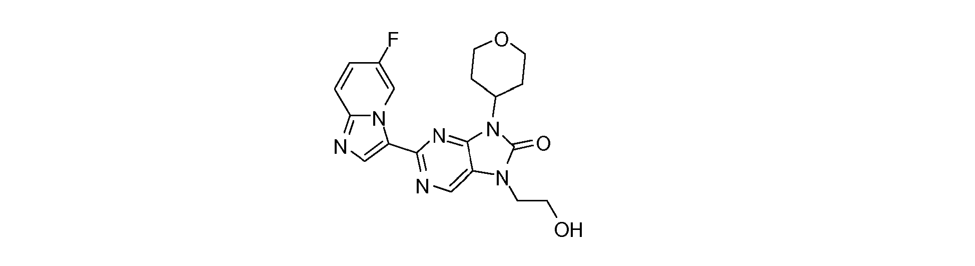

Obtained as a solid (46%) from 2-chloro-9-(tetrahydro-2*H-*pyran-4-yl)-7-[2-(tetrahydro-2*H-*pyran-2-yloxy)ethyl]-7,9-dihydro-8*H-*purin-8-one **(Preparation 15)** and 6-fluoroimidazo[1,2*-a*]pyridine **(Preparation 21)** following the experimental procedure as described in **Example 9.**
LRMS (m/z): 399 (M+1)⁺.
1H NMR (300 MHz, DMSO-*d₆*) δ ppm 1.78 (dd, 2H), 2.61 (dd, 2H), 3.51 (t, 2H), 3.71 (t, 2H), 4.04 (dd, 4H), 4.59 (td, 1H), 7.90 - 8.00 (m, 1H), 8.02 - 8.11 (m, 1H), 8.66 (s, 1H), 8.80 (s, 1H), 10.11 (br s, 1H).

### EXAMPLE 21

### 9-Cyclohexyl-2-(6-fluoroimidazo[1,2-a]pyridin-3-yl)-7H-purin-8(9H)-one

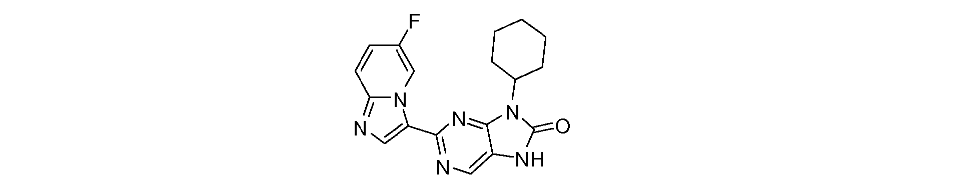

Obtained as a white solid (60%) from 9-cyclohexyl-2-(6-fluoroimidazo[1,2-a]pyridin-3-yl)-7-((2-(trimethylsilyl)ethoxy)methyl)-7H-purin-8(9H)-one **(Preparation 23)** following the experimental procedure as described in **Example 19** followed by purification of the crude product by flash chromatography (97:3 dichloromethane/methanol)
LRMS (m/z): 353 (M+1)⁺.
1H NMR (300 MHz, DMSO-d₆) δ ppm 1.05 - 1.57 (m, 4H), 1.62 - 2.05 (m, 4H), 2.19 - 2.52 (m, 2H), 4.28 (t, 1H), 7.54 (t, 1H), 7.84 (dd, 1H), 8.22 - 8.58 (m, 2H), 9.93 (d, 1H).

### EXAMPLE 22

### 9-[(4R)-8-Fluoro-3,4-dihydro-2H-chromen-4-yl]-2-(6-fluoroimidazo[1,2-a]pyridin-3-yl)-7,9-dihydro-8H-purin-8-one

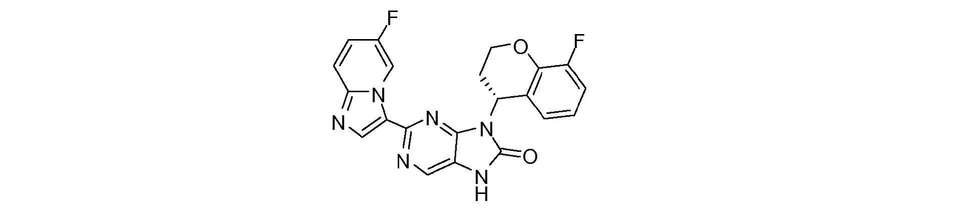

Obtained as a white solid (32%) from (R)-9-(8-fluorochroman-4-yl)-2-(6-fluoroimidazo[1,2-a]pyridin-3-yl)-7-((2-(trimethylsilyl)ethoxy)methyl)-7H-purin-8(9H)-one **(Preparation 24)** following the experimental procedure as described in **Example 19.** The crude product was washed exhaustively with water and then a small amount of methanol to give the pure title compound.
LRMS (m/z): 421 (M+1)⁺.
1H NMR (300 MHz, DMSO-*d₆*) δ ppm 2.18 - 2.38 (m, 1H), 2.68 - 2.88 (m, 1H), 4.46 (t, 1H), 4.57 (t, 1H), 5.85 (dd, 1H), 6.64 - 6.86 (m, 2H), 7.01 - 7.19 (m, 1H), 7.49 (m, 1H), 7.78 (dd, 1H), 8.23 (s, 1H), 8.43 (s, 1H), 9.31 (d, 1H), 11.71 (s, 1H).

### EXAMPLE 23

### 2-(6-Fluoroimidazo[1,2-a]pyridin-3-yl)-9-[(1 S)-1-phenylethy)]-7,9-dihydro-8H-purin-8-one

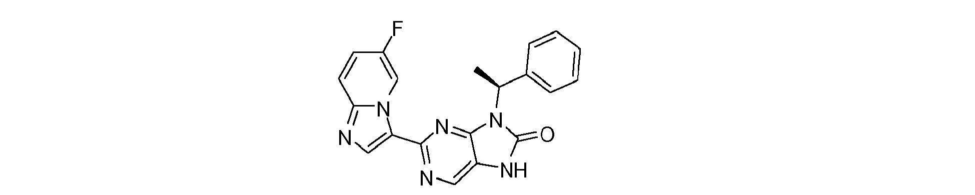

Obtained as a pale pink solid (53%) from 2-(6-fluoroimidazo[1,2*-a*]pyridin-3-yl)-N-[(1*R*)-1-pyridin-3-ylethyl]pyrimidine-4,5-diamine **(Preparation 26b)** and 1,1'-carbonylbis-1*H-*imidazole following the experimental procedure as described in **Preparation 42d.**
LRMS (m/z): 375 (M+1)⁺.
1H NMR (300 MHz, DMSO-*d₆*) δ ppm 2.01 (d, 3H), 5.75 (q, 1H), 7.36 (t, 1H), 7.51 (d, 2H), 7.47 - 7.57 (m, 3H), 7.76 - 7.91 (m, 1H), 8.39 (d, 2H), 9.65 - 9.80 (m, 1H), 11.60 (br s, 1H).

### EXAMPLE 24

### 2-(6-Fluoroimidazo[1,2-a]pyridin-3-yl)-9-[(1R)-1-phenylethyl]-7,9-dihydro-8H-purin-8-one

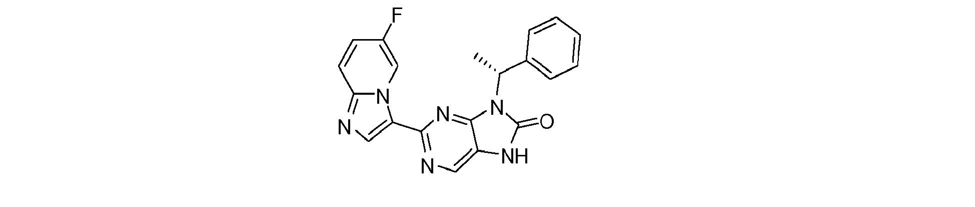

Obtained as a white solid (30%) from 2-(6-fluoroimidazo[1,2*-a*]pyridin-3-yl)-N⁺-[(1*R*)-1-phenylethyl]pyrimidine-4,5-diamine **(Preparation 27b)** and 1,1'-carbonylbis-1*H-*imidazole following the experimental procedure as described in **Preparation 42d** followed by purification of the crude product by flash chromatography (97:3 dichloromethane/methanol).
LRMS (m/z): 375 (M+1)⁺.
1H NMR (300 MHz, DMSO-*d₆*) δ ppm 2.01 (d, 3H), 5.75 (q, 1H), 7.36 (t, 1H), 7.51 (d, 2H), 7.47 - 7.57 (m, 3H), 7.76 - 7.91 (m, 1H), 8.39 (d, 2H), 9.65 - 9.80 (m, 1H), 11.60 (br s, 1H).

### EXAMPLE 25

### 2-(6-Fluoroimidazo[1,2-a]pyridin-3-yl)-9-(pyridin-3-ylmethyl)-7,9-dihydro-8H-purin-8-one

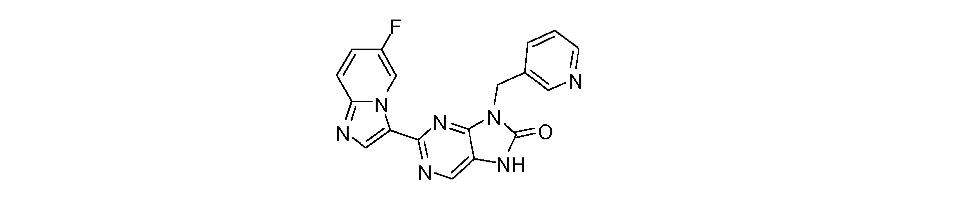

Obtained as a pale pink solid (27%) from 2-(6-fluoroimidazo[1,2*-a*]pyridin-3-yl)-N-(pyridin-3-ylmethyl)pyrimidine-4,5-diamine **(Preparation 28b)** and 1,1'-carbonylbis-1*H-*imidazole following the experimental procedure as described in **Preparation 42d** followed by purification of the crude product by flash chromatography (95:5 dichloromethane/methanol).
LRMS (m/z): 362 (M+1)⁺.
1H NMR (300 MHz, DMSO-*d₆*) δ ppm 5.28 (s, 2H), 7.72 - 7.92 (m, 2H), 8.00 (dd, 1H), 8.33 (d, 1H), 8.45 (s, 1H), 8.67 - 8.80 (m, 2H), 8.95 (br s, 1H), 10.03 (br s, 1H).

### EXAMPLE 26

### 2-(6-Fluoroimidazo[1,2-a]pyridin-3-yl)-9-(5,6,7,8-tetrahydroquinolin-5-yl)-7H-purin-8(9H)-one

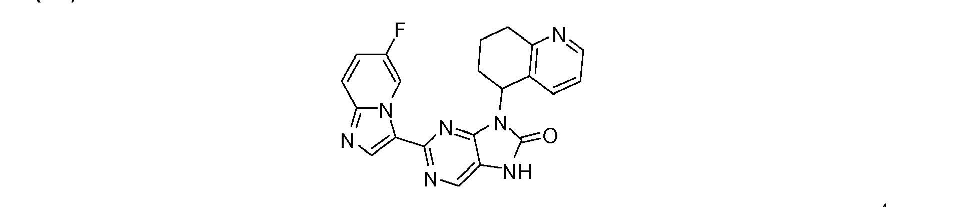

Obtained as a pale pink solid (50%) from 2-(6-fluoroimidazo[1,2-a]pyridin-3-yl)-N⁴-(5,6,7,8-tetrahydroquinolin-5-yl)pyrimidine-4,5-diamine **(Preparation 29b)** and 1,1'-carbonylbis-1*H-*imidazole following the experimental procedure as described in **Preparation 42d** followed by purification of the crude product by flash chromatography (99:1 to 90:10 dichloromethane/methanol).
LRMS (m/z): 402 (M+1)⁺.
1H NMR (300 MHz, DMSO-*d₆*) δ ppm 1.82 - 2.27 (m, 4H), 2.88 - 3.24 (m, 2H), 5.72 (dd, 1H), 7.06 (dd, 1H), 7.35 (d, 1H), 7.42 - 7.57 (m, 1H), 7.78 (dd, 1H), 8.18 (s, 1H), 8.30 - 8.51 (m, 2H), 9.40 (d, 1H).

### EXAMPLE 27

### 2-(6-Fluoroimidazo[1,2-a]pyridin-3-yl)-9-(pyridin-2-ylmethyl)-7H-purin-8(9H)-one

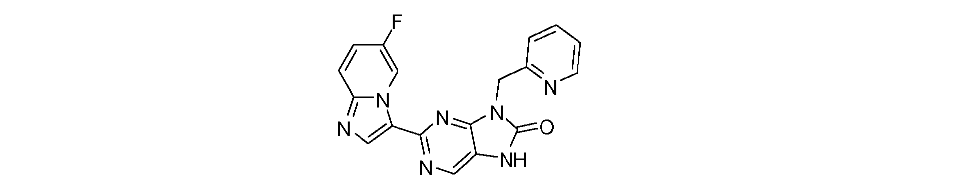

Obtained as a white solid (40%) from 2-(6-fluoroimidazo[1,2-a]pyridin-3-yl)-N⁴-(pyridin-2-ylmethyl)pyrimidine-4,5-diamine **(Preparation 30b)** and 1,1'-carbonylbis-1*H-*imidazole following the experimental procedure as described in **Preparation 42d** followed by purification of the crude product by flash chromatography (98:2 to 92:8 dichloromethane/methanol).
LRMS (m/z): 362 (M+1)⁺.
1H NMR (300 MHz, DMSO-*d₆*) δ ppm 5.27 (s, 2H), 7.33 (dd, 1H), 7.42 - 7.64 (m, 2H), 7.69 - 7.95 (m, 2H), 8.36 (s, 1H), 8.44 (s, 1H), 8.51 (d, 1H), 9.77 (dd, 1H).

### EXAMPLE 28

### 2-(6-Fluoroimidazo[1,2-a]pyridin-3-yl)-9-((5-fluoropyridin-2-yl)methyl)-7H-purin-8(9H)-one

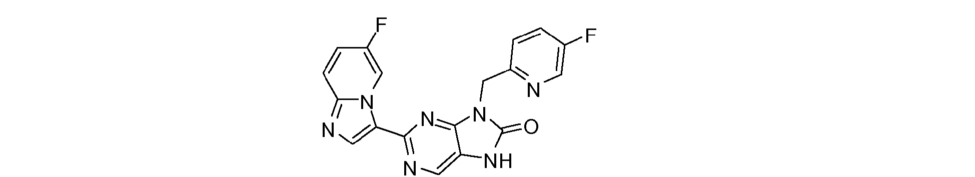

Obtained as a pale pink solid (70%) from 2-(6-fluoroimidazo[1,2-a]pyridin-3-yl)-N⁴-((5-fluoropyridin-2-yl)methyl)pyrimidine-4,5-diamine **(Preparation 31 b)** and 1,1'-carbonylbis-1*H-*imidazole following the experimental procedure as described in **Preparation 42d.**
LRMS (m/z): 380 (M+1)⁺.
1H NMR (300 MHz, DMSO-d₆) δ ppm 5.23 (s, 2H), 7.51 (m, 1H), 7.61 (dd, 1H), 7.72 - 7.82 (m, 2H), 8.34 (s, 1H), 8.41 (s, 1H), 8.48 (d, 1H), 9.71 (dd, 1H), 11.62 (br s, 1H).

### EXAMPLE 29

### (R)-2-(6-Fluoroimidazo[1,2-a]pyridin-3-yl)-9-(1-(5-fluoropyridin-2-yl)ethyl)-7H-purin-8(9H)-one

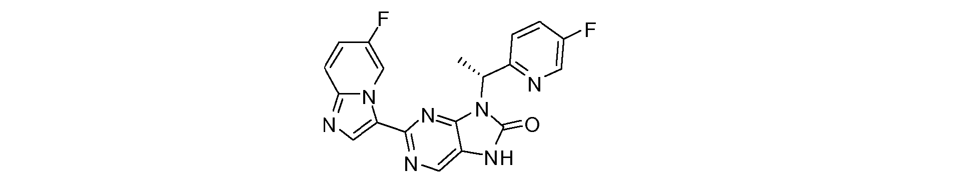

Obtained as a white solid (75%) from (R)-2-(6-fluoroimidazo[1,2-a]pyridin-3-yl)-N⁴-(1-(5-fluoropyridin-2-yl)ethyl)pyrimidine-4,5-diamine **(Preparation 32b)** and 1,1'-carbonylbis-1*H-*imidazole following the experimental procedure as described in **Preparation 42d.** The crude product was suspended in methanol and treated with 4M hydrogen chloride in 1,4-dioxane and concentrated to give the bishydrochloride salt of the title compound.
LRMS (m/z): 394 (M+1)⁺.
1H NMR (600 MHz, DMSO-*d₆*) δ ppm 2.01 (d, 3H), 5.82 (q, 1H), 7.66 (dd, 1H), 7.75 (td, 1H), 8.01 - 8.16 (m, 2H), 8.42 - 8.53 (m, 2H), 8.80 (s, 1H), 9.94 (dd, 1H).

### EXAMPLE 30

### (R)-2-(6-Fluoroimidazo[1,2-a]pyridin-3-yl)-9-(1-(5-fluoropyridin-2-yl)ethyl)-7-(2-hydroxyethyl)-7H-purin-8(9H)-one

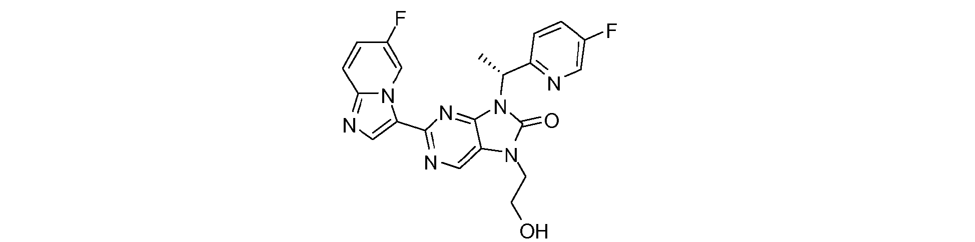

Obtained as a white solid (46%) from (R)-2-(6-fluoroimidazo[1,2-a]pyridin-3-yl)-9-(1-(5-fluoropyridin-2-yl)ethyl)-7H-purin-8(9H)-one **(Example 29)** and 2-bromoethanol following the experimental procedure as described in **Example 15a** followed by purification of the crude product by flash chromatography (98:2 to 95:5 dichloromethane/methanol).
LRMS (m/z): 438 (M+1)⁺.
1H NMR (300 MHz, DMSO-d₆) δ ppm 2.01 (d, 3H), 3.74 (t, 2H), 3.99 (t, 2H), 5.04 (br s, 1H), 5.84 (q, 1H), 7.46 - 7.84 (m, 4H), 8.31 (s, 1H), 8.50 (s, 1H), 8.58 (s, 1H), 9.57 (m, 1H).

### EXAMPLE 31

### 2-(6-Fluoroimidazo[1,2-a]pyridin-3-yl)-9-(1-(5-fluoropyridin-2-yl)-2-methoxyethyl)-7H-purin-8(9H)-one

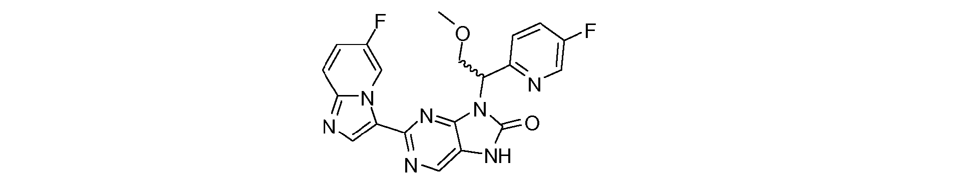

Obtained as an a pale pink solid (61 %) from 2-(6-fluoroimidazo[1,2-a]pyridin-3-yl)-N⁴-(1-(5-fluoropyridin-2-yl)-2-methoxyethyl)pyrimidine-4,5-diamine (Preparation 35b) following the experimental procedure as described in Preparation 42d.
LRMS (m/z): 424 (M+1)⁺.
1H NMR (300 MHz, DMSO-*d₆*) δ ppm 3.31 (s, 3H), 4.32 (dd, 1H), 4.59 (t, 1H), 5.88 (dd, 1H), 7.52 (m, 1H), 7.64 (dd, 1H), 7.73 - 7.83 (m, 2H), 8.34 (s, 1H), 8.42 (s, 1H), 8.53 (d, 1H), 9.66 (dd, 1H), 11.66 (s, 1H).

### EXAMPLE 32

### (R)-2-(6-Fluoroimidazo[1,2-a]pyridin-3-yl)-9-(1-(pyridin-2-yl)ethyl)-7H-purin-8(9H)-one

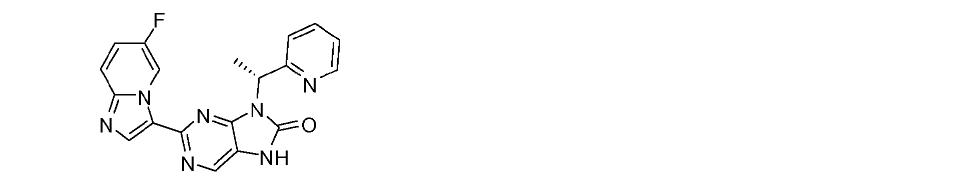

Obtained as a white solid (59%) from (R)-2-(6-fluoroimidazo[1,2-a]pyridin-3-yl)-N⁴-(1-(pyridin-2-yl)ethyl)pyrimidine-4,5-diamine **(Preparation 37b)** and 1,1'-carbonylbis-1*H-*imidazole following the experimental procedure as described in **Preparation 42d.** The crude product was suspended in methanol and treated with 4M hydrogen chloride in 1,4-dioxane and concentrated to give the bishydrochloride salt of the title compound.
LRMS (m/z): 376 (M+1)⁺.
1H NMR (300 MHz, DMSO-d₆) δ ppm 2.05 (d, 3H), 5.90 (q, 1H), 7.44 (dd, 1H), 7.69 (d, 2H), 7.87 - 8.19 (m, 3H), 8.49 (s, 1H), 8.57 (d, 1H), 8.81 (s, 1H).

### EXAMPLE 33

### (R)-2-(6-Fluoroimidazo[1,2-a]pyridin-3-yl)-9-(1-(5-fluoropyrimidin-2-yl)ethyl)-7H-purin-8(9H)-one

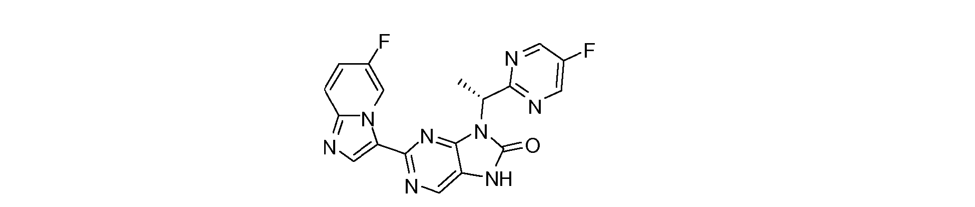

Obtained as a white solid (69%) from (R)-2-(6-fluoroimidazo[1,2-a]pyridin-3-yl)-N⁴-(1-(5-fluoropyrimidin-2-yl)ethyl)pyrimidine-4,5-diamine **(Preparation 39b)** and 1,1'-carbonylbis-1*H-*imidazole following the experimental procedure as described in **Preparation 42d.** The crude product was suspended in methanol and treated with 4M hydrogen chloride in 1,4-dioxane and concentrated to give the bishydrochloride salt of the title compound.
LRMS (m/z): 395 (M+1)⁺.
1H NMR (300 MHz, DMSO-d₆) δ ppm 2.09 (d, 3H), 5.96 (q, 1H), 8.16 (br s, 2H), 8.53 (s, 1H), 8.70 - 9.09 (m, 3H), 9.93 (br s, 1H).

### EXAMPLE 34

### (S)-2-(6-Fluoroimidazo[1,2-a]pyridin-3-yl)-9-(1-(5-fluoropyridin-2-yl)ethyl)-7H-purin-8(9H)-one

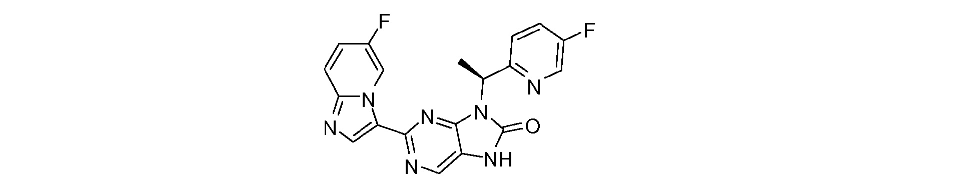

Obtained as a white solid (75%) from (S)-2-(6-fluoroimidazo[1,2-a]pyridin-3-yl)-N⁴-(1-(5-fluoropyridin-2-yl)ethyl)pyrimidine-4,5-diamine **(Preparation 40b)** and 1,1'-carbonylbis-1*H-*imidazole following the experimental procedure as described in **Preparation 42d.** The crude product was suspended in methanol and treated with 4M hydrogen chloride in 1,4-dioxane and concentrated to give the bishydrochloride salt of the title compound.
LRMS (m/z): 394 (M+1)⁺.
1H NMR (300 MHz, DMSO-*d₆*) δ ppm 2.03 (d, 3H), 5.83 (q, 1H), 7.60 - 7.71 (m, 1H), 7.75 (dd, 1H), 7.91 - 8.18 (m, 2H), 8.47 (s, 1H), 8.50 (d, 1H), 8.73 (s, 1H), 9.90 (dd, 1H).

### EXAMPLE 35

### (S)-2-(6-Fluoroimidazo[1,2-a]pyridin-3-yl)-9-(1-(5-fluoropyridin-2-yl)-2-hydroxyethyl)-7H-purin-8(9H)-one

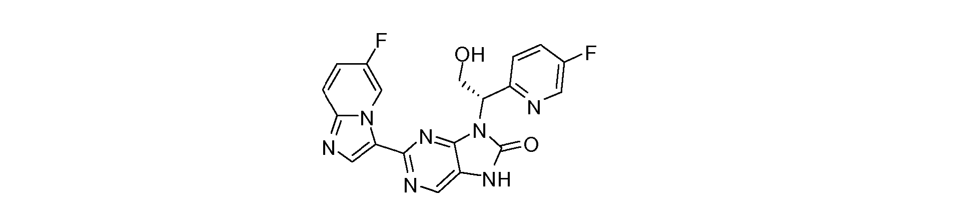

(S)-9-(2-(*tert*-Butyldiphenylsilyloxy)-1-(5-fluoropyridin-2-yl)ethyl)-2-(6-fluoroimidazo[1,2-a]pyridin-3-yl)-7H-purin-8(9H)-one **(Preparation 42d,** 0.200 g, 0.32 mmol) was dissolved in a 1.25 M solution of hydrogen chloride in methanol (5 mL) and the mixture was stirred overnight. The mixture was concentrated to dryness and the residue was triturated with ether and dried to give the hydrochloride salt of the title compound (0.092 g, 64%) as a beige solid.
LRMS (m/z): 410 (M+1)⁺.
1H NMR (300 MHz, DMSO-d₆) δ ppm 4.38 (dd, 1H), 4.61 (t, 1H), 5.73 (dd, 1H), 7.67 (dd, 1H), 7.76 (m, 1H), 8.13 (m, 2H), 8.50 (s, 1H), 8.52 (d, 1H), 8.86 (s, 1H), 10.02 (m, 1H), 11.93 (s, 1H).

### EXAMPLE 36

### 1-(2-(6-Fluoroimidazo[1,2-a]pyridin-3-yl)-8-oxo-7H-purin-9(8H)-yl)cyclohexanecarbonitrile

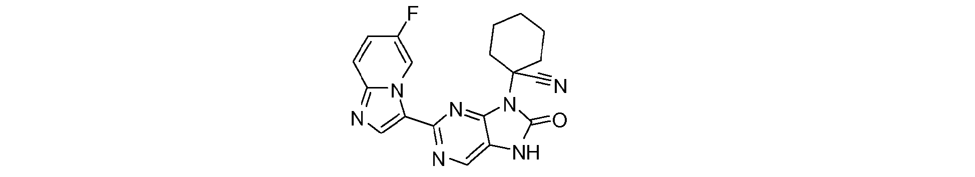

Obtained as an off-white solid (53%) from 1-(5-amino-2-(6-fluoroimidazo[1,2-a]pyridin-3-yl)pyrimidin-4-ylamino)cyclohexanecarbonitrile **(Preparation 43b)** following the experimental procedure as described in **Preparation 42d.**
LRMS (m/z): 378 (M+1)⁺.
1H NMR (300 MHz, DMSO-*d₆*) δ ppm 1.29 - 1.99 (m, 6H), 2.67 (m, 4H), 7.55 (m, 1H), 7.84 (dd, 1H), 8.42 (s, 1H), 8.44 (s, 1H), 9.88 (dd, 1H), 11.79 (s, 1H).

### EXAMPLE 37

### (1s,4s)-Ethyl 4-(2-(6-fluoroimidazo[1,2-a]pyridin-3-yl)-8-oxo-7H-purin-9(8H)-yl)cyclohexanecarboxylate

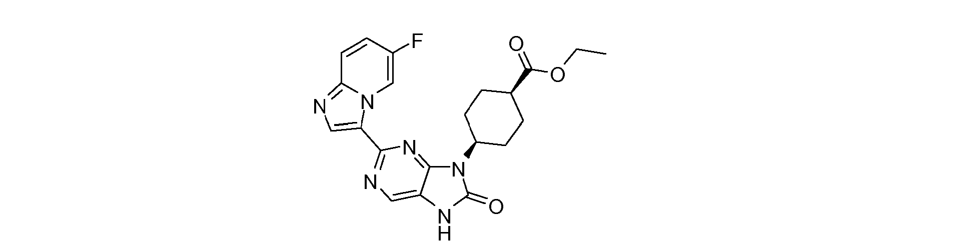

Obtained as an off-white solid (63%) from (1s,4s)-ethyl 4-(5-amino-2-(6-fluoroimidazo[1,2-a]pyridin-3-yl)pyrimidin-4-ylamino)cyclohexanecarboxylate **(Preparation 44b)** following the experimental procedure as described in **Preparation 42d** followed by purification of the crude product by flash chromatography (1 - 4% methanol in dichloromethane).
LRMS (m/z): 425 (M+1)⁺.
1H NMR (300 MHz, DMSO-*d₆*) δ ppm 1.23 (t, 3H), 1.59 - 1.76 (m, 4H), 2.23 (d, 2H), 2.55 (m, 2H), 2.78 (m, 1H), 4.19 - 4.30 (m, 3H), 7.53 (m, 1H), 7.82 (dd, 1H), 8.35 (s, 1H), 8.43 (s, 1H), 9.92 (dd, 1H).

### EXAMPLE 38

### (1 r,4r)-Ethyl 4-(2-(6-fluoroimidazo[1,2-a]pyridin-3-yl)-8-oxo-7H-purin-9(8H)-yl)cyclohexanecarboxylate

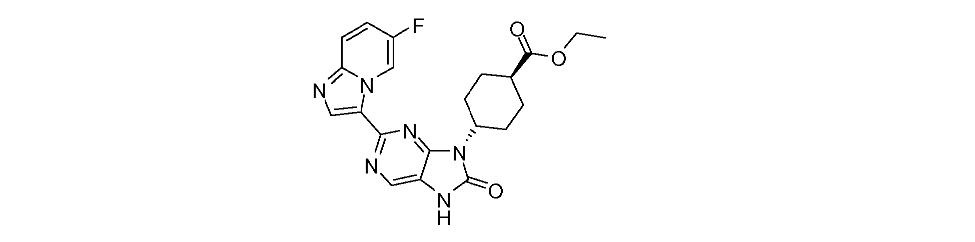

Obtained as an off-white solid (73%) from (1r,4r)-ethyl 4-(5-amino-2-(6-fluoroimidazo[1,2-a]pyridin-3-yl)pyrimidin-4-ylamino)cyclohexanecarboxylate **(Preparation 45b)** following the experimental procedure as described in **Preparation 42d** followed by purification of the crude product by flash chromatography (1 - 3% methanol in dichloromethane).
LRMS (m/z): 425 (M+1)⁺.
1H NMR (300 MHz, DMSO-*d₆*) δ ppm 1.21 (t, 3H), 1.54 (m, 2H), 1.88 (m, 2H), 2.10 (d, 2H), 2.44 - 2.60 (m, 3H), 4.09 (q, 2H), 4.26 (m, 1H), 7.54 (m, 1H), 7.83 (dd, 1H), 8.36 (s, 1H), 8.52 (s, 1H), 9.94 (dd, 1H).

### EXAMPLE 39

### (1 r,4r)-4-(2-(6-Fluoroimidazo[1,2-a]pyridin-3-yl)-8-oxo-7H-purin-9(8H)-yl)cyclohexanecarboxylic acid

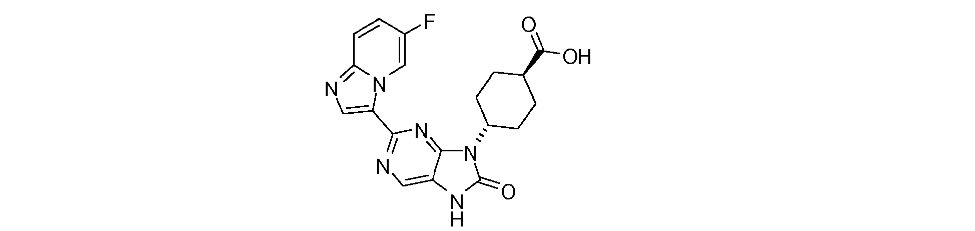

Aqueous sodium hydroxide solution (2M, 0.048 mL, 0.39 mmol) was added to a suspension of (1r,4r)-ethyl 4-(2-(6-fluoroimidazo[1,2-a]pyridin-3-yl)-8-oxo-7H-purin-9(8H)-yl)cyclohexanecarboxylate **(Example 38,** 0.050 g, 0.12 mmol) in ethyl alcohol (1.5 mL) and the mixture was stirred and heated to 80 °C. After HPLC indicated complete reaction, the mixture was evaporated to dryness and the residue was dissolved in water. The pH was adjusted to ca. 5 with aqueous hydrogen chloride solution and the precipitate was filtered and dried to give the title compound (0.030 g, 64%) as a white solid.
LRMS (m/z): 395 (M-1)⁺.
1H NMR (300 MHz, DMSO-*d₆*) δ ppm 1.52 (m, 2H), 1.87 (m, 2H), 2.10 (d, 2H), 2.42 - 2.60 (m, 3H), 4.27 (m, 1H), 7.63 (m, 1H), 7.88 (dd, 1H), 8.38 (s, 1H), 8.58 (s, 1H), 9.98 (m, 1H).

### EXAMPLE 40

### (1r,4r)-4-(2-(6-Fluoroimidazo[1,2-a]pyridin-3-yl)-8-oxo-7H-purin-9(8H)-yl)-N-methylcyclohexanecarboxamide

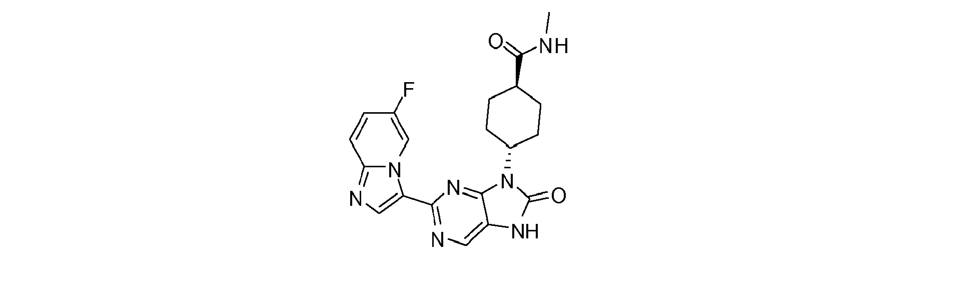

O-(7-Azabenzotriazol-1-yl)-N,N,N',N'-tetramethyluronium hexafluorophosphate (0.081 g, 0.21 mmol) and N,N-diisopropylethylamine (0.058 g, 0.45 mmol) were added to a suspension of ((1r,4r)-4-(2-(6-fluoroimidazo[1,2-a]pyridin-3-yl)-8-oxo-7H-purin-9(8H)-yl)cyclohexanecarboxylic acid **(Example 39,** 0.060 g, 0.15 mmol) in N,N'-dimethylformamide (0.5 mL). After stirring at ambient temperature for 5 minutes, methyl amine hydrochloride (0.011 g, 0.16 mmol) was added and the mixture was stirred overnight. The mixture was evaporated to dryness and the residue was partitioned between aqueous sodium hydroxide solution and ethyl acetate. The insoluble precipitate was filtered, washed with water and dried to give the title compound (0.031 g, 50%) as a white solid.
LRMS (m/z): 410 (M+1)⁺.
1H NMR (300 MHz, DMSO-*d₆*) δ ppm 1.54 (m, 2H), 1.74 - 1.96 (m, 5H), 2.21 - 2.50 (m, 3H), 2.59 (d, 2H), 4.23 (m, 1H), 7.52 (m, 1H), 7.72 - 7.86 (m, 2H), 8.28 (s, 1H), 8.42 (s, 1H), 9.96 (m, 1H).

### EXAMPLE 41

### 9-(2,2-Dimethyltetrahydro-2H-pyran-4-yl)-2-(6-fluoroimidazo[1,2-a]pyridin-3-yl)-7,9-dihydro-8H-purin-8-one

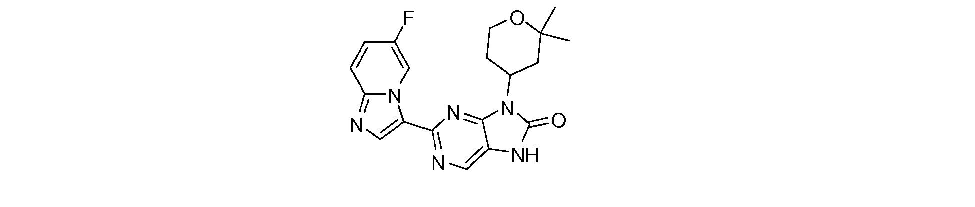

Obtained as a pale pink solid (55%) from *N⁴*-(2,2-dimethyltetrahydro-2*H-*pyran-4-yl)-2-(6-fluoroimidazo[1,2*-a*]pyridin-3-yl)pyrimidine-4,5-diamine **(Preparation 46b)** and 1,1'-carbonylbis-1*H-*imidazole following the experimental procedure as described in **Preparation 42d** followed by purification of the crude product by flash chromatography (98:2 to 96:4 dichloromethane/methanol).
LRMS (m/z): 383 (M+1)⁺.
1H NMR (300 MHz, DMSO-*d₆*) δ ppm 1.25 (br s, 3H), 1.31 (br s, 3H), 1.74 (d, 2H), 2.36 - 2.62 (m, 2H), 3.66 - 3.92 (m, 2H), 4.54 - 4.80 (m, 1H), 7.54 (dd, 1H), 7.79 - 7.88 (m, 1H), 8.37 (s, 1H), 8.41 (s, 1H), 9.87 (br s, 1H), 11.52 (br s, 1H).

### EXAMPLE 42

### 9-(2,2-Dimethyl-3,4-dihydro-2H-pyrano[2,3-b]pyridin-4-yl)-2-(6-fluoroimidazo[1,2-a]pyridin-3-yl)-7H-purin-8(9H)-one

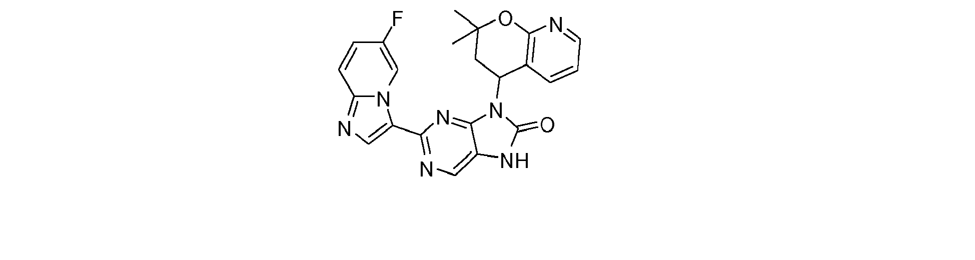

Obtained as an off-white solid (27%) from N⁴-(2,2-dimethyl-3,4-dihydro-2H-pyrano[2,3-b]pyridin-4-yl)-2-(6-fluoroimidazo[1,2-a]pyridin-3-yl)pyrimidine-4,5-diamine (Preparation 48b) following the experimental procedure as described in Preparation 42d followed by purification of the crude product by flash chromatography (2 - 5% methanol in dichloromethane).
LRMS (m/z): 432 (M+1)⁺.
1H NMR (300 MHz, CDCl₃) b ppm 1.55 (s, 3H), 1.65 (s, 3H), 2.18 (dd, 1H), 3.03 (t, 1H), 5.95 (dd, 1H), 6.82 (dd, 1H), 7.20 - 7.30 (m, 2H), 7.67 (dd, 1H), 7.72 (s, 1H), 8.21 (m, 1H), 8.36 (br s, 1H), 8.38 (s, 1H), 9.46 (br m, 1H).

### EXAMPLE 43

### 2-(6-Fluoroimidazo[1,2-a]pyridin-3-yl)-9-(1,4-dioxaspiro[4.5]decan-8-yl)-7H-purin-8(9H)-one

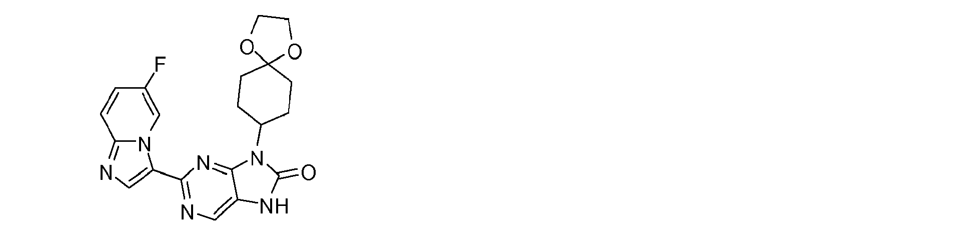

Obtained as a pale pink solid (60%) from 2-(6-fluoroimidazo[1,2-a]pyridin-3-yl)-N⁴-(1,4-dioxaspiro[4.5]decan-8-yl)pyrimidine-4,5-diamine **(Preparation 49b)** and 1,1'-carbonylbis-1*H-*imidazole following the experimental procedure as described in **Preparation 42d** followed by purification of the crude product by flash chromatography (98:2 to 95:5 dichloromethane/methanol).
LRMS (m/z): 411 (M+1)⁺.
1H NMR (300 MHz, DMSO-*d₆*) δ ppm 1.67 - 1.86 (m, 6H), 2.61 - 2.75 (m, 2H), 3.89 - 4.00 (m, 4H), 4.37 (m, 1H), 7.54 (dd, 1H), 7.83 (dd, 1H), 8.36 (s, 1H), 8.41 (s, 1H), 9.83 (dd, 1H), 11.49 (br s, 1H).

### EXAMPLE 44

### 2-(6-Fluoroimidazo[1,2-a]pyridin-3-yl)-9-(4-oxocyclohexyl)-7H-purin-8(9H)-one

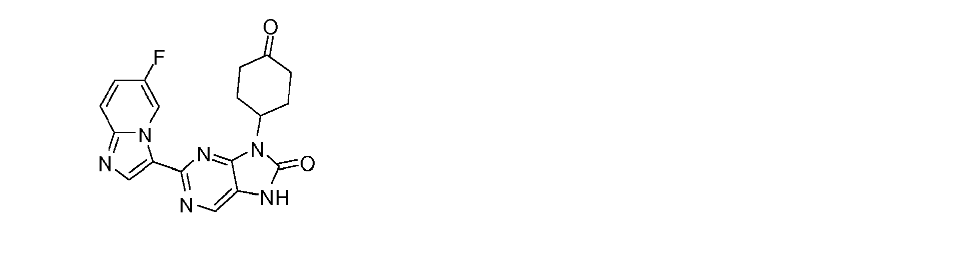

4-Methylbenzenesulfonic acid monohydrate (0.350 g, 1.8 mmol) was added to a suspension of 2-(6-fluoroimidazo[1,2-a]pyridin-3-yl)-9-(1,4-dioxaspiro[4.5]decan-8-yl)-7H-purin-8(9H)-one **(Example 43,** 0.780 g, 1.9 mmol) in acetone (20 mL) and water (20 mL) and the mixture was stirred and heated to 70 °C. After 40 hours, the acetone was removed to give a suspension which was made basic with solid sodium hydrogen carbonate. The suspension was filtered and the solid was washed with water and dried in vacuo to give the title compound (0.660 g, 95%) as a pink solid.
LRMS (m/z): 367 (M+1)⁺.
1H NMR (300 MHz, DMSO-*d₆*) δ ppm 2.13 (m, 2H), 2.38 (m, 2H), 2.66 - 2.85 (m, 4H), 4.87 (m, 1H), 7.54 (m, 1H), 7.84 (dd, 1H), 8.39 (s, 1H), 8.47 (s, 1H), 9.89 (dd, 1H).

### EXAMPLE 45

### 2-((1r,4r)-4-(2-(6-Fluoroimidazo[1,2-a]pyridin-3-yl)-8-oxo-7H-purin-9(8H)-yl)cyclohexyl)acetonitrile

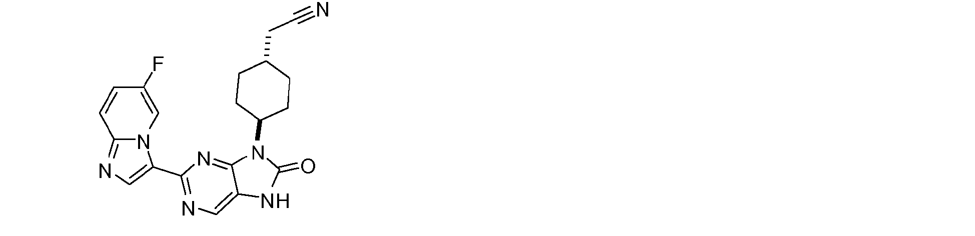

Obtained as a pale pink solid (48%) from 2-((1r,4r)-4-(5-amino-2-(6-fluoroimidazo[1,2-a]pyridin-3-yl)pyrimidin-4-ylamino)cyclohexyl)acetonitrile **(Preparation 51b)** and 1,1'-carbonylbis-1*H-*imidazole following the experimental procedure as described in **Preparation 42d** followed by purification of the crude product by flash chromatography (100:0 to 90:10 dichloromethane/methanol).
LRMS (m/z): 392 (M+1)⁺.
1H NMR (300 MHz, DMSO-*d₆*) ppm 1.07 - 1.50 (m, 2H), 1.69 - 2.13 (m, 6H), 2.32 - 2.70 (m, 4H), 4.16 - 4.41 (m, 1H), 7.44 - 7.74 (m, 1H), 7.87 (d, 1H), 8.41 (s, 1H), 8.52 (s, 1H), 9.98 (br s, 1H).

### EXAMPLE 46

### 2-((1r,4r)-4-(2-(6-Fluoroimidazo[1,2-a]pyridin-3-yl)-7-(2-hydroxyethyl)-8-oxo-7H-purin-9(8H)-yl)cyclohexyl)acetonitrile

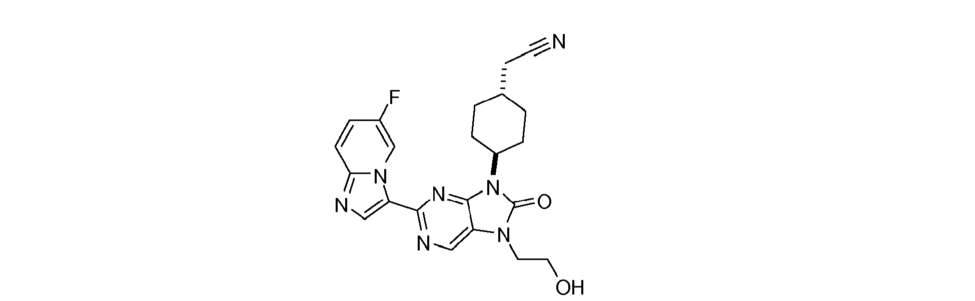

Obtained as an off-white solid (58%) from 2-((1r,4r)-4-(2-(6-fluoroimidazo[1,2-a]pyridin-3-yl)-8-oxo-7H-purin-9(8H)-yl)cyclohexyl)acetonitrile (**Example 45**) and 2-bromoethanol following the experimental procedure as described in **Example 15a** followed by purification of the crude product by flash chromatography (98:2 to 95:5 dichloromethane/methanol).
LRMS (m/z): 436 (M+1)+.
1H NMR (300 MHz, DMSO-d₆) δ ppm 1.18-1.41 (m, 3H), 1.78-2.05 (m, 6H), 2.56 (d, 2H), 3.70 (m, 2H), 3.95 (t, 2H), 4.29 (m, 1H), 4.99 (t, 1H), 7.55 (m, 1H), 7.84 (dd, 1H), 8.50 (s, 1H), 8.57 (s, 1H), 9.93 (m, 1H).

### EXAMPLE 47

### 2-(6-Fluoroimidazo[1,2-a]pyridin-3-yl)-9-((1r,4r)-4-(hydroxymethyl)cyclohexyl)-7H-purin-8(9H)-one

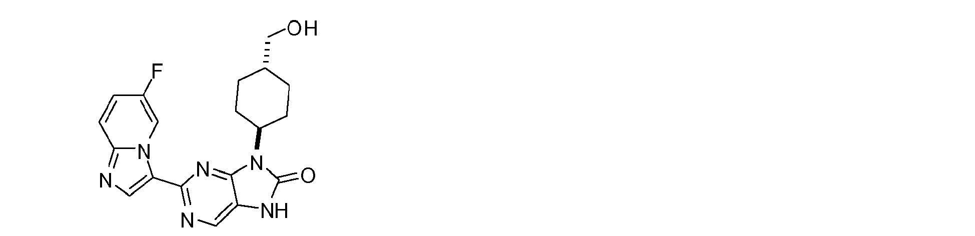

Obtained as a white solid (52%) from 9-((1r,4r)-4-((*tert-*butyldiphenylsilyloxy)methyl)cyclohexyl)-2-(6-fluoroimidazo[1,2-a]pyridin-3-yl)-7H-purin-8(9H)-one (**Preparation 53c**) following the experimental procedure as described in **Example 35.**
LRMS (m/z): 383 (M+1)⁺.
1H NMR (300 MHz, DMSO*-d*₆) δ ppm 0.92 - 1.35 (m, 3H), 1.59 (m, 1H), 1.72 - 2.12 (m, 3H), 2.40 (d, 1H), 3.40 (m, 2H), 4.24 (m, 1H), 4.58 (br s, 1H), 7.56 (t, 1H), 7.85 (m, 1H), 8.35 (s, 1H), 8.44 (s, 1H), 9.99 (m, 1H).

### EXAMPLE 48

### 2-(6-Fluoroimidazo[1,2-a]pyridin-3-yl)-9-((1r,4r)-4-hydroxycyclohexyl)-7H-purin-8(9H)-one

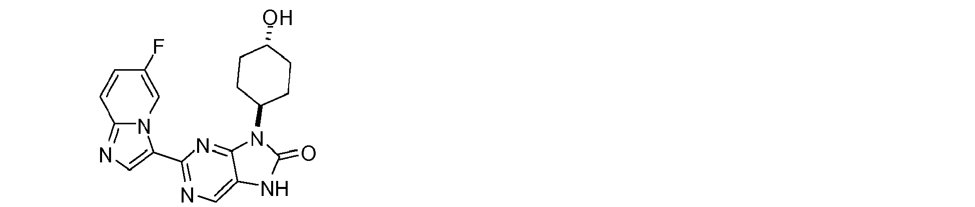

Obtained as a white solid (55%) from 9-((1r,4r)-4-(*tert*-butyldiphenylsilyloxy)cyclohexyl)-2-(6-fluoroimidazo[1,2-a]pyridin-3-yl)-7H-purin-8(9H)-one **(Preparation 55c)** following the experimental procedure as described in **Example 35.**
LRMS (m/z): 369 (M+1)⁺.
1H NMR (300 MHz, DMSO-d₆) δ ppm 1.36 (m, 2H), 1.78 (d, 2H), 1.99 (m, 2H), 2.43 (m, 2H), 3.61 (m, 1H), 4.23 (m, 1H), 4.73 (br s, 1H), 7.53 (m, 1H), 7.82 (m, 1H), 8.33 (s, 1H), 8.43 (s, 1H), 9.91 (m, 1H).

### EXAMPLE 49

### 2-(6-Fluoroimidazo[1,2-a]pyridin-3-yl)-9-((1R,4R)-4-hydroxy-1,2,3,4-tetrahydronaphthalen-1-yl)-7H-purin-8(9H)-one

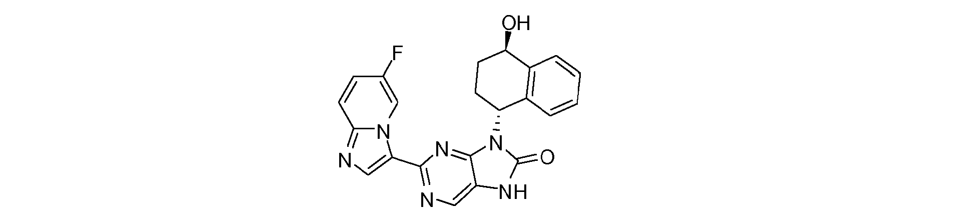

Caesium fluoride (0.274 g, 1.80 mmol) was added to a solution of (9-((1R,4R)-4-(tert-butyldiphenylsilyloxy)-1,2,3,4-tetrahydronaphthalen-1-yl)-2-(6-fluoroimidazo[1,2-a]pyridin-3-yl)-7H-purin-8(9H)-one (**Preparation 56c,** 0.118 g, 0.18 mmol) in N,N-dimethylformamide (6 mL) and the mixture was stirred and heated to 60 °C. After 30 hours, the mixture was partitioned between ethyl acetate and water and the organic extract was dried (MgSO₄ and evaporated and the residue was purified by flash chromatography (0 - 6% methanol in dichloromethane) to give the title compound (0.041 g, 47%) as a cream solid.
LRMS (m/z): 417 (M+1)⁺.
1H NMR (300 MHz, DMSO*-d*₆) δ ppm 1.76-1.90 (m, 1H), 2.12-2.33 (m, 2H), 2.40-2.55 (m, 1H), 4.94 (m, 1H), 5.50 (d, 1H), 5.70 (m, 1H), 6.85 (d, 1H), 7.07 (t, 1H), 7.23 (t, 1H), 7.48 (t, 1H), 7.68 (d, 1H), 7.78 (dd, 1H), 8.22 (s, 1H), 8.40 (s, 1H), 9.20 (m, 1H).

### EXAMPLE 50

### 2-(6-Fluoroimidazo[1,2-a]pyridin-3-yl)-9-((1r,4r)-4-hydroxy-4-methylcyclohexyl)-7H-purin-8(9H)-one

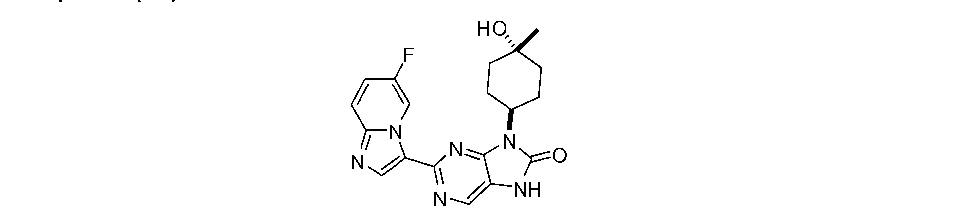

Obtained as a pale pink solid (65%) from (1r,4r)-4-(5-amino-2-(6-fluoroimidazo[1,2-a]pyridin-3-yl)pyrimidin-4-ylamino)-1-methylcyclohexanol (**Preparation 57b**) and 1,1'-carbonylbis-1*H-*imidazole following the experimental procedure as described in **Preparation 42d.**
LRMS (m/z): 383 (M+1)⁺.
1H NMR (300 MHz, DMSO*-d*₆) δ ppm 1.20 - 1.75 (m, 11H), 4.25 (m, 1H), 4.55 (br s, 1 ), 7.55 (m, 1H), 7.85 (m, 1H), 8.40 (m, 2H), 9.90 (m, 1H), 11.55 (br s, 1H).

### EXAMPLE 51

### 9-((1r,4r)-4-(Aminomethyl)cyclohexyl)-2-(6-fluoroimidazo[1,2-a]pyridin-3-yl)-7H-purin-8(9H)-one

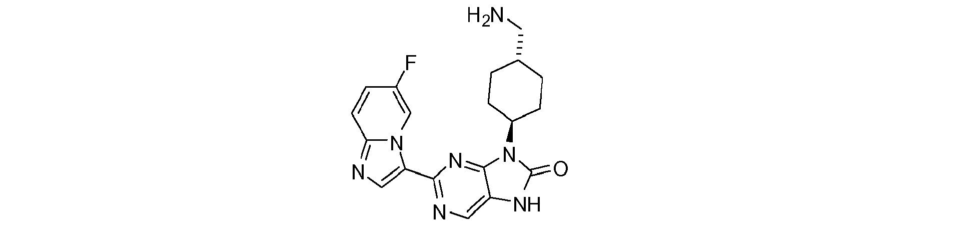

A suspension of tert-butyl ((1r,4r)-4-(2-(6-fluoroimidazo[1,2-a]pyridin-3-yl)-8-oxo-7H-purin-9(8H)-yl)cyclohexyl)methylcarbamate (**Preparation 58**, 0.225 g, 0.47 mmol) in a 4M solution of hydrogen chloride in 1,4-dioxane (5 mL) was stirred overnight. The suspension was filtered and the solid was suspended in warm dimethylsulphoxide (0.5 mL) and methanol (1.0 mL) and filtered. The solid was washed with methanol/diethyl ether (1:2), diethyl ether and dried to give the dihydrochloride salt of the title compound (0.190 g, 89%) as a white solid.
LRMS (m/z): 382 (M+1)⁺.
1H NMR (300 MHz, DMSO*-d*₆) δ ppm 1.16 (q, 2H), 1.68 - 1.98 (m, 5H), 2.36 (q, 2H), 2.71 (m, 2H), 4.24 (m, 1H), 7.84 (m, 1H), 7.95 (br s, 3H), 7.99 (dd, 1H), 8.39 (s, 1H), 8.68 (s, 1H), 10.05 (dd, 1H), 11.64 (s, 1H).

### EXAMPLE 52

### 9-((1r,4r)-4-Aminocyclohexyl)-2-(6-fluoroimidazo[1,2-a]pyridin-3-yl)-7H-purin-8(9H)-one

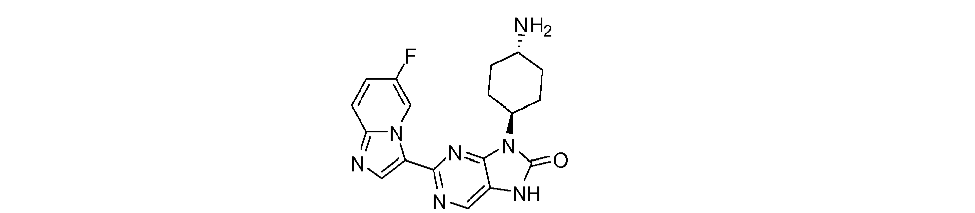

A suspension of tert-butyl (1r,4r)-4-(2-(6-fluoroimidazo[1,2-a]pyridin-3-yl)-8-oxo-7H-purin-9(8H)-yl)cyclohexylcarbamate (**Preparation 59**, 0.204 g, 0.44 mmol) in a 4M solution of hydrogen chloride in 1,4-dioxane (10 mL) was stirred overnight. The mixture was evaporated and the residue was purified by reverse phase chromatography (C-18 silica from Waters©, water/acetonitrile/methanol as eluents [0.1% v/v formic acid buffered] 0% to 100%) to give the title compound (0.070 g, 44%) as a white solid.
LRMS (m/z): 368 (M+1)⁺.
1H NMR (300 MHz, DMSO*-d*₆) δ ppm 1.29 (q, 2H), 1.78 (m, 2H), 1.97 (m, 2H), 2.45 (m, 2H), 2.85 (m, 1H), 4.23 (m, 1H), 7.52 (m, 1H), 7.82 (dd, 1H), 8.28 (s, 1H), 8.41 (s, 1H), 9.95 (dd, 1H).

### EXAMPLE 53

### 9-Cyclobutyl-2-(6-fluoroimidazo[1,2-a]pyridin-3-yl)-7H-purin-8(9H)-one

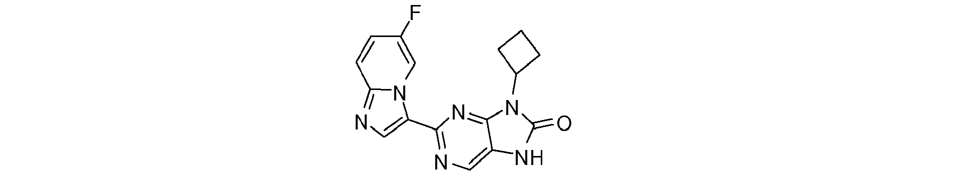

Obtained as an off-white solid (63%) from N⁴-cyclobutyl-2-(6-fluoroimidazo[1,2-a]pyridin-3-yl)pyrimidine-4,5-diamine (**Preparation 60b**) and 1,1'-carbonylbis-1*H-*imidazole following the experimental procedure as described in **Preparation 42d** followed by purification of the crude product by flash chromatography (99:1 to 90:10 dichloromethane/methanol).
LRMS (m/z): 325 (M+1)⁺.
1H NMR (300 MHz, DMSO*-d*₆) δ ppm 1.74 - 2.19 (m, 2H), 2.22 - 2.49 (m, 2H), 2.96 - 3.26 (m, 2H), 4.80 - 5.12 (m, 1H), 7.06 (br s, 1H), 7.42 - 7.77 (m, 2H), 7.89 (dd, 1H), 8.41 (s, 1H), 8.49 (s, 1H).

### EXAMPLE 54

### (R)-2-(6-Fluoroimidazo[1,2-a]pyridin-3-yl)-9-(3-methylbutan-2-yl)-7H-purin-8(9H)-one

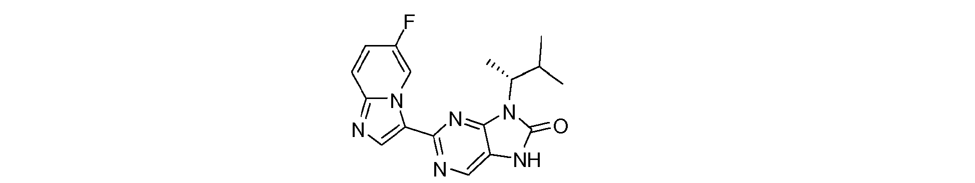

Obtained as an off-white solid (22%) from (R)-2-(6-fluoroimidazo[1,2-a]pyridin-3-yl)-N⁴-(3-methylbutan-2-yl)pyrimidine-4,5-diamine (**Preparation 61b**) following the experimental procedure as described in **Preparation 42d** followed by purification of the crude product by flash chromatography (5-10% methanol in dichloromethane).
LRMS (m/z): 341 (M+1)⁺.
1H NMR (300 MHz, DMSO*-d*₆) δ ppm 0.76 (d, 3H), 1.06 (d, 3H), 1.58 (d, 3H), 2.52 (m, 1H), 4.12 (m, 1H), 7.54 (m, 1H), 7.82 (dd, 1H), 8.38 (s, 1H), 8.42 (s, 1H), 9.91 (dd, 1H).

### EXAMPLE 55

### 2-(6-Fluoroimidazo[1,2-a]pyridin-3-yl)-9-(1-methoxypropan-2-yl)-7H-purin-8(9H)-one

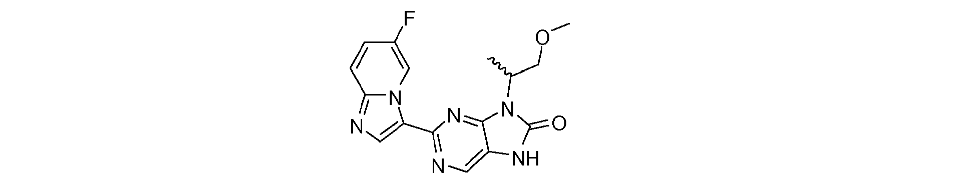

Obtained as an off-white solid (60%) from 2-(6-fluoroimidazo[1,2-a]pyridin-3-yl)-N⁴-(1-methoxypropan-2-yl)pyrimidine-4,5-diamine (**Preparation 62b**) following the experimental procedure as described in **Preparation 42d** followed by purification of the crude product by flash chromatography (5 - 10% methanol in dichloromethane).
LRMS (m/z): 343 (M+1)⁺.
1H NMR (300 MHz, DMSO*-d*₆) δ ppm 1.52 (d, 3H), 3.23 (s, 3H), 3.65 (dd, 1H), 4.10 (t, 1H), 4.74 (m, 1H), 7.55 (m, 1H), 7.83 (dd, 1H), 8.38 (s, 1H), 8.43 (s, 1H), 9.92 (dd, 1H).

### EXAMPLE 56

### (R)-tert-Butyl 3-(2-(6-fluoroimidazo[1,2-a]pyridin-3-yl)-8-oxo-7H-purin-9(8H)-yl)butanoate

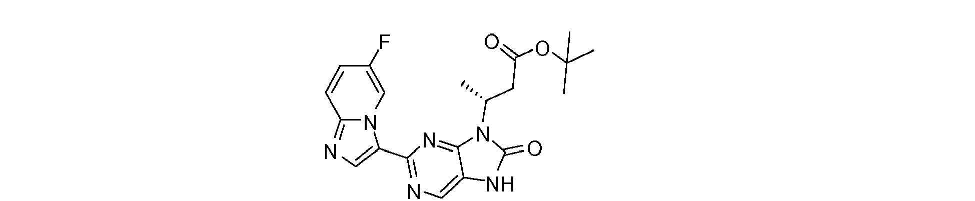

Obtained as an off-white solid (58%) from (R)-tert-butyl 3-(5-amino-2-(6-fluoroimidazo[1,2-a]pyridin-3-yl)pyrimidin-4-ylamino)butanoate (**Preparation 63b**) following the experimental procedure as described in **Preparation 42d** followed by purification of the crude product by flash chromatography (0 - 10% methanol in dichloromethane).
LRMS (m/z): 413 (M+1)⁺.
1 H NMR (300 MHz, DMSO*-d*₆) δ ppm 1.19 (s, 9H), 1.55 (d, 3H), 2.87 (dd, 1 H), 3.23 (dd, 1H), 4.88 (m, 1H), 7.55 (m, 1H), 7.85 (dd, 1H), 8.39 (s, 1H), 8.43 (s, 1H), 9.91 (dd, 1H), 11.52 (br s, 1H).

### EXAMPLE 57

### (R)-3-(2-(6-Fluoroimidazo[1,2-a]pyridin-3-yl)-8-oxo-7H-purin-9(8H)-yl)butanoic acid

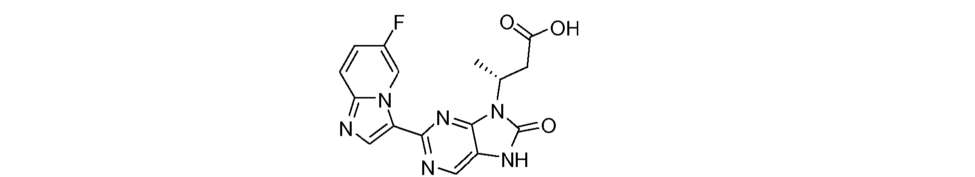

A 4M solution of hydrogen chloride in 1,4-dioxane (9 mL) was added to a cold (0 °C) suspension of (R)-tert-butyl 3-(2-(6-fluoroimidazo[1,2-a]pyridin-3-yl)-8-oxo-7H-purin-9(8H)-yl)butanoate (**Example 56**, 0.189 g, 0.46 mmol) in 1,4-dioxane (5 mL) and water (0.9 mL) and the mixture was stirred and warmed to room temperature. After 3 hours, the mixture was evaporated in vacuo to give the dihydrochloride salt of the title compound (0.189 g, 96%) as a beige solid.
LRMS (m/z): 355 (M-1)⁺.
1H NMR (300 MHz, DMSO*-d*₆) δ ppm 1.54 (d, 3H), 2.98 (dd, 1H), 3.24 (dd, 1H), 4.89 (m, 1H), 8.05 - 8.17 (m, 2H), 8.45 (s, 1H), 8.97 (s, 1H), 10.21 (m, 1H), 11.74 (br s, 1H).

### EXAMPLE 58

### (R)-3-(2-(6-Fluoroimidazo[1,2-a]pyridin-3-yl)-8-oxo-7H-purin-9(8H)-yl)butanamide

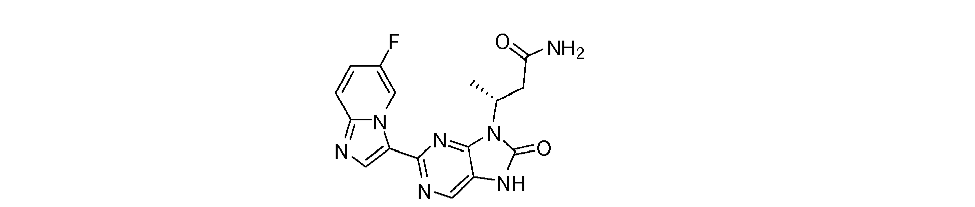

Concentrated aqueous ammonium hydroxide solution (0.076 mL) was added to a mixture of (R)-3-(2-(6-fluoroimidazo[1,2-a]pyridin-3-yl)-8-oxo-7H-purin-9(8H)-yl)butanoic acid dihydrochloride (**Example 57,** 0.066 g, 0.15 mmol), N-(3-dimethylaminopropyl)-N'-ethylcarbodiimide hydrochloride (0.044 g, 0.23 mmol) and 1-hydroxybenzotriazole hydrate (0.031 g, 0.23 mmol) in N,N-dimethylformamide (1.0 mL) and the mixture was stirred and heated to 60 °C. After 4 hours, further N-(3-dimethylaminopropyl)-N'-ethylcarbodiimide hydrochloride (0.015 g, 0.08 mmol), 1-hydroxybenzotriazole hydrate (0.010 g, 0.08 mmol) and concentrated aqueous ammonium hydroxide solution (0.076 mL) were added and heating was continued for a further 3 hours. The mixture was diluted with water and the precipitate was filtered. The solid was dissolved in 2M aqueous sodium hydroxide solution and then the mixture was neutralized with 2M aqueous hydrogen chloride solution and the fine precipitate was filtered and dried to give the title compound (0.026 g, 45%) as a white solid.
LRMS (m/z): 356 (M+1)⁺.
1H NMR (300 MHz, DMSO*-d*₆) δ ppm 1.51 (d, 3H), 2.90 (m, 2H), 4.92 (m, 1H), 6.85 (s, 1H), 7.51 (m, 2H), 7.84 (m, 1H), 8.35 (s, 1H), 8.49 (s, 1H), 9.95 (m, 1H).

### EXAMPLE 59

### 9-(1-(2,2-Difluoroethyl)piperidin-4-yl)-2-(6-fluoroimidazo[1,2-a]pyridin-3-yl)-7H-purin-8(9H)-one

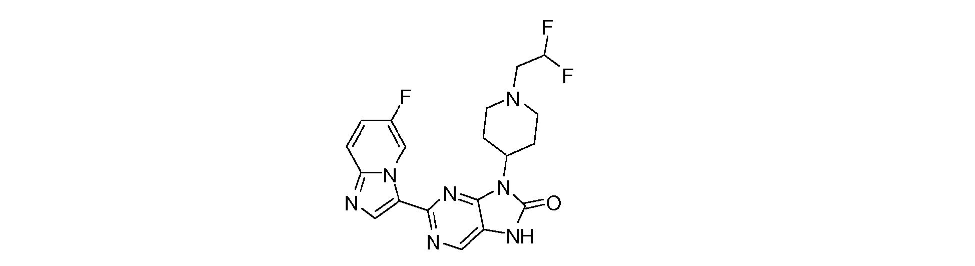

Obtained as a pale pink solid (88%) from N⁴-(1-(2,2-difluoroethyl)piperidin-4-yl)-2-(6-fluoroimidazo[1,2-a]pyridin-3-yl)pyrimidine-4,5-diamine (**Preparation 64b**) and 1,1'-carbonylbis-1*H-*imidazole following the experimental procedure as described in **Preparation 42d.**
LRMS (m/z): 418 (M+1)⁺.
1H NMR (300 MHz, DMSO*-d*₆) δ ppm 1.75 (d, 2H), 2.38 (t, 2H), 2.61 (m, 2H), 2.84 (dt, 2H), 3.09 (d, 2H), 4.28 (m, 1H), 6.18 (tt, 1H), 7.55 (m, 1H), 7.84 (dd, 1H), 8.38 (s, 1H), 8.41 (s, 1H), 9.90 (m, 1H), 11.52 (br s, 1H).

### EXAMPLE 60

### 9-(4,4-Difluorocyclohexyl)-2-(6-fluoroimidazo[1,2-a]pyridin-3-yl)-7,9-dihydro-8H-purin-8-one

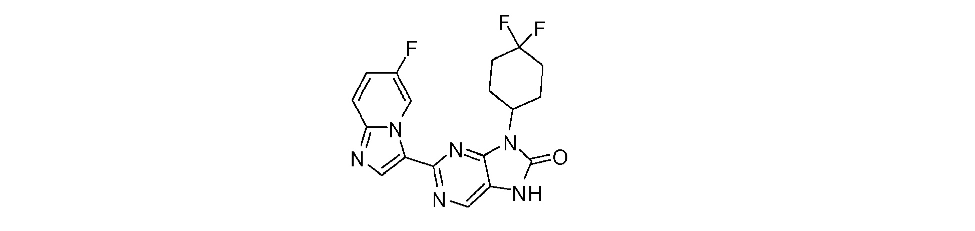

Obtained as a solid (34%) from *N*⁴-(4,4-difluorocyclohexyl)-2-(6-fluoroimidazo[1,2-a]pyridin-3-yl)pyrimidine-4,5-diamine (**Preparation 65b**) and 1,1'-carbonylbis-1*H-*imidazole following the experimental procedure as described in **Preparation 42d** followed by purification of the crude product by flash chromatography (98:2 to 95:5 dichloromethane/methanol).
LRMS (m/z): 389 (M+1)⁺.
1H NMR (300 MHz, DMSO*-d*₆) δ ppm 1.79 - 1.97 (m, 2H), 1.98 - 2.23 (m, 4H), 2.61 (dd, 2H), 4.39 - 4.62 (m, 1H), 7.81 - 7.93 (m, 1H), 8.01 (dd, 1H), 8.40 (s, 1H), 8.64 (s, 1H), 9.97 (dd, 1H), 11.69 (s, 1H).

### EXAMPLE 61

### 2-(6-Fluoroimidazo[1,2-a]pyridin-3-yl)-9-[(3R)-piperidin-3-yl]-7,9-dihydro-8H-purin-8-one

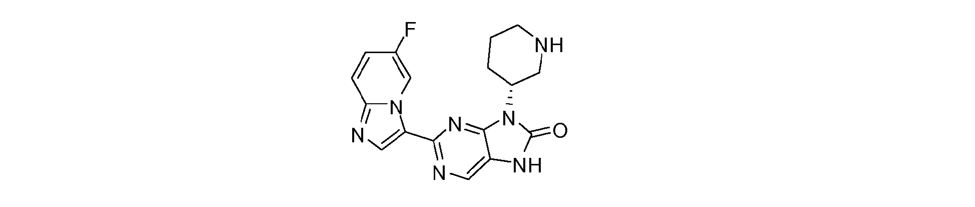

(*R*)-*tert*-butyl-3-(2-(6-fluoroimidazo[1,2-a]pyridin-3-yl)-8-oxo-7-((2-(trimethylsilyl)ethoxy)methyl)-7H-purin-9(8H)-yl)piperidine-1-carboxylate (**Preparation 67**, 4.20 g, 5.76 mmol) was suspended in trifluoroacetic acid (20 mL) and the mixture was stirred and heated to 50 °C. After 2 hours, water was added and the mixture was neutralized with solid sodium hydrogen carbonate and then extracted with several portions of ethyl acetate. The organic layer was dried (MgSO₄) and concentrated and the residue was purified by reverse phase chromatography (C-18 silica from Waters@, water/acetonitrile/methanol as eluents [0.1% v/v formic acid buffered] 0% to 100%) to give the title compound (0.45 g, 26%) as a white solid.
LRMS (m/z): 354 (M+1)⁺.
1H NMR (300 MHz, DMSO-d₆) δ ppm 1.86 (m, 2H), 2.67 (m, 2H), 3.07 (m, 2H), 3.25-3.51 (m, 2H), 4.39 (m, 1H), 7.59 (m, 1H), 7.85 (m, 1H), 8.22 (s, 1H), 8.39 (s, 1H), 8.48 (s, 1H), 9.98 (m, 1H).

### EXAMPLE 62

### 2-(6-Fluoroimidazo[1,2-a]pyridin-3-yl)-9-[(3R)-1-(methylsulfonyl)piperidin-3-yl]-7,9-dihydro-8H-purin-8-one

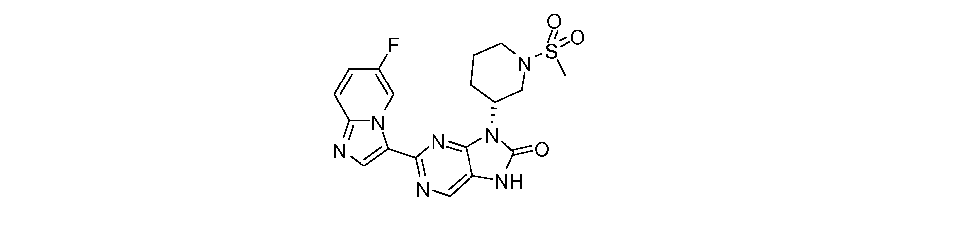

Obtained as a white solid (31%) from (R)-2-(6-fluoroimidazo[1,2-a]pyridin-3-yl)-9-(1-(methylsulfonyl)piperidin-3-yl)-7-((2-(trimethylsilyl)ethoxy)methyl)-7H-purin-8(9H)-one (**Preparation 68**) following the experimental procedure as described in **Example 19** followed by purification of the crude product by flash chromatography (98:2 dichloromethane/methanol).
LRMS (m/z): 432 (M+1)⁺.
1H NMR (300 MHz, DMSO-d₆) δ ppm 2.00 (m, 2H), 2.84 (m, 2H), 2.95 (s, 3H), 3.29 (m, 2H), 3.73 (m, 2H), 4.42 (m, 1H), 7.55 (m, 1H), 7.84 (m, 1H), 8.40 (s, 1H), 8.52 (s, 1H), 9.94 (m, 1H).

### EXAMPLE 63

### 2-(6-Fluoroimidazo[1,2-a]pyridin-3-yl)-9-[(3R)-1-(methylsulfonyl)piperidin-3-yl]-7,9-dihydro-8H-purin-8-one

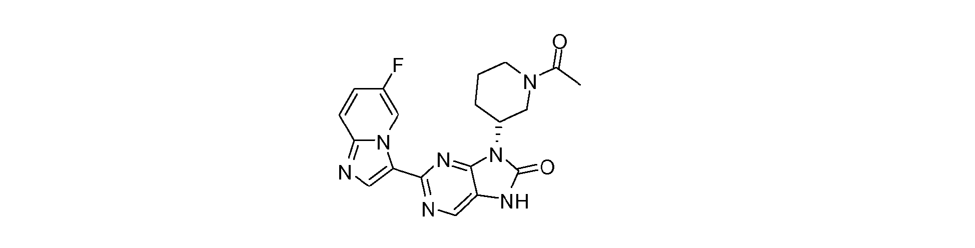

Obtained as a white solid (46%) from (R)-9-(1-acetylpiperidin-3-yl)-2-(6-fluoroimidazo[1,2-a]pyridin-3-yl)-7-((2-(trimethylsilyl)ethoxy)methyl)-7H-purin-8(9H)-one (**Preparation 69**) following the experimental procedure as described in **Example 19** followed by purification of the crude product by flash chromatography (98:2 dichloromethane/methanol).
LRMS (m/z): 396 (M+1)⁺.
1H NMR (300 MHz, DMSO*-d*₆) δ ppm 1.89 (m, 2H), 2.00 - 2.10 (2 singlets due to presence of rotamers, 3H in total), 2.60 (m, 2H), 3.15 (m, 1H), 3.88 - 4.31 (m, 3H), 4.50 (m, 1H), 7.53 (m, 1H), 7.83 (dd, 1H), 8.37 (s, 1H), 8.51 (s, 1H), 9.93 (dd, 1H).

### EXAMPLE 64

### 3-[2-Oxo-3-(tetrahydro-2H-pyran-4-yl)-2,3-dihydro-1H-imidazo[4,5-b]pyridin-5-yl]imidazo[1,2-a]pyridine-6-carbonitrile

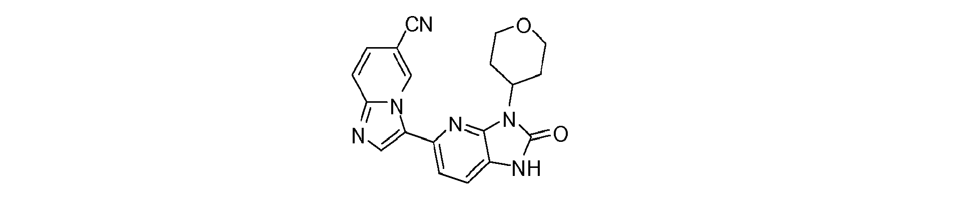

Obtained (69%) from 3-(2-oxo-3-(tetrahydro-2H-pyran-4-yl)-1-((2-(trimethylsilyl)ethoxy)methyl)-2,3-dihydro-1H-imidazo[4,5-b]pyridin-5-yl)imidazo[1,2-a]pyridine-6-carbonitrile (**Preparation 71**) following the experimental procedure as described in **Example 1**.
LRMS (m/z): 361 (M+1)⁺.
1H NMR (300 MHz, DMSO-d₆) δ ppm 1.90 (m, 2H), 3.23 (m, 2H), 4.12 (m, 2H), 4.67 (m, 2H), 5.39 (m, 1H), 7.59 (d, 1H), 7.78 (d, 1H), 7.90 (s, 1H), 8.06 (m, 1H), 8.64 (m, 1H), 10.36 (s, 1H), 11.55 (br s, 1H).

### EXAMPLE 65

### 3-[1-Methyl-2-oxo-3-(tetrahydro-2H-pyran-4-yl)-2,3-dihydro-1H-imidazo[4,5-b]pyridin-5-yl]imidazo[1,2-a]pyridine-6-carbonitrile

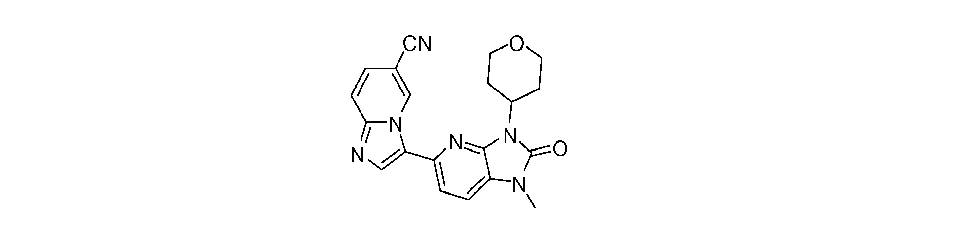

Obtained as a pale yellow solid (31%) from 5-chloro-1-methyl-3-(tetrahydro-2*H-*pyran-4-yl)-1,3-dihydro-2*H-*imidazo[4,5-b]pyridin-2-one (**Preparation 72**) and imidazo[1,2-a]pyridine-6-carbonitrile (**Preparation 1**) following the experimental procedure as described in **Preparation 4.**
LRMS (m/z): 375 (M+1)⁺.
1H NMR (300 MHz, DMSO-d₆) δ ppm 1.85 (m, 2H), 2.65 (m, 2H), 3.46 (s, 3H), 3.59 (m, 2H), 4.08 (m, 2H), 4.66 (m, 1H), 7.77-7.87 (m, 3H), 8.00 (d, 1H), 9.60 (s, 1H), 10.33 (s, 1H).

### EXAMPLE 66

### 5-(6-Fluoroimidazo[1,2-a]pyridin-3-yl)-3-(tetrahydro-2H-pyran-4-yl)-1,3-dihydro-2H-imidazo[4,5-b]pyridin-2-one

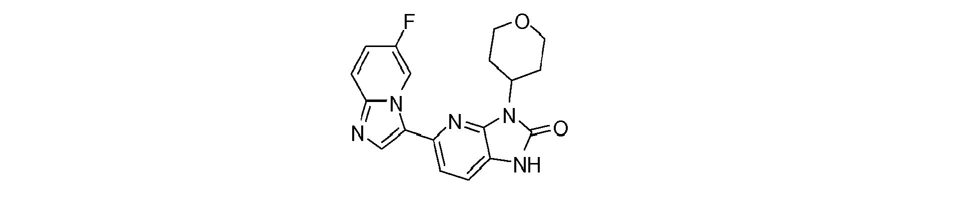

Obtained from 5-(6-fluoroimidazo[1,2-a]pyridin-3-yl)-3-(tetrahydro-2H-pyran-4-yl)-1-((2-(trimethylsilyl)ethoxy)methyl)-1H-imidazo[4,5-b]pyridin-2(3H)-one (**Preparation 73**) following the experimental procedure as described in **Example 19.**
LRMS (m/z): 354 (M+1)⁺.
1H NMR (300 MHz, DMSO*-d*₆) δ ppm 1.77 (d, 2H), 2.54 - 2.75 (m, 2H), 3.48 (t, 2H), 4.02 (dd, 2H), 4.43 - 4.65 (m, 1H), 7.37 - 7.52 (m, 2H), 7.60 - 7.69 (m, 1H), 7.78 (dd, 1H), 8.30 (s, 1H), 9.74 (d, 1H).

### EXAMPLE 67

### 5-(6-Fluoroimidazo[1,2-a]pyridin-3-yl)-1-methyl-3-(tetrahydro-2H-pyran-4-yl)-1,3-dihydro-2H-imidazo[4,5-b]pyridin-2-one

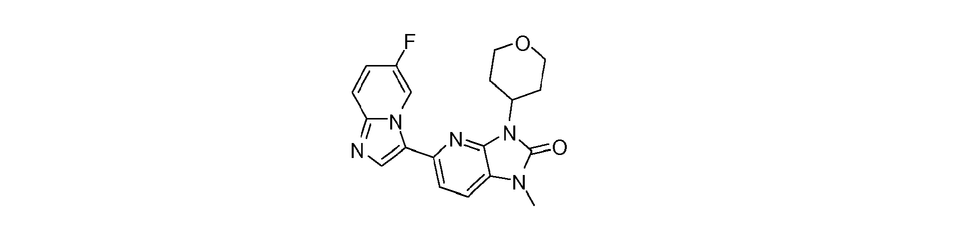

Obtained (32%) from 5-chloro-1-methyl-3-(tetrahydro-2*H-*pyran-4-yl)-1,3-dihydro-2*H-*imidazo[4,5-b]pyridin-2-one **(Preparation 72)** and 6-fluoroimidazo[1,2*-a*]pyridine (**Preparation 21**) following the experimental procedure as described in **Preparation 4.**
LRMS (m/z): 368 (M+1)⁺.
1H NMR (300 MHz, CDCl₃) δ ppm 1.85 (dd, 2H), 2.87 (qd, 2H), 3.49 (s, 3H), 3.60 (t, 2H), 4.20 (dd, 2H), 4.62 - 4.82 (m, 1H), 7.16 - 7.34 (m, 2H), 7.51 (d, 1H), 7.68 (dd, 1H), 8.10 (s, 1H), 9.70 (dd, 1H).

### EXAMPLE 68

### 5-(6-Fluoroimidazo[1,2-a]pyridin-3-yl)-1-(2-hydroxyethyl)-3-(tetrahydro-2H-pyran-4-yl)-1,3-dihydro-2H-imidazo[4,5-b]pyridin-2-one

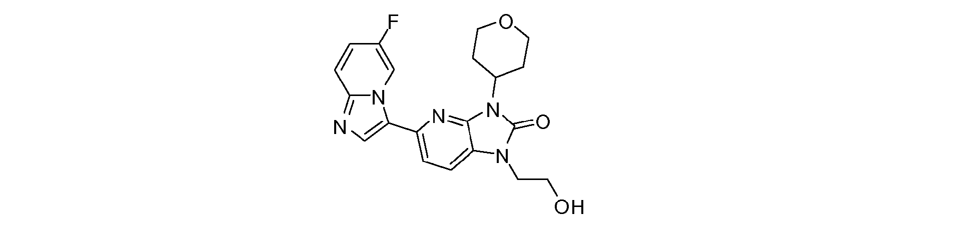

Obtained (13%) from 5-chloro-3-(tetrahydro-2*H-*pyran-4-yl)-1-[2-(tetrahydro-2*H-*pyran-2-yloxy)ethyl]-1,3-dihydro-2*H-*imidazo[4,5-*b*]pyridin-2-one (**Preparation 74**) and 6-fluoroimidazo[1,2*-a*]pyridine (**Preparation 21**) following the experimental procedure as described in **Example 9.**
LRMS (m/z): 398 (M+1)⁺.
1H NMR (300 MHz, DMSO-d₆) δ ppm 1.86 (dd, 2H), 2.68 (dq, 2H), 3.20-3.46 (m, 4H), 3.99-4.07 (m, 4H), 4.65 (m, 1H), 7.51 (m, 1H), 7.75-7.85 (m, 3H), 8.38 (m, 1H), 9.79 (m, 1H).

### EXAMPLE 69

### tert-Butyl 4-[5-(6-fluoroimidazo[1,2-a]pyridin-3-yl)-2-oxo-1,2-dihydro-3H-imidazo[4,5-b]pyridin-3-yl]piperidine-1-carboxylate

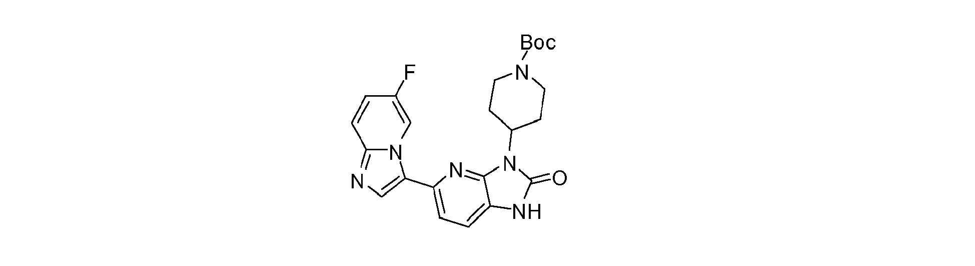

Obtained as a white solid (84%) from *tert*-butyl 4-(5-(6-fluoroimidazo[1,2-a]pyridin-3-yl)-2-oxo-1-((2-(trimethylsilyl)ethoxy)methyl)-1H-imidazo[4,5-b]pyridin-3(2H)-yl)piperidine-1-carboxylate (**Preparation 76**) following the experimental procedure as described in **Example 19** followed by purification of the crude product by flash chromatography (98:2 to 95:5 dichloromethane/methanol).
LRMS (m/z): 453 (M+1)⁺.
1H NMR (300 MHz, DMSO*-d*₆) δ ppm 1.39 (s, 9H), 1.84 (d, 2H), 2.32 - 2.60 (m, 2H), 2.76 - 3.02 (m, 2H), 4.16 (d, 2H), 4.32 - 4.61 (m, 1H), 4.32 - 4.56 (m, 1H), 7.45 (d, 2H), 7.62 (d, 1H), 7.76 (dd, 1H), 8.26 (s, 1H), 9.56 (dd, 1H).

### EXAMPLE 70

### 5-(6-Fluoroimidazo[1,2-a]pyridin-3-yl)-3-piperidin-4-yl-1,3-dihydro-2H-imidazo[4,5-b]pyridin-2-one

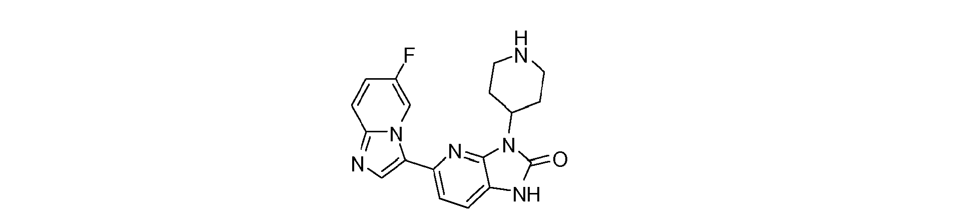

A suspension of *tert*-butyl 4-[5-(6-fluoroimidazo[1,2*-a*]pyridin-3-yl)-2-oxo-1,2-dihydro-3*H-*imidazo[4,5-*b*]pyridin-3-yl]piperidine-1-carboxylate (**Example 69**, 0.025 g, 0.06 mmol) in 5N hydrochloric acid (0.70 mL) was heated at 80 °C for 1 hour. The mixture was then partitioned between water and diethyl ether and the organic layer was separated. The aqueous layer was neutralized with solid sodium hydrogen carbonate and extracted with chloroform. The combined organic extract was dried (MgSO₄ and the solvent was evaporated to yield the title compound (0.015 g, 73%) as a beige solid.
LRMS (m/z): 353 (M+1)⁺.
1H NMR (300 MHz, DMSO-d₆) δ ppm 1.35 (m, 2H), 1.78 (m, 2H), 2.63 (m, 2H), 3.15 (m, 2H), 4.37 (m, 1H), 7.40-7.52 (m, 3H), 7.66 (m, 1H), 7.79 (m, 1H), 8.31 (s, 1H), 9.78 (m, 1H).

### EXAMPLE 71

### 5-(6-Fluoroimidazo[1,2-a]pyridin-3-yl)-3-[1-(methylsulfonyl)piperidin-4-yl]-1,3-dihydro-2H-imidazo[4,5-b]pyridin-2-one

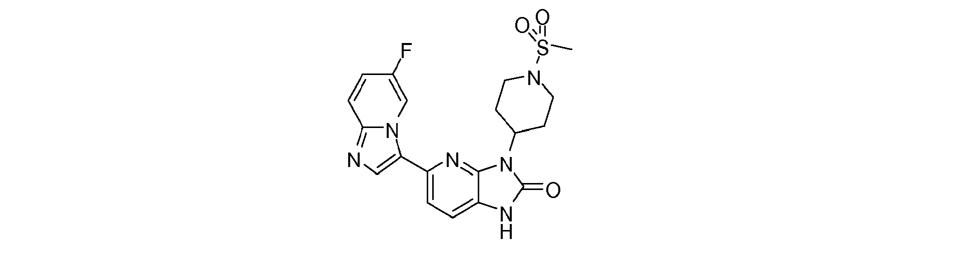

Obtained as a white solid (33%) from 5-(6-fluoroimidazo[1,2-a]pyridin-3-yl)-3-(1-(methylsulfonyl)piperidin-4-yl)-1-((2-(trimethylsilyl)ethoxy)methyl)-1H-imidazo[4,5-b]pyridin-2(3H)-one (**Preparation 77**) following the experimental procedure as described in **Example 19.**
LRMS (m/z): 431 (M+1)⁺.
1H NMR (300 MHz, DMSO*-d*₆) δ ppm 1.96 (dd, 2H), 2.63 (dd, 2H), 2.82 - 3.02 (m, 5H), 3.75 (d, 2H), 4.45 (td, 1H), 7.38 - 7.51 (m, 2H), 7.65 (d, 1H), 7.78 (dd, 1H), 8.29 (s, 1H), 9.64 (dd, 1H).

### EXAMPLE 72

### 3-(1-Acetylpiperidin-4-yl)-5-(6-fluoroimidazo[1,2-a]pyridin-3-yl)-1,3-dihydro-2H-imidazo[4,5-b]pyridin-2-one

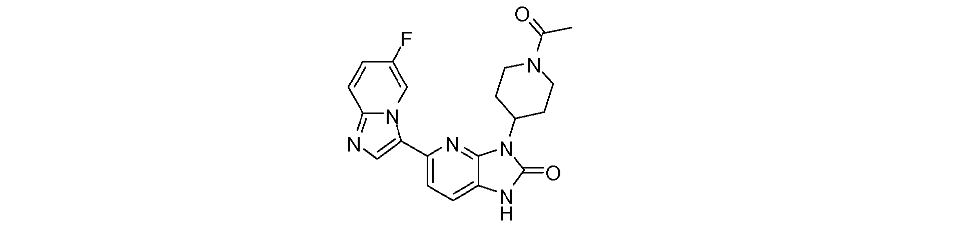

Obtained as a white solid (20%) from 3-(1-acetylpiperidin-4-yl)-5-(6-fluoroimidazo[1,2-a]pyridin-3-yl)-1-((2-(trimethylsilyl)ethoxy)methyl)-1H-imidazo[4,5-b]pyridin-2(3H)-one (**Preparation 78**) following the experimental procedure as described in **Example 19** followed by purification of the crude product by flash chromatography (100:8:1 dichloromethane/methanol/c. NH₄OH).
LRMS (m/z): 395 (M+1)⁺.
1H NMR (300 MHz, DMSO*-d*₆) δ ppm 1.75 - 1.98 (m, 2H), 2.13 (s, 3H), 2.22 - 2.44 (m, 1H), 2.56 - 2.74 (m, 2H), 3.95 - 4.19 (m, 2H), 4.42 - 4.73 (m, 2H), 7.41 - 7.52 (m, 2H), 7.66 (d, 1H), 7.77 (d, 1H), 8.30 (s, 1H), 9.63 (dd, 1H), 11.33 (br s, 1H).

### EXAMPLE 73

### 6-Fluoro-5-(6-fluoroimidazo[1,2-a]pyridin-3-yl)-3-(tetrahydro-2H-pyran-4-yl)-1,3-dihydro-2H-imidazo[4,5-b]pyridin-2-one

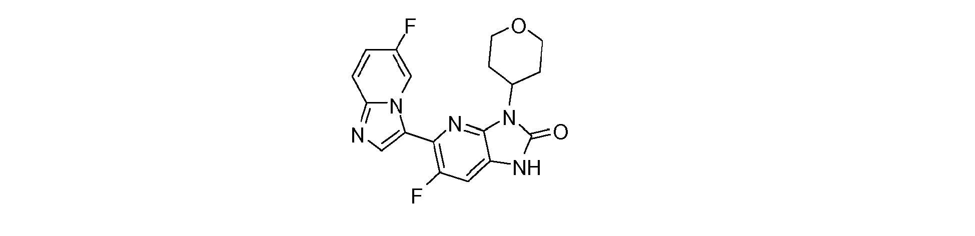

Obtained as a white solid (57%) from 6-fluoro-5-(6-fluoroimidazo[1,2-a]pyridin-3-yl)-3-(tetrahydro-2H-pyran-4-yl)-1-((2-(trimethylsilyl)ethoxy)methyl)-1H-imidazo[4,5-b]pyridin-2(3H)-one (**Preparation 80**) following the experimental procedure as described in **Example 19.**
LRMS (m/z): 372 (M+1)⁺.
1H NMR (300 MHz, DMSO*-d*₆) δ ppm 1.65 - 1.84 (m, 2H), 2.57 (dd, 2H), 3.41 - 3.55 (m, 2H), 4.00 (dd, 2H), 4.38 - 4.62 (m, 1H), 7.46 - 7.55 (m, 1H), 7.58 (d, 1H), 7.81 (dd, 1H), 8.12 (d, 1H), 9.59 (dd, 1H).

### EXAMPLE 74

### 6-(6-Fluoroimidazo[1,2-a]pyridin-3-yl)-1-(tetrahydro-2H-pyran-4-yl)-1,3-dihydro-2H-imidazo[4,5-b]pyrazin-2-one

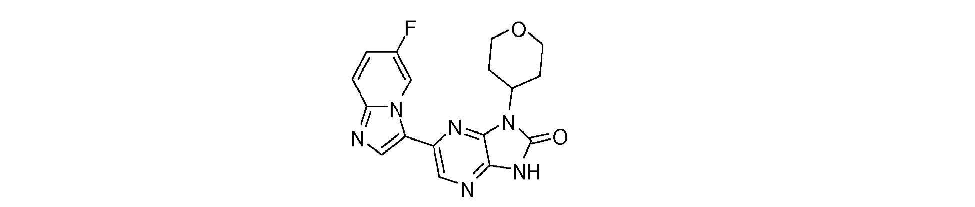

Obtained (18%) from 5-(6-fluoroimidazo[1,2-a]pyridin-3-yl)-3-(tetrahydro-2H-pyran-4-yl)-1-((2-(trimethylsilyl)ethoxy)methyl)-1H-imidazo[4,5-b]pyrazin-2(3H)-one (**Preparation 82**) following the experimental procedure as described in **Example 19** followed by purification of the crude product by reverse phase chromatography (C-18 silica from Waters©, water/acetonitrile/methanol as eluents [0.1% v/v formic acid buffered] 0% to 100%).
LRMS (m/z): 355 (M+1)⁺.
1H NMR (300 MHz, CDCl₃ + CD3OD) δ ppm 1.85 (dd, 2H), 2.72 (dq, 2H), 3.55 (t, 2H), 4.11 (dd, 2H), 4.62 (m, 1H), 7.26 (m, 1H), 7.64 (dd, 1H), 8.03 (s, 1H), 8.33 (s, 1H), 9.31 (dd, 1H).

### EXAMPLE 75

### 2-(2-Amino-6-fluoroimidazo[1,2-a]pyridin-3-yl)-9-(tetrahydro-2H-pyran-4-yl)-7H-purin-8(9H)-one

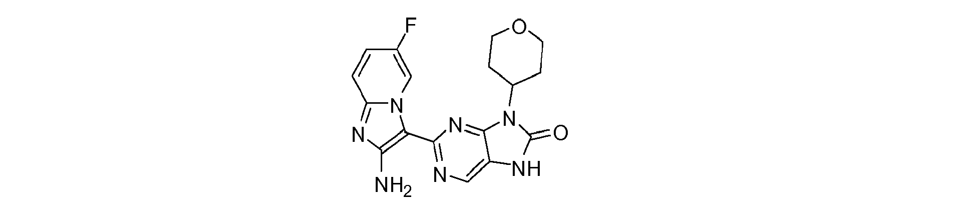

Obtained (63%) from 2-(2-amino-6-fluoroimidazo[1,2-a]pyridin-3-yl)-9-(tetrahydro-2H-pyran-4-yl)-7-((2-(trimethylsilyl)ethoxy)methyl)-7H-purin-8(9H)-one (**Preparation 84**) following the experimental procedure as described in **Example 19**. After the reaction was complete, the solvent was evaporated and water was added. The solid was filtered and, after washing with water, the crude product was dissolved in a mixture of methanol and 4M hydrogen chloride in 1,4-dioxane and then concentrated to give the bis-hydrochloride salt of the title compound.
LRMS (m/z): 370 (M+1)⁺.
1H NMR (300 MHz, DMSO*-d*₆) δ ppm 1.81 (d, 2H), 2.55 (m, 2H), 3.50 (t, 2H), 4.02 (m, 2H), 4.55 (m, 1H), 7.73 (m, 2H), 8.40 (s, 1H), 10.07 (m, 1H), 11.57 (s, 1H).

### EXAMPLE 76

### 2-(Imidazo[1,2-a]pyrazin-3-yl)-9-(tetrahydro-2H-pyran-4-yl)-7H-purin-8(9H)-one

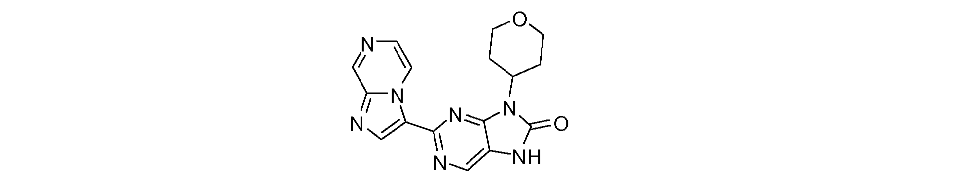

Obtained (79%) from 2-(imidazo[1,2-a]pyrazin-3-yl)-9-(tetrahydro-2H-pyran-4-yl)-7-((2-(trimethylsilyl)ethoxy)methyl)-7H-purin-8(9H)-one (**Preparation 85**) following the experimental procedure as described in **Example 19.** After the reaction was complete, the solvent was evaporated and water was added. The solid was filtered and washed several times with water and dried to give the title compound.
LRMS (m/z): 338 (M+1)⁺.
1H NMR (300 MHz, DMSO*-d*₆) δ ppm 1.78 (d, 2H), 2.63 (m, 2H), 3.54 (t ,2H), 4.06 (m, 2H), 4.58 (m, 1H), 8.17 (d, 1H), 8.43 (s, 1H), 8.56 (s, 1H), 9.26 (s, 1H), 9.71 (d, 1H), 11.63 (br s, 1H).

### EXAMPLE 77

### 2-Pyrazolo[1,5-a]pyrazin-3-yl-9-(tetrahydro-2H-pyran-4-yl)-7,9-dihydro-8H-purin-8-one

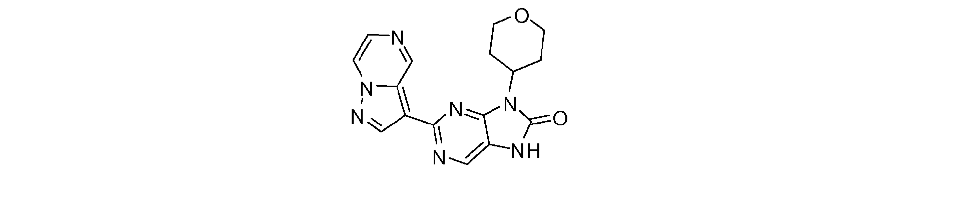

Obtained as a pale yellow solid (74%) from 2-(pyrazolo[1,5-a]pyrazin-3-yl)-9-(tetrahydro-2H-pyran-4-yl)-7-((2-(trimethylsilyl)ethoxy)methyl)-7H-purin-8(9H)-one (**Preparation 87**) following the experimental procedure described in **Example 19** followed by purification of the crude product by flash chromatography (95:5 dichloromethane/methanol).
LRMS (m/z): 338 (M+1)⁺.
1H NMR (300 MHz, CDCl₃) δ ppm 1.85 (d, 2H), 2.91 (dd, 2H), 3.52 - 3.71 (m, 2H), 4.16 - 4.31 (m, 2H), 4.58 - 4.81 (m, 1H), 8.02 (d, 1H), 8.36 (s, 1H), 8.45 (d, 1H), 8.76 (s, 1H), 10.03 (s, 1H).

### EXAMPLE 78

### 9-[(1R)-1-Phenylethyl]-2-pyrazolo[1,5-a]pyrazin-3-yl-7,9-dihydro-8H-purin-8-one

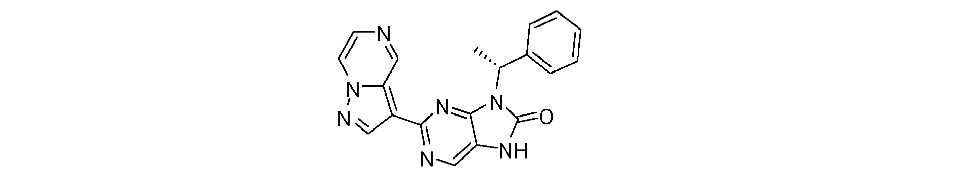

Obtained as a white solid (68%) from (R)-9-(1-phenylethyl)-2-(pyrazolo[1,5-a]pyrazin-3-yl)-7-((2-(trimethylsilyl)ethoxy)methyl)-7H-purin-8(9H)-one (**Preparation 89**) following the experimental procedure as described in **Example 19** followed by purification of the crude product by flash chromatography (98:2 to 95:5 dichloromethane/methanol).
LRMS (m/z): 358 (M+1)⁺.
1H NMR (300 MHz, DMSO*-d*₆) δ ppm 2.03 (d, 3H), 5.79 (q, 1H), 7.16 - 7.42 (m, 3H), 7.44 - 7.61 (m, 2H), 8.03 (d, 1H), 8.34 (s, 1H), 8.69 (s, 1H), 8.87 (dd, 1H), 9.68 (d, 1H).

### EXAMPLE 79

### (R)-9-(1-(5-Fluoropyridin-2-yl)ethyl)-2-(pyrazolo[1,5-a]pyrazin-3-yl)-7H-purin-8(9H)-one

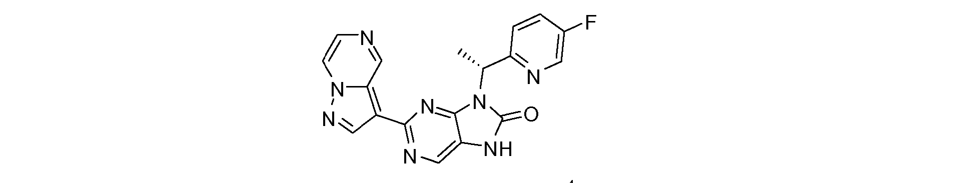

Obtained as a beige solid (64%) from (R)-N⁴-(1-(5-fluoropyridin-2-yl)ethyl)-2-(pyrazolo[1,5-a]pyrazin-3-yl)pyrimidine-4,5-diamine (**Preparation 91**) and 1,1'-carbonylbis-1*H-*imidazole following the experimental procedure as described in **Preparation 42d** followed by purification of the crude product by flash chromatography (9:1 dichloromethane/methanol).
LRMS (m/z): 377 (M+1)⁺.
1H NMR (300 MHz, DMSO*-d*₆) δ ppm 2.02 (d, 3H), 5.83 (q, 1H), 7.64 (dd, 1H), 7.75 (m, 1H), 8.34 (s, 1H), 8.01 (d, 1H), 8.51 (d, 1H), 8.61 (s, 1H), 8.85 (d, 1H), 9.55 (s, 1H), 11.54 (br s, 1H).

### EXAMPLE 80

### 2-(Pyrazolo[1,5-a]pyrazin-3-yl)-9-((tetrahydro-2H-pyran-4-yl)methyl)-7H-purin-8(9H)-one

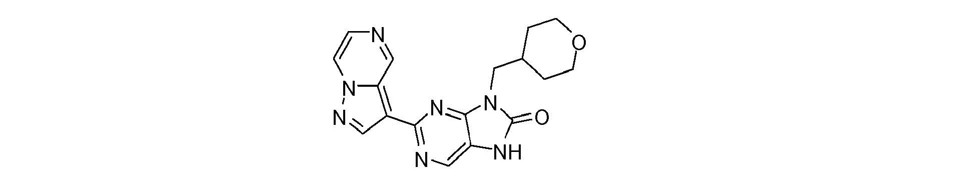

Obtained as a white solid (48%) from 2-(pyrazolo[1,5-a]pyrazin-3-yl)-N⁴-((tetrahydro-2H-pyran-4-yl)methyl)pyrimidine-4,5-diamine (**Preparation 92**) following the experimental procedure as described in **Preparation 42d** followed by purification of the crude product by flash chromatography (98:2 methanol/dichloromethane).
LRMS (m/z): 352 (M+1)⁺.
1H NMR (300 MHz, DMSO*-d*₆) δ ppm 1.34 (dq, 2H), 1.57 (d, 2H), 2.16 (m, 1H), 3.25 (t, 2H), 3.82 (m, 4H), 8.04 (d, 1H), 8.31 (s, 1H), 8.71 (s, 1H), 8.88 (d, 1H), 9.91 (s, 1H).

### EXAMPLE 81

### 9-(4,4-Difluorocyclohexyl)-2-(pyrazolo[1,5-a]pyrazin-3-yl)-7H-purin-8(9H)-one

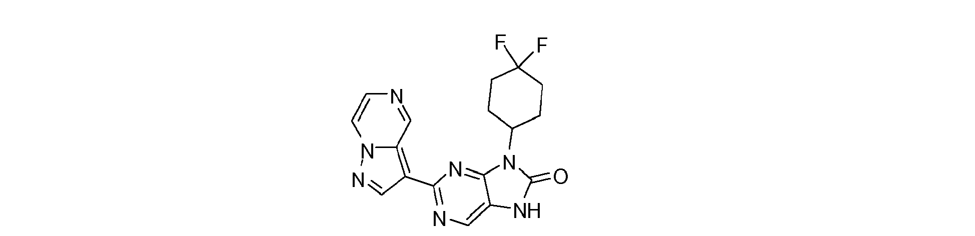

Obtained as a beige solid (65%) from N⁴-(4,4-difluorocyclohexyl)-2-(pyrazolo[1,5-a]pyrazin-3-yl)pyrimidine-4,5-diamine (**Preparation 93**) and 1,1'-carbonylbis-1*H-*imidazole following the experimental procedure as described in **Preparation 42d** followed by trituration of the crude product with diethyl ether.
LRMS (m/z): 372 (M+1)⁺.
1H NMR (300 MHz, DMSO*-d*₆) ppm 1.80 - 2.01 (m, 2H), 2.05 - 2.34 (m, 4H), 2.57 - 2.88 (m, 2H), 4.38 - 4.67 (m, 1H), 8.05 (d, 1H), 8.33 (s, 1H), 8.69 (s, 1H), 8.90 (d, 1H), 9.90 (s, 1H).

### EXAMPLE 82

### 9-(2,2-Dimethyltetrahydro-2H-pyran-4-yl)-2-(pyrazolo[1,5-a]pyrazin-3-yl)-7H-purin-8(9H)-one

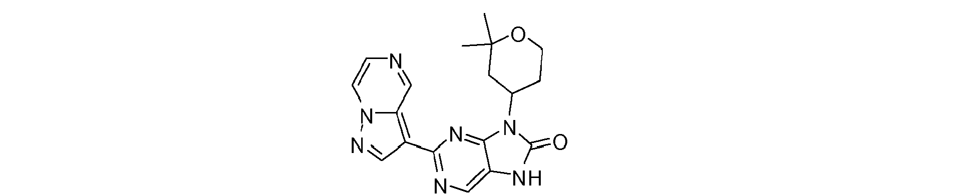

Obtained as an off-white solid (62%) from N⁴-(4,4-difluorocyclohexyl)-2-(pyrazolo[1,5-a]pyrazin-3-yl)pyrimidine-4,5-diamine (**Preparation 94**) and 1,1'-carbonylbis-1*H-*imidazole following the experimental procedure as described in **Preparation 42d** followed by purification of the crude product by flash chromatography (98:2 dichloromethane/methanol).
LRMS (m/z): 404 (M+1)⁺.
1H NMR (300 MHz, CDCl₃) ppm 1.40 (s, 3H), 1.42 (s, 3H), 1.82 (t, 2H), 2.60 - 3.02 (m, 2H), 3.74 - 4.12 (m, 2H), 4.77 - 4.99 (m, 1H), 8.02 (m, 1H), 8.38 (d, 1H), 8.46 (d, 1H), 8.75 (d, 1H), 9.03 (br s, 1H), 10.05 (s, 1H).

### EXAMPLE 83

### (R)-9-(8-Fluorochroman-4-yl)-2-(pyrazolo[1,5-a]pyrazin-3-yl)-7H-purin-8(9H)-one

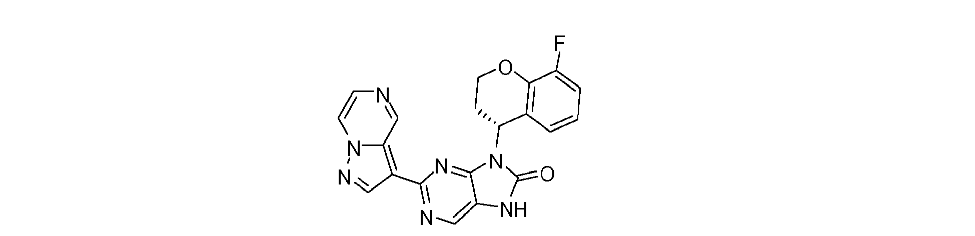

Obtained as an off-white solid (27%) from (R)-N⁴-(8-fluorochroman-4-yl)-2-(pyrazolo[1,5-a]pyrazin-3-yl)pyrimidine-4,5-diamine (**Preparation 95**) and 1,1'-carbonylbis-1*H-*imidazole following the experimental procedure as described in **Preparation 42d** followed by purification of the crude product by flash chromatography (98:2 to 95:5 dichloromethane/methanol).
LRMS (m/z): 366 (M+1)⁺.
1H NMR (300 MHz, CDCl₃) δ ppm 2.41 (m, 1H), 3.03 (m, 1H), 4.47 (t, 1H), 4.74 (m, 1H), 5.97 (t, 1H), 6.74 (m, 2H), 7.04 (m, 1H), 8.00 (d, 1H), 8.40 (m, 2H), 8.57 (s, 1H), 9.56 (s, 1H), 9.73 (s, 1H).

### EXAMPLE 84

### 2-((1r,4r)-4-(8-Oxo-2-(pyrazolo[1,5-a]pyrazin-3-yl)-7H-purin-9(8H)-yl)cyclohexyl)acetonitrile

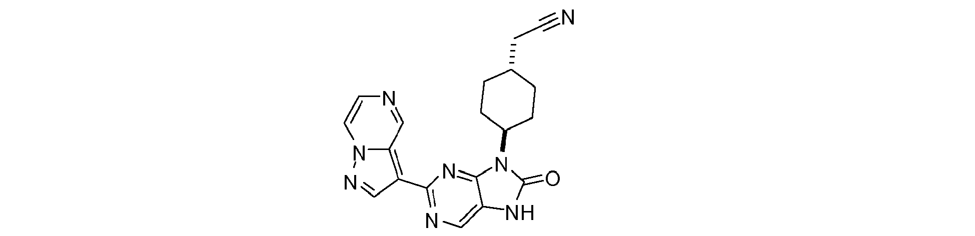

Obtained as a beige solid (70%) from 2-((1r,4r)-4-(5-amino-2-(pyrazolo[1,5-a]pyrazin-3-yl)pyrimidin-4-ylamino)cyclohexyl)acetonitrile (**Preparation 96**) and 1,1'-carbonylbis-1*H-*imidazole following the experimental procedure as described in **Preparation 42d.**
LRMS (m/z): 375 (M+1)⁺.
1H NMR (300 MHz, DMSO*-d*₆) δ ppm 1.20 -1.40 (m, 3H), 1.76 - 2.03 (m, 6H), 2.57 (d, 2H), 4.26 (m, 1H), 8.04 (d, 1H), 8.32 (s, 1H), 8.76 (s, 1H), 8.89 (dd, 1H), 9.89 (d, 1H), 11.45 (br s, 1H).

### EXAMPLE 85

### 2-((1r,4r)-4-(7-(2-Hydroxyethyl)-8-oxo-2-(pyrazolo[1,5-a]pyrazin-3-yl)-7H-purin-9(8H)-yl)cyclohexyl)acetonitrile

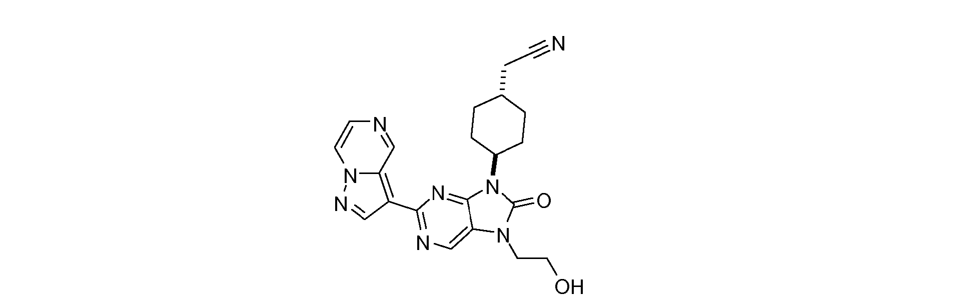

Obtained as a pale orange solid (70%) from 2-((1r,4r)-4-(8-Oxo-2-(pyrazolo[1,5-a]pyrazin-3-yl)-7H-purin-9(8H)-yl)cyclohexyl)acetonitrile (**Example 84**) and 2-bromoethanol following the experimental procedure as described in **Example 15a** followed by purification of the crude product by flash chromatography (98:2 to 95:5 dichloromethane/methanol).
LRMS (m/z): 419 (M+1)⁺.
1H NMR (300 MHz, DMSO*-d*₆) δ ppm 1.20-1.42 (m, 3H), 1.76-2.04 (m, 6H), 2.58 (d, 2H), 3.70 (m, 2H), 3.95 (t, 2H), 4.31 (m, 1H), 4.97 (t, 1H), 8.05 (d, 1H), 8.54 (s, 1H), 8.78 (s, 1H), 8.91 (d, 1H), 9.90 (s, 1H).

### EXAMPLE 86

### 5-(Pyrazolo[1,5-a]pyrazin-3-yl)-3-(tetrahydro-2H-pyran-4-yl)-1H-imidazo[4,5-b]pyridin-2(3H)-one

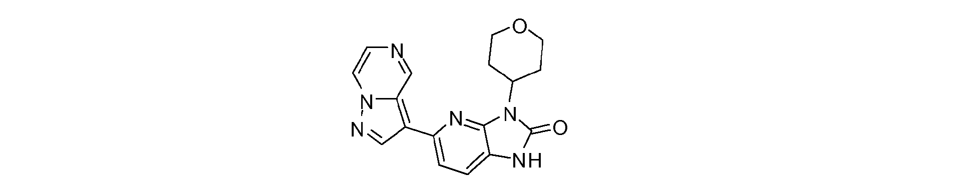

Obtained as yellow solid (35%) from 5-(pyrazolo[1,5-a]pyrazin-3-yl)-3-(tetrahydro-2H-pyran-4-yl)-1-((2-(trimethylsilyl)ethoxy)methyl)-1H-imidazo[4,5-b]pyridin-2(3H)-one **(Preparation 98)** following the experimental procedure as described in **Example 19.**
LRMS (m/z): 337 (M+1)⁺.
1H NMR (300 MHz, DMSO*-d*₆) ppm 1.73 (dd, 2H), 2.71 (dq, 2H), 3.50 (t, 2H), 4.04 (m, 2H), 4.56 (m, 1H), 7.40 (d, 1H), 7.65 (d, 1H), 7.97 (d, 1H), 8.76 (s, 1H), 8.82 (dd, 1H), 9.90 (s, 1H), 11.23 (br s, 1H).

### EXAMPLE 87

### 6-Fluoro-5-pyrazolo[1,5-a]pyrazin-3-yl-3-(tetrahydro-2H-pyran-4-yl)-1,3-dihydro-2H-imidazo[4,5-b]pyridin-2-one

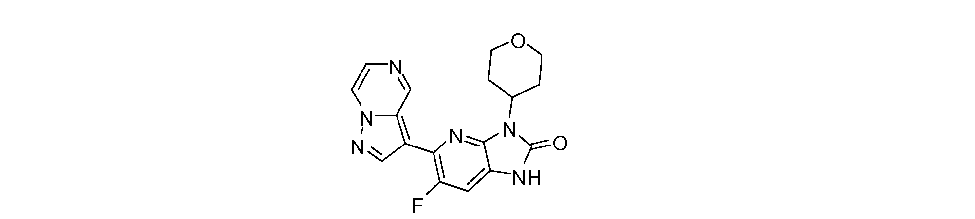

Obtained as yellow solid (31%) from 6-fluoro-5-(pyrazolo[1,5-a]pyrazin-3-yl)-3-(tetrahydro-2H-pyran-4-yl)-1-((2-(trimethylsilyl)ethoxy)methyl)-1H-imidazo[4,5-b]pyridin-2(3H)-one (**Preparation 99**) following the experimental procedure as described in **Example 19** followed by purification of the crude product by flash chromatography (98:2 to 95:5 dichloromethane/methanol).
LRMS (m/z): 355 (M+1)⁺.
1H NMR (300 MHz, DMSO-d₆) δ ppm 1.77 (m, 2H), 2.65 (m, 2H), 3.50 (m, 2H), 4.03 (m, 2H), 4.55 (m, 1H), 7.50 (d, 1H), 8.04 (d, 1H), 8.57 (s, 1H), 8.88 (d, 1H), 9.89 (s, 1H), 11.47 (s, 1H).

### EXAMPLE 88

### 2-(Pyrazolo[1,5-a]pyridin-3-yl)-9-(tetrahydro-2H-pyran-4-yl)-7H-purin-8(9H)-one

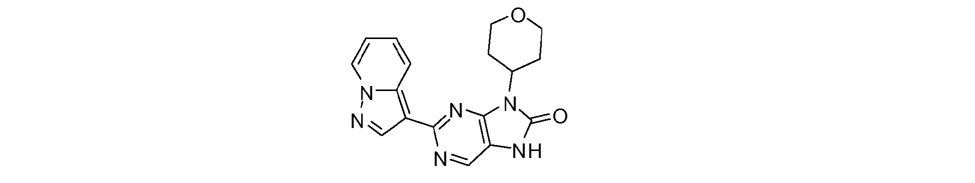

Obtained as yellow solid (42%) from 2-(pyrazolo[1,5-a]pyridin-3-yl)-9-(tetrahydro-2H-pyran-4-yl)-7-((2-(trimethylsilyl)ethoxy)methyl)-7H-purin-8(9H)-one (**Preparation 101**) following the experimental procedure as described in **Example 19**. The crude product was washed with dichloromethane.
LRMS (m/z): 337 (M+1)⁺.
1H NMR (300 MHz, DMSO*-d*₆) δ ppm 1.75 (dd, 2H), 2.56 - 2.82 (m, 2H), 3.51 (t, 2H), 4.05 (dd, 2H), 4.53 (tt, 1H), 7.05 (td, 1H), 7.47 (ddd, 1H), 8.28 (s, 1H), 8.54 (d, 1H), 8.60 (s, 1H), 8.80 (d, 1H).

### EXAMPLE 89

### 2-(6-Fluoropyrazolo[1,5-a]pyridin-3-yl)-9-(tetrahydro-2H-pyran-4-yl)-7H-purin-8(9H)-one

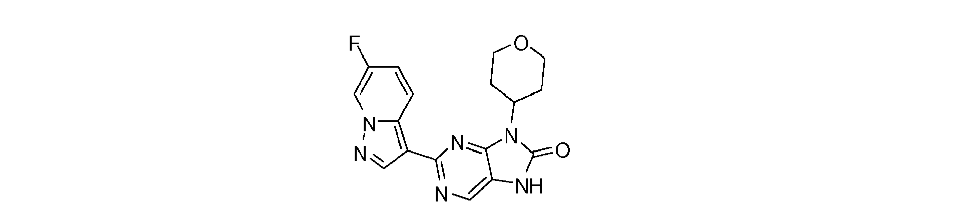

Obtained as yellow solid (48%) from 2-(6-fluoropyrazolo[1,5-a]pyridin-3-yl)-9-(tetrahydro-2H-pyran-4-yl)-7-((2-(trimethylsilyl)ethoxy)methyl)-7H-purin-8(9H)-one (**Preparation 104**) following the experimental procedure as described in **Example 19.** The crude product was washed with diethyl ether.
LRMS (m/z): 355 (M+1)⁺.
1H NMR (300 MHz, DMSO-d₆) δ ppm 1.74 (dd, 2H), 2.64 (dd, 2H), 3.47 - 3.58 (m, 2H), 4.05 (dd, 2H), 4.38 - 4.64 (m, 1H), 7.54 - 7.67 (m, 1H), 8.28 (s, 1H), 8.56 (dd, 1H), 8.62 (s, 1H), 9.14 (dd, 1H).

### EXAMPLE 90

### 2-(4-Fluoropyrazolo[1,5-a]pyridin-3-yl)-9-(tetrahydro-2H-pyran-4-yl)-7H-purin-8(9H)-one

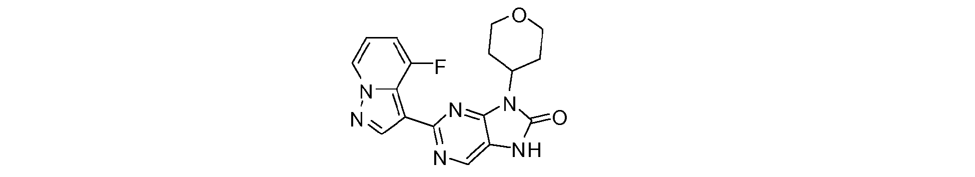

Obtained as yellow solid (42%) from 2-(4-fluoropyrazolo[1,5-a]pyridin-3-yl)-9-(tetrahydro-2H-pyran-4-yl)-7-((2-(trimethylsilyl)ethoxy)methyl)-7H-purin-8(9H)-one (**Preparation 106**) following the experimental procedure as described in **Example 19** followed by purification of the crude product by reverse phase chromatography (C-18 silica from Waters©, water/acetonitrile/methanol as eluents [0.1% v/v formic acid buffered] 0% to 100%).
LRMS (m/z): 355 (M+1)⁺.
1H NMR (300 MHz, CDCl₃) δ ppm 1.78 (d, 2H), 2.76 - 3.02 (m, 2H), 3.58 (t, 2H), 4.03 - 4.28 (m, 2H), 4.63 (t, 1H), 6.72 - 6.91 (m, 1H), 7.02 (t, 1H), 7.28 (s, 1H), 8.30 (s, 1H), 8.39 (d, 1H), 8.60 (s, 1H).

### EXAMPLE 91

### 2-(Pyrazolo[1,5-a]pyrimidin-3-yl)-9-(tetrahydro-2H-pyran-4-yl)-7H-purin-8(9H)-one

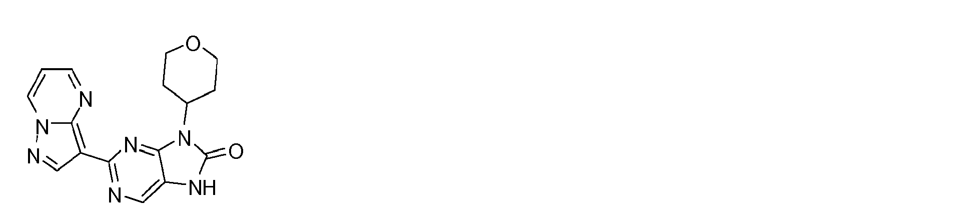

Obtained as yellow solid (20%) from 2-(pyrazolo[1,5-a]pyrimidin-3-yl)-9-(tetrahydro-2H-pyran-4-yl)-7-((2-(trimethylsilyl)ethoxy)methyl)-7H-purin-8(9H)-one (**Preparation 108**) following the experimental procedure as described in **Example 19** followed by purification of the crude product by flash chromatography (98:2 to 95:5 dichloromethane/methanol).
LRMS (m/z): 338 (M+1)⁺.
1H NMR (300 MHz, DMSO*-d*₆) δ ppm 1.78 (d, 2H), 2.64 - 2.90 (m, 2H), 3.54 (t, 2H), 4.06 - 4.12 (m, 2H), 4.45 - 4.67 (m, 1H), 5.83 (s, 1H), 7.23 (dd, 1H), 8.38 (s, 1H), 8.82 (m, 2H), 9.28 (d, 1H).

### EXAMPLE 92

### 6-(Pyrazolo[1,5-a]pyrimidin-3-yl)-1-(tetrahydro-2H-pyran-4-yl)-1H-imidazo[4,5-c]pyridin-2(3H)-one

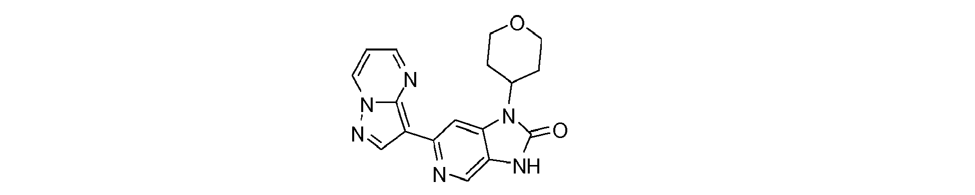

Triethylamine (0.042 mL, 0.3 mmol) and diphenylphosphoryl azide (0.040 mL, 0.19 mmol) were added to a suspension of crude 6-(pyrazolo[1,5-a]pyrimidin-3-yl)-4-(tetrahydro-2H-pyran-4-ylamino)nicotinic acid (**Preparation 109c**, ca. 0.15 mmol) in 1,4-dioxane (1.5 mL) and the mixture was stirred and heated to 110 °C. Further portions of triethylamine (0.042 mL, 0.3 mmol) and diphenylphosphoryl azide (0.040 mL, 0.19 mmol) were added after 2 hours and again after 7 hours and stirring was continued. After 24 hours, the solvent was evaporated and the residue was taken up with 2M aqueous sodium hydroxide solution (3 mL) and the resultant solution was washed with diethyl ether. The pH of the aqueous layer was adjusted to 6-7 with 2M aqueous hydrogen chloride solution and the mixture was extracted with dichloromethane. The organic extract was dried (MgSO₄ and evaporated and the residue was purified by ion-exchange chromatography using a Varian Bond Elute® SCX cartridge (eluting with dichloromethane/methanol to elute impurities followed by elution of the desired product with a 7M solution of ammonia in methanol) to give the title compound (0.030 g, 59%) as a yellow solid.
LRMS (m/z): 337 (M+1)⁺.
1H NMR (300 MHz, DMSO*-d*₆) δ ppm 1.80 (m, 2H), 2.46 - 2.64 (m, 2H), 3.59 (m, 2H), 4.17 (dd, 2H), 4.55 (m, 1H), 6.93 (dd, 1H), 8.26 (s, 1H), 8.37 (s, 1H), 8.66 (dd, 1H), 8.74 (dd, 1H), 8.77 (s, 1H).

### EXAMPLE 93

### 3-[8-Oxo-9-(tetrahydro-2H-pyran-4-yl)-8,9-dihydro-7H-purin-2-yl]imidazo[1,2-a]pyridine-6-carboxamide

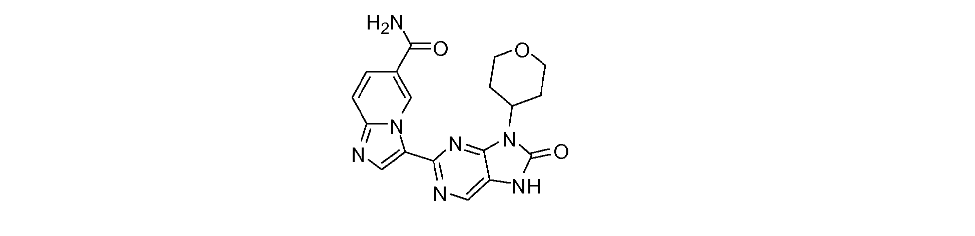

A mixture of 3-[7-(2,4-dimethoxybenzyl)-8-oxo-9-(tetrahydro-2*H-*pyran-4-yl)-8,9-dihydro-7*H-*purin-2-yl]imidazo[1,2*-a*]pyridine-6-carbonitrile (**Example 18**, 0.29 g, 0.57 mmol) and thioanisole (0.67 mL, 5.7 mmol) in trifluoroacetic acid (1.30 mL) was stirred and heated to 100 °C in a sealed tube. After 20 hours, water was added and the mixture was neutralized with solid sodium hydrogencarbonate. The resultant precipitate was filtered, washed with water and diethyl ether and dried to give the title compound (0.040 g, 20%) as a beige solid.
LRMS (m/z): 380 (M+1)⁺.
1H NMR (300 MHz, DMSO-d₆) δ ppm 1.80 (m, 2H), 2.57 (m, 2H), 3.45 (m, 2H), 4.03 (m, 2H), 4.60 (m, 1H), 7.85 (s, 1H), 7.98 (m, 1H), 8.11 (m, 1H), 8.35 (s, 1H), 8.45 (s, 1H), 8.68 (s, 1H), 10.54 (s, 1H), 11.67 (s, 1H).

### PHARMACOLOGICAL ACTIVITY

### In vitro JAK kinase Assays

Compounds were screened for their ability to inhibit JAK1, JAK2 , JAK3 and Tyk2 using the assays as indicated below.
The catalytic domains of human JAK1 (aa 850-1154), JAK2 (aa 826-1132), JAK3 (aa 795-1124) and Tyk2 (aa 871-1187) were expressed as N-terminal GST-fusion proteins using a baculovirus expression system and were purchased from Carna Biosciences. The enzymatic activity was assayed using as substrate a biotinylated peptide, poly (GT)-Biotin (CisBio). The peptide concentration in the reactions was 60 nM for JAK1, 20 nM for JAK2, 140 nM for JAK3 and 50 nM for Tyk2. The degree of phosphorylation was detected by TR-FRET (time-resolved fluorescence energy transfer).
IC₅₀S of compounds were measured for each kinase in a reaction mixture containing the enzyme, ATP and the peptide in 8 mM MOPS (pH 7.0), 10 mM MgCl₂, 0.05% β-mercaptoethanol, 0.45 mg/mL BSA. The ATP concentration in the reactions was 3 µM for JAK1, 0.2 µM for JAK2, 0.6 µM for JAK3 and 1.8 µM for Tyk2. The enzymatic reactions took place for 30 minutes at room temperature. Then, the reactions were stopped with 20 µL of quench detection buffer (50 mM HEPES, 0.5 M KF, EDTA 0.25 M, 0.1 % (w/v) BSA, pH 7.5) containing 0.115 µg/mL of anti-phosphoTyr (PT66)-Cryptate (CisBio) and a variable concentration of SA-XL665 (CisBio) to keep the SA-B ratio constant. Incubate for 3 h and read on Victor 2V spectrofluorometer (PerkinElmer) set to read fluorescence resonance energy transfer.

Some of the acronyms used above have the following meaning:
- AA:: aminoacids
- GST:: glutathione-S-transferase
- MOPS:: 3-(N-morpholino)propane sulfonic acid
- BSA:: bovine serum albumin
- ATP:: adenosine tri-phosphate
- EDTA:: ethylenediaminetetraacetic acid
- HEPES:: 4-(2-hydroxyethyl)-1-piperazineethanesulfonic acid

Table 1 depicts IC₅₀ values for certain exemplary compounds described in the invention. In Table 1, "A" represents an IC₅₀ value of less than 0.1 µM (100 nM), "B" represents an IC₅₀ value in the range of 0.1 µM (100 nM) to 1 µM, and C represents an IC₅₀ value higher than 1 µM.

**Table 1**

| Example No. | IC₅₀ JAK3 | IC₅₀ JAK2 | IC₅₀ JAK1 |
|---|---|---|---|
| | (µM) | (µM) | (µM) |
| 3 | A | A | B |
| 5 | A | B | C |
| 10 | A | B | C |
| 20 | A | A | C |
| 24 | A | A | A |
| 35 | B | A | B |
| 36 | B | A | C |
| 42 | B | B | C |
| 48 | A | A | B |
| 58 | B | B | C |
| 62 | B | A | C |
| 71 | A | A | C |
| 73 | A | A | B |
| 79 | A | A | A |
| 80 | B | B | B |
| 81 | A | A | A |
| 88 | A | A | A |
| 89 | A | A | C |
| 92 | B | A | B |

It can be seen from Table 1 that the compounds of formula (I) are potent inhibitors of JAK1, JAK2 and JAK3 kinases. Preferred heteroaryl imidazolone derivatives of the invention possess an IC₅₀ value for the inhibition of JAK1, JAK2 and JAK3 kinases (determined as defined above) of less than 1 nM, preferably less than 0.5 nM for each JAK kinase.

### Combinations

The heteroaryl imidazolone derivatives of the invention may also be combined with other active compounds in the treatment of a pathological condition or disease susceptible to amelioration by inhibition of Janus Kinases.

The combinations of the invention can optionally comprise of one or more additional active substances which are known to be useful in the treatment of myeloproliferative disorders (such as polycythemia vera, essential thrombocythemia or myelofibrosis), leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis, such as (a) Dihydrofolate reductase inhibitors, such as Methotrexate or CH-1504; (b) DHODH inhibitors such as leflunomide, teriflunomide, or the compounds described in the International Patent Application Nos. WO2008/077639 and WO2009021696; (c) Immunomodulators such as Glatiramer acetate (Copaxone), Laquinimod or Imiquimod; (d) Inhibitors of DNA synthesis and repair, such as Mitoxantrone or Cladribine; (e) Anti-alpha 4 integrin antibodies, such as Natalizumab (Tysabri); (f) Alpha 4 integrin antagonists such as R-1295, TBC-4746, CDP-323, ELND-002, Firategrast or TMC-2003; (g) Corticoids and glucocorticoids such as prednisone or methylprednisolone, fluticasone, mometasone, or beta-metasone; (h) Fumaric acid esters, such as *BG-12;* (i) Anti-TNF alpha antibodies, such as Infliximab, Adalimumab, or Certolizumab pegol; (j) Soluble TNF alpha receptors such as Ethanercept; (k) Anti-CD20 monoclonal antibodies such as Rituximab, Ocrelizumab Ofatumumab or TRU-015; (I) Anti-CD52 such as alemtuzumab; (m) Anti-CD25 such as daclizumab; (n) Anti-CD88, such as eculizumab or pexilizumab; (o) Anti-IL12R /IL23R, such as ustekinumab; (p) Calcineurin inhibitors such as cyclosporine A or tacrolimus; (q) IMPDH inhibitors, such as mycophenolate mophetyl; (r) Cannabinoid receptor agonists such as Sativex; (s) Chemokine CCR1 antagonists such as MLN-3897 or PS-031291; (t) Chemokine CCR2 antagonists such as INCB-8696; (u) NF-kappaB activation inhibitors such as MLN-0415; (v) S1 P receptor agonists such as fingolimod, BAF-312, ACT128800 or the compounds described in the International Patent Application Nos. PCT/EP2009/007348 and PCT/EP2009/008968; (w) S1 P liase inhibitors such as LX2931; (x) Syk inhibitors, such as R-112; (y) PKC inhibitors, such as NVP-AEB071; (z) M3 antagonists such as tiotropium or aclidinium; (aa) Long-acting beta adrenergic agonists such as salmeterol, formoterol or indacaterol; (bb) Vitamin D derivatives like calcipotriol (Daivonex); (cc) Phosphosdiesterase IV inhibitors such as roflumilast or GRC-4039; (dd) p38 Inhibitors such as ARRY-797; (ee) MEK inhibitors, such as ARRY-142886 or ARRY-438162; (ff) PI3Kδγ inhibitors; (gg) Interferons comprising Interferon beta 1a such as Avonex from Biogen Idec, CinnoVex from CinnaGen and Rebif from EMD Serono, and Interferon beta 1 b such as Betaferon from Schering and Betaseron from Berlex; and (hh) Interferon alpha such as Sumiferon MP.

Specific examples of suitable corticoids and glucocorticoids that can be combined with the JAK inhibitors of the present invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Specific examples of suitable Syk kinase inhibitors that can be combined with the JAK inhibitors of the present invention are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Examples of suitable M3 antagonists (anticholinergics) that can be combined with the JAK inhibitors of the present invention are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-1 04135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred..

Specific examples of suitable long-acting beta adrenergic agonists (β2-agonists) that can be combined with the JAK inhibitors of the present invention are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in international patent applications Nos. WO2007/124898, W02006/122788A1, WO2008/046598 and WO2008095720.

Specific examples of suitable Phosphosdiesterase IV inhibitors that can be combined with the JAK inhibitors of the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid (MK-0873), CDC-801, UK-500001, BLX-914, 2-carbomethoxy-4-cyano-4-(3-cyclopropylmethoxy-4-difluroromethoxyphenyl)cyclohexan1-one, cis [4-cyano-4-(3-cyclopropylmethoxy-4-difluoromethoxyphenyl)cyclohexan-1-ol, GRC-4039, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the International Patent Applications Nos. WO03/097613, WO2004/058729, WO 2005/049581, WO 2005/123693 and WO 2005/123692.

Examples of suitable PI3Kδγ inhibitors that can be combined with the JAK inhibitors of the present invention are 2-Methyl-2-[4-[3-methyl-2-oxo-8-(3-quinolinyl)-2,3-dihydro-1H-imidazo[4,5-c]quinolin-1-yl]phenyl]propanenitrile (BEZ-235 from Novartis), CAL-101 (from Calistoga Pharmaceuticals) and N-Ethyl-N'-[3-(3,4,5-trimethoxyphenylamino)pyrido[2,3-b]pyrazin-6-yl]thiourea (AEZS-126 from Aeterna Zentaris).

The compounds of formula (I) and the combinations of the invention may be used in the treatment of myeloproliferative disorders, leukemia, lymphoid malignancies and solid tumors; bone marrow and organ transplant rejection; immune-mediated diseases and inflammatory diseases, wherein the use of a JAK inhibitor is expected to have a beneficial effect, for example rheumatoid arthritis, multiple sclerosis, inflammatory bowel disease (such as ulcerative colitis or Crohn's disease), dry eye, uveitis, allergic conjunctivitis, allergic rhinitis, asthma, chronic obstructive pulmonary disease (COPD), atopic dermatitis and psoriasis.

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two active agents could be taken in the morning and the other(s) later in the day. Or in another scenario, one or two active agents could be taken twice daily and the other(s) once daily, either at the same time as one of the twice-a-day dosing occurred, or separately. Preferably at least two, and more preferably all, of the active agents would be taken together at the same time. Preferably, at least two, and more preferably all active agents would be administered as an admixture.

The invention is also directed to a combination product of the compounds of the invention together with one or more other therapeutic agents for use in the treatment of a pathological condition or disease susceptible to amelioration by inhibition of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

The invention also encompasses the use of a combination of the compounds of the invention together with one or more other therapeutic agents for the manufacture of a formulation or medicament for treating these diseases.

The invention also provides a method of treatment of a pathological condition or disease susceptible to amelioration by inhibition of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis; comprising administering a therapeutically effective amount of a combination of the compounds of the invention together with one or more other therapeutic agents.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the heteroaryl imidazolone derivatives of the invention, and the other optional active compounds may be administered together in the same pharmaceutical composition or in different compositions intended for separate, simultaneous, concomitant or sequential administration by the same or a different route.

One execution of the present invention consists of a kit of parts comprising of a heteroaryl imidazolone derivative of the invention together with instructions for simultaneous, concurrent, separate or sequential use in combination with another active compound useful in the treatment of myeloproliferative disorders, leukemia, lymphoid malignancies and solid tumors; bone marrow and organ transplant rejection; immune-mediated diseases and inflammatory diseases, more in particular useful in the treatment of rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

Another execution of the present invention consists of a package comprising of a heteroaryl imidazolone derivative of the invention and another active compound useful in the treatment of myeloproliferative disorders, leukemia, lymphoid malignancies and solid tumors; bone marrow and organ transplant rejection; immune-mediated diseases and inflammatory diseases, more in particular useful in the treatment of rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

### Pharmaceutical Compositions

Pharmaceutical compositions according to the present invention comprise the compounds of the invention in association with a pharmaceutically acceptable diluent or carrier.

The invention further provides pharmaceutical compositions comprising the compounds of the invention in association with a pharmaceutically acceptable diluent or carrier together with one or more other therapeutic agents for use in the treatment of a pathological condition or disease susceptible to amelioration by inhibition of Janus Kinases (JAK), such as the ones previously described.

The invention is also directed to pharmaceutical compositions of the invention for use in the treatment of a pathological condition or disease susceptible to amelioration by inhibition of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis. The invention also encompasses the use of a pharmaceutical composition of the invention for the manufacture of a medicament for treating these diseases.

The invention also provides a method of treatment of a pathological condition or disease susceptible to amelioration by inhibition of Janus Kinases (JAK), in particular wherein the pathological condition or disease is selected from myeloproliferative disorders, leukemia, lymphoid malignancies and solid tumors; bone marrow and organ transplant rejection; immune-mediated diseases and inflammatory diseases, more in particular wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis, comprising administering a therapeutically effective amount of a pharmaceutical composition of the invention.

The present invention also provides pharmaceutical compositions which comprise, as an active ingredient, at least an heteroaryl imidazolone of formula (I) or a pharmaceutically acceptable salt thereof in association with a pharmaceutically acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

The pharmaceutically acceptable excipients which are admixed with the active compound or salts of such compound, to form the compositions of this invention are well-known per se and the actual excipients used depend inter alia on the intended method of administering the compositions.

Compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 0.01-3000 mg, more preferably 0.5-1000 mg of active ingredient or the equivalent amount of a pharmaceutically acceptable salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Compositions for topical administration may take the form of ointments, creams or lotions, all containing the compound of the invention; such preparations may be made by methods well-known in the art.

Effective doses are normally in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical compositions may conveniently be presented in unit dosage form and may be prepared by any of the methods well known in the art of pharmacy.

The pharmaceutical compositions of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of, for example, gelatine or blisters of, for example, laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2 µg and 150 µg of each therapeutically active ingredient. Alternatively, the active ingredient (s) may be presented without excipients.

Packaging of the formulation for inhalation may be carried out by using suitable inhaler devices such as Genuair® (formerly Novolizer® SD2FL) which is described in the following patent applications: WO 97/000703, WO 03/000325 and WO 03/061742 and W02006/008027.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

The following preparations forms are cited as formulation examples:

### COMPOSITION EXAMPLE 1

50,000 capsules, each containing 100 mg of 2-(6-Fluoroimidazo[1,2*-a*]pyridin-3-yl)-9-[(1*R*)-1-phenylethyl]-7,9-dihydro-8*H-*purin-8-one (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 2-(6-Fluoroimidazo[1,2*-a*]pyridin-3-yl)-9-[(1*R*)-1-phenylethyl]-7,9-dihydro-8*H-*purin-8-one (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, or solvate, or N-oxide, or stereoisomer or deuterated derivative thereof: 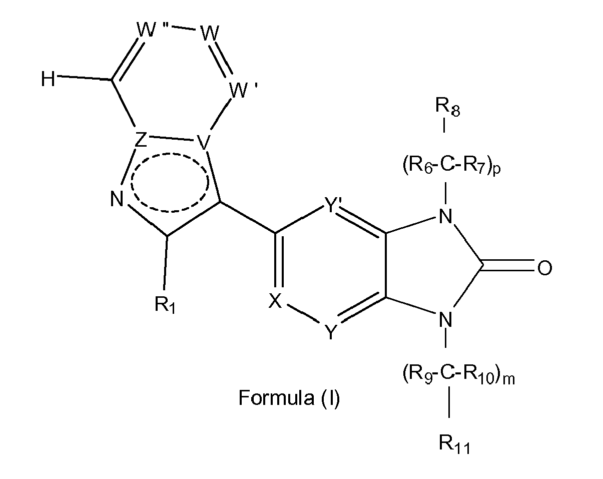 wherein
m is 0 or an integer from 1 to 3;
p is 0 or an integer from 1 to 3;
Z and V independently represent a nitrogen atom or a carbon atom, wherein at least one of Z and V represents a nitrogen atom, and the other represents a carbon atom;
W represents a nitrogen atom or a -CR₃ group;
W' represents a nitrogen atom or a -CR₂ group;
W" represents a nitrogen atom or a -CR₄ group;
X and Y independently represent a nitrogen atom or a -CR₅ group, wherein at least one of X and Y represents a -CR₅ group;
Y' represents a nitrogen atom or a -CR_{5'} group;
R₁, R₂, R₃, R₄ and R₅ independently represent a hydrogen atom, a halogen atom, a hydroxyl group, a cyano group, a linear or branched C₁₋C₆ alkyl group, a C₂₋C₄ alkenyl group, a C₂₋C₄ alkynyl group, a C₁₋C₄ haloalkyl group, a C₁₋C₄ hydroxyalkyl group, a C₃₋C₁₀ cycloalkyl group, a C₃₋C₁₀ cycloalkenyl group, a monocyclic or polycyclic C₅₋C₁₄ aryl group, a 5- to 14- membered heteroaryl group containing at least one heteroatom selected from O, S and N, a 5- to 14- membered heterocyclyl group containing at least one heteroatom selected from O, S and N, a bicyclyl group containing a monocyclic C₅₋C₉ aryl or heteroaryl group bonded directly to a 5- to 9- membered cycloalkyl or heterocyclyl group, said heteroaryl or heterocyclyl group containing at least one heteroatom selected from O, S and N, an aza-bicycloalkyl group having up to 12 carbon atoms or an aza-bicycloalkenyl group having up to 12 carbon atoms, a -(CH₂)_{q}SR₁₅ group, a -(CH₂)_{q}SOR₁₅ group, a -(CH₂)_{q}S(O)₂R₁₅ group, a -(CH₂)_{q}S(O)₂NR₁₅R₁₆ group, a -(CH₂)_{q}NR₁₅S(O)₂R₁₆ group, a -(CH₂)_{q}NR₁₅S(O)₂NR₁₆ group, a -(CH₂)_{q}OR₁₅ group, a -(CH₂)_{q}C(O)OR₁₅ group, a -(CH₂)_{q}O-C(O)R₁₅ group, a -(CH₂)_{q'}C(O)-(CH₂)_{q}-R₁₅ group, a -(CH₂)_{q}NR₁₅R₁₆ group, a -(CH₂)_{q}CH(R₁₅)NR₁₆R₁₇ group, a -(CH₂)_{q'}C(O)-(CH₂)_{q}-NR₁₅R₁₆ group, a -(CH₂)_{q},NR₁₅C(O)-(CH₂)_{q}-R₁₆ group or a -(CH₂)_{q'}NR₁₅C(O)-(CH₂)_{q}-NR₁₆R₁₇ group, wherein each q and q' are independently 0, 1 or 2,
wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from substituents Ra, and the alkyl groups are unsubstituted or substituted by one or more substituents selected from Rb;
R₅, represents a hydrogen atom, a halogen atom, a hydroxyl group, a cyano group, a linear or branched C₁₋C₆ alkyl group, a C₂₋C₄ alkenyl group, a C₂₋C₄ alkynyl group, a C₁₋C₄ haloalkyl group, a C₁₋C₄ hydroxyalkyl group or a C₃₋C₁₀ cycloalkyl group;
R₆, R₇, R₉ and R₁₀ each independently represent a hydrogen atom, a hydroxyl group, a C₁₋C₄ hydroxyalkyl group, a -(C₁₋C₄ alkyl)-O-(C₁₋C₄ alkyl) group or a linear or branched C₁₋C₆ alkyl group, wherein said alkyl group is optionally substituted by one or more substituents selected from a cyano group, a C₁₋C₄ haloalkyl group, a C₁₋C₄ hydroxyalkyl group, a C₁₋C₄ alkoxycarbonyl group, a C₃₋C₇ cycloalkyl group, a phenyl group or a 6 membered, saturated N-containing heterocyclyl ring;
R₈ and R₁₁ each independently represent a hydrogen atom, a halogen atom, a cyano group, a linear or branched C₁₋C₆ alkyl group, a C₂₋C₄ alkenyl group, a C₂₋C₄ alkynyl group, a C₁₋C₄ haloalkyl group, a C₁₋C₄ hydroxyalkyl group, a C₃₋C₁₀ cycloalkyl group, a C₃₋C₁₀ cycloalkenyl group, a monocyclic or polycyclic C₅₋C₁₄ aryl group, a 5- to 14-membered heteroaryl group containing at least one heteroatom selected from O, S and N, a 5- to 14- membered heterocyclyl group containing at least one heteroatom selected from O, S and N, a bicyclyl group containing a monocyclic C₅₋C₉ aryl or heteroaryl group fused to a 5- to 9- membered cycloalkyl or heterocyclyl group, said heteroaryl or heterocyclyl group containing at least one heteroatom selected from O, S and N, a bicyclyl group containing a C₃₋C₁₀ cycloalkyl group fused to a 5- to 9-membered heterocyclyl group containing at least one heteroatom selected from O, S and N, an aza-bicycloalkyl group having up to 12 carbon atoms or a aza-bicycloalkenyl group having up to 12 carbon atoms, a -(CH₂)ₙSR₁₅ group, a -(CH₂)ₙSOR₁₅ group, a -(CH₂)ₙS(O)₂R₁₅ group, a -(CH₂)ₙS(O)₂NR₁₅R₁₆ group, a -(CH₂)ₙNR₁₅S(O)₂R₁₆ group, a -(CH₂)ₙNR₁₅S(O)₂NR₁₆ group, a -(CH₂)ₙOR₁₅ group, a -(CH₂)ₙC(O)OR₁₅ group, a -(CH₂)ₙO-C(O)R₁₅ group, a -(CH₂)_{n'}C(O)-(CH₂)ₙ-R₁₅ group, a -(CH₂)_{n'}C(OH)(J)-(CH₂)ₙK group, a -(CH₂)ₙNR₁₅R₁₆ group, a -(CH₂)ₙCH(R₁₅)NR₁₆R₁₇ group, a -(CH₂)_{n'}C(O)-(CH₂)ₙ-NR₁₅R₁₆ group, a -(CH₂)_{n'}NR₁₅C(O)-(CH₂)ₙ-R₁₆ group or a -(CH₂)_{n'}NR₁₅C(O)-(CH₂)ₙ-NR₁₆R₁₇ group, wherein each n and n' are independently 0 or 1; and J represents a hydrogen atom or a methyl group, K represents a hydroxyl group, a methyl group or a -NR'R" group, and R' and R" each independently represent a hydrogen atom, a linear or branched C₁₋C₆ alkyl group, a C₁₋C₄ haloalkyl group or a C₁₋C₄ hydroxyalkyl group,
wherein the alkenyl, alkynyl, haloalkyl, hydroxyalkyl, cycloalkyl, cycloalkenyl, aryl, heteroaryl, heterocyclyl, bicyclyl, aza-bicycloalkyl and aza-bicycloalkenyl groups are unsubstituted or substituted by one or more substituents selected from Ra, -(C₁₋C₄ alkyl)-CN groups, or -(C₁₋C₄ alkyl)-C(O)N R'R" groups wherein R' and R" are the same or different and are selected from hydrogen atoms and linear or branched C₁₋C₄ alkyl groups; and the alkyl groups are unsubstituted or substituted by one or more substituents selected from Rb;
Ra is a halogen atom, a cyano group, a hydroxyl group, an oxo group, a linear or branched C₁₋C₆ alkyl group, a C₁₋C₄ haloalkyl group, C₁₋C₄ alkoxy group, a C₁₋C₄ hydroxyalkyl group, a C₃₋C₇ cycloalkyl or a C₃₋C₇ cycloalkenyl group unsubstituted or substituted by one or more substituents selected from substituents Re, a monocyclic or polycyclic C₅₋C₁₄ aryl group unsubstituted or substituted by one or more substituents selected from substituents Re, a 5- to 14- membered heteroaryl group containing at least one heteroatom selected from O, S and N unsubstituted or substituted by one or more substituents selected from substituents Re, a 5- to 14- membered heterocyclyl group containing at least one heteroatom selected from O, S and N unsubstituted or substituted by one or more substituents selected from substituents Re, a -(CH₂)_{q}SR₁₂ group, a -(CH₂)_{q}SOR₁₂ group, a -(CH₂)_{q}S(O)₂R₁₂ group, a -(CH₂)_{q}S(O)₂NR₁₂R₁₃ group, a -(CH₂)_{q}NR₁₂S(O)₂R₁₃ group, a -(CH₂)_{q}NR₁₂S(O)₂NR₁₃ group, a -(CH₂)_{q}OR₁₂ group, a -(CH₂)_{q}C(O)OR₁₂ group, a -(CH₂)_{q}O-C(O)R₁₂ group, a -(CH₂)_{q'}C(O)-(CH₂)_{q}-R₁₂ group, a -(CH₂)_{q}NR₁₂R₁₃ group, a -(CH₂)_{q}CH(R₁₂)NR₁₃R₁₄ group, a -(CH₂)_{q'}C(O)-(CH₂)_{q}-NR₁₂R₁₃ group, a -(CH₂)_{q'}NR₁₂C(O)-(CH₂)_{q}-R₁₃ group or a -NR₁₂C(O)-(CH₂)_{q}-NR₁₃R₁₄ group, wherein each q and q' are independently 0, 1 or 2;
Rb is a cyano group, a C₁₋C₄ haloalkyl group, C₁₋C₄ alkoxy group, a C₁₋C₄ hydroxyalkyl group, a C₃₋C₇ cycloalkyl or a C₃₋C₇ cycloalkenyl group unsubstituted or substituted by one or more substituents selected from substituents Re, a monocyclic or polycyclic C₅₋C₁₄ aryl group unsubstituted or substituted by one or more substituents selected from substituents Re, a 5- to 14- membered heteroaryl group containing at least one heteroatom selected from O, S and N unsubstituted or substituted by one or more substituents selected from substituents Re, a 5- to 14- membered heterocyclyl group containing at least one heteroatom selected from O, S and N unsubstituted or substituted by one or more substituents selected from substituents Re, a -(CH₂)_{q}SR₁₂ group, a -(CH₂)_{q}SOR₁₂ group, a -(CH₂)_{q}S(O)₂R₁₂ group, a -(CH₂)_{q}S(O)₂NR₁₂R₁₃ group, a -(CH₂)_{q}NR₁₂S(O)₂R₁₃ group, a -(CH₂)_{q}NR₁₂S(O)₂NR₁₃ group, a -(CH₂)_{q}OR₁₂ group, a -(CH₂)_{q}C(O)OR₁₂ group, a -(CH₂)_{q}O-C(O)R₁₂ group, a -(CH₂)_{q'}C(O)-(CH₂)_{q}-R₁₂ group, a -(CH₂)_{q}NR₁₂R₁₃ group, a -(CH₂)_{q}CH(R₁₂)NR₁₃R₁₄ group, a -(CH₂)_{q'}C(O)-(CH₂)_{q}-NR₁₂R₁₃ group, a -(CH₂)_{q'}NR₁₂C(O)-(CH₂)_{q}-R₁₃ group or a -NR₁₂C(O)-(CH₂)_{q}-NR₁₃R₁₄ group, wherein each q and q' are independently 0, 1 or 2;
R₁₂, R₁₃ and R₁₄ each independently represents a hydrogen atom, a cyano group, a linear or branched C₁₋C₆ alkyl group, a C₁₋C₄ haloalkyl group, a C₁₋C₄ hydroxyalkyl group, a C₁₋C₄ alkoxycarbonyl group, a C₃₋C₇ cycloalkyl group, a phenyl group, a 5- to 6-membered monocyclic heteroaryl group containing 1, 2 or 3 heteroatoms selected from N, O and S, a 5- to 6- membered, heterocyclyl group containing 1, 2 or 3 nitrogen atoms, a bicyclyl group containing a monocyclic C₅₋C₆ aryl or heteroaryl group bonded directly to a 5- to 6- membered cycloalkyl or heterocyclyl group which heteroaryl or heterocyclyl group contains 1, 2 or 3 nitrogen atoms, the haloalkyl, hydroxyalkyl, alkoxycarbonyl, cycloalkyl, phenyl, heteroaryl, heterocyclyl and bicyclyl group being unsubstituted or substituted by one or more substituents selected from substituents Rc, and the alkyl groups being unsubstituted or substituted by one or more substituents selected from substituents Rd;
Rc is a halogen atom, a hydroxyl group, a cyano group, a linear or branched C₁₋C₆ alkyl group, a C₁₋C₄ haloalkyl group, a C₁₋C₄ alkoxy group, a C₁₋C₄ hydroxyalkyl group, a C₃₋C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃₋C₇ heterocycloalkyl ketone group containing 1, 2 or 3 nitrogen atoms, wherein said phenyl group is unsubstituted or substituted by one or more halogen atoms, and wherein said heteroaryl, heterocyclyl and heterocycloalkyl ketone groups are unsubstituted or substituted by one or more linear or branched C₁₋C₃ alkyl groups;
Rd is a cyano group, a C₁₋C₄ haloalkyl group, a C₁₋C₄ alkoxy group, a C₁₋C₄ hydroxyalkyl group, a C₃₋C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, or a C₃₋C₇ heterocycloalkyl ketone group containing 1, 2 or 3 nitrogen atoms, wherein said phenyl group is unsubstituted or substituted by one or more halogen atoms, and wherein said heteroaryl, heterocyclyl and heterocycloalkyl ketone groups are unsubstituted or substituted by one or more linear or branched C₁₋C₃ alkyl groups;
Re is a halogen atom, a hydroxyl group, a cyano group, a linear or branched C₁₋C₆ alkyl group or a C₁₋C₄ haloalkyl group;
R₁₅, R₁₆, and R₁₇ each independently represents a hydrogen atom, a cyano group, a linear or branched C₁₋C₆ alkyl group, a C₁₋C₄ haloalkyl group, a C₁₋C₄ hydroxyalkyl group, a C₁₋C₄ alkoxycarbonyl group, a C₃₋C₇ cycloalkyl group, a monocyclic or polycyclic C₅₋C₁₄ aryl group, a 5- to 14- membered heteroaryl group containing at least one heteroatom selected from O, S and N, or a 5- to 14- membered heterocyclyl group containing at least one heteroatom selected from O, S and N, wherein the haloalkyl, hydroxyalkyl, alkoxycarbonyl, cycloalkyl, aryl, heteroaryl and heterocyclyl groups are unsubstituted or substituted by one or more substituents selected from the substituents Ra, and the alkyl groups are unsubstituted or substituted by one or more substitutents selected from Rb;
provided that the compound of formula (1) does not carry a -(CH₂)ₙSR₁₅ group, a - (CH₂)ₙSOR₁₅ group, a -(CH₂)ₙS(O)₂R₁₅ group, a -(CH₂)ₙ S(O)₂NR₁₅R₁₆ group, a - (CH₂)ₙNR₁₅S(O)₂R₁₆ group, a -(CH₂)ₙNR₁₅S(O)₂NR₁₆ group, a -(CH₂)ₙOR₁₅ group, a - (CH2)ₙC(O)OR₁₅ group,
a -(CH₂)ₙO-C(O)R₁₅ group, a -(CH₂)_{n'}C(O)-(CH₂)ₙ-R₁₅ group, a -(CH₂)ₙNR₁₅R₁₆ group, a -(CH₂)ₙCH(R₁₅)NR₁₆R₁₇ group, a -(CH₂)_{n'}C(O)-(CH₂)ₙ-NR₁₅R₁₆ group,
a -(CH₂)_{n'}NR₁₅C(O)-(CH₂)ₙ-R₁₆ group or a -(CH₂)_{n'}NR₁₅C(O)-(CH₂)ₙ-NR₁₆R₁₇ group bonded directly to an imidazolone nitrogen atom.

2. A compound according to claim 1, wherein
R₁ represents a hydrogen atom, a halogen atom, a hydroxyl group, a cyano group, a linear or branched C₁₋C₆ alkyl group, a C₁₋C₄ haloalkyl group, a C₁₋C₄ hydroxyalkyl group, a C₃₋C₁₀ cycloalkyl group or a -(CH₂)ₙNR'R" group; wherein n is 0 or 1, and R' and R" are the same or different and each represents a hydrogen atom, a linear or branched C₁₋C₆ alkyl group, a C₁₋C₄ haloalkyl group or C₁₋C₄ hydroxyalkyl group;
R₂ and R₄ are the same or different and each represent a hydrogen atom, a halogen atom, a hydroxyl group, a cyano group, a linear or branched C₁₋C₆ alkyl group, a C₁₋C₄ haloalkyl group, a C₁₋C₄ hydroxyalkyl group, or a C₃₋C₁₀ cycloalkyl group;
R₃ represents a hydrogen atom, a halogen atom, a hydroxyl group, a cyano group, a linear or branched C₁₋C₆ alkyl group, a C₁₋C₄ haloalkyl group, a C₁₋C₄ hydroxyalkyl group, a C₃₋C₁₀ cycloalkyl group, a -(CH₂)_{q'}C(O)-(CH₂)_{q}-R group or a -(CH₂)_{q'}C(O)-(CH₂)_{q}-NR'R" group, wherein each q and q' are independently 0, 1 or 2, R represents a hydrogen atom, or a linear or branched C₁₋C₆ alkyl group, a C₁₋C₄ haloalkyl group, a C₁₋C₄ hydroxyalkyl group, or a cyano group and R' and R" are the same or different and each represents a hydrogen atom, a linear or branched C₁₋C₆ alkyl group, a C₁₋C₄ haloalkyl group, or C₁₋C₄ hydroxyalkyl group;
R₅ represents a hydrogen atom, a halogen atom, a hydroxyl group, a cyano group, a linear or branched C₁₋C₆ alkyl group, a C₁₋C₄ haloalkyl group, a C₁₋C₄ hydroxyalkyl group, a C₃₋C₁₀ cycloalkyl group, a 5- to 10- membered heterocyclyl group, a C₆₋C₁₀ aryl group, or a 5- to 10- membered heteroaryl group, wherein said heterocyclyl, aryl and heteroaryl groups are unsubstituted or substituted by one or more substituents selected from a halogen atom, a linear or branched C₁₋C₆ alkyl group, a cyano group, a hydroxyl group or a C₁₋C₄ alkoxy group;
R₅, represents a hydrogen atom, a halogen atom, a hydroxyl group, a cyano group, a linear or branched C₁-C₆ alkyl group, a C₁-C₄ haloalkyl group, a C₁-C₄ hydroxyalkyl group or a C₃-C₇ cycloalkyl group;
R₆, R₇, R₉ and R₁₀ are the same or different and each represent a hydrogen atom, a C₁-C₄ haloalkyl group, a C₁-C₄ hydroxyalkyl group, a -(C₁-C₄ alkyl)-O-(C₁-C₄ alkyl) group or a linear or branched C₁-C₆ alkyl group;
R₈ and R₁₁ each independently represent a hydrogen atom, a linear or branched C₁-C₆ alkyl group, a C₁-C₄ haloalkyl group, a C₁-C₄ hydroxyalkyl group, a C₃-C₁₀ cycloalkyl group, a C₃-C₁₀ cycloalkenyl group, a C₆-C₁₀ aryl group, a 5- to 10- membered heteroaryl group containing 1, 2 or 3 heteroatoms selected from N, O and S, a 5- to 10-membered heterocyclyl group containing 1, 2 or 3 heteroatoms selected from N, O and S, -L-Het-R"', -L-NR'R", -L-A, -A-SO₂-R', -A-SO-R"', -A-A', -A-L'-C(O)NR'R", -A-(C₁-C₄ alkyl)-CN, -A-L'-NR'R", -A-L'-OR', -A-NR'R", -A-C(O)-Het'-L-CN, -A-C(O)-NR'R", -A-C(O)-(O)_{z}-A", -A-C(O)-(O)_{z}-R', -A-C(O)-(O)_{z}-L-A"', -A-C(O)-(O)_{z}R"', -A-C(O)-(O)_{z}-L-CN, -A-C(O)-L'-Het-R' group, or a -(CH₂)ₙ,C(OH)(J)-(CH₂)ₙK group, wherein z is 0 or 1, n and n' are independently 0 or 1, J represents a hydrogen atom or a methyl group, K represents a hydroxyl group, a methyl group or a -NR'R" group; and wherein R' and R" are the same or different and each represents a hydrogen atom or linear or a branched C₁-C₆ alkyl group, a C₁-C₄ haloalkyl group or a C₁-C₄ hydroxyalkyl group, and R'" represents a linear or branched C₁-C₆ alkyl group, a C₁-C₄ haloalkyl group or a C₁-C₄ hydroxyalkyl group; the heterocyclyl and heteroaryl groups being optionally fused to a phenyl group or to a pyridyl group; the cycloalkyl group being optionally fused to a 1,3-dioxolane group; and wherein the cycloalkyl, cycloalkenyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group, and wherein
L is a linear or branched C₁-C₆ alkylene group, L' is a linear C₁-C₂ alkylene group;
Het represents O or NR^{IV}, and Het' represents NR^{IV}, wherein R^{IV} is a hydrogen atom, a linear or branched C₁-C₄ alkyl group, a C₁-C₄ haloalkyl group, or C₁-C₄ hydroxyalkyl group;
A represents a C₃-C₁₀ cycloalkyl group, C₃-C₁₀ cycloalkenyl group, a 5- to 10-membered heterocyclyl group, a C₆-C₁₀ aryl group, or a 5- to 10- membered heteroaryl group, wherein the cycloalkyl, cycloalkenyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group;
A' represents a C₃-C₇ cycloalkyl group, C₃-C₇ cycloalkenyl group, a 5- to 10- membered heterocyclyl group, a C₆-C₁₀ aryl group, or a 5- to 10- membered heteroaryl group, wherein the cycloalkyl, cycloalkenyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group; A" represents a C₃-C₇ cycloalkyl group, a phenyl group, a 5- to 6-membered monocyclic heteroaryl group containing 1, 2 or 3 heteroatoms selected from N, O and S, a 5- to 6- membered heterocyclyl group containing 1, 2 or 3 nitrogen atoms, wherein the cycloalkyl, phenyl, heteroaryl and heterocyclyl groups are unsubstituted or substituted by one or more substituents selected from a halogen atom, a hydroxyl group, a cyano group, a linear or branched C₁-C₆ alkyl group, or a C₁-C₄ alkoxy group;
A"' represents a C₃-C₇ cycloalkyl group, a phenyl group, a 5- to 6-membered monocyclic heteroaryl group containing 1, 2 or 3 nitrogen atoms, a 5- to 6-membered heterocyclyl group containing 1, 2 or 3 nitrogen atoms, wherein said phenyl group is unsubstituted or substituted by one or more halogen atoms, and wherein said heteroaryl and heterocyclyl groups are unsubstituted or substituted by one or more linear or branched C₁-C₃ alkyl groups.

3. A compound according to claim 1 or 2, wherein R₈ or R₁₁ independently represent a hydrogen atom, a linear or branched C₁-C₆ alkyl group, a C₁-C₄ haloalkyl group, a C₁-C₄ hydroxyalkyl group, a C₃-C₁₀ cycloalkyl group, a C₆-C₁₀ aryl group, a 5- to 10-membered heteroaryl group containing 1, 2 or 3 heteroatoms selected from N, O and S, a 5- to 10- membered heterocyclyl group containing 1, 2 or 3 heteroatoms selected from N, O and S, -L-Het-R"', -L-A, -A-SO₂-R', -A-A', -A-L-CN, -A-L'-NR'R", -A-L'-OR', -A-NR'R", -A-C(O)-NR'R", -A-C(O)-(O)_{z}-R', -A-C(O)-L'-Het-R' group, or a -(CH₂)ₙ,C(OH)(J)-(CH₂)ₙK group, wherein z is 0 or 1, n and n' are independently 0 or 1, J represents a hydrogen atom or a methyl group, K represents a hydroxyl group, a methyl group or a -NR'R" group; and wherein R' and R" are the same or different and each represents a hydrogen atom or linear or a branched C₁-C₃ alkyl group, a C₁-C₃ haloalkyl group or a C₁-C₃ hydroxyalkyl group, and R"' represents a linear or branched C₁-C₃ alkyl group, a C₁-C₃ haloalkyl group or a C₁-C₃ hydroxyalkyl group; the heterocyclyl and heteroaryl groups being optionally fused to a phenyl group or to a pyridyl group; the cycloalkyl group being optionally fused to a 1,3-dioxolane group; and wherein the cycloalkyl, heterocyclyl, aryl and heteroaryl groups are unsubstituted or substituted by one or more substituents selected from a halogen atom, a hydroxyl group, a cyano group, a linear or branched C₁-C₆ alkyl group, or a C₁-C₄ alkoxy group, and wherein
L is a linear or branched C₁-C₃ alkylene group, L' is a linear C₁-C₂ alkylene group;
Het represents O or NR^{IV}, wherein R^{IV} is a hydrogen atom, a linear or branched C₁-C₄ alkyl group, a C₁-C₄ haloalkyl group, or C₁-C₄ hydroxyalkyl group,
A represents a C₃-C₁₀ cycloalkyl group, a 5- to 10- membered heterocyclyl group, a C₆-C₁₀ aryl group, or a 5- to 10- membered heteroaryl group, the cycloalkyl, heterocyclyl, aryl and heteroaryl groups being unsubstituted or substituted by one or more substituents selected from a halogen atom, a hydroxyl group, a cyano group, a linear or branched C₁-C₆ alkyl group, or a
C₁-C₄ alkoxy group;
A' represents a C₃-C₇ cycloalkyl group, a 5- to 10- membered heterocyclyl group, a C₆-C₁₀ aryl group, or a 5- to 10- membered heteroaryl group, the cycloalkyl, heterocyclyl, aryl and heteroaryl groups being unsubstituted or substituted by one or more substituents selected from a halogen atom, a hydroxyl group, a cyano group, a linear or branched C₁-C₆ alkyl group, or a C₁-C₄ alkoxy group.

4. A compound according to any one of the preceding claims, wherein in the compound of formula (I) Z represents a nitrogen atom and V represents a carbon atom.

5. A compound according to claims 1 to 3, wherein in the compound of formula (I) Z represents a carbon atom and V represents a nitrogen atom.

6. A compound according to any one of the preceding claims, wherein R₁ represents a hydrogen atom, a halogen atom, a cyano group, a linear or branched C₁-C₆ alkyl group, a C₃-C₇cycloalkyl group or a -(CH₂)ₙ-NR'R" group, wherein n is 0 or 1, and R' and R" are the same or different and each represents a hydrogen atom, a linear or branched C₁-C₆ alkyl group, a C₁-C₄ haloalkyl group or C₁-C₄ hydroxyalkyl group; preferably R₁ represents a hydrogen atom, a linear or branched C₁-C₃ alkyl group or a -NR'R" group, wherein R' and R" are the same or different and each represents a hydrogen atom or a linear or branched C₁-C₃ alkyl group; more preferably R₁ represents a hydrogen atom or a -NH₂ group; most preferably R₁ represents a hydrogen atom.

7. A compound according to any one of the preceding claims, wherein R₂ represents a hydrogen atom, a halogen atom, a cyano group, a linear or branched C₁-C₆ alkyl group or a C₃-C₇cycloalkyl group; preferably R₂ represents a hydrogen atom or a halogen atom; more preferably R₂ represents a hydrogen atom.

8. A compound according to any one of the preceding claims, wherein R₃ represents a hydrogen atom, a halogen atom, a cyano group, a linear or branched C₁-C₆ alkyl group, a C₃-C₇cycloalkyl group or a -(CH₂)q^{,}C(O)-(CH₂)_{q}-NR'R" group, wherein q and q' are independently 0 or 1, and R' and R" are the same or different and each represents a hydrogen atom, a linear or branched C₁-C₆ alkyl group or a C₁-C₄ haloalkyl group; preferably R₃ represents a hydrogen atom, a halogen atom, a cyano group or a -C(O)-NH₂ group; more preferably R₃ represents a hydrogen atom, a halogen atom or a cyano group; most preferably R₃ represents a halogen atom or a cyano group.

9. A compound according to any one of the preceding claims, wherein R₄ represents a hydrogen atom, a halogen atom, a hydroxyl group, a cyano group, a linear or branched C₁-C₆ alkyl group or a C₃-C₇cycloalkyl group; preferably R₄ represents a hydrogen atom, a halogen atom or a hydroxyl group; more preferably R₄ represents a hydrogen atom or a halogen atom; most preferably R₄ represents a hydrogen atom.

10. A compound according to any one of the preceding claims, wherein R₅ and R₅' each independently represent a hydrogen atom, a halogen atom, a hydroxyl group, a cyano group or a linear or branched C₁-C₆ alkyl group; preferably R₅ and R₅' independently represent a hydrogen atom, a halogen atom or a linear or branched C₁-C₃ alkyl group; more preferably R₅ and R₅' independently represent a hydrogen atom or a halogen atom; most preferably R₅ and R₅' independently represent a hydrogen atom.

11. A compound according to any one of the preceding claims, wherein R₆ and R₇ each independently represent a hydrogen atom, a hydroxyl group, a C₁-C₄ hydroxyalkyl group, a -(C₁-C₄ alkyl)-O-(C₁-C₄ alkyl) group or a linear or branched C₁-C₄ alkyl group; preferably R₆ and R₇ independently represent a hydrogen atom, a hydroxyl group, a C₁-C₃ hydroxyalkyl group, a -(C₁-C₃ alkyl)-O-(C₁-C₃ alkyl) group or a linear or branched C₁-C₃ alkyl group; more preferably R₆ and R₇ independently represent a hydrogen atom, a C₁-C₂ hydroxyalkyl group, a -(C₁-C₂ alkyl)-O-(C₁-C₂ alkyl) group or a methyl group; most preferably R₆ and R₇ independently represent a hydrogen atom or a methyl group.

12. A compound according to any one of the preceding claims, wherein R₉ and R₁₀ each independently represent a hydrogen atom, a hydroxyl group, a C₁-C₄ hydroxyalkyl group, a -(C₁-C₄ alkyl)-O-(C₁-C₄ alkyl) group or a linear or branched C₁-C₄ alkyl group; preferably R₉ and R₁₀ independently represent a hydrogen atom, a hydroxyl group, a C₁-C₃ hydroxyalkyl group, a -(C₁-C₃ alkyl)-O-(C₁-C₃ alkyl) group or a linear or branched C₁-C₃ alkyl group; more preferably R₉ and R₁₀ independently represent a hydrogen atom, a C₁-C₂ hydroxyalkyl group, a -(C₁-C₂ alkyl)-O-(C₁-C₂ alkyl) group or a methyl group; most preferably R₉ and R₁₀ independently represent a hydrogen atom or a methyl group.

13. A compound according to any one of the preceding claims, wherein R₈ and R₁₁ each independently represent a hydrogen atom, a linear or branched C₁-C₆ alkyl group, a C₁-C₄ hydroxyalkyl group, a C₃-C₇ cycloalkyl group, a C₃-C₇ cycloalkenyl group, a phenyl group, a 5- to 6-membered monocyclic heteroaryl group containing 1, 2 or 3 heteroatoms selected from N, O and S, a 5- to 7- membered heterocyclyl group containing 1, 2 or 3 heteroatoms selected from N, O and S, a bicyclyl group containing a phenyl group or a pyridyl group fused to a 5- to 7- membered heterocyclyl group containing at least one heteroatom selected from O, S and N, a bicyclyl group containing a phenyl group or a pyridyl group fused to a C₅-C₇ cycloalkyl group, a bicyclyl group containing a C₅-C₇ cycloalkyl group fused to a 1,3 dioxolane group, a -S(O)R' group, a -S(O)₂R' group, a -(CH₂)ₙOR"' group, a -(CH₂)ₙC(O)OR' group, a -(CH₂)ₙ,C(O)-(CH₂)ₙ-R' group, a -(CH₂)ₙ,C(OH)(J)-(CH₂)ₙK group, a -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₆ alkyl group, a
C₁-C₄ haloalkyl group or a C₁-C₄ hydroxyalkyl group; and R'" represents a linear or branched C₁-C₆ alkyl group, a C₁-C₄ haloalkyl group or a C₁-C₄ hydroxyalkyl group; and wherein the cycloalkyl, cycloalkenyl, phenyl, heteroaryl, heterocyclyl and bicyclyl groups are unsubstituted or substituted by one or more substituents selected from a halogen atom, a linear or branched C₁-C₆ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 4- to 5- membered heterocyclyl group containing 1, 2 or 3 heteroatoms selected from N, O and S, a - S(O)R' group, a -S(O)₂R' group, a -(CH₂)ₙOR' group, a -(CH₂)ₙC(O)OR' group, a -(CH₂)_{n'}C(O)-(CH₂)ₙ-R' group, a -(CH₂)ₙNR'R" group, a -(CH₂)ₙCH(R"')NR'R" group, a -(CH₂)_{n'}C(O)(CH₂)ₙNR'R" group or a -(CH₂)ₙCN group; wherein each n and n' are independently 0 or 1, and R' and R" each independently represent a hydrogen atom, a linear or branched C₁-C₆ alkyl group, a C₁-C₄ haloalkyl group or a C₁-C₄ hydroxyalkyl group, and R'" represents a linear or branched C₁-C₆ alkyl group, C₁-C₄ haloalkyl group or a C₁-C₄ hydroxyalkyl group.

14. A compound according to claim 13, wherein R₈ and R₁₁ each independently represent a hydrogen atom, a linear or branched C₁-C₆ alkyl group, a C₁-C₄ hydroxyalkyl group, a C₃-C₇ cycloalkyl group, a C₃-C₇ cycloalkenyl group, a phenyl group, a pyridyl group, a 5- to 7- membered heterocyclyl group containing 1, 2 or 3 heteroatoms selected from N, O and S, a bicyclyl group containing a phenyl group or a pyridyl group bounded directly to a 5- to 7- membered heterocyclyl group containing at least one heteroatom selected from O, S and N, a bicyclyl group containing a phenyl group or a pyridyl group fused to a C₅-C₇ cycloalkyl group, a bicyclyl group containing a C₅-C₇ cycloalkyl group fused to a 1,3 dioxolane group, a -(CH₂)ₙOR"' group, a -(CH₂)ₙC(O)OR' group, a -(CH₂)_{n'}C(O)-(CH₂)ₙ-R' group, a -(CH₂)_{n'}C(OH)(J)-(CH₂)ₙK group, a -(CH₂)ₙNR'R" group, a -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₆ alkyl group, a C₁-C₄ haloalkyl group or a C₁-C₄ hydroxyalkyl group, and R'" represents a linear or branched C₁-C₆ alkyl group, a C₁-C₄ haloalkyl group or a C₁-C₄ hydroxyalkyl group; and wherein the cycloalkyl, cycloalkenyl, phenyl, pyridyl, heterocyclyl and bicyclyl groups are unsubstituted or substituted by one or more substituents selected from a halogen atom, a linear or branched C₁-C₃ alkyl group, a C₁-C₄ haloalkyl group, a C₃-C₇ cycloalkyl group, a phenyl group, a 5- to 6-membered monocyclic heteroaryl group containing 1, 2 or 3 heteroatoms selected from N, O and S, a 4- to 5- membered heterocyclyl group containing 1, 2 or 3 heteroatoms selected from N, O and S, a -S(O)R' group, a -S(O)₂R' group, a -(CH₂)ₙOR' group, a -C(O)OR' group, a -(CH₂)_{n'}C(O)-(CH₂)ₙ-R' group, a -(CH₂)ₙNR'R" group, a -(CH₂)_{n'}C(O)(CH₂)ₙNR'R" group or a -(CH₂)ₙCN group; wherein each n and n' are independently 0 or 1, and R' and R" each independently represent a hydrogen atom, a linear or branched C₁-C₆ alkyl group, a C₁-C₄ haloalkyl group or a C₁-C₄ hydroxyalkyl group.

15. A compound according to claim 1, of formula (I): 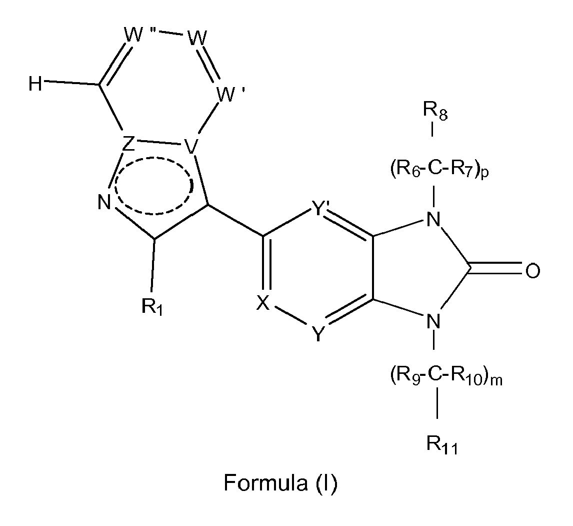 wherein
m is 0, 1 or 2;
p is 0, 1 or 2;
one of Z and V represents a nitrogen atom, and the other represents a carbon atom;
W represents a nitrogen atom or a -CR₃ group;
W' represents a nitrogen atom or a -CR₂ group;
W" represents a nitrogen atom or a -CR₄ group;
X and Y independently represent a nitrogen atom or a -CR₅ group, wherein at least one of X and Y represents a -CR₅ group;
Y' represents a nitrogen atom or a -CR₅, group;
R₁ represents a hydrogen atom or a -NH₂ group;
R₂ represents a hydrogen atom or a halogen atom;
R₃ represents a hydrogen atom, a halogen atom, a cyano group or a -C(O)-NH₂ group; R₄ represents a hydrogen atom or a halogen atom;
R₅ represents a hydrogen atom or a halogen atom;
R₅, represents a hydrogen atom;
R₆ and R₇ independently represent a hydrogen atom, a methyl group, a C₁-C₂ hydroxyalkyl group or a -(C₁-C₂ alkyl)-O-(C₁-C₂ alkyl) group;
R₉ and R₁₀ independently represent a hydrogen atom, a methyl group, a C₁-C₂ hydroxyalkyl group or a -(C₁-C₂ alkyl)-O-(C₁-C₂ alkyl) group;
R₈ and R₁₁ independently represent a hydrogen atom, a linear or branched C₁-C₃ alkyl group, a C₁-C₄ hydroxyalkyl group, a C₃-C₇ cycloalkyl group, a C₃-C₇ cycloalkenyl group, a phenyl group, a pyridyl group, a pyrimidinyl group, a morpholinyl group, a piperidyl group, a tetrahydropyranyl group, a chromanyl group,
a 3,4-dihydro-2H-pyrano[2,3-b]pyridyl group, a 1,2,3,4-tetrahydronaphthalenyl group, a 5,6,7,8-tetrahydroquinolinyl group, a 1,4-dioxaspiro[4.5]decanyl group, a -(CH₂)ₙOR"' group, a -(CH₂)ₙC(O)OR' group, a -(CH₂)_{n'}C(O)-(CH₂)ₙ-R' group,
a -(CH₂)_{n'}C(OH)(J)-(CH₂)ₙK group, a -(CH₂)ₙNR'R" group, a -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₄ alkyl group or a C₁-C₄ haloalkyl group, and R'" represents a linear or branched C₁-C₃ alkyl group or a C₁-C₄ haloalkyl group; and wherein the cycloalkyl, cycloalkenyl, phenyl, pyridyl, pyrimidinyl, morpholinyl, piperidyl, tetrahydropyranyl, chromanyl, 3,4-dihydro-2H-pyrano[2,3-b]pyridyl 1,2,3,4-tetrahydronaphthalenyl, 5,6,7,8-tetrahydroquinolinyl and 1,4-dioxaspiro[4.5]decanyl groups are unsubstituted or substituted by one or two substituents selected from a halogen atom, a linear or branched C₁-C₃ alkyl group, a linear or branched C₁-C₃ haloalkyl group, an oxo group, a -S(O)₂R' group, a -(CH₂)ₙOR' group, a -(CH₂)ₙC(O)OR' group, a -(CH₂)_{n'}C(O)-(CH₂)ₙ-R' group, a -(CH₂)_{n'}C(O)(CH₂)ₙNR'R" group, a -(CH₂)ₙNR'R" group or a -(CH₂)ₙCN group; wherein each n and n' are independently 0 or 1, and R' and R" each independently represent a hydrogen atom, a linear or branched C₁-C₄ alkyl group or a C₁-C₄ haloalkyl group.

16. A compound according to claim 15, of Formula (I-a): 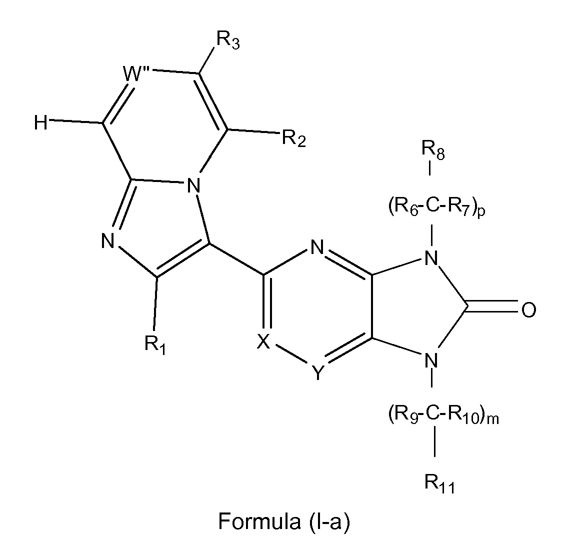 wherein
m is 0, 1 or 2;
p is 0, 1 or 2;
W" represents a nitrogen atom or a -CR₄ group; preferably W" represents a -CR₄ group;
X and Y independently represent a nitrogen atom or a -CR₅ group, wherein at least one of X and Y represents a -CR₅ group; preferably X represents a nitrogen atom or a -CR₅ group and Y represents a -CR₅ group;
R₁ represents a hydrogen atom or a -NH₂ group;
R₂ represents a hydrogen atom or a halogen atom;
R₃ represents a hydrogen atom, a halogen atom, a cyano group or a -C(O)-NH₂ group;
R₄ represents a hydrogen atom;
R₅ represents a hydrogen atom or a halogen atom; preferably R₅ represents a hydrogen atom;
R₆ and R₇ independently represent a hydrogen atom, a methyl group, a C₁-C₂ hydroxyalkyl group or a -(C₁-C₂ alkyl)-O-(C₁-C₂ alkyl) group; preferably R₆ and R₇ independently represent a hydrogen atom or a methyl group;
R₉ and R₁₀ independently represent a hydrogen atom, a methyl group, a C₁-C₂ hydroxyalkyl group or a -(C₁-C₂ alkyl)-O-(C₁-C₂ alkyl) group; preferably R₉ and R₁₀ independently represent a hydrogen atom or a methyl group;
R₈ represents a linear or branched C₁-C₃ alkyl group, a C₁-C₄ hydroxyalkyl group, a C₃-C₇ cycloalkyl group, a C₃-C₇ cycloalkenyl group, a phenyl group, a pyridyl group, a pyrimidinyl group, a morpholinyl group, a piperidyl group, a tetrahydropyranyl group, a chromanyl group, a 3,4-dihydro-2H-pyrano[2,3-b]pyridyl group, a 1,2,3,4-tetrahydronaphthalenyl group, a 5,6,7,8-tetrahydroquinolinyl group, a 1,4-dioxaspiro[4.5]decanyl group, a -(CH₂)ₙOR"' group, a -(CH₂)ₙC(O)OR' group, a -(CH₂)ₙ,C(O)-(CH₂)ₙ-R' group, a -(CH₂)ₙ.C(OH)(J)-(CH₂)ₙK group, a -(CH₂)ₙNR'R" group, a -(CH₂)ₙCH(R"')NR'R" group, or a -(CH₂)_{n'}C(O)(CH₂)ₙNR'R" group; wherein each n and n' are independently 0 or 1; and J represents a hydrogen atom or a methyl group; and K represents a hydroxyl group, a methyl group or a -NR'R" group; and R' and R" each independently represent a hydrogen atom, a linear or branched C₁-C₃ alkyl group or a C₁-C₄ haloalkyl group, and R'" represents a linear or branched C₁-C₃ alkyl group or a C₁-C₄ haloalkyl group; and wherein the cycloalkyl, cycloalkenyl, phenyl, pyridyl, pyrimidinyl, morpholinyl, piperidyl, tetrahydropyranyl, chromanyl and 3,4-dihydro-2H-pyrano[2,3-b]pyridyl 1,2,3,4-tetrahydronaphthalenyl, 5,6,7,8-tetrahydroquinolinyl and 1,4-dioxaspiro[4.5]decanyl groups are unsubstituted or substituted by one or more substituents selected from a halogen atom, a linear or branched C₁-C₃ alkyl group, a -S(O)₂R' group, a -(CH₂)ₙOR' group, a -(CH₂)ₙC(O)OR' group, a -(CH₂)_{n'}C(O)-(CH₂)ₙ-R' group, a -(CH₂)_{n'}C(O)(CH₂)ₙNR'R" group, a -(CH₂)ₙNR'R" group or a -(CH₂)ₙCN group; wherein each n and n' are independently 0 or 1, and R' and R" each independently represent a hydrogen atom, a linear or branched C₁-C₃ alkyl group or a C₁-C₄ haloalkyl group;
R₁₁ represents a hydrogen atom, a linear or branched C₁-C₃ alkyl group, a C₁-C₄ hydroxyalkyl group, a phenyl group, a morpholinyl group, a tetrahydropyranyl group, a -(CH₂)ₙOR"' group, a -(CH₂)_{n'}C(OH)(J)-(CH₂)ₙK group, a -(CH₂)ₙNR'R" group or a -(CH₂)ₙCH(R"')NR'R" group; wherein each n and n' are 0 or 1; and J represents a hydrogen atom or a methyl group; and K represents a hydroxyl group, a methyl group or a -NR'R" group; and R' and R" each independently represent a hydrogen atom, a linear or branched C₁-C₃ alkyl group or a C₁-C₄ haloalkyl group, and R'" represents a linear or branched C₁-C₃ alkyl group or a C₁-C₄ haloalkyl group; and wherein the phenyl, morpholinyl and tetrahydropyranyl groups are unsubstituted or substituted by one or more substituents selected from a halogen atom, a linear or branched C₁-C₃ alkyl group or a -(CH₂)ₙOR' group; wherein n is 0 or 1, and R' represents a hydrogen atom, a linear or branched C₁-C₃ alkyl group or a C₁-C₄ haloalkyl group.

17. A compound according to claim 15, of formula (I-b): 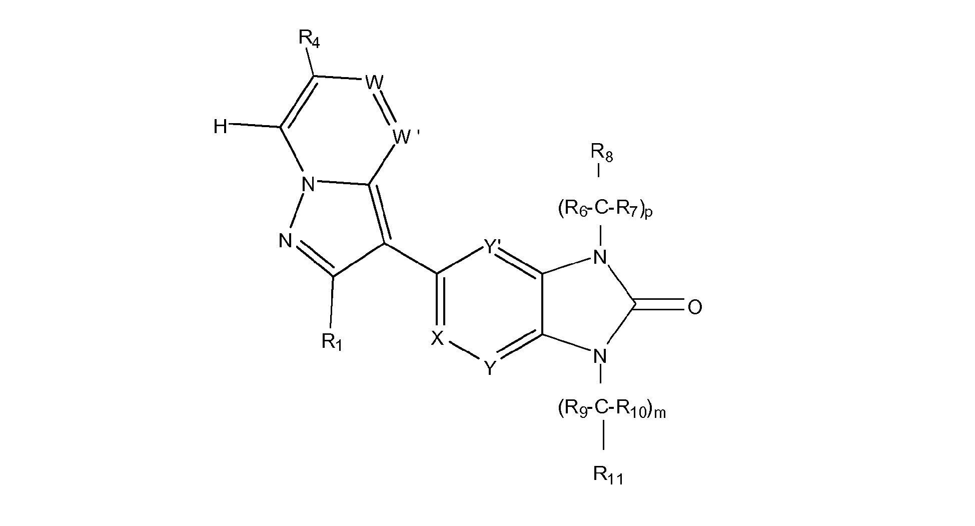 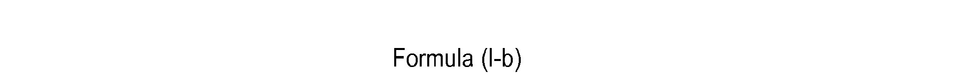 wherein
m is 0;
p is 0 or 1;
W represents a nitrogen atom or a -CR₃ group;
W' represents a nitrogen atom or a -CR₂ group; preferably W' represents a -CR₂ group;
X represents a nitrogen atom or a -CR₅ group;
Y represents a -CR₅ group;
Y' represents a nitrogen atom or a -CR₅, group;
R₁ represents a hydrogen atom;
R₂ represents a hydrogen atom or a halogen atom;
R₃ represents a hydrogen atom;
R₄ represents a hydrogen atom or a halogen atom;
R₅ represents a hydrogen atom or a halogen atom;
R₅, represents a hydrogen atom;
R₆ and R₇ independently represent a hydrogen atom or a methyl group;
R₈ represents a cyclohexyl group, a phenyl group, a pyridyl group, a chromanyl group, a tetrahydropyranyl group, wherein the cyclohexyl, phenyl, pyridyl, chromanyl and tetrahydropyranyl groups are unsubstituted or substituted by one or more substituents selected from a halogen atom, a linear or branched C₁-C₃ alkyl group or a -(CH₂)ₙCN group, wherein n is 0 or 1;
R₁₁ represents a hydrogen atom or a C₁-C₄ hydroxyalkyl group.

18. A compound according to claim 1 which is one of:
3-[8-Oxo-9-(tetrahydro-2*H-*pyran-4-yl)-8,9-dihydro-7*H-*purin-2-yl]imidazo[1,2-*a*]pyridine-6-carbonitrile;
3-(9-Cyclohexyl-8-oxo-8,9-dihydro-7*H*-purin-2-yl)imidazo[1,2*-a*]pyridine-6-carbonitrile;
3-[3-(2-Methylcyclohexyl)-2-oxo-2,3-dihydro-1*H*-imidazo[4,5*-b*]pyridin-5-yl]imidazo[1,2-*a*]pyridine-6-carbonitrile;
3-{9-[(4*R*)-8-Fluoro-3,4-dihydro-2*H-*chromen-4-yl]-8-oxo-8, 9-dihydro-7*H-*purin-2-yl}imidazo[1,2*-a*]pyridine-6-carbonitrile;
3-[7-Methyl-8-oxo-9-(tetrahydro-2*H-*pyran-4-yl)-8,9-dihydro-7*H-*purin-2-yl]imidazo[1,2-*a*]pyridine-6-carbonitrile;
3-(9-Benzyl-7-methyl-8-oxo-8,9-dihydro-7*H-*purin-2-yl)imidazo[1,2*-a*]pyridine-6-carbonitrile;
3-[7-(2-Morpholin-4-ylethyl)-8-oxo-9-(tetrahydro-2*H-*pyran-4-yl)-8,9-dihydro-7*H-*purin-2-yl]imidazo[1,2*-a*]pyridine-6-carbonitrile;
3-[7-[2-(Dimethylamino)ethyl]-8-oxo-9-(tetrahydro-2*H-*pyran-4-yl)-8,9-dihydro-7*H-*purin-2-yl]imidazo[1,2*-a*]pyridine-6-carbonitrile;
3-[7-(2-Hydroxyethyl)-8-oxo-9-(tetrahydro-2*H-*pyran-4-yl)-8,9-dihydro-7*H-*purin-2-yl]imidazo[1,2*-a*]pyridine-6-carbonitrile;
3-[7-(2-Hyd roxy-2-methyl propyl )-8-oxo-9-(tetrahyd ro-2*H-*pyran-4-yl )-8, 9-d i hyd ro-7*H-*purin-2-yl]imidazo[1,2*-a*]pyridine-6-carbonitrile;
3-[7-[(2*R*)-2,3-Dihydroxypropyl]-8-oxo-9-(tetrahydro-2*H-*pyran-4-yl)-8,9-dihydro-7*H-*purin-2-yl]imidazo[1,2*-a*]pyridine-6-carbonitrile;
3-[7-[(2*S*)-3-(Di methylamino)-2-hyd roxypropyl]-8-oxo-9-(tetra hyd ro-2*H-*pyran-4-yl )-8, 9-dihydro-7*H-*purin-2-yl]imidazo[1,2*-a*]pyridine-6-carbonitrile;
3-[7-(2-Methoxyethyl)-8-oxo-9-(tetrahydro-2*H-*pyran-4-yl)-8,9-dihydro-7*H-*purin-2-yl]imidazo[1,2*-a*]pyridine-6-carbonitrile;
3-[7-(2-Aminoethyl)-8-oxo-9-(tetrahydro-2*H-*pyran-4-yl)-8,9-dihydro-7*H-*purin-2-yl]imidazo[1,2*-a*]pyridine-6-carbonitrile;
3-(8-Oxo-9-(tetrahydro-2*H-*pyran-4-yl)-7-{2-[(2,2,2-trifluoroethyl)amino]ethyl}-8,9-dihydro-7*H-*purin-2-yl)imidazo[1,2*-a*]pyridine-6-carbonitrile;
2-(2-(6-Cyanoimidazo[1,2-*a*]pyridin-3-yl)-8-oxo-9-(tetrahydro-2*H-*pyran-4-yl)-8,9-dihydro-7*H-*purin-7-yl)acetic acid;
3-[7-(2,4-Dimethoxybenzyl)-8-oxo-9-(tetrahydro-2*H-*pyran-4-yl)-8,9-dihydro-7*H-*purin-2-yl]imidazo[1,2*-a*]pyridine-6-carbonitrile;
2-(6-Fluoroimidazo[1,2*-a*]pyridin-3-yl)-9-(tetrahydro-2*H-*pyran-4-yl)-7,9-dihydro-8*H-*purin-8-one;
3-[8-Oxo-9-(tetrahydro-2*H-*pyran-4-yl)-8,9-dihydro-7*H-*purin-2-yl]imidazo[1,2*-a*]pyridine-6-carbonitrile;
9-Cyclohexyl-2-(6-fluoroimidazo[1,2-a]pyridin-3-yl)-7H-purin-8(9H)-one;
9-[(4*R*)-8-Fluoro-3,4-dihydro-2*H-*chromen-4-yl]-2-(6-fluoroimidazo[1,2*-a*]pyridin-3-yl)-7,9-dihydro-8*H-*purin-8-one;
2-(6-Fluoroimidazo[1,2*-a*]pyridin-3-yl)-9-[(1*S*)-1-phenylethyl]-7,9-dihydro-8*H-*purin-8-one;
2-(6-Fluoroimidazo[1,2*-a*]pyridin-3-yl)-9-[(1*R*)-1-phenylethyl]-7,9-dihydro-8*H-*purin-8-one;
2-(6-Fluoroimidazo[1,2-a]pyridin-3-yl)-9-(pyridin-3-ylmethyl)-7,9-dihydro-8H-purin-8-one;
2-(6-Fluoroimidazo[1,2-a]pyridin-3-yl)-9-(5,6,7,8-tetrahydroquinolin-5-yl)-7H-purin-8(9H)-one;
2-(6-Fluoroimidazo[1,2-a]pyridin-3-yl)-9-(pyridin-2-ylmethyl)-7H-purin-8(9H)-one;
2-(6-Fluoroimidazo[1,2-a]pyridin-3-yl)-9-((5-fluoropyridin-2-yl)methyl)-7H-purin-8(9H)-one;
(R)-2-(6-Fluoroimidazo[1,2-a]pyridin-3-yl)-9-(1-(5-fluoropyridin-2-yl)ethyl)-7H-purin-8(9H)-one;
(R)-2-(6-Fluoroimidazo[1,2-a]pyridin-3-yl)-9-(1-(5-fluoropyridin-2-yl)ethyl)-7-(2-hydroxyethyl)-7H-purin-8(9H)-one;
2-(6-Fluoroimidazo[1,2-a]pyridin-3-yl)-9-(1-(5-fluoropyridin-2-yl)-2-methoxyethyl)-7H-purin-8(9H)-one;
(R)-2-(6-Fluoroimidazo[1,2-a]pyridin-3-yl)-9-(1-(pyridin-2-yl)ethyl)-7H-purin-8(9H)-one;
(R)-2-(6-Fluoroimidazo[1,2-a]pyridin-3-yl)-9-(1-(5-fluoropyrimidin-2-yl)ethyl)-7H-purin-8(9H)-one;
(S)-2-(6-Fluoroimidazo[1,2-a]pyridin-3-yl)-9-(1-(5-fluoropyridin-2-yl)ethyl)-7H-purin-8(9H)-one;
(S)-2-(6-Fluoroimidazo[1,2-a]pyridin-3-yl)-9-(1-(5-fluoropyridin-2-yl)-2-hydroxyethyl)-7H-purin-8(9H)-one;
1-(2-(6-Fluoroimidazo[1,2-a]pyridin-3-yl)-8-oxo-7H-purin-9(8H)-yl)cyclohexanecarbonitrile;
(1s,4s)-Ethyl 4-(2-(6-fluoroimidazo[1,2-a]pyridin-3-yl)-8-oxo-7H-purin-9(8H)-yl)cyclohexanecarboxylate;
(1r,4r)-4-(2-(6-Fluoroimidazo[1,2-a]pyridin-3-yl)-8-oxo-7H-purin-9(8H)-yl)cyclohexanecarboxylic acid;
(1r,4r)-4-(2-(6-Fluoroimidazo[1,2-a]pyridin-3-yl)-8-oxo-7H-purin-9(8H)-yl)-N-methylcyclohexanecarboxamide;
9-(2,2-Dimethyltetrahydro-2*H-*pyran-4-yl)-2-(6-fluoroimidazo[1,2*-a*]pyridin-3-yl)-7,9-dihydro-8*H*-purin-8-one;
9-(2,2-Dimethyl-3,4-dihydro-2H-pyrano[2,3-b]pyridin-4-yl)-2-(6-fluoroimidazo[1,2-a]pyridin-3-yl)-7H-purin-8(9H)-one;
2-(6-Fluoroimidazo[1,2-a]pyridin-3-yl)-9-(1,4-dioxaspiro[4.5]decan-8-yl)-7H-purin-8(9H)-one;
2-(6-Fluoroimidazo[1,2-a]pyridin-3-yl)-9-(4-oxocyclohexyl)-7H-purin-8(9H)-one;
2-((1r,4r)-4-(2-(6-Fluoroimidazo[1,2-a]pyridin-3-yl)-8-oxo-7H-purin-9(8H)-yl)cyclohexyl)acetonitrile;
2-((1r,4r)-4-(2-(6-Fluoroimidazo[1,2-a]pyridin-3-yl)-7-(2-hydroxyethyl)-8-oxo-7H-purin-9(8H)-yl)cyclohexyl)acetonitrile;
2-(6-Fluoroimidazo[1,2-a]pyridin-3-yl)-9-((1r,4r)-4-(hydroxymethyl)cyclohexyl)-7H-purin-8(9H)-one;
2-(6-Fluoroimidazo[1,2-a]pyridin-3-yl)-9-((1r,4r)-4-hydroxycyclohexyl)-7H-purin-8(9H)-one;
2-(6-Fluoroimidazo[1,2-a]pyridin-3-yl)-9-((1R,4R)-4-hydroxy-1,2,3,4-tetrahydronaphthalen-1-yl)-7H-purin-8(9H)-one;
2-(6-Fluoroimidazo[1,2-a]pyridin-3-yl)-9-((1r,4r)-4-hydroxy-4-methylcyclohexyl)-7H-purin-8(9H)-one;
9-((1r,4r)-4-(Aminomethyl)cyclohexyl)-2-(6-fluoroimidazo[1,2-a]pyridin-3-yl)-7H-purin-8(9H)-one;
9-((1r,4r)-4-Aminocyclohexyl)-2-(6-fluoroimidazo[1,2-a]pyridin-3-yl)-7H-purin-8(9H)-one;
9-Cyclobutyl-2-(6-fluoroimidazo[1,2-a]pyridin-3-yl)-7H-purin-8(9H)-one; (R)-2-(6-Fluoroimidazo[1,2-a]pyridin-3-yl)-9-(3-methylbutan-2-yl)-7H-purin-8(9H)-one;
2-(6-Fluoroimidazo[1,2-a]pyridin-3-yl)-9-(1-methoxypropan-2-yl)-7H-purin-8(9H)-one; (R)-tert-Butyl 3-(2-(6-fluoroimidazo[1,2-a]pyridin-3-yl)-8-oxo-7H-purin-9(8H)-yl)butanoate;
(R)-3-(2-(6-Fluoroimidazo[1,2-a]pyridin-3-yl)-8-oxo-7H-purin-9(8H)-yl)butanoic acid;
(R)-3-(2-(6-Fluoroimidazo[1,2-a]pyridin-3-yl)-8-oxo-7H-purin-9(8H)-yl)butanamide;
9-(1-(2,2-Difluoroethyl)piperidin-4-yl)-2-(6-fluoroimidazo[1,2-a]pyridin-3-yl)-7H-purin-8(9H)-one;
9-(4,4-Difluorocyclohexyl)-2-(6-fluoroimidazo[1,2-a]pyridin-3-yl)-7,9-dihydro-8H-purin-8-one;
2-(6-Fluoroimidazo[1,2*-a*]pyridin-3-yl)-9-[(3*R*)-piperidin-3-yl]-7,9-dihydro-8*H*-purin-8-one;
2-(6-Fluoroimidazo[1,2*-a*]pyridin-3-yl)-9-[(3*R*)-1-(methylsulfonyl)piperidin-3-yl]-7,9-dihydro-8*H-*purin-8-one;
3-[2-Oxo-3-(tetrahydro-2*H-*pyran-4-yl)-2,3-dihydro-1*H-*imidazo[4,5*-b*]pyridin-5-yl]imidazo[1,2*-a*]pyridine-6-carbonitrile;
3-[1-Methyl-2-oxo-3-(tetrahydro-2*H-*pyran-4-yl)-2,3-dihydro-1*H-*imidazo[4,5*-b*]pyridin-5-yl]imidazo[1,2*-a*]pyridine-6-carbonitrile;
5-(6-Fluoroimidazo[1,2*-a*]pyridin-3-yl)-3-(tetrahydro-2*H-*pyran-4-yl)-1,3-dihydro-2*H-*imidazo[4,5-*b*]pyridin-2-one;
5-(6-Fluoroimidazo[1,2*-a*]pyridin-3-yl)-1-methyl-3-(tetrahydro-2*H-*pyran-4-yl)-1,3-dihydro-2*H-*imidazo[4,5-*b*]pyridin-2-one;
5-(6-Fluoroimidazo[1,2*-a*]pyridin-3-yl)-1-(2-hydroxyethyl)-3-(tetrahydro-2*H-*pyran-4-yl)-1,3-dihydro-2*H-*imidazo[4,5-*b*]pyridin-2-one;
*tert*-Butyl 4-[5-(6-fluoroimidazo[1,2*-a*]pyridin-3-yl)-2-oxo-1,2-dihydro-3*H-*imidazo[4,5-b]pyridin-3-yl]piperidine-1-carboxylate;
5-(6-Fluoroimidazo[1,2-a]pyridin-3-yl)-3-piperidin-4-yl-1,3-dihydro-2H-imidazo[4,5-b]pyridin-2-one;
5-(6-Fluoroimidazo[1,2*-a*]pyridin-3-yl)-3-[1-(methylsulfonyl)piperidin-4-yl]-1,3-dihydro-2*H-*imidazo[4,5*-b*]pyridin-2-one;
3-(1-Acetylpiperidin-4-yl)-5-(6-fluoroimidazo[1,2*-a*]pyridin-3-yl)-1,3-dihydro-2*H-*imidazo[4,5*-b*]pyridin-2-one;
6-Fluoro-5-(6-fluoroimidazo[1,2-a]pyridin-3-yl)-3-(tetrahydro-2H-pyran-4-yl)-1,3-dihydro-2H-imidazo[4,5-b]pyridin-2-one;
6-(6-Fluoroimidazo[1,2*-a*]pyridin-3-yl)-1-(tetrahydro-2*H-*pyran-4-yl)-1,3-dihydro-2*H-*imidazo[4,5*-b*]pyrazin-2-one;
2-(2-Amino-6-fluoroimidazo[1,2-a]pyridin-3-yl)-9-(tetrahydro-2H-pyran-4-yl)-7H-purin-8(9H)-one;
2-(Imidazo[1,2-a]pyrazin-3-yl)-9-(tetrahydro-2H-pyran-4-yl)-7H-purin-8(9H)-one;
2-Pyrazolo[1,5*-a*]pyrazin-3-yl-9-(tetrahydro-2*H-*pyran-4-yl)-7,9-dihydro-8*H-*purin-8-one;
9-[(1 *R*)-1-Phenylethyl]-2-pyrazolo[1,5*-a*]pyrazin-3-yl-7,9-dihydro-8*H-*purin-8-one; (R)-9-(1-(5-Fluoropyridin-2-yl)ethyl)-2-(pyrazolo[1,5-a]pyrazin-3-yl)-7H-purin-8(9H)-one;
2-(Pyrazolo[1,5-a]pyrazin-3-yl)-9-((tetrahydro-2H-pyran-4-yl)methyl)-7H-purin-8(9H)-one;
9-(4,4-Difluorocyclohexyl)-2-(pyrazolo[1,5-a]pyrazin-3-yl)-7H-purin-8(9H)-one;
9-(2,2-Dimethyltetrahydro-2H-pyran-4-yl)-2-(pyrazolo[1,5-a]pyrazin-3-yl)-7H-purin-8(9H)-one;
(R)-9-(8-Fluorochroman-4-yl)-2-(pyrazolo[1,5-a]pyrazin-3-yl)-7H-purin-8(9H)-one; 2-((1r,4r)-4-(8-Oxo-2-(pyrazolo[1,5-a]pyrazin-3-yl)-7H-purin-9(8H)-yl)cyclohexyl)acetonitrile;
2-((1r,4r)-4-(7-(2-Hydroxyethyl)-8-oxo-2-(pyrazolo[1,5-a]pyrazin-3-yl)-7H-purin-9(8H)-yl)cyclohexyl)acetonitrile;
5-(Pyrazolo[1,5-a]pyrazin-3-yl)-3-(tetrahydro-2H-pyran-4-yl)-1H-imidazo[4,5-b]pyridin-2(3H)-one;
6-Fluoro-5-pyrazolo[1,5-a]pyrazin-3-yl-3-(tetrahydro-2H-pyran-4-yl)-1,3-dihydro-2H-imidazo[4,5-b]pyridin-2-one;
2-(Pyrazolo[1,5-a]pyridin-3-yl)-9-(tetrahydro-2H-pyran-4-yl)-7H-purin-8(9H)-one;
2-(6-Fluoropyrazolo[1,5-a]pyridin-3-yl)-9-(tetrahydro-2H-pyran-4-yl)-7H-purin-8(9H)-one;
2-(4-Fluoropyrazolo[1,5-a]pyridin-3-yl)-9-(tetrahydro-2H-pyran-4-yl)-7H-purin-8(9H)-one;
2-(Pyrazolo[1,5-a]pyrimidin-3-yl)-9-(tetrahydro-2H-pyran-4-yl)-7H-purin-8(9H)-one;
6-(Pyrazolo[1,5-a]pyrimidin-3-yl)-1-(tetrahydro-2H-pyran-4-yl)-1H-imidazo[4,5-c]pyridin-2(3H)-one;
3-[8-Oxo-9-(tetrahydro-2*H-*pyran-4-yl)-8,9-dihydro-7*H-*purin-2-yl]imidazo[1,2*-a*]pyridine-6-carboxamide;
and pharmaceutically acceptable salts, or solvates, or N-oxides, or stereoisomers or deuterated derivatives thereof:

19. A compound according to any one of claims 1 to 18, for use in the treatment of a pathological condition or disease susceptible to amelioration by inhibition of Janus kinases.

20. A compound according to claim 19, wherein the pathological condition or disease is selected from myeloproliferative disorders, leukemia, lymphoid malignancies and solid tumors; bone marrow and organ transplant rejection; immune-mediated diseases and inflammatory diseases.

21. A compound according to claims 19 or 20, wherein the pathological condition or disease is selected from rheumatoid arthritis, multiple sclerosis, inflammatory bowel disease, dry eye, uveitis, allergic conjunctivitis, allergic rhinitis, asthma, chronic obstructive pulmonary disease (COPD), atopic dermatitis and psoriasis.

22. A pharmaceutical composition comprising a compound as defined in any one of claims 1 to 18 in association with a pharmaceutically acceptable diluent or carrier.

23. Use of a compound as defined in any one of claims 1 to 18, for the manufacture of a medicament for the treatment of a pathological condition or disease as defined in any one of claims 19 to 21.

24. A method for treating a subject afflicted with a pathological condition or disease as defined in any one of claims 19 to 21, which comprises administering to said subject a therapeutically effective amount of a compound as defined in any one of claims 1 to 18, or a pharmaceutical composition as defined in claim 22.

25. A combination product comprising (i) a compound as defined in any one of claims 1 to 18; and (ii) another compound selected from:
a) Dyhydrofolate reductase inhibitors, such as Methotrexate or CH-1504;
b) DHODH inhibitors such as leflunomide, teriflunomide, or the compounds described in the International Patent Application Nos. WO2008/077639 and WO2009021696;
c) Immunomodulators such as Glatiramer acetate or Laquinimod;
d) Inhibitors of DNA synthesis and repair, such as Mitoxantrone or Cladribine;
e) Anti-alpha 4 integrin antibodies, such as Natalizumab (Tysabri);
f) Alpha 4 integrin antagonists such as R-1295, TBC-4746, CDP-323, ELND-002, Firategrast or TMC-2003;
g) Corticoids and glucocorticoids such as prednisone or methylprednisolone, fluticasone, mometasone, or beta-metasone;
h) Fumaric acid esters, such as *BG-12;*
i) Anti-TNF alpha antibodies, such as Infliximab, Adalimumab, or Certolizumab pegol;
j) Soluble TNF alpha receptors such as Ethanercept;
k) Anti-CD20 monoclonal antibodies such as Rituximab, Ocrelizumab Ofatumumab or TRU-015;
l) Anti-CD52 such as alemtuzumab;
m) Anti-CD25 such as daclizumab;
n) Anti-CD88, such as eculizumab or pexilizumab;
o) Anti-IL12R /IL23R, such as ustekinumab;
p) Calcineurin inhibitors such as cyclosporine A or tacrolimus;
q) IMPDH inhibitors, such as mycophenolate mophetyl;
r) Cannabinoid receptor agonists such as Sativex;
s) Chemokine CCR1 antagonists such as MLN-3897 or PS-031291;
t) Chemokine CCR2 antagonists such as INCB-8696;
u) NF-kappaB activation inhibitors such as MLN-0415;
v) S1 P receptor agonists such as fingolimod, BAF-312, ACT128800, or the compounds described in the International Patent Application Nos. PCT/EP2009/007348 and PCT/EP2009/008968;
w) S1P liase inhibitors such as LX2931;
x) Syk inhibitors, such as R-112;
y) PKC inhibitors, such as NVP-AEB071;
z) M3 antagonist such as tiotropium or aclidinium;
aa) Long-acting beta adrenergic agonist such as formoterol;
bb) Vitamin D derivatives like calcipotriol (Daivonex);
cc) Phosphosdiesterase IV inhibitors such as roflumilast or GRC-4039;
dd) p38 Inhibitors such as ARRY-797;
ee) MEK inhibitors, such as ARRY-142886 or ARRY-438162;
ff) PI3Kδγ inhibitors;
gg) Interferons comprising Interferon beta 1 a such as Avonex from Biogen Idec, CinnoVex from CinnaGen and Rebif from EMD Serono, and Interferon beta 1 b such as Betaferon from Schering and Betaseron from Berlex; and
hh) Interferon alpha such as Sumiferon MP,
for simultaneous, separate or sequential use in the treatment of the human or animal body.
